# EUROPEAN PATENT APPLICATION

(11) **EP 1 233 014 A1**
(43) Date of publication of application: **21.08.2002**
(21) Application number: 00970223.4
(22) Date of filing: 30.10.2000
(51) Int. Cl.: C07C 409/32, C08K 5/14, C08L 83/04

(54) **ASYMMETRIC ORGANIC PEROXIDE, CROSSLINKING AGENT COMPRISING THE SAME, AND METHOD OF CROSSLINKING WITH THE SAME**

(30) Priority: 05.11.1999 JP 31542299; 31.03.2000 JP 2000996630
(71) Applicant: NOF CORPORATION, Tokyo 150-0013 (JP)
(72) Inventor: KOJIMA, Tatsuya, Chita-gun, Aichi 470-2362 (JP); TAURA, Katsuki, Chita-gun, Aichi 470-2362 (JP); UJIKAWA, Norihisa, Chita-gun, Aichi 470-2362 (JP)
(74) Representative: Müller-Boré & Partner Patentanwälte
(86) International application number: JP0007646
(87) International publication number: WO01034564

(57) **Abstract**

A crosslinking agent comprising an asymmetry organic peroxide having at least one structure unit of (substituted)benzoylcarbonyloxy group represented by the following formula (1) in the molecule thereof.

Environmentally friendly crosslinking agents and crosslinked silicone rubber moldings are provided thereby.

Specifically, useful crosslinking agents and crosslinking processes for silicone rubber are provided.

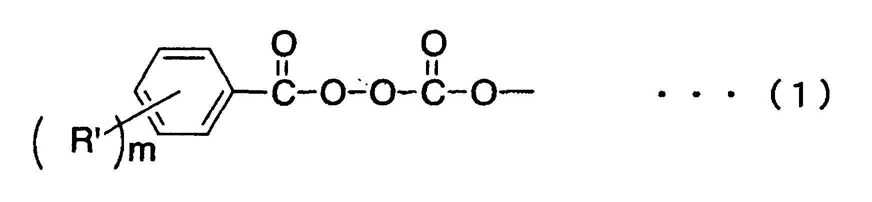

## Description

### TECHNICAL FIELD AND INDUSTRIAL APPLICABILITY OF THE INVENTION

The present invention relates to an asymmetry organic peroxide, a crosslinking agent comprising the same and a crosslinking process employing the same.

More particularly, the present invention relates to a crosslinking agent for silicone rubber and to a crosslinking process which causes no sanitarily problem and has excellent productivity, because the crosslinking efficiency and the crosslinking speed thereof are high.

### BACKGROUND OF THE INVENTION

Silicone rubber, natural rubber, ethylene-propylene rubber and ethylene-propylene-diene rubber can be crosslinked by mixing organic peroxides as crosslinking agents with the corresponding un-crosslinking compositions and heating them. Crosslinked rubbers are used as automotive rubber parts such as hose, cap, packing, oil seal and belt; golf balls, electrical wire and cable coatings, tires, and so on.
Among the rubbers, silicone rubber is the most widely used rubber in the various fields, as electrical wire and cable coatings, automotive parts, electronic parts and medical products, because silicone rubber has excellent characters such as high heat-resistance, wetherability and transparency as well as good electrical properties.

Benzoyl peroxide, 4-chlorobenzoyl peroxide, 2,4-dichlorobenzoyl peroxide, di-tert-butyl peroxide, dicumyl peroxide, tert-butylperoxy benzoate, and so on are used as the above-described crosslinking agent.

Especially, only 2,4-dichlorobenzoyl peroxide has been used as a crosslinking agent of silicone rubber for electrical wire and cable coatings and tubes produced by extrusion molding applications in which previously shaped silicone rubber is continuously extruded to an air oven and crosslinked by hot air.

However, the use of 2,4-dichlorobenzoyl peroxide as the crosslinking agent for hot air crosslinking of silicone rubber moldings might cause blooming, the gradual bleeding out of crosslinking agent decomposition products to whiten the surfaces of the moldings. Therefore, the moldings require aftercrosslinking at high temperature for a long time to prevent from blooming.
Furthermore, the use of 2,4-dichlorobenzoyl peroxide might also cause sanitary problems because of the production of polychlorinatedbiphenyl as decomposition products.

It was reported that organic peroxides such as 2-methylbenzoyl peroxide (Japanese Published Unexamined Application No.59-18758), substituted-dimethylbenzoyl peroxide (Japanese Published Unexamined Application No.60-16968), substituted-trimethylbenzoyl peroxide (Japanese Published Unexamined Application No.60-163860), 2-methoxbenzoyl peroxide (Japanese Published Unexamined Application No. 59-232145), derivative of benzoyl peroxide such as 4-methylbenzoyl peroxide (Japanese Published Unexamined Application No.62-185750), and acylperoxy carboxylate (U.S. patent 4,051,310) would be useful in place of the 2,4-dichlorobenzoyl peroxide as the crosslinking agent.

However, there was a problem that in cases where 2-methylbenzoyl peroxide, substituted dimethylbenzoyl peroxide, substituted trimethylbenzoyl peroxide, 4-methylbenzoyl peroxide or acylperoxy carboxylate were used, the crosslinking speeds would become slower than the case where 2,4-dichlorobenzoyl peroxide was used, which resulting in lower productivities.
On the other hand, there was a problem that in case where 2-methoxybenzoyl peroxide is used, the crosslinking efficiency would become lower than that in case where 2,4-dichlorobenzoyl peroxide is used. Therefore, neither of them could be an alternative crosslinking agent to 2,4-dichlorobenzoyl peroxide.

In consideration of the above situations, the purpose of the present invention is to provide an asymmetry organic peroxide having a specific structure, a crosslinking agent for silicone rubber comprising the same and a crosslinking process of silicone rubber employing the same.
According to the present invention, high productivity can be attained because of the high crosslinking efficiency and the high crosslinking speed, and aftercrosslinking is not necessary because no blooming, and no sanitarily problems can be occur since there is no possibility of forming any high toxic decomposition product.

### SUMMARY OF THE INVENTION

After examining intensively the above-described problems, the inventors found that they can be solved by what described hereinafter;
The asymmetry organic peroxide having at least one structure unit of (substituted)benzoylperoxy carbonyloxy group represented by the following formula (1) in the molecule thereof, 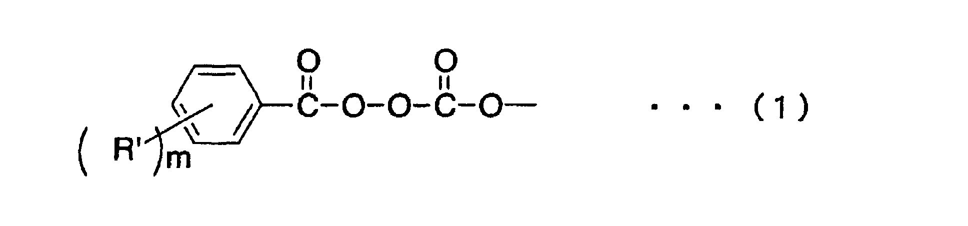 more particularly, the asymmetry organic peroxide comprising a (substituted)benzoylperoxyalkyl carbonate, or a (substituted)benzoylperoxyalkylene carbonate represented by the following formula (2) 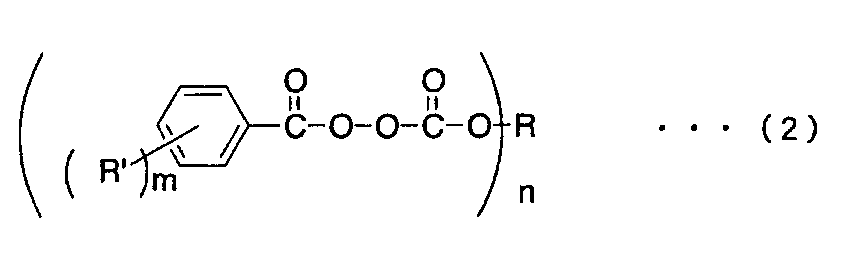 (wherein n stands for an integer of 1 to 6; m stands for an integer of 0 to 5; n and R corresponds to either of the following (a), (b) or (c), and m and R' corresponds to either of the following (A), (B) or (C) :
(a) in case of n is 1, R stands for an alkyl group of 1 to 20 carbon atoms, whereas in case of n is in a range of 2 to 6, R stands for a linear or branched hydrocarbon group of 1 to 20 carbon atoms.
(b) in case of n is 1, R stands for a cycloalkyl group of 3 to 10 carbon atoms, or a substituted cycloalkyl group of 4 to 10 carbon atoms substituted by an alkyl group of 1 to 4 carbon atoms, whereas in case of n is in a range of 2 to 4, R stands for a cyclohexanediyldimethylene group, a cyclohexylene group, a bicyclohexylene group, or a hydrocarbon group of 13 to 25 carbon atoms substituted by 2 to 4 of cyclohexylene groups.
(c) in case of n is 1, R stands for an alkoxyalkyl group represented by the formula -R¹-O-R² (wherein R¹ stands for an alkylene group of 2 to 5 carbon atoms, R² stands for an alkyl group of 1 to 8 carbon aoms.), whereas in case of n is 2, R stands for an oxoalkylene group represented by the formula - (R³-O-)ₖ-R³- (wherein k stands for 1 or 2, R³ stands for an alkylene group of 2 to 5 carbon atoms).

(A) m stands for 0, in other words, R' stands for a hydrogen atom.
(B) m stands for an integer of 1 to 5, and R' stands for an alkyl group of 1 to 6 carbon atoms, an alkoxy group of 1 to 6 carbon atoms, or a phenyl group.
(C) m stands for an integer of 1 to 5, and R' stands for a silyl group substituted by either of an alkyl group of 1 to 6 carbon atoms, an alkoxy group of 1 to 6 carbon atoms, a phenyl group, or a vinyl group.
wherein, R' can be the same or different in each (substituted)benzoyl group, and in each (substituted)benzoyl group ) in one molecule,
and the crosslinking agent comprising the above asymmetry organic peroxide, and the crosslinking agent comprising the above asymmetry organic peroxide and other conventional organic peroxide, and either one of the above-described crosslinking agents further comprising silicone oil singly or in combination with microparticulate silica.

In case where either one of the above crosslinking agent was mixed with a rubber and heated, unlike the case 2,4-dichlorobenzoyl peroxide was used, aftercrosslinking was not necessary because no blooming and no sanitarily problems could be occur since there was no possibility of forming any high toxic decomposition product,
and higher productivity could be attained than in the case 2-methylbenzoyl peroxide, dimethyl-substitutedbenzoyl peroxide, trimethyl-substitutedbenzoyl peroxide, 4-methylbenzoyl peroxide or acylperoxycarboxylate was used because of the high crosslinking speed , and higher crosslinking efficiency could be attained than in the case 2-methoxybenzoyl peroxide was used, and further, the materiality of the obtained rubber was excellent.
The present invention was completed as a result.

### DETAILED DESCRIPTION OF THE PREFERED EMBODIMENTS

The present invention will be described hereinafter in detail.

The asymmetry organic peroxide of the present invention has at least one structure unit of (substituted)benzoylperoxycarbonyloxy group represented by the following formula (1) in the molecule thereof. 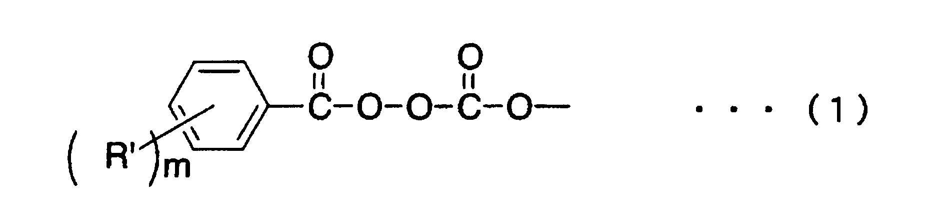 Herein, "an asymmetry organic peroxide" indicates an organic peroxide having an asymmetrical structure in which different substituted groups of a (substituted)benzoyl group and a carbonyloxy group are bonded to each peroxide bond (-OO-) thereof, including an organic peroxide of a symmetrical structure as the compound.

More particularly, the asymmetry organic peroxide comprises a (substituted)benzoylperoxyalkyl carbonate, or a (substituted)benzoylperoxyalkylene carbonate represented by the following formula (2)

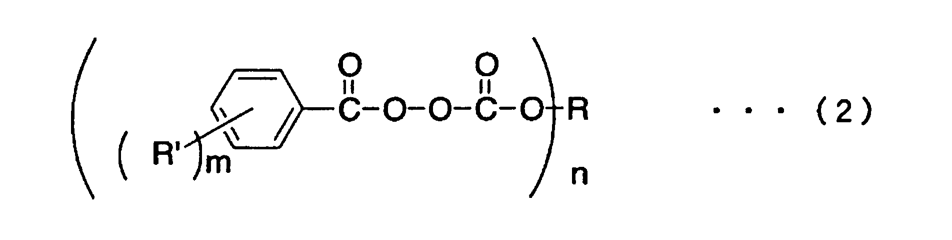

In the formula (2), n stands for an integer of 1 to 6; m stands for an integer of 0 to 5; n and R correspond to either of the following (a), (b) or (c), and m and R' correspond to either of the following (A), (B) or (C):
(a) in case of n is 1, R stands for an alkyl group of 1 to 20 carbon atoms, whereas in case of n is in a range of 2 to 6, R stands for a linear or branched hydrocarbon group of 1 to 20 carbon atoms.
(b) in case of n is 1, R stands for a cycloalkyl group of 3 to 10 carbon atoms, or a substituted cycloalkyl group of 4 to 10 carbon atoms substituted by an alkyl group of 1 to 4 carbon atoms, whereas in case of n is in a range of 2 to 4, R stands for a cyclohexanediyldimethylene group, a cyclohexylene group, a bicyclohexylene group, or a hydrocarbon group of 13 to 25 carbon atoms substituted by 2 to 4 of cyclohexylene groups.
(c) in case of n is 1, R stands for an alkoxyalkyl group represented by the formula -R¹-O-R² (wherein R¹ stands for an alkylene group of 2 to 5 carbon atoms, R² stands for an alkyl group of 1 to 8 carbon aoms), whereas in case of n is 2, R stands for an oxoalkylene group represented by the formula- (R³-O-)ₖ-R³- (wherein k stands for 1 or 2, and R³ stands for an alkylene group of 2 to 5 carbon atoms).

(A) m stands for 0, in other words, R' stands for a hydrogen atom.
(B) m stands for an integer of 1 to 5, and R' stands for an alkyl group of 1 to 6 carbon atoms, an alkoxy group of 1 to 6 carbon atoms, or a phenyl group.
(C) mstands for an integer of 1 to 5, and R' stands for a silyl group substituted by either of an alkyl group having 1 to 6 carbon atoms, an alkoxy group of 1 to 6 carbon atoms, a phenyl group or a vinyl group.
wherein, R' can be the same or different in each (substituted) benzoyl group, and in each (substituted)benzoyl group in one molecule.

R in the above formula (2) will be described below in more detail.

In the compound that n and R correspond to the above (a), R in case of n is 1 includes methyl, ethyl, n-propyl, isopropyl, n-butyl, sec-butyl, isobutyl, n-pentyl, neopentyl, hexyl, heptyl, octyl, 1-methylheptyl, 2-ethylhexyl, nonyl, decyl, myristyl, cetyl, stearyl, and so on.

R in case of (a) and n is in a range of 2 to 6 includes ethylene, trimethylene, 2-methyltrimethylene, 2,2-dimethyltrimethylene, 2,2-diethyltrimethylene, tetramethylene, pentamethylene, 3-methylpentamethylene, hexamethylene, heptamethylene, octamethylene, nonamethylene, decamethylene, dodecamethylene, hexadecamethylene, 1,1,1-ethylidyne, 1,1,1-plopylidine, 1,2,3-propanetriyl, 1,1,1-butylidine, 1,1,1,1-tetramethylidenemethine, 1,2,3,4-butanetetrayl, 1,2,3,4,5-pentanepentayl, 1,2,3,4,5,6-hexanehexayl, and so on.

R in case of (b) and n is 1 includes cyclopentyl, cyclohexyl, 2-methylcyclohexyl, 3-methylcyclohexyl, 4-methylcyclohexyl, 2,3-dimethylcyclohexyl, 2,4-dimethylcyclohexyl, 2,5-dimethylcyclohexyl, 2,6-dimethylcyclohexyl, 3,4-dimethylcyclohexyl, 3,5-dimethylcyclohexyl, 2-ethylcyclohexyl, 4-ethylcyclohexyl, 4-butylcyclohexyl, 3,3,5-trimethylcyclohexyl, 2-tert-butylcyclohexyl, 4-tert-butylcyclohexyl, menthyl, cyclododecyl, and so on.

R in case of (b) and n is in a range of 2 to 4 includes 1,2-cyclohexanediyldimethylene, 1,4-cyclohexanediyldimethylene, 1,2-cyclohexylene, 1,3-cyclohexylene, 1,4-cyclohexylene, 4,4'-bicyclohexylene, bis(1,4-cyclohexylene)methane, 2,2-bis(1,4-cyclohexylene)propane, tris(1,4-cyclohexylene)methane, tetrakis(1,4-cyclohexylene)methane, and so on.

R in case of (c) and n is 1 includes 2-methoxyethyl, 2-ethoxyethyl, 2-propyloxyethyl, 2-butoxyethyl, 1-methyl-2-methoxyethyl, 3-methoxybutyl, 3-methoxy-3-methylbutyl, 2-tert-butoxy -1-methylethyl, 2-(2-ethylhexyloxy)ethyl, and so on.

R in case of (c) and n is 2 includes 3-oxapentamethylene, 3,6-dioxaoctamethylene, 4-oxaheptamethylene, 4,8-dioxaundecamethylene, and so on.

Next, the (substituted)benzoyl group containing R' in the above formula (2) will be described below in more detail.

R' is a hydrogen atom when m and R' correspond to the above (A), and the applicable group in this case is benzoyl group.

When m and R' correspond to the above (B), R' includes methyl, ethyl, n-propyl, isopropyl, n-butyl, isobutyl, sec-butyl, tert-butyl, n-octyl, methoxy, ethoxy, butoxy, phenyl, and so on.

The substituted benzoyl group in this case includes 2-methylbenzoyl, 3-methylbenzoyl, 4-methylbenzoyl, 2-ethylbenzoyl, 3-ethylbenzoyl, 4-ethylbenzoyl, 2,3-dimethylbenzoyl, 2,4-dimethylbenzoyl, 2,5-dimethylbenzoyl, 2,6-dimethylbenzoyl, 3,4-dimethylbenzoyl, 3,5-dimethylbenzoyl, 2-n-propylbenzoyl, 3-n-propylbenzoyl, 4-n-propylbenzoyl, 2-isopropylbenzoyl, 3-isopropylbenzoyl, 4-isopropylbenzoyl, 2,3,4-trimethylbenzoyl, 2,3,5-trimethylbenzoyl, 2,3,6-trimethylbenzoyl, 2,4,5-trimethylbenzoyl, 2,4,6-trimethylbenzoyl, 3,4,5-trimethylbenzoyl, 2-n-butylbenzoyl, 3-n-butylbenzoyl, 4-n-butylbenzoyl, 2-tert-butylbenzoyl, 3-tert-butylbenzoyl, 4-tert-butylbenzoyl, 2-n-pentylbenzoyl, 3-n-pentylbenzoyl, 4-n-pentylbenzoyl, 2,3,4,5,6-pentamethylbenzoyl, 3-n-hexylbenzoyl, 4-n-hexylbenzoyl, 2-methoxybenzoyl, 3-methoxybenzoyl, 4-methoxybenzoyl, 2-ethoxybenzoyl, 3-ethoxybenzoyl, 4-ethoxybenzoyl, 2,3-dimethoxybenzoyl, 2,4-dimethoxybenzoyl, 2,5-dimethoxybenzoyl, 2,6-dimethoxybenzoyl, 3,4-dimethoxybenzoyl, 3,5-dimethoxybenzoyl, 2-n-propyloxybenzoyl, 3-n-propyloxybenzoyl, 4-n-propyloxybenzoyl, 2-isopropyloxybenzoyl, 3-isopropyloxybenzoyl, 4-isopropyloxybenzoyl, 2,3,4-trimethoxybenzoyl, 2,3,5-trimethoxybenzoyl, 2,3,6-trimethoxybenzoyl, 2,4,5-trimethoxybenzoyl, 2,4,6-trimethoxybenzoyl, 3,4,5-trimethoxybenzoyl, 2-n-butoxybenzoyl, 3-n-butoxybenzoyl, 4-n-butoxybenzoyl, 3-n-hexyloxybenzoyl, 4-n-hexyloxybenzoyl, 2-phenylbenzoyl, 3-phenylbenzoyl, 4-phenylbenzoyl, and so on.

When m and R' correspond to the above (C), R' includes trimethylsilyl, trimethoxysilyl, triphenylsilyl, dimethylvinylsilyl, diethylvinylsilyl, dimethoxyvinylsilyl, diphenylvinylsilyl, methyldivinylsilyl, ethyldivinylsilyl, methoxydivinylsilyl, phenyldivinylsilyl, trivinylsilyl, and so on.

The substituted benzoyl group in this case includes 2-trimethylsilylbenzoyl, 3-trimethylsilylbenzoyl, 4-trimethylsilylbenzoyl, 2-dimethylvinylsilylbenzoyl, 3-dimethylvinylsilylbenzoyl, 4-dimethylvinylsilylbenzoyl, 2-diethylvinylsilylbenzoyl, 3-diethylvinylsilylbenzoyl, 4-diethylvinylsilylbenzoyl, 2-methyldivinylsilylbenzoyl, 3-methyldivinylsilylbenzoyl, 4-methyldivinylsilylbenzoyl, 2-ethyldivinylsilylbenzoyl, 3-ethyldivinylsilylbenzoyl, 4-ethyldivinylsilylbenzoyl, and so on.

Wherein, R' can be the same or different in each (substituted)benzoyl group, and in each (substituted)benzoyl group in one molecule.

(Substituted)benzoylperoxy alkyl carbonate or (substituted)benzoylperoxy alkylene carbonate represented by the above formula (2) will be described below in more detail.

In the case of (a) and (A) including but not limited to the following, benzoylperoxy methyl monocarbonate, benzoylperoxy ethyl monocarbonate, benzoylperoxy n-propyl monocarbonate, benzoylperoxy isopropyl monocarbonate, benzoylperoxy n-butyl monocarbonate, benzoylperoxy sec-butyl monocarbonate, benzoylperoxy isobutyl monocarbonate, benzoylperoxy n-pentyl monocarbonate, benzoylperoxy neopentyl monocarbonate, benzoylperoxy hexyl monocarbonate, benzoylperoxy heptyl monocarbonate, benzoylperoxy octyl monocarbonate, benzoylperoxy 1-methylheptyl monocarbonate, benzoylperoxy 2-ethylhexyl monocarbonate, benzoylperoxy nonyl monocarbonate, benzoylperoxy decyl monocarbonate, benzoylperoxy myristyl monocarbonate, benzoylperoxy cetyl monocarbonate, benzoylperoxy stearyl monocarbonate, dibenzoylperoxy ethylene biscarbonate, dibenzoylperoxy trimethylene biscarbonate, dibenzoylperoxy 2-methyltrimethylene biscarbonate, dibenzoylperoxy 2,2-dimethyltrimethylene biscarbonate, dibenzoylperoxy 2,2-diethyltrimethylene biscarbonate, dibenzoylperoxy tetramethylene biscarbonate, dibenzoylperoxy pentamethylene biscarbonate, dibenzoylperoxy 3-methylpentamethylene biscarbonate, dibenzoylperoxy hexamethylene biscarbonate, dibenzoylperoxy heptamethylene biscarbonate, dibenzoylperoxy octamethylene biscarbonate, dibenzoylperoxy nonamethylene biscarbonate, dibenzoylperoxy decamethylene biscarbonate, dibenzoylperoxy dodecamethylene biscarbonate, dibenzoylperoxy hexadecamethylene biscarbonate, 1,1,1-tris(benzoylperoxycarbonyloxymethyl)ethane, 1,1,1-tris(benzoylperoxycarbonyloxymethyl)propane, 1,2,3-tris(benzoylperoxycarbonyloxymethyl)propane, 1,1,1-tris(benzoylperoxycarbonyloxymethyl)butane, 1,1,1,1-tetrakis(benzoylperoxycarbonyloxymethyl)methane, 1,2,3,4-tetrakis(benzoylperoxycarbonyloxymethyl)butane, 1,2,3,4,5-penta(benzoylperoxycarbonyloxymethyl)pentane, 1,2,3,4,5,6-hexa(benzoylperoxycarbonyloxymethyl)hexane

In the cases of (a) and (B) including but not limited to the following, 2-methylbenzoylperoxy methyl monocarbonate, 2-methylbenzoylperoxy ethyl monocarbonate, 2-methylbenzoylperoxy n-propyl monocarbonate, 2-methylbenzoylperoxy isopropyl monocarbonate, 2-methylbenzoylperoxy n-butyl monocarbonate, 2-methylbenzoylperoxy sec-butyl monocarbonate, 2-methylbenzoylperoxy isobutyl monocarbonate, 2-methylbenzoylperoxy n-pentyl monocarbonate, 2-methylbenzoylperoxy neopentyl monocarbonate, 2-methylbenzoylperoxy hexyl monocarbonate, 2-methylbenzoylperoxy heptyl monocarbonate, 2-methylbenzoylperoxy octyl monocarbonate, 2-methylbenzoylperoxy 1-methylheptyl monocarbonate, 2-methylbenzoylperoxy 2-ethylhexyl monocarbonate, 2-methylbenzoylperoxy nonyl monocarbonate, 2-methylbenzoylperoxy decyl monocarbonate, 2-methylbenzoylperoxy myristyl monocarbonate, 2-methylbenzoylperoxy cetyl monocarbonate, 2-methylbenzoylperoxy stearyl monocarbonate, di(2-methylbenzoylperoxy) ethylene biscarbonate, di(2-methylbenzoylperoxy) trimethylene biscarbonate, di(2-methylbenzoylperoxy) 2-methyltrimethylene biscarbonate, di(2-methylbenzoylperoxy) 2,2-dimethyltrimethylene biscarbonate, di(2-methylbenzoylperoxy) 2,2-diethyltrimethylene biscarbonate, di(2-methylbenzoylperoxy) tetramethylene biscarbonate, di(2-methylbenzoylperoxy) pentamethylene biscarbonate, di(2-methylbenzoylperoxy) 3-methylpentamethylene biscarbonate, di(2-methylbenzoylperoxy) hexamethylene biscarbonate, di(2-methylbenzoylperoxy) heptamethylene biscarbonate, di(2-methylbenzoylperoxy) octamethylene biscarbonate, di(2-methylbenzoylperoxy) nonamethylene biscarbonate, di(2-methylbenzoylperoxy) decamethylene biscarbonate, di(2-methylbenzoylperoxy) dodecamethylene biscarbonate, di(2-methylbenzoylperoxy) hexadecamethylene biscarbonate, 1,1,1-tris(2-methylbenzoylperoxycarbonyloxymethyl)ethane, 1,1,1-tris(2-methylbenzoylperoxycarbonyloxymethyl)propane, 1,2,3-tris(2-methylbenzoylperoxycarbonyloxymethyl)propane, 1,1,1-tris(2-methylbenzoylperoxycarbonyloxymethyl)butane, 1,1,1,1-tetrakis(2-methylbenzoylperoxycarbonyloxymethyl)methane, 1,2,3,4-tetrakis(2-methylbenzoylperoxycarbonyloxymethyl)butane, 1,2,3,4,5-penta-(2-methylbenzoylperoxycarbonyloxymethyl)pentane, 1,2,3,4,5,6-hexa(2-methylbenzoylperoxycarbonyloxymethyl)hexane,
3-methylbenzoylperoxy methyl monocarbonate, 3-methylbenzoylperoxy ethyl monocarbonate, 3-methylbenzoylperoxy n-propyl monocarbonate, 3-methylbenzoylperoxy isopropyl monocarbonate, 3-methylbenzoylperoxy n-butyl monocarbonate, 3-methylbenzoylperoxy sec-butyl monocarbonate, 3-methylbenzoylperoxy isobutyl monocarbonate, 3-methylbenzoylperoxy n-pentyl monocarbonate, 3-methylbenzoylperoxy neopentyl monocarbonate, 3-methylbenzoylperoxy hexyl monocarbonate, 3-methylbenzoylperoxy heptyl monocarbonate, 3-methylbenzoylperoxy octyl monocarbonate, 3-methylbenzoylperoxy 1-methylheptyl monocarbonate, 3-methylbenzoylperoxy 2-ethylhexyl monocarbonate, 3-methylbenzoylperoxy nonyl monocarbonate, 3-methylbenzoylperoxy decyl monocarbonate, 3-methylbenzoylperoxy myristyl monocarbonate, 3-methylbenzoylperoxy cetyl monocarbonate, 3-methylbenzoylperoxy stearyl monocarbonate, di(3-methylbenzoylperoxy) ethylene biscarbonate, di(3-methylbenzoylperoxy) trimethylene biscarbonate, di(3-methylbenzoylperoxy) 2-methyltrimethylene biscarbonate, di(3-methylbenzoylperoxy) 2,2-dimethyltrimethylene biscarbonate, di(3-methylbenzoylperoxy) 2,2-diethyltrimethylene biscarbonate, di(3-methylbenzoylperoxy) tetramethylene biscarbonate, di(3-methylbenzoylperoxy) pentamethylene biscarbonate, di(3-methylbenzoylperoxy) 3-methylpentamethylene biscarbonate, di(3-methylbenzoylperoxy) hexamethylene biscarbonate, di(3-methylbenzoylperoxy) heptamethylene biscarbonate, di(3-methylbenzoylperoxy) octamethylene biscarbonate, di(3-methylbenzoylperoxy) nonamethylene biscarbonate, di(3-methylbenzoylperoxy) decamethylene biscarbonate, di(3-methylbenzoylperoxy) dodecamethylene biscarbonate, di(3-methylbenzoylperoxy) hexadecamethylene biscarbonate, 1,1,1-tris(3-methylbenzoylperoxycarbonyloxymethyl)ethane, 1,1,1-tris(3-methylbenzoylperoxycarbonyloxymethyl)propane, 1,2,3-tris(3-methylbenzoylperoxycarbonyloxymethyl)propane, 1,1,1-tris(3-methylbenzoylperoxycarbonyloxymethyl)butane, 1,1,1,1-tetrakis(3-methylbenzoylperoxycarbonyloxymethyl)methane, 1,2,3,4-tetrakis(3-methylbenzoylperoxycarbonyloxymethyl)butane, 1,2,3,4,5-penta(3-methylbenzoylperoxycarbonyloxymethyl)pentane, 1,2,3,4,5,6-hexa(3-methylbenzoylperoxycarbonyloxymethyl)hexane,
4-methylbenzoylperoxy methyl monocarbonate, 4-methylbenzoylperoxy ethyl monocarbonate, 4-methylbenzoylperoxy n-propyl monocarbonate, 4-methylbenzoylperoxy isopropyl monocarbonate, 4-methylbenzoylperoxy n-butyl monocarbonate, 4-methylbenzoylperoxy sec-butyl monocarbonate, 4-methylbenzoylperoxy isobutyl monocarbonate, 4-methylbenzoylperoxy n-pentyl monocarbonate, 4-methylbenzoylperoxy neopentyl monocarbonate, 4-methylbenzoylperoxy hexyl monocarbonate, 4-methylbenzoylperoxy heptyl monocarbonate, 4-methylbenzoylperoxy octyl monocarbonate, 4-methylbenzoylperoxy 1-methylheptyl monocarbonate, 4-methylbenzoylperoxy 2-ethylhexyl monocarbonate, 4-methylbenzoylperoxy nonyl monocarbonate, 4-methylbenzoylperoxy decyl monocarbonate, 4-methylbenzoylperoxy myristyl monocarbonate, 4-methylbenzoylperoxy cetyl monocarbonate, 4-methylbenzoylperoxy stearyl monocarbonate, di(4-methylbenzoylperoxy) ethylene biscarbonate, di(4-methylbenzoylperoxy) trimethylene biscarbonate, di(4-methylbenzoylperoxy) 2-methyltrimethylene biscarbonate, di(4-methylbenzoylperoxy) 2,2-dimethyltrimethylene biscarbonate, di(4-methylbenzoylperoxy) 2,2-diethyltrimethylene biscarbonate, di(4-methylbenzoylperoxy) tetramethylene biscarbonate, di(4-methylbenzoylperoxy) pentamethylene biscarbonate, di(4-methylbenzoylperoxy) 3-methylpentamethylene biscarbonate, di(4-methylbenzoylperoxy) hexamethylene biscarbonate, di(4-methylbenzoylperoxy) heptamethylene biscarbonate di(4-methylbenzoylperoxy) octamethylene biscarbonate, di(4-methylbenzoylperoxy) nonamethylene biscarbonate, di(4-methylbenzoylperoxy) decamethylene biscarbonate, di(4-methylbenzoylperoxy) dodecamethylene biscarbonate, di(4-methylbenzoylperoxy) hexadecamethylene biscarbonate, 1,1,1-tris(4-methylbenzoylperoxycarbonyloxymethyl)ethane, 1,1,1-tris(4-methylbenzoylperoxycarbonyloxymethyl)propane, 1,2,3-tris(4-methylbenzoylperoxycarbonyloxymethyl)propane, 1,1,1-tris(4-methylbenzoylperoxycarbonyloxymethyl)butane, 1,1,1,1-tetrakis(4-methylbenzoylperoxycarbonyloxymethyl)methane, 1,2,3,4-tetrakis(4-methylbenzoylperoxycarbonyloxymethyl)butane, 1,2,3,4,5-penta(4-methylbenzoylperoxycarbonyloxymethyl)pentane, 1,2,3,4,5,6-hexa(4-methylbenzoylperoxycarbonyloxymethyl)hexane,
4-ethylbenzoylperoxy methyl monocarbonate, 4-ethylbenzoylperoxy ethyl monocarbonate, 4-ethylbenzoylperoxy n-propyl monocarbonate, 4-ethylbenzoylperoxy isopropyl monocarbonate, 4-ethylbenzoylperoxy n-butyl monocarbonate, 4-ethylbenzoylperoxy sec-butyl monocarbonate, 4-ethylbenzoylperoxy isobutyl monocarbonate, 4-ethylbenzoylperoxy n-pentyl monocarbonate, 4-ethylbenzoylperoxy neopentyl monocarbonate, 4-ethylbenzoylperoxy hexyl monocarbonate, 4-ethylbenzoylperoxy heptyl monocarbonate, 4-ethylbenzoylperoxy octyl monocarbonate, 4-ethylbenzoylperoxy 1-methylheptyl monocarbonate, 4-ethylbenzoylperoxy 2-ethylhexyl monocarbonate, 4-ethylbenzoylperoxy nonyl monocarbonate, 4-ethylbenzoylperoxy decyl monocarbonate, 4-ethylbenzoylperoxy myristyl monocarbonate, 4-ethylbenzoylperoxy cetyl monocarbonate, 4-ethylbenzoylperoxy stearyl monocarbonate, di(4-ethylbenzoylperoxy) ethylene biscarbonate, di(4-ethylbenzoylperoxy) trimethylene biscarbonate, di(4-ethylbenzoylperoxy) 2-methyltrimethylene biscarbonate, di(4-ethylbenzoylperoxy) 2,2-dimethyltrimethylene biscarbonate, di(4-ethylbenzoylperoxy) 2,2-diethyltrimethylene biscarbonate, di(4-ethylbenzoylperoxy) tetramethylene biscarbonate, di(4-ethylbenzoylperoxy) pentamethylene biscarbonate, di(4-ethylbenzoylperoxy) 3-methylpentamethylene biscarbonate, di(4-ethylbenzoylperoxy) hexamethylene biscarbonate, di(4-ethylbenzoylperoxy) heptamethylene biscarbonate, di(4-ethylbenzoylperoxy) octamethylene biscarbonate, di(4-ethylbenzoylperoxy) nonamethylene biscarbonate, di(4-ethylbenzoylperoxy) decamethylene biscarbonate, di(4-ethylbenzoylperoxy) dodecamethylene biscarbonate, di(4-ethylbenzoylperoxy) hexadecamethylene biscarbonate, 1,1,1-tris(4-ethylbenzoylperoxycarbonyloxymethyl)ethane, 1,1,1-tris(4-ethylbenzoylperoxycarbonyloxymethyl)propane, 1,2,3-tris(4-ethylbenzoylperoxycarbonyloxymethyl)propane, 1,1,1-tris(4-ethylbenzoylperoxycarbonyloxymethyl)butane, 1,1,1,1-tetrakis(4-ethylbenzoylperoxycarbonyloxymethyl)methane, 1,2,3,4-tetrakis(4-ethylbenzoylperoxycarbonyloxymethyl)butane, 1,2,3,4,5-penta(4-ethylbenzoylperoxycarbonyloxymethyl)pentane, 1,2,3,4,5,6-hexa(4-ethylbenzoylperoxycarbonyloxymethyl)hexane,
2,3-dimethylbenzoylperoxymethylmonocarbonate, 2, 3-dimethylbenzoylperoxy ethyl monocarbonate, 2,3-dimethylbenzoylperoxy n-propyl monocarbonate, 2,3-dimethylbenzoylperoxy isopropyl monocarbonate, 2,3-dimethylbenzoylperoxy n-butyl monocarbonate, 2,3-dimethylbenzoylperoxy sec-butyl monocarbonate, 2,3-dimethylbenzoylperoxy isobutyl monocarbonate, 2,3-dimethylbenzoylperoxy n-pentyl monocarbonate, 2,3-dimethylbenzoylperoxy neopentyl monocarbonate, 2,3-dimethylbenzoylperoxy 1-methylheptyl monocarbonate, 2,3-dimethylbenzoylperoxy 2-ethylhexyl monocarbonate, 2,3-dimethylbenzoylperoxy decyl monocarbonate, 2,3-dimethylbenzoylperoxy myristyl monocarbonate, 2,3-dimethylbenzoylperoxy myristyl monocarbonate, 2,3-dimethylbenzoylperoxy stearyl monocarbonate, di(2,3-dimethylbenzoylperoxy) ethylene biscarbonate, di(2,3-dimethylbenzoylperoxy) trimethylene biscarbonate, di(2,3-dimethylbenzoylperoxy) tetramethylene biscarbonate, di(2,3-dimethylbenzoylperoxy) pentamethylene biscarbonate, di(2,3-dimethylbenzoylperoxy) hexamethylene biscarbonate, di(2,3-dimethylbenzoylperoxy) decamethylene biscarbonate, di(2,3-dimethylbenzoylperoxy) dodecamethylene biscarbonate, 1,1,1-tris(2,3-dimethylbenzoylperoxycarbonyloxymethyl)ethane, 1,1,1-tris(2,3-dimethylbenzoylperoxycarbonyloxymethyl)propane, 1,2,3-tris(2,3-dimethylbenzoylperoxycarbonyloxymethyl)propane, 1,1,1-tris(2,3-dimethylbenzoylperoxycarbonyloxymethyl)butane, 1,1,1,1-tetrakis(2,3-dimethylbenzoylperoxycarbonyloxymethyl)methane, 1,2,3,4-tetrakis(2,3-dimethylbenzoylperoxycarbonyloxymethyl)butane, 1,2,3,4,5-penta(2,3-dimethylbenzoylperoxycarbonyloxymethyl)pentane, 1,2,3,4,5,6-hexa(2,3-dimethylbenzoylperoxycarbonyloxymethyl)hexane,
2,4-dimethylbenzoylperoxymethyl monocarbonate, 2,4-dimethylbenzoylperoxy ethyl monocarbonate, 2,4-dimethylbenzoylperoxy n-propyl monocarbonate, 2,4-dimethylbenzoylperoxy isopropyl monocarbonate, 2,4-dimethylbenzoylperoxy n-butyl monocarbonate, 2,4-dimethylbenzoylperoxy sec-butyl monocarbonate, 2,4-dimethylbenzoylperoxy isobutyl monocarbonate, 2,4-dimethylbenzoylperoxy n-pentyl monocarbonate, 2,4-dimethylbenzoylperoxy neopentyl monocarbonate, 2,4-dimethylbenzoylperoxy hexyl monocarbonate, 2,4-dimethylbenzoylperoxy heptyl monocarbonate, 2,4-dimethylbenzoylperoxy octyl monocarbonate, 2,4-dimethylbenzoylperoxy 1-methylheptyl monocarbonate, 2,4-dimethylbenzoylperoxy 2-ethylhexyl monocarbonate, 2,4-dimethylbenzoylperoxy nonyl monocarbonate, 2,4-dimethylbenzoylperoxy decyl monocarbonate, 2,4-dimethylbenzoylperoxy myristyl monocarbonate, 2,4-dimethylbenzoylperoxy myristyl monocarbonate, 2,4-dimethylbenzoylperoxy stearyl monocarbonate, di (2,4-dimethylbenzoylperoxy) ethylene biscarbonate, di(2,4-dimethylbenzoylperoxy) trimethylene biscarbonate, di(2,4-dimethylbenzoylperoxy) 2-methyltrimethylene biscarbonate, di (2,4-dimethylbenzoylperoxy) 2,2-dimethyltrimethylene biscarbonate, di(2,4dimethylbenzoylperoxy) 2,2-diethyltrimethylene biscarbonate, di(2,4-dimethylbenzoylperoxy) tetramethylene biscarbonate, di(2,4-dimethylbenzoylperoxy) pentamethylene biscarbonate, di(2,4-dimethylbenzoylperoxy) 3-methylpentamethylene biscarbonate, di (2,4-dimethylbenzoylperoxy) hexamethylene biscarbonate, di(2,4-dimethylbenzoylperoxy) heptamethylene biscarbonate, di (2,4-dimethylbenzoylperoxy) octamethylene biscarbonate, di(2,4-dimethylbenzoylperoxy) nonamethylene biscarbonate, di(2,4-dimethylbenzoylperoxy) decamethylene biscarbonate, di(2,4-dimethylbenzoylperoxy) dodecamethylene biscarbonate, di(2,4-dimethylbenzoylperoxy) hexadecamethylene biscarbonate, 1,1,1-tris (2,4-dimethylbenzoylperoxycarbonyloxymethyl)ethane, 1,1,1-tris(2,4-dimethylbenzoylperoxycarbonyloxymethyl)propane, 1,2,3-tris(2,4-dimethylbenzoylperoxycarbonyloxymethyl)propane, 1,1,1-tris(2,4-dimethylbenzoylperoxycarbonyloxymethyl)butane, 1,1,1,1-tetrakis(2,4-dimethylbenzoylperoxycarbonyloxymethyl)methane, 1,2,3,4-tetrakis(2,4-dimethylbenzoylperoxycarbonyloxymethyl)butane, 1,2,3,4,5-,penta(2,4-dimethylbenzoylperoxycarbonyloxymethyl)pentane, 1,2,3,4,5,6-hexa(2,4-dimethylbenzoylperoxycarbonyloxymethyl)hexane,
2,5-dimethylbenzoylperoxy methyl monocarbonate, 2,5-dimethylbenzoylperoxy ethyl monocarbonate, 2,5-dimethylbenzoylperoxy n-propyl monocarbonate, 2,5-dimethylbenzoylperoxy isopropylmonocarbonate, 2,5-dimethylbenzoylperoxy n-butyl monocarbonate, 2,5-dimethylbenzoylperoxy sec-butyl monocarbonate, 2,5-dimethylbenzoylperoxy isobutyl monocarbonate, 2,5-dimethylbenzoylperoxy n-pentyl monocarbonate, 2,5-dimethylbenzoylperoxy neopentyl monocarbonate, 2,5-dimethylbenzoylperoxy 1-methylheptyl monocarbonate, 2,5-dimethylbenzoylperoxy 2-ethylhexyl monocarbonate, 2,5-dimethylbenzoylperoxy decyl monocarbonate, 2,5-dimethylbenzoylperoxy myristyl monocarbonate, 2,5-dimethylbenzoylperoxy myristyl monocarbonate, 2,5-dimethylbenzoylperoxy stearyl monocarbonate, di(2,5-dimethylbenzoylperoxy) ethylene biscarbonate, di(2,5-dimethylbenzoylperoxy) trimethylene biscarbonate, di(2,5-dimethylbenzoylperoxy) tetramethylene biscarbonate, di(2,5-dimethylbenzoylperoxy) pentamethylene biscarbonate, di(2,5-dimethylbenzoylperoxy) hexamethylene biscarbonate, di(2,5-dimethylbenzoylperoxy) decamethylene biscarbonate, di(2,5-dimethylbenzoylperoxy) dodecamethylene biscarbonate, 1,1,1-tris(2,5-dimethylbenzoylperoxycarbonyloxymethyl)ethane, 1,1,1-tris(2,5-dimethylbenzoylperoxycarbonyloxymethyl)propane, 1,2,3-tris(2,5-dimethylbenzoylperoxycarbonyloxymethyl)propane, 1,1,1-tris(2,5-dimethylbenzoylperoxycarbonyloxymethyl)butane, 1,1,1,1-tetrakis(2,5-dimethylbenzoylperoxycarbonyloxymethyl)methane, 1,2,3,4-tetrakis(2,5-dimethylbenzoylperoxycarbonyloxymethyl)butane, 1,2,3,4,5-penta(2,5-dimethylbenzoylperoxycarbonyloxymethyl)pentane, 1,2,3,4,5,6-hexa(2,5-dimethylbenzoylperoxycarbonyloxymethyl)hexane,
2,6-dimethylbenzoylperoxymethyl monocarbonate, 2,6-dimethylbenzoylperoxy ethyl monocarbonate, 2,6-dimethylbenzoylperoxy n-propyl monocarbonate, 2,6-dimethylbenzoylperoxy isopropyl monocarbonate, 2,6-dimethylbenzoylperoxy n-butyl monocarbonate, 2,6-dimethylbenzoylperoxy sec-butyl monocarbonate, 2,6-dimethylbenzoylperoxy isobutyl monocarbonate, 2,6-dimethylbenzoylperoxy n-pentyl monocarbonate, 2,6-dimethylbenzoylperoxy neopentyl monocarbonate, 2,6-dimethylbenzoylperoxy 1-methylheptyl monocarbonate, 2,6-dimethylbenzoylperoxy 2-ethylhexyl monocarbonate, 2,6-dimethylbenzoylperoxy decyl monocarbonate, 2,6-dimethylbenzoylperoxy myristyl monocarbonate, 2,6-dimethylbenzoylperoxy myristyl monocarbonate, 2,6-dimethylbenzoylperoxy stearyl monocarbonate, di(2,6-dimethylbenzoylperoxy) ethylene biscarbonate, di(2,6-dimethylbenzoylperoxy) trimethylene biscarbonate, di(2,6-dimethylbenzoylperoxy) tetramethylene biscarbonate, di(2,6-dimethylbenzoylperoxy) pentamethylene biscarbonate, di(2,6-dimethylbenzoylperoxy) hexamethylene biscarbonate, di(2,6-dimethylbenzoylperoxy) decamethylene biscarbonate, di(2,6-dimethylbenzoylperoxy) dodecamethylene biscarbonate, 1,1,1-tris(2,6-dimethylbenzoylperoxycarbonyloxymethyl)ethane, 1,1,1-tris(2,6-dimethylbenzoylperoxycarbonyloxymethyl)propane, 1,2,3-tris(2,6-dimethylbenzoylperoxycarbonyloxymethyl)propane, 1,1,1-tris(2,6-dimethylbenzoylperoxycarbonyloxymethyl)butane, 1,1,1,1-tetrakis(2,6-dimethylbenzoylperoxycarbonyloxymethyl)methane, 1,2,3,4-tetrakis(2,6-dimethylbenzoylperoxycarbonyloxymethyl)butane, 1,2,3,4,5-penta(2,6-dimethylbenzoylperoxycarbonyloxymethyl)pentane, 1,2,3,4,5,6-hexa(2,6-dimethylbenzoylperoxycarbonyloxymethyl)hexane,
3,4-dimethylbenzoylperoxymethyl monocarbonate, 3,4-dimethylbenzoylperoxy ethyl monocarbonate, 3,4-dimethylbenzoylperoxy n-propyl monocarbonate, 3,4-dimethylbenzoylperoxy isopropyl monocarbonate, 3,4-dimethylbenzoylperoxy n-butyl monocarbonate, 3,4-dimethylbenzoylperoxy sec-butyl monocarbonate, 3,4-dimethylbenzoylperoxy isobutyl monocarbonate, 3,4-dimethylbenzoylperoxy n-pentyl monocarbonate, 3,4-dimethylbenzoylperoxy neopentyl monocarbonate, 3,4-dimethylbenzoylperoxy 1-methylheptyl monocarbonate, 3,4-dimethylbenzoylperoxy 2-ethylhexyl monocarbonate, 3,4-dimethylbenzoylperoxy decyl monocarbonate, 3,4-dimethylbenzoylperoxy myristyl monocarbonate, 3,4-dimethylbenzoylperoxy myristyl monocarbonate, 3,4-dimethylbenzoylperoxy stearyl monocarbonate, di(3,4-dimethylbenzoylperoxy) ethylene biscarbonate, di (3,4-dimethylbenzoylperoxy) trimethylene biscarbonate, di(3,4-dimethylbenzoylperoxy) tetramethylene biscarbonate, di(3,4-dimethylbenzoylperoxy) pentamethylene biscarbonate, di(3,4-dimethylbenzoylperoxy) hexamethylene biscarbonate, di(3,4-dimethylbenzoylperoxy) decamethylene biscarbonate, di(3,4-dimethylbenzoylperoxy) dodecamethylene biscarbonate, 1,1,1-tris(3,4-dimethylbenzoylperoxycarbonyloxymethyl)ethane, 1,1,1-tris(3,4-dimethylbenzoylperoxycarbonyloxymethyl)propane, 1,2,3-tris(3,4-dimethylbenzoylperoxycarbonyloxymethyl)propane, 1,1,1-tris(3,4-dimethylbenzoylperoxycarbonyloxymethyl)butane, 1,1,1,1-tetrakis(3,4-dimethylbenzoylperoxycarbonyloxymethyl)methane, 1,2,3,4-tetrakis(3,4-dimethylbenzoylperoxycarbonyloxymethyl)butane, 1,2,3,4,5-penta(3,4-dimethylbenzoylperoxycarbonyloxymethyl)pentane, 1,2,3,4,5,6-hexa(3,4-dimethylbenzoylperoxycarbonyloxymethyl)hexane,
3,5-dimethylbenzoylperoxymethyl monocarbonate, 3,5-dimethylbenzoylperoxy ethyl monocarbonate, 3,5-dimethylbenzoylperoxy n-propyl monocarbonate, 3,5-dimethylbenzoylperoxy isopropyl monocarbonate, 3,5-dimethylbenzoylperoxy n-butyl monocarbonate, 3,5-dimethylbenzoylperoxy sec-butyl monocarbonate, 3,5-dimethylbenzoylperoxy isobutyl monocarbonate, 3,5-dimethylbenzoylperoxy n-pentyl monocarbonate, 3,5-dimethylbenzoylperoxy neopentyl monocarbonate, 3,5-dimethylbenzoylperoxy 1-methylheptyl monocarbonate, 3,5-dimethylbenzoylperoxy 2-ethylhexyl monocarbonate, 3,5-dimethylbenzoylperoxy decyl monocarbonate, 3,5-dimethylbenzoylperoxy myristyl monocarbonate, 3,5-dimethylbenzoylperoxy myristyl monocarbonate, 3,5-dimethylbenzoylperoxy stearyl monocarbonate, di (3,5-dimethylbenzoylperoxy) ethylene biscarbonate, di(3,5-dimethylbenzoylperoxy) trimethylene biscarbonate, di(3,5-dimethylbenzoylperoxy) tetramethylene biscarbonate, di(3,5-dimethylbenzoylperoxy) pentamethylene biscarbonate, di(3,5-dimethylbenzoylperoxy) hexamethylene biscarbonate, di (3,5-dimethylbenzoylperoxy) decamethylene biscarbonate, di (3,5-dimethylbenzoylperoxy) dodecamethylene biscarbonate, 1,1,1-tris(3,5-dimethylbenzoylperoxycarbonyloxymethyl)ethane, 1,1,1-tris(3,5-dimethylbenzoylperoxycarbonyloxymethyl)propane, 1,2,3-tris(3,5-dimethylbenzoylperoxycarbonyloxymethyl)propane, 1,1,1-tris(3,5-dimethylbenzoylperoxycarbonyloxymethyl)butane, 1,1,1,1-tetrakis(3,5-dimethylbenzoylperoxycarbonyloxymethyl)methane, 1,2,3,4-tetrakis(3,5-dimethylbenzoylperoxycarbonyloxymethyl)butane, 1,2,3,4,5-penta(3,5-dimethylbenzoylperoxycarbonyloxymethyl)pentane, 1,2,3,4,5,6-hexa(3,5-dimethylbenzoylperoxycarbonyloxymethyl)hexane,
4-n-propylbenzoylperoxy methyl monocarbonate, 4-n-propylbenzoylperoxy ethyl monocarbonate, 4-n-propylbenzoylperoxy n-propyl monocarbonate, 4-n-propylbenzoylperoxy isopropyl monocarbonate, 4-n-propylbenzoylperoxy n-butyl monocarbonate, 4-n-propylbenzoylperoxy sec-butyl monocarbonate, 4-n-propylbenzoylperoxy isobutyl monocarbonate, 4-n-propylbenzoylperoxy n-pentyl monocarbonate, 4-n-propylbenzoylperoxy neopentyl monocarbonate, 4-n-propylbenzoylperoxy hexyl monocarbonate, 4-n-propylbenzoylperoxy heptyl monocarbonate, 4-n-propylbenzoylperoxy octyl monocarbonate, 4-n-propylbenzoylperoxy 1-methylheptyl monocarbonate, 4-n-propylbenzoylperoxy 2-ethylhexyl monocarbonate, 4-n-propylbenzoylperoxy nonyl monocarbonate, 4-n-propylbenzoylperoxy decyl monocarbonate, 4-n-propylbenzoylperoxy myristyl monocarbonate, 4-n-propylbenzoylperoxy myristyl monocarbonate, 4-n-propylbenzoylperoxy stearyl monocarbonate, di(4-n-propylbenzoylperoxy) ethylene biscarbonate, di(4-n-propylbenzoylperoxy) trimethylene biscarbonate, di(4-n-propylbenzoylperoxy) 2-methyltrimethylene biscarbonate, di(4-n-propylbenzoylperoxy) 2,2-diethyltrimethylene biscarbonate, di(4-n-propylbenzoylperoxy) tetramethylene biscarbonate, di(4-n-propylbenzoylperoxy) pentamethylene biscarbonate, di(4-n-propylbenzoylperoxy) 3-methylpentamethylene biscarbonate, di(4-n-propylbenzoylperoxy) hexamethylene biscarbonate, di(4-n-propylbenzoylperoxy) heptamethylene biscarbonate, di(4-n-propylbenzoylperoxy) octamethylene biscarbonate, di(4-n-propylbenzoylperoxy) nonamethylene biscarbonate, di(4-n-propylbenzoylperoxy) decamethylene biscarbonate, di(4-n-propylbenzoylperoxy) dodecamethylene biscarbonate, di(4-n-propylbenzoylperoxy) hexadecamethylene biscarbonate, 1,1,1-tris (4-n-propylbenzoylperoxycarbonyloxymethyl)ethane, 1,1,1-tris(4-n-propylbenzoylperoxycarbonyloxymethyl)propane, 1,2,3-tris(4-n-propylbenzoylperoxycarbonyloxymethyl)propane, 1,1,1-tris(4-n-propylbenzoylperoxycarbonyloxymethyl)butane, 1,1,1,1-tetrakis(4-n-propylbenzoylperoxycarbonyloxymethyl)methane, 1,2,3,4-tetrakis(4-n-propylbenzoylperoxycarbonyloxymethyl)butane, 1,2,3,4,5-penta(4-n-propylbenzoylperoxycarbonyloxymethyl)pentane, 1,2,3,4,5,6-hexa(4-n-propylbenzoylperoxycarbonyloxymethyl)hexane,
4-isopropylbenzoylperoxy methyl monocarbonate, 4-isopropylbenzoylperoxy ethyl monocarbonate, 4-isopropylbenzoylperoxy n-propyl monocarbonate, 4-isopropylbenzoylperoxy isopropyl monocarbonate, 4-isopropylbenzoylperoxy n-butyl monocarbonate, 4-isopropylbenzoylperoxy sec-butyl monocarbonate, 4-isopropylbenzoylperoxy isobutyl monocarbonate, 4-isopropylbenzoylperoxy n-pentyl monocarbonate, 4-isopropylbenzoylperoxy neopentyl monocarbonate, 4-isopropylbenzoylperoxy hexyl monocarbonate, 4-isopropylbenzoylperoxy heptyl monocarbonate, 4-isopropylbenzoylperoxy octyl monocarbonate, 4-isopropylbenzoylperoxy 1-methylheptyl monocarbonate, 4-isopropylbenzoylperoxy 2-ethylhexyl monocarbonate, 4-isopropylbenzoylperoxy nonyl monocarbonate, 4-isopropylbenzoylperoxy decyl monocarbonate, 4-isopropylbenzoylperoxy myristyl monocarbonate, 4-isopropylbenzoylperoxy myristyl monocarbonate, 4-isopropylbenzoylperoxy stearyl monocarbonate, di(4-isopropylbenzoylperoxy) ethylene biscarbonate, di(4-isopropylbenzoylperoxy) trimethylene biscarbonate, di(4-isopropylbenzoylperoxy) 2-methyltrimethylene biscarbonate, di(4-isopropylbenzoylperoxy) 2,2-dimethyltrimethylene biscarbonate, di(4-isopropylbenzoylperoxy) 2,2-diethyltrimethylene biscarbonate, di(4-isopropylbenzoylperoxy) tetramethylene biscarbonate, di(4-isopropylbenzoylperoxy) pentamethylene biscarbonate, di(4-isopropylbenzoylperoxy) 3-methylpentamethylene biscarbonate, di(4-isopropylbenzoylperoxy) hexamethylene biscarbonate, di(4-isopropylbenzoylperoxy) heptamethylene biscarbonate, di(4-isopropylbenzoylperoxy) octamethylene biscarbonate, di(4-isopropylbenzoylperoxy) nonamethylene biscarbonate, di(4-isopropylbenzoylperoxy) decamethylene biscarbonate, di(4-isopropylbenzoylperoxy) dodecamethylene biscarbonate, di(4-isopropylbenzoylperoxy) hexadecamethylene biscarbonate, 1,1,1-tris(4-isopropylbenzoylperoxycarbonyloxymethyl)ethane, 1,1,1-tris(4-isopropylbenzoylperoxycarbonyloxymethyl)propane, 1,2,3-tris(4-isopropylbenzoylperoxycarbonyloxymethyl)propane, 1,1,1-tris(4-isopropylbenzoylperoxycarbonyloxymethyl)butane, 1,1,1,1-tetrakis(4-isopropylbenzoylperoxycarbonyloxymethyl)methane, 1,2,3,4-tetrakis(4-isopropylbenzoylperoxycarbonyloxymethyl)butane, 1,2,3,4,5-penta(4-isopropylbenzoylperoxycarbonyloxymethyl)pentane, 1,2,3,4,5,6-hexa(4-isopropylbenzoylperoxycarbonyloxymethyl)hexane,
2,4,6-trimethylbenzoylperoxy methyl monocarbonate, 2,4,6-trimethylbenzoylperoxy ethyl monocarbonate, 2,4,6-trimethylbenzoylperoxy n-propyl monocarbonate, 2,4,6-trimethylbenzoylperoxy isopropyl monocarbonate, 2,4,6-trimethylbenzoylperoxy n-butyl monocarbonate, 2,4,6-trimethylbenzoylperoxy sec-butyl monocarbonate, 2,4,6-trimethylbenzoylperoxy isobutyl monocarbonate, 2,4,6-trimethylbenzoylperoxy n-pentyl monocarbonate, 2,4,6-trimethylbenzoylperoxy neopentyl monocarbonate, 2,4,6-trimethylbenzoylperoxy 1-methylheptyl monocarbonate, 2,4,6-trimethylbenzoylperoxy 2-ethylhexyl monocarbonate, 2,4,6-trimethylbenzoylperoxy decyl monocarbonate, 2,4,6-trimethylbenzoylperoxy myristyl monocarbonate, 2,4,6-trimethylbenzoylperoxy myristyl monocarbonate, 2,4,6-trimethylbenzoylperoxy stearyl monocarbonate, di(2,4,6-trimethylbenzoylperoxy) ethylene biscarbonate, di(2,4,6-trimethylbenzoylperoxy) trimethylene biscarbonate, di(2,4,6-trimethylbenzoylperoxy) tetramethylene biscarbonate, di(2,4,6-trimethylbenzoylperoxy) pentamethylene biscarbonate, di(2,4,6-trimethylbenzoylperoxy) hexamethylene biscarbonate, di(2,4,6-trimethylbenzoylperoxy) decamethylene biscarbonate, di(2,4,6-trimethylbenzoylperoxy) dodecamethylene biscarbonate, 1,1,1-tris(2,4,6-trimethylbenzoylperoxycarbonyloxymethyl)ethane, 1,1,1-tris(2,4,6-trimethylbenzoylperoxycarbonyloxymethyl)propane, 1,2,3-tris (2,4,6-trimethylbenzoylperoxycarbonyloxymethyl)propane, 1,1,1-tris(2,4,6-trimethylbenzoylperoxycarbonyloxymethyl)butane, 1,1,1,1-tetrakis(2,4,6-trimethylbenzoylperoxycarbonyloxymethyl)methane, 1,2,3,4-tetrakis(2,4,6-trimethylbenzoylperoxycarbonyloxymethyl)butane, 1,2,3,4,5-penta(2,4,6-trimethylbenzoylperoxycarbonyloxymethyl)pentane, 1,2,3,4,5,6-hexa(2,4,6-trimethylbenzoylperoxycarbonyloxymethyl)hexane,
4-n-butylbenzoylperoxy methyl monocarbonate, 4-n-butylbenzoylperoxy ethyl monocarbonate, 4-n-butylbenzoylperoxy n-propyl monocarbonate, 4-n-butylbenzoylperoxy isopropyl monocarbonate, 4-n-butybenzoylperoxy n-butyl monocarbonate, 4-n-butylbenzoylperoxy sec-butyl monocarbonate, 4-n-butylbenzoylperoxy isobutyl monocarbonate, 4-n-butylbenzoylperoxy n-pentyl monocarbonate, 4-n-butylbenzoylperoxy neopentyl monocarbonate, 4-n-butylbenzoylperoxy hexyl monocarbonate, 4-n-butylbenzoylperoxy heptyl monocarbonate, 4-n-butylbenzoylperoxy octyl monocarbonate, 4-n-butylbenzoylperoxy 1-methylheptyl monocarbonate, 4-n-butylbenzoylperoxy 2-ethylhexyl monocarbonate, 4-n-butylbenzoylperoxy nonyl monocarbonate, 4-n-butylbenzoylperoxy decyl monocarbonate, 4-n-butylbenzoylperoxy myristyl monocarbonate, 4-n-butylbenzoylperoxy myristyl monocarbonate, 4-n-butylbenzoylperoxy stearyl monocarbonate, di(4-n-butylbenzoylperoxy) ethylene biscarbonate, di(4-n-butylbenzoylperoxy) trimethylene biscarbonate, di(4-n-butylbenzoylperoxy) 2-methyltrimethylene biscarbonate, di(4-n-butylbenzoylperoxy) 2,2-dimethyltrimethylene biscarbonate, di(4-n-butylbenzoylperoxy) 2,2-diethyltrimethylene biscarbonate, di (4-n-butylbenzoylperoxy) tetramethylene biscarbonate, di(4-n-butylbenzoylperoxy) pentamethylene biscarbonate, di(4-n-butylbenzoylperoxy) 3-methylpentamethylene biscarbonate, di(4-n-butylbenzoylperoxy) hexamethylene biscarbonate, di(4-n-butylbenzoylperoxy) heptamethylene biscarbonate, di(4-n-butylbenzoylperoxy) octamethylene biscarbonate, di(4-n-butylbenzoylperoxy) nonamethylene biscarbonate, di(4-n-butylbenzoylperoxy) decamethylene biscarbonate, di(4-n-butylbenzoylperoxy) dodecamethylene biscarbonate, di(4-n-butylbenzoylperoxy) hexadecamethylene biscarbonate, 1,1,1-tris(4-n-butylbenzoylperoxycarbonyloxymethyl)ethan, 1,1,1-tris(4-n-butylbenzoylperoxycarbonyloxymethyl)propane, 1,2,3-tris(4-n-butylbenzoylperoxycarbonyloxymethyl)propane, 1,1,1-tris(4-n-butylbenzoylperoxycarbonyloxymethyl)butane, 1,1,1,1-tetrakis(4-n-butylbenzoylperoxycarbonyloxymethyl)methane, 1,2,3,4-tetrakis(4-n-butylbenzoylperoxycarbonyloxymethyl)butane, 1,2,3,4,5-penta (4-n-butylbenzoylperoxycarbonyloxymethyl)pentane, 1,2,3,4,5,6-hexa(4-n-butylbenzoylperoxycarbonyloxymethyl)hexane,
4-tert-butylbenzoylperoxy methyl monocarbonate, 4-tert-butylbenzoylperoxy ethyl monocarbonate, 4-tert-butylbenzoylperoxy n-propyl monocarbonate, 4-tert-butylbenzoylperoxy isopropyl monocarbonate, 4-tert-butylbenzoylperoxy n-butyl monocarbonate, 4-tert-butylbenzoylperoxy sec-butyl monocarbonate, 4-tert-butylbenzoylperoxy isobutyl monocarbonate, 4-tert-butylbenzoylperoxy n-pentyl monocarbonate, 4-tert-butylbenzoylperoxy neopentyl monocarbonate, 4-tert-butylbenzoylperoxy hexyl monocarbonate, 4-tert-butylbenzoylperoxy heptyl monocarbonate, 4-tert-butylbenzoylperoxy octyl monocarbonate, 4-tert-butylbenzoylperoxy 1-methylheptyl monocarbonate, 4-tert-butylbenzoylperoxy 2-ethylhexyl monocarbonate, 4-tert-butylbenzoylperoxy nonyl monocarbonate, 4-tert-butylbenzoylperoxy decyl monocarbonate, 4-tert-butylbenzoylperoxy myristyl monocarbonate, 4-tert-butylbenzoylperoxy myristyl monocarbonate, 4-tert-butylbenzoylperoxy stearyl monocarbonate, di(4-tert-butylbenzoylperoxy) ethylene biscarbonate, di(4-tert-butylbenzoylperoxy) trimethylene biscarbonate, di(4-tert-butylbenzoylperoxy) 2-methyltrimethylene biscarbonate, di(4-tert-butylbenzoylperoxy) 2,2-dimethyltrimethylene biscarbonate, di(4-tert-butylbenzoylperoxy) 2,2-diethyltrimethylene biscarbonate, di(4-tert-butylbenzoylperoxy) tetramethylene biscarbonate, di(4-tert-butylbenzoylperoxy) pentamethylene biscarbonate, di(4-tert-butylbenzoylperoxy) 3-methylpentamethylene biscarbonate, di(4-tert-butylbenzoylperoxy) hexamethylene biscarbonate, di(4-tert-butylbenzoylperoxy) heptamethylene biscarbonate, di(4-tert-butylbenzoylperoxy) octamethylene biscarbonate, di(4-tert-butylbenzoylperoxy) nonamethylene biscarbonate, di(4-tert-butylbenzoylperoxy) decamethylene biscarbonate, di(4-tert-butylbenzoylperoxy) dodecamethylene biscarbonate, di(4-tert-butylbenzoylperoxy) hexadecamethylene biscarbonate, 1,1,1-tris(4-tert-butylbenzoylperoxycarbonyloxymethyl)ethane, 1,1,1-tris(4-tert-butylbenzoylperoxycarbonyloxymethyl)propane, 1,2,3-tris(4-tert-butylbenzoylperoxycarbonyloxymethyl)propane, 1,1,1-tris(4-tert-butylbenzoylperoxycarbonyloxymethyl)butane, 1,1,1,1-tetrakis(4-tert-butylbenzoylperoxycarbonyloxymethyl)methane, 1,2,3,4-tetrakis(4-tert-butylbenzoylperoxycarbonyloxyethyl)butane, 1,2,3,4,5-penta(4-tert-butylbenzoylperoxycarbonyloxymethyl)pentane, 1,2,3,4,5,6-hexa(4-tert-butylbenzoylperoxycarbonyloxymethyl)hexane,
4-n-pentylbenzoylperoxy methyl monocarbonate, 4-n-pentylbenzoylperoxy ethyl monocarbonate, 4-n-pentylbenzoylperoxy n-propyl monocarbonate, 4-n-pentylbenzoylperoxy isopropyl monocarbonate, 4-n-pentylbenzoylperoxy n-butyl monocarbonate, 4-n-pentylbenzoylperoxy sec-butyl monocarbonate, 4-n-pentylbenzoylperoxy isobutyl monocarbonate, 4-n-pentylbenzoylperoxy n-pentyl monocarbonate, 4-n-pentylbenzoylperoxy neopentyl monocarbonate, 4-n-pentylbenzoylperoxy hexyl monocarbonate, 4-n-pentylbenzoylperoxy heptyl monocarbonate, 4-n-pentylbenzoylperoxy octyl monocarbonate, 4-n-pentylbenzoylperoxy 1-methylheptyl monocarbonate, 4-n-pentylbenzoylperoxy 2-ethylhexyl monocarbonate, 4-n-pentylbenzoylperoxy nonyl monocarbonate, 4-n-pentylbenzoylperoxy decyl monocarbonate, 4-n-pentylbenzoylperoxy myristyl monocarbonate, 4-n-pentylbenzoylperoxy myristyl monocarbonate, 4-n-pentylbenzoylperoxy stearyl monocarbonate, di(4-n-pentylbenzoylperoxy) ethylene biscarbonate, di(4-n-pentylbenzoylperoxy) trimethylene biscarbonate, di(4-n-pentylbenzoylperoxy) 2-methyltrimethylene biscarbonate, di(4-n-pentylbenzoylperoxy) 2,2-dimethyltrimethylene biscarbonate, di(4-n-pentylbenzoylperoxy) 2,2-diethyltrimethylene biscarbonate, di(4-n-pentylbenzoylperoxy) tetramethylene biscarbonate, di(4-n-pentylbenzoylperoxy) pentamethylene biscarbonate, di(4-n-pentylbenzoylperoxy) 3-methylpentamethylene biscarbonate, di(4-n-pentylbenzoylperoxy) hexamethylene biscarbonate, di(4-n-pentylbenzoylperoxy) heptamethylene biscarbonate, di(4-n-pentylbenzoylperoxy) octamethylene biscarbonate, di(4-n-pentylbenzoylperoxy) nonamethylene biscarbonate, di(4-n-pentylbenzoylperoxy) decamethylene biscarbonate, di(4-n-pentylbenzoylperoxy) dodecamethylene biscarbonate, di(4-n-pentylbenzoylperoxy) hexadecamethylene biscarbonate, 1,1,1-tris (4-n-pentylbenzoylperoxycarbonyloxymethyl)ethane, 1,1,1-tris(4-n-pentylbenzoylperoxycarbonyloxymethyl)propane, 1,2,3-tris(4-n-pentylbenzoylperoxycarbonyloxy methyl)propane, 1,1,1-tris(4-n-pentylbenzoylperoxycarbonyloxymethyl)butane, 1,1,1,1-tetrakis(4-n-pentylbenzoylperoxycarbonyloxymethyl)methane, 1,2,3,4-tetrakis(4-n-pentylbenzoylperoxycarbonyloxymethyl)butane, 1,2,3,4,5-penta(4-n-pentylbenzoylperoxycarbonyloxymethyl)pentane, 1,2,3,4,5,6-hexa(4-n-pentylbenzoylperoxycarbonyloxymethyl)hexane,
2,3,4,5,6-pentamethylbenzoylperoxy methyl monocarbonate, 2,3,4,5,6-pentamethylbenzoylperoxy ethyl monocarbonate, 2,3,4,5,6-pentamethylbenzoylperoxy n-propyl monocarbonate, 2,3,4,5,6-pentamethylbenzoylperoxy isopropyl monocarbonate, 2,3,4,5,6-pentamethylbenzoylperoxy n-butyl monocarbonate, 2,3,4,5,6-pentamethylbenzoylperoxy sec-butyl monocarbonate, 2,3,4,5,6-pentamethylbenzoylperoxy isobutyl monocarbonate, 2,3,4,5,6-pentamethylbenzoylperoxy n-pentyl monocarbonate, 2,3,4,5,6-pentamethylbenzoylperoxy neopentyl monocarbonate, 2,3,4,5,6-pentamethylbenzoylperoxy 1-methylheptyl monocarbonate, 2,3,4,5,6-pentamethylbenzoylperoxy 2-ethylhexyl monocarbonate, 2,3,4,5,6-pentamethylbenzoylperoxy decyl monocarbonate, 2,3,4,5,6-pentamethylbenzoylperoxy myristyl monocarbonate, 2,3,4,5,6-pentamethylbenzoylperoxy myristyl monocarbonate, 2,3,4,5,6-pentamethylbenzoylperoxy stearyl monocarbonate, di(2,3,4,5,6-pentamethylbenzoylperoxy) ethylene biscarbonate, di(2,3,4,5,6-pentamethylbenzoylperoxy) trimethylene biscarbonate, di(2,3,4,5,6-pentamethylbenzoylperoxy) tetramethylene biscarbonate, di(2,3,4,5,6-pentamethylbenzoylperoxy) pentamethylene biscarbonate, di(2,3,4,5,6-pentamethylbenzoylperoxy) hexamethylene biscarbonate, di(2,3,4,5,6-pentamethylbenzoylperoxy) decamethylene biscarbonate, di(2,3,4,5,6-pentamethylbenzoylperoxy) dodecamethylene biscarbonate, 1,1,1-tris(2,3,4,5,6-pentamethylbenzoylperoxycarbonyloxymethyl)ethane, 1,1,1-tris(2,3,4,5,6-pentamethylbenzoylperoxycarbonyloxymethyl)propane, 1,2,3-tris(2,3,4,5,6-pentamethylbenzoylperoxycarbonyloxymethyl)propane, 1,1,1-tris(2,3,4,5,6-pentamethylbenzoylperoxycarbonyloxymethyl)butane, 1,1,1,1-tetrakis(2,3,4,5,6-pentamethylbenzoylperoxycarbonyloxymethyl)meth ane, 1,2,3,4-tetrakis(2,3,4,5,6-pentamethylbenzoylperoxycarbonyloxymethyl)buta ne, 1,2,3,4,5-penta(2,3,4,5,6-pentamethylbenzoylperoxycarbonyloxymethyl)penta ne, 1,2,3,4,5,6-hexa(2,3,4,5,6-pentamethylbenzoylperoxycarbonyloxymethyl)hexa ne,
4-n-hexylbenzoylperoxy methyl monocarbonate, 4-n-hexylbenzoylperoxy ethyl monocarbonate, 4-n-hexylbenzoylperoxy n-propyl monocarbonate, 4-n-hexylbenzoylperoxy isopropyl monocarbonate, 4-n-hexylbenzoylperoxy n-butyl monocarbonate, 4-n-hexylbenzoylperoxy sec-butyl monocarbonate, 4-n-hexylbenzoylperoxy isobutyl monocarbonate, 4-n-hexylbenzoylperoxy n-pentyl monocarbonate, 4-n-hexylbenzoylperoxy neopentyl monocarbonate, 4-n-hexylbenzoylperoxy hexyl monocarbonate, 4-n-hexylbenzoylperoxy heptyl monocarbonate, 4-n-hexylbenzoylperoxy octyl monocarbonate, 4-n-hexylbenzoylperoxy 1-methylheptyl monocarbonate, 4-n-hexylbenzoylperoxy 2-ethylhexyl monocarbonate, 4-n-hexylbenzoylperoxy nonyl monocarbonate, 4-n-hexylbenzoylperoxy decyl monocarbonate, 4-n-hexylbenzoylperoxy myristyl monocarbonate, 4-n-hexylbenzoylperoxy myristyl monocarbonate, 4-n-hexylbenzoylperoxy stearyl monocarbonate, di(4-n- hexylbenzoylperoxy) ethylene biscarbonate, di(4-n-hexylbenzoylperoxy) trimethylene biscarbonate, di(4-n-hexylbenzoylperoxy) 2-methyltrimethylene biscarbonate, di(4-n-hexylbenzoylperoxy) 2,2-dimethyltrimethylene biscarbonate, di(4-n-hexylbenzoylperoxy) 2,2-diethyltrimethylene biscarbonate, di(4-n-hexylbenzoylperoxy) tetramethylene biscarbonate, di(4-n-hexylbenzoylperoxy) pentamethylene biscarbonate, di(4-n-hexylbenzoylperoxy) 3-methylpentamethylene biscarbonate, di(4-n-hexylbenzoylperoxy) hexamethylene biscarbonate, di(4-n-hexylbenzoylperoxy) heptamethylene biscarbonate, di(4-n-hexylbenzoylperoxy) octamethylene biscarbonate, di(4-n-hexylbenzoylperoxy) nonamethylene biscarbonate, di(4-n-hexylbenzoylperoxy) decamethylene biscarbonate, di(4-n-hexylbenzoylperoxy) dodecamethylene biscarbonate, di(4-n-hexylbenzoylperoxy) hexadecamethylene biscarbonate, 1,1,1-tris(4-n-hexylbenzoylperoxycarbonyloxymethyl)ethane, 1,1/1-tris(4-n-hexylbenzoylperoxycarbonyloxymethyl)propane, 1,2,3-tris(4-n-hexylbenzoylperoxycarbonyloxymethyl)propane, 1,1,1-tris(4-n-hexylbenzoylperoxycarbonyloxymethyl)butane, 1,1,1,1-tetrakis(4-n-hexylbenzoylperoxycarbonyloxymethyl)methane, 1,2,3,4-tetrakis(4-n-hexylbenzoylperoxycarbonyloxymethyl)butane, 1,2,3,4,5-penta(4-n-hexylbenzoylperoxycarbonyloxymethyl)pentane, 1,2,3,4,5,6-hexa(4-n-hexylbenzoylperoxycarbonyloxymethyl)hexane,
2-methoxybenzoylperoxy methyl monocarbonate, 2-methoxybenzoylperoxy ethyl monocarbonate, 2-methoxybenzoylperoxy n-propyl monocarbonate, 2-methoxybenzoylperoxy isopropyl monocarbonate, 2-methoxybenzoylperoxy n-butyl monocarbonate, 2-methoxybenzoylperoxy sec-butyl monocarbonate, 2-methoxybenzoylperoxy isobutyl monocarbonate, 2-methoxybenzoylperoxy n-pentyl monocarbonate, 2-methoxybenzoylperoxy neopentyl monocarbonate, 2-methoxybenzoylperoxy hexyl monocarbonate, 2-methoxybenzoylperoxy heptyl monocarbonate, 2-methoxybenzoylperoxy octyl monocarbonate, 2-methoxybenzoylperoxy 1-methylheptyl monocarbonate, 2-methoxybenzoylperoxy 2-ethylhexyl monocarbonate, 2-methoxybenzoylperoxy nonyl monocarbonate, 2-methoxybenzoylperoxy decyl monocarbonate, 2-methoxybenzoylperoxy myristyl monocarbonate, 2-methoxybenzoylperoxy myristyl monocarbonate, 2-methoxybenzoylperoxy stearyl monocarbonate, di(2-methoxybenzoylperoxy) ethylene biscarbonate, di(2-methoxybenzoylperoxy) trimethylene biscarbonate, di(2-methoxybenzoylperoxy) 2-methyltrimethylene biscarbonate, di(2-methoxybenzoylperoxy) 2,2-dimethyltrimethylene biscarbonate, di(2-methoxybenzoylperoxy) 2,2-diethyltrimethylene biscarbonate, di(2-methoxybenzoylperoxy) tetramethylene biscarbonate, di(2-methoxybenzoylperoxy) pentamethylene biscarbonate, di(2-methoxybenzoylperoxy) 3-methylpentamethylene biscarbonate, di(2-methoxybenzoylperoxy) hexamethylene biscarbonate, di(2-methoxybenzoylperoxy) heptamethylene biscarbonate, di(2-methoxybenzoylperoxy) octamethylene biscarbonate, di (2-methoxybenzoylperoxy) nonamethylene biscarbonate, di (2-methoxybenzoylperoxy) decamethylene biscarbonate, di(2-methoxybenzoylperoxy) dodecamethylene biscarbonate, di(2-methoxybenzoylperoxy) hexadecamethylene biscarbonate, 1,1,1-tris(2-methoxybenzoylperoxycarbonyloxymethyl)ethane, 1,1,1-tris(2-methoxybenzoylperoxycarbonyloxymethyl)propane, 1,2,3-tris(2-methoxybenzoylperoxycarbonyloxymethyl)propane, 1,1,1-tris(2-methoxybenzoylperoxycarbonyloxymethyl)butane, 1,1,1,1-tetrakis(2-methoxybenzoylperoxycarbonyloxymethyl)methane, 1,2,3,4-tetrakis(2-methoxybenzoylperoxycarbonyloxymethyl)butane, 1,2,3,4,5-penta(2-methoxybenzoylperoxycarbonyloxymethyl)pentane, 1,2,3,4,5,6-hexa(2-methoxybenzoylperoxycarbonyloxymethyl) hexane,
3-methoxybenzoylperoxy methyl monocarbonate, 3-methoxybenzoylperoxy ethyl monocarbonate, 3-methoxybenzoylperoxy n-propyl monocarbonate, 3-methoxybenzoylperoxy isopropyl monocarbonate, 3-methoxybenzoylperoxy n-butyl monocarbonate, 3-methoxybenzoylperoxy sec-butyl monocarbonate, 3-methoxybenzoylperoxy isobutyl monocarbonate, 3-methoxybenzoylperoxy n-pentyl monocarbonate, 3-methoxybenzoylperoxy neopentyl monocarbonate, 3-methoxybenzoylperoxy 1-methylheptyl monocarbonate, 3-methoxybenzoylperoxy 2-ethylhexyl monocarbonate, 3-methoxybenzoylperoxy decyl monocarbonate, 3-methoxybenzoylperoxy myristyl monocarbonate, 3-methoxybenzoylperoxy myristyl monocarbonate, 3-methoxybenzoylperoxy stearyl monocarbonate, di (3-methoxybenzoylperoxy)ethylenebiscarbonate, di(3-methoxybenzoylperoxy) trimethylene biscarbonate, di(3-methoxybenzoylperoxy) tetramethylene biscarbonate, di(3-methoxybenzoylperoxy) pentamethylene biscarbonate, di(3-methoxybenzoylperoxy) hexamethylene biscarbonate, di(3-methoxybenzoylperoxy) decamethylene biscarbonate, di(3-methoxybenzoylperoxy) dodecamethylene biscarbonate, 1,1,1-tris(3-methoxybenzoylperoxycarbonyloxymethyl)ethane, 1,1,1-tris(3-methoxybenzoylperoxycarbonyloxymethyl)propane, 1,2,3-tris(3-methoxybenzoylperoxycarbonyloxymethyl)propane, 1,1,1-tris(3-methoxybenzoylperoxycarbonyloxymethyl)butane, 1,1,1,1-tetrakis(3-methoxybenzoylperoxycarbonyloxymethyl)methane, 1,2,3,4-tetrakis(3-methoxybenzoylperoxycarbonyloxymethyl)butane, 1,2,3,4,5-penta(3-methoxybenzoylperoxycarbonyloxymethyl)pentane, 1,2,3,4,5,6-hexa(3-methoxybenzoylperoxycarbonyloxymethyl)hexane,
4-methoxybenzoylperoxy methyl monocarbonate, 4-methoxybenzoylperoxy ethyl monocarbonate, 4-methoxybenzoylperoxy n-propyl monocarbonate, 4-methoxybenzoylperoxy isopropyl monocarbonate, 4-methoxybenzoylperoxy n-butyl monocarbonate, 4-methoxybenzoylperoxy sec-butyl monocarbonate, 4-methoxybenzoylperoxy isobutyl monocarbonate, 4-methoxybenzoylperoxy n-pentyl monocarbonate, 4-methoxybenzoylperoxy neopentyl monocarbonate, 4-methoxybenzoylperoxy hexyl monocarbonate, 4-methoxybenzoylperoxy heptyl monocarbonate, 4-methoxybenzoylperoxy octyl monocarbonate, 4-methoxybenzoylperoxy 1-methylheptyl monocarbonate, 4-methoxybenzoylperoxy 2-ethylhexyl monocarbonate, 4-methoxybenzoylperoxy nonyl monocarbonate, 4-methoxybenzoylperoxy decyl monocarbonate, 4-methoxybenzoylperoxy myristyl monocarbonate, 4-methoxybenzoylperoxy myristyl monocarbonate, 4-methoxybenzoylperoxy stearyl monocarbonate, di(4-methoxybenzoylperoxy) ethylene biscarbonate, di(4-methoxybenzoylperoxy) trimethylene biscarbonate, di(4-methoxybenzoylperoxy) 2-methyltrimethylene biscarbonate, di(4-methoxybenzoylperoxy) 2,2-dimethyltrimethylene biscarbonate, di(4-methoxybenzoylperoxy) 2,2-diethyltrimethylene biscarbonate, di(4-methoxybenzoylperoxy) tetramethylene biscarbonate, di(4-methoxybenzoylperoxy) pentamethylene biscarbonate, di(4-methoxybenzoylperoxy) 3-methylpentamethylene biscarbonate, di(4-methoxybenzoylperoxy) hexamethylene biscarbonate, di(4-methoxybenzoylperoxy) heptamethylene biscarbonate, di(4-methoxybenzoylperoxy) octamethylene biscarbonate, di(4-methoxybenzoylperoxy) nonamethylene biscarbonate, di(4-methoxybenzoylperoxy) decamethylene biscarbonate, di(4-methoxybenzoylperoxy) dodecamethylene biscarbonate, di(4-methoxybenzoylperoxy) hexadecamethylene biscarbonate, 1,1,1-tris(4-methoxybenzoylperoxycarbonyloxymethyl)ethane, 1,1,1-tris(4-methoxybenzoylperoxycarbonyloxymethyl)propane, 1,2,3-tris(4-methoxybenzoylperoxycarbonyloxymethyl)propane, 1,1,1-tris(4-methoxybenzoylperoxycarbonyloxymethyl)butane, 1,1,1,1-tetrakis(4-methoxybenzoylperoxycarbonyloxymethyl)methane, 1,2,3,4-tetrakis(4-methoxybenzoylperoxycarbonyloxymethyl)butane, 1,2,3,4,5-penta(4-methoxybenzoylperoxycarbonyloxymethyl)pentane, 1,2,3,4,5,6-hexa(4-methoxybenzoylperoxycarbonyloxymethyl)hexane,
2-ethoxybenzoylperoxy methyl monocarbonate, 2-ethoxybenzoylperoxy ethyl monocarbonate, 2-ethoxybenzoylperoxy n-propyl monocarbonate, 2-ethoxybenzoylperoxy isopropyl monocarbonate, 2-ethoxybenzoylperoxy n-butyl monocarbonate, 2-ethoxybenzoylperoxy sec-butyl monocarbonate, 2-ethoxybenzoylperoxy isobutyl monocarbonate, 2-ethoxybenzoylperoxy n-pentyl monocarbonate, 2-ethoxybenzoylperoxy neopentyl monocarbonate, 2-ethoxybenzoylperoxy 1-methylheptyl monocarbonate, 2-ethoxybenzoylperoxy 2-ethylhexyl monocarbonate, 2-ethoxybenzoylperoxy decyl monocarbonate, 2-ethoxybenzoylperoxy myristyl monocarbonate, 2-ethoxybenzoylperoxy myristyl monocarbonate, 2-ethoxybenzoylperoxy stearyl monocarbonate, di(2-ethoxybenzoylperoxy) ethylene biscarbonate, di(2-ethoxybenzoylperoxy) trimethylene biscarbonate, di(2-ethoxybenzoylperoxy) tetramethylene biscarbonate, di(2-ethoxybenzoylperoxy) pentamethylene biscarbonate, di (2-ethoxybenzoylperoxy) hexamethylene biscarbonate, di(2-ethoxybenzoylperoxy) decamethylene biscarbonate, di(2-ethoxybenzoylperoxy) dodecamethylene biscarbonate, 1,1,1-tris(2-ethoxybenzoylperoxycarbonyloxymethyl)ethane, 1,1,1-tris(2-ethoxybenzoylperoxycarbonyloxymethyl)propane, 1,2,3-tris(2-ethoxybenzoylperoxycarbonyloxymethyl)propane, 1,1,1-tris(2-ethoxybenzoylperoxycarbonyloxymethyl)butane, 1,1,1,1-tetrakis(2-ethoxybenzoylperoxycarbonyloxymethyl)methane, 1,2,3,4-tetrakis(2-ethoxybenzoylperoxycarbonyloxymethyl)butane, 1,2,3,4,5-penta(2-ethoxybenzoylperoxycarbonyloxymethyl)pentane, 1,2,3,4,5,6-hexa(2-ethoxybenzoylperoxycarbonyloxymethyl)hexane,
3-ethoxybenzoylperoxy methyl monocarbonate, 3-ethoxybenzoylperoxy ethyl monocarbonate, 3-ethoxybenzoylperoxy n-propyl monocarbonate, 3-ethoxybenzoylperoxy isopropyl monocarbonate, 3-ethoxybenzoylperoxy n-butyl monocarbonate, 3-ethoxybenzoylperoxy sec-butyl monocarbonate, 3-ethoxybenzoylperoxy isobutyl monocarbonate, 3-ethoxybenzoylperoxy n-pentyl monocarbonate, 3-ethoxybenzoylperoxy neopentyl monocarbonate, 3-ethoxybenzoylperoxy 1-methylheptyl monocarbonate, 3-ethoxybenzoylperoxy 2-ethylhexyl monocarbonate, 3-ethoxybenzoylperoxy decyl monocarbonate, 3-ethoxybenzoylperoxy myristyl monocarbonate, 3-ethoxybenzoylperoxy myristyl monocarbonate, 3-ethoxybenzoylperoxy stearyl monocarbonate, di(3-ethoxybenzoylperoxy) ethylene biscarbonate, di(3-ethoxybenzoylperoxy) trimethylene biscarbonate, di(3-ethoxybenzoylperoxy) tetramethylene biscarbonate, di(3-ethoxybenzoylperoxy) pentamethylene biscarbonate, di(3-ethoxybenzoylperoxy) hexamethylene biscarbonate, di(3-ethoxybenzoylperoxy) decamethylene biscarbonate, di(3-ethoxybenzoylperoxy) dodecamethylene biscarbonate, 1,1,1-tris(3-ethoxybenzoylperoxycarbonyloxymethyl)ethane, 1,1,1-tris(3-ethoxybenzoylperoxycarbonyloxymethyl)propane, 1,2,3-tris(3-ethoxybenzoylperoxycarbonyloxymethyl)propane, 1,1,1-tris(3-ethoxybenzoylperoxycarbonyloxymethyl)butane, 1,1,1,1-tetrakis(3-ethoxybenzoylperoxycarbonyloxymethyl)methane, 1,2,3,4-tetrakis(3-ethoxybenzoylperoxycarbonyloxymethyl)butane, 1,2,3,4,5-penta(3-ethoxybenzoylperoxycarbonyloxymethyl)pentane, 1,2,3,4,5,6-hexa(3-ethoxybenzoylperoxycarbonyloxymethyl)hexane,
4-ethoxybenzoylperoxy methyl monocarbonate, 4-ethoxybenzoylperoxy ethyl monocarbonate, 4-ethoxybenzoylperoxy n-propyl monocarbonate, 4-ethoxybenzoylperoxy isopropyl monocarbonate, 4-ethoxybenzoylperoxy n-butyl monocarbonate, 4-ethoxybenzoylperoxy sec-butyl monocarbonate, 4-ethoxybenzoylperoxy isobutyl monocarbonate, 4-ethoxybenzoylperoxy n-pentyl monocarbonate, 4-ethoxybenzoylperoxy neopentyl monocarbonate, 4-ethoxybenzoylperoxy 1-methylheptyl monocarbonate, 4-ethoxybenzoylperoxy 2-ethylhexyl monocarbonate, 4-ethoxybenzoylperoxy decyl monocarbonate, 4-ethoxybenzoylperoxy myristyl monocarbonate, 4-ethoxybenzoylperoxy myristyl monocarbonate, 4-ethoxybenzoylperoxy stearyl monocarbonate, di(4-ethoxybenzoylperoxy) ethylene biscarbonate, di(4-ethoxybenzoylperoxy) trimethylene biscarbonate, di(4-ethoxybenzoylperoxy) tetramethylene biscarbonate, di(4-ethoxybenzoylperoxy) pentamethylene biscarbonate, di(4-ethoxybenzoylperoxy) hexamethylene biscarbonate, di(4-ethoxybenzoylperoxy) decamethylene biscarbonate, di(4-ethoxybenzoylperoxy) dodecamethylene biscarbonate, 1,1,1-tris(4-ethoxybenzoylperoxycarbonyloxymethyl)ethane, 1,1,1-tris(4-ethoxybenzoylperoxycarbonyloxymethyl)propane, 1,2,3-tris(4-ethoxybenzoylperoxycarbonyloxymethyl)propane, 1,1,1-tris(4-ethoxybenzoylperoxycarbonyloxymethyl)butane, 1,2,3,4-tetrakis(4-ethoxybenzoylperoxycarbonyloxymethyl)butane, 1,2,3,4,5-penta(4-ethoxybenzoylperoxycarbonyloxymethyl)pentane, 1,2,3,4,5,6-hexa(4-ethoxybenzoylperoxycarbonyloxymethyl)hexane,
2,3-dimethoxybenzoylperoxy methyl monocarbonate, 2,3-dimethoxybenzoylperoxy ethyl monocarbonate, 2,3-dimethoxybenzoylperoxy n-propyl monocarbonate, 2,3-dimethoxybenzoylperoxy isopropyl monocarbonate, 2,3-dimethoxybenzoylperoxy n-butyl monocarbonate, 2,3-dimethoxybenzoylperoxy sec-butyl monocarbonate, 2,3-dimethoxybenzoylperoxy isobutyl monocarbonate, 2,3-dimethoxybenzoylperoxy n-pentyl monocarbonate, 2,3-dimethoxybenzoylperoxy neopentyl monocarbonate, 2,3-dimethoxybenzoylperoxy 1-methylheptyl monocarbonate, 2,3-dimethoxybenzoylperoxy 2-ethylhexyl monocarbonate, 2,3-dimethoxybenzoylperoxy decyl monocarbonate, 2,3-dimethoxybenzoylperoxy myristyl monocarbonate, 2,3-dimethoxybenzoylperoxy myristyl monocarbonate, 2,3-dimethoxybenzoylperoxy stearyl monocarbonate, di (2,3-dimethoxybenzoylperoxy) ethylene biscarbonate, di(2,3-dimethoxybenzoylperoxy) trimethylene biscarbonate, di(2,3-dimethoxybenzoylperoxy) tetramethylene biscarbonate, di (2,3-dimethoxybenzoylperoxy) pentamethylene biscarbonate, di(2,3-dimethoxybenzoylperoxy) hexamethylene biscarbonate, di(2,3-dimethoxybenzoylperoxy) decamethylene biscarbonate, di (2,3-dimethoxybenzoylperoxy) dodecamethylene biscarbonate, 1,1,1-tris(2,3-dimethoxybenzoylperoxycarbonyloxymethyl)ethane, 1,1,1-tris(2,3-dimethoxybenzoylperoxycarbonyloxymethyl)propane, 1,2,3-tris(2,3-dimethoxybenzoylperoxycarbonyloxymethyl)propane, 1,1,1-tris(2,3-dimethoxybenzoylperoxycarbonyloxymethyl)butane, 1,1,1,1-tetrakis(2,3-dimethoxybenzoylperoxycarbonyloxymethyl)methane, 1,2,3,4-tetrakis(2,3-dimethoxybenzoylperoxycarbonyloxymethyl)butane, 1,2,3,4,5-penta(2,3-dimethoxybenzoylperoxycarbonyloxymethyl)pentane, 1,2,3,4,5,6-hexa(2,3-dimethoxybenzoylperoxycarbonyloxymethyl)hexane,
2,4-dimethoxybenzoylperoxy methyl monocarbonate, 2,4-dimethoxybenzoylperoxy ethyl monocarbonate, 2,4-dimethoxybenzoylperoxy n-propyl monocarbonate, 2,4-dimethoxybenzoylperoxy isopropyl monocarbonate, 2,4-dimethoxybenzoylperoxy n-butyl monocarbonate, 2,4-dimethoxybenzoylperoxy sec-butyl monocarbonate, 2,4-dimethoxybenzoylperoxy isobutyl monocarbonate, 2,4-dimethoxybenzoylperoxy n-pentyl monocarbonate, 2,4-dimethoxybenzoylperoxy neopentyl monocarbonate, 2,4-dimethoxybenzoylperoxy 1-methylheptyl monocarbonate, 2,4-dimethoxybenzoylperoxy 2-ethylhexyl monocarbonate, 2,4-dimethoxybenzoylperoxy decyl monocarbonate, 2,4-dimethoxybenzoylperoxy myristyl monocarbonate, 2,4-dimethoxybenzoylperoxy myristyl monocarbonate, 2,4-dimethoxybenzoylperoxy stearyl monocarbonate, di(2,4-dimethoxybenzoylperoxy) ethylene biscarbonate, di(2,4-dimethoxybenzoylperoxy) trimethylene biscarbonate, di(2,4-dimethoxybenzoylperoxy) tetramethylene biscarbonate, di(2,4-dimethoxybenzoylperoxy) pentamethylene biscarbonate, di(2,4-dimethoxybenzoylperoxy) hexamethylene biscarbonate, di(2,4-di methoxybenzoylperoxy) decamethylene biscarbonate, di (2,4-dimethoxybenzoylperoxy) dodecamethylene biscarbonate, 1,1,1-tris(2,4-dimethoxybenzoylperoxycarbonyloxymethyl)ethane, 1,1,1-tris(2,4-dimethoxybenzoylperoxycarbonyloxymethyl)propane, 1,2,3-tris(2,4-dimethoxybenzoylperoxycarbonyloxymethyl)propane, 1,1,1-tris(2,4-dimethoxybenzoylperoxycarbonyloxymethyl)butane, 1,1,1,1-tetrakis(2,4-dimethoxybenzoylperoxycarbonyloxymethyl)methane, 1,2,3,4-tetrakis(2,4-dimethoxybenzoylperoxycarbonyloxymethyl)butane, 1,2,3,4,5-penta(2,4-dimethoxybenzoylperoxycarbonyloxymethyl)pentane, 1,2,3,4,5,6-hexa(2,4-dimethoxybenzoylperoxycarbonyloxmethyl)hexane,
2,5-dimethoxybenzoylperoxy methyl monocarbonate, 2,5-dimethoxybenzoylperoxy ethyl monocarbonate, 2,5-dimethoxybenzoylperoxy n-propyl monocarbonate, 2,5-dimethoxybenzoylperoxy isopropyl monocarbonate, 2,5-dimethoxybenzoylperoxy n-butyl monocarbonate, 2,5-dimethoxybenzoylperoxy sec-butyl monocarbonate, 2,5-dimethoxybenzoylperoxy isobutyl monocarbonate, 2,5-dimethoxybenzoylperoxy n-pentyl monocarbonate, 2,5-dimethoxybenzoylperoxy neopentyl monocarbonate, 2,5-dimethoxybenzoylperoxy 1-methylheptyl monocarbonate, 2,5-dimethoxybenzoylperoxy 2-ethylhexyl monocarbonate, 2,5-dimethoxybenzoylperoxy decyl monocarbonate, 2,5-dimethoxybenzoylperoxy myristyl monocarbonate, 2,5-dimethoxybenzoylperoxy myristyl monocarbonate, 2,5-dimethoxybenzoylperoxy stearyl monocarbonate, di(2,5-dimethoxybenzoylperoxy) ethylene biscarbonate, di(2,5-dimethoxybenzoylperoxy) trimethylene biscarbonate, di(2,5-dimethoxybenzoylperoxy) tetramethylene biscarbonate, di(2,5-dimethoxybenzoylperoxy) pentamethylene biscarbonate, di(2,5-dimethoxybenzoylperoxy) hexamethylene biscarbonate, di(2,5-dimethoxybenzoylperoxy) decamethylene biscarbonate, di(2,5-dimethoxybenzoylperoxy) dodecamethylene biscarbonate, 1,1,1-tris(2,5-dimethoxybenzoylperoxycarbonyloxymethyl)ethane, 1,1,1-tris(2,5-dimethoxybenzoylperoxycarbonyloxymethyl)propane, 1,2,3-tris(2,5-dimethoxybenzoylperoxycarbonyloxymethyl)propane, 1,1,1-tris(2,5-dimethoxybenzoylperoxycarbonyloxymethyl)butane, 1,1,1,1-tetrakis(2,5-dimethoxybenzoylperoxycarbonyloxymethyl)methane, 1,2,3,4-tetrakis(2,5-dimethoxybenzoylperoxycarbonyloxymethyl)butane, 1,2,3,4,5-penta(2,5-dimethoxybenzoylperoxycarbonyloxymethyl)pentane, 1,2,3,4,5,6-hexa(2,5-dimethoxybenzoylperoxycarbonyloxymethyl)hexane,
2,6-dimethoxybenzoylperoxy methyl monocarbonate, 2,6-dimethoxybenzoylperoxy ethyl monocarbonate, 2,6-dimethoxybenzoylperoxy n-propyl monocarbonate, 2,6-dimethoxybenzoylperoxy isopropyl monocarbonate, 2,6-dimethoxybenzoylperoxy n-butyl monocarbonate, 2,6-dimethoxybenzoylperoxy sec-butyl monocarbonate, 2,6-dimethoxybenzoylperoxy isobutyl monocarbonate, 2,6-dimethoxybenzoylperoxy n-pentyl monocarbonate, 2,6-dimethoxybenzoylperoxy neopentyl monocarbonate, 2,6-dimethoxybenzoylperoxy 1-methylheptyl monocarbonate, 2,6-dimethoxybenzoylperoxy 2-ethylhexyl monocarbonate, 2,6-dimethoxybenzoylperoxy decyl monocarbonate, 2,6-dimethoxybenzoylperoxy myristyl monocarbonate, 2,6-dimethoxybenzoylperoxy myristyl monocarbonate, 2,6-dimethoxybenzoylperoxy stearyl monocarbonate, di(2,6-dimethoxybenzoylperoxy) ethylene biscarbonate, di(2,6-dimethoxybenzoylperoxy) trimethylene biscarbonate, di (2,6-dimethoxybenzoylperoxy) tetramethylene biscarbonate, di(2,6-dimethoxybenzoylperoxy) pentamethylene biscarbonate, di(2,6-dimethoxybenzoylperoxy) hexamethylene biscarbonate, di(2,6-dimethoxybenzoylperoxy) decamethylene biscarbonate, di(2,6-dimethoxybenzoylperoxy) dodecamethylene biscarbonate, 1,1,1-tris(2,6-dimethoxybenzoylperoxycarbonyloxymethyl)ethane, 1,1,1-tris(2,6-dimethoxybenzoylperoxycarbonyloxymethyl)propane, 1,2,3-tris(2,6-dimethoxybenzoylperoxycarbonyloxymethyl)propane, 1,1,1-tris(2,6-dimethoxybenzoylperoxycarbonyloxymethyl)butane, 1,1,1,1-tetrakis(2,6-dimethoxybenzoylperoxycarbonyloxymethyl)methane, 1,2,3,4-tetrakis(2,6-dimethoxybenzoylperoxycarbonyloxymethyl)butane, 1,2,3,4,5-penta(2,6-dimethoxybenzoylperoxycarbonyloxymethyl)pentane, 1,2,3,4,5,6-hexa(2,6-dimethoxybenzoylperoxycarbonyloxymethyl)hexane,
3,4-dimethoxybenzoylperoxy methyl monocarbonate, 3,4-dimethoxybenzoylperoxy ethyl monocarbonate, 3,4-dimethoxybenzoylperoxy n-propyl monocarbonate, 3,4-dimethoxybenzoylperoxy isopropyl monocarbonate, 3,4-dimethoxybenzoylperoxy n-butylmonocarbonate, 3,4-dimethoxybenzoylperoxy sec-butyl monocarbonate, 3,4-dimethoxybenzoylperoxy isobutyl monocarbonate, 3,4-dimethoxybenzoylperoxy n-pentyl monocarbonate, 3,4-dimethoxybenzoylperoxy neopentyl monocarbonate, 3,4-dimethoxybenzoylperoxy 1-methylheptyl monocarbonate, 3,4-dimethoxybenzoylperoxy 2-ethylhexyl monocarbonate, 3,4-dimethoxybenzoylperoxy decyl monocarbonate, 3,4-dimethoxybenzoylperoxy myristyl monocarbonate, 3,4-dimethoxybenzoylperoxy myristyl monocarbonate, di (3,4-dimethoxybenzoylperoxy) trimethylene biscarbonate, di(3,4-dimethoxybenzoylperoxy) tetramethylene biscarbonate, di(3,4-dimethoxybenzoylperoxy) pentamethylene biscarbonate, di(3,4-dimethoxybenzoylperoxy) hexamethylene biscarbonate, di(3,4-dimethoxybenzoylperoxy) decamethylene biscarbonate, di (3,4-dimethoxybenzoylperoxy) dodecamethylene biscarbonate, 1,1,1-tris(3,4-dimethoxybenzoylperoxycarbonyloxymethyl)ethane, 1,1,1-tris(3,4-dimethoxybenzoylperoxycarbonyloxymethyl)propane, 1,2,3-tris(3,4-dimethoxybenzoylperoxycarbonyloxymethyl)propane, 1,1,1-tris(3,4-dimethoxybenzoylperoxycarbonyloxymethyl)butane, 1,1,1,1-tetrakis(3,4-dimethoxybenzoylperoxycarbonyloxymethyl)methane, 1,2,3,4-tetrakis(3,4-dimethoxybenzoylperoxycarbonyloxymethyl)butane, 1,2,3,4,5-penta(3,4-dimethoxybenzoylperoxycarbonyloxymethyl)pentane, 1,2,3,4,5,6-hexa(3,4-dimethoxybenzoylperoxycarbonyloxymethyl)hexane,
3,5-dimethoxybenzoylperoxy methyl monocarbonate, 3,5-dimethoxybenzoylperoxy ethyl monocarbonate, 3,5-dimethoxybenzoylperoxy n-propyl monocarbonate, 3,5-dimethoxybenzoylperoxy isopropyl monocarbonate, 3,5-dimethoxybenzoylperoxy n-butylmonocarbonate, 3,5-dimethoxybenzoylperoxy sec-butyl monocarbonate, 3,5-dimethoxybenzoylperoxy isobutyl monocarbonate, 3,5-dimethoxybenzoylperoxy n-pentyl monocarbonate, 3,5-dimethoxybenzoylperoxy neopentyl monocarbonate, 3,5-dimethoxybenzoylperoxy 1-methylheptyl monocarbonate, 3,5-dimethoxybenzoylperoxy 2-ethylhexyl monocarbonate, 3,5-dimethoxybenzoylperoxy decyl monocarbonate, 3,5-dimethoxybenzoylperoxy myristyl monocarbonate, 3,5-dimethoxybenzoylperoxy myristyl monocarbonate, 3,5-dimethoxybenzoylperoxy stearyl monocarbonate, di(3,5-dimethoxybenzoylperoxy) ethylene biscarbonate, di(3,5-dimethoxybenzoylperoxy) trimethylene biscarbonate, di(3,5-dimethoxybenzoylperoxy) tetramethylene biscarbonate, di(3,5-dimethoxybenzoylperoxy) pentamethylene biscarbonate, di(3,5-dimethoxybenzoylperoxy) hexamethylene biscarbonate, di(3,5-dimethoxybenzoylperoxy) decamethylene biscarbonate, di(3,5-dimethoxybenzoylperoxy) dodecamethylene biscarbonate, 1,1,1-tris(3,5-dimethoxybenzoylperoxycarbonyloxymethyl)ethane, 1,1,1-tris(3,5-dimethoxybenzoylperoxycarbonyloxymethyl)propane, 1,2,3-tris(3,5-dimethoxybenzoylperoxycarbonyloxymethyl)propane, 1,1,1-tris(3,5-dimethoxybenzoylperoxycarbonyloxymethyl)butane, 1,1,1,1-tetrakis(3,5-dimethoxybenzoylperoxycarbonyloxymethyl)methane, 1,2,3,4-tetrakis(3,5-dimethoxybenzoylperoxycarbonyloxymethyl)butane, 1,2,3,4,5-penta(3,5-dimethoxybenzoylperoxycarbonyloxymethyl)pentane, 1,2,3,4,5,6-hexa(3,5-dimethoxybenzoylperoxycarbonyloxymethyl)hexane,
4-n-propyloxybenzoylperoxy methyl monocarbonate, 4-n-propyloxybenzoylperoxy ethyl monocarbonate, 4-n-propyloxybenzoylperoxy n-propyl monocarbonate, 4-n-propyloxybenzoylperoxy isopropyl monocarbonate, 4-n-propyloxybenzoylperoxy n-butyl monocarbonate, 4-n-propyloxybenzoylperoxy sec-butyl monocarbonate, 4-n-propyloxybenzoylperoxy isobutyl monocarbonate, 4-n-propyloxybenzoylperoxy n-pentyl monocarbonate, 4-n-propyloxybenzoylperoxy neopentyl monocarbonate, 4-n-propyloxybenzoylperoxy 1-methylheptyl monocarbonate, 4-n-propyloxybenzoylperoxy 2-ethylhexyl monocarbonate, 4-n-propyloxybenzoylperoxy decyl monocarbonate, 4-n-propyloxybenzoylperoxy myristyl monocarbonate, 4-n-propyloxybenzoylperoxy myristyl monocarbonate, 4-n-propyloxybenzoylperoxy stearyl monocarbonate, di(4-n-propyloxybenzoylperoxy) ethylene biscarbonate, di(4-n-propyloxybenzoylperoxy) trimethylene biscarbonate, di(4-n-propyloxybenzoylperoxy) tetramethylene biscarbonate, di(4-n-propyloxybenzoylperoxy) pentamethylene biscarbonate, di(4-n-propyloxybenzoylperoxy) hexamethylene biscarbonate, di(4-n-propyloxybenzoylperoxy) decamethylene biscarbonate, di(4-n-propyloxybenzoylperoxy) dodecamethylene biscarbonate, 1,1,1-tris(4-n-propylbenzoylperoxycarbonyloxymethyl)ethane, 1,1,1-tris(4-n-propylbenzoylperoxycarbonyloxymethyl)propane, 1,2,3-tris(4-n-propylbenzoylperoxycarbonyloxymethyl)propane, 1,1,1-tris(4-n-propylbenzoylperoxycarbonyloxymethyl)butane, 1,1,1,1-tetrakis(4-n-propylbenzoylperoxycarbonyloxymethyl)methane, 1,2,3,4-tetrakis(4-n-propylbenzoylperoxycarbonyloxymethyl)butane, 1,2,3,4,5-penta(4-n-propylbenzoylperoxycarbonyloxymethyl)pentane, 1,2,3,4,5,6-hexa(4-n-propylbenzoylperoxycarbonyloxymethyl)hexane,
4-isopropyloxybenzoylperoxy methyl monocarbonate, 4-isopropyloxybenzoylperoxy ethyl monocarbonate, 4-isopropyloxybenzoylperoxy n-propyl monocarbonate, 4-isopropyloxybenzoylperoxy isopropyl monocarbonate, 4-isopropyloxybenzoylperoxy n-butyl monocarbonate, 4-isopropyloxybenzoylperoxy sec-butyl monocarbonate, 4-isopropyloxybenzoylperoxy isobutyl monocarbonate, 4-isopropyloxybenzoylperoxy n-pentyl monocarbonate, 4-isopropyloxybenzoylperoxy neopentyl monocarbonate, 4-isopropyloxybenzoylperoxy 1-methylheptyl monocarbonate, 4-isopropyloxybenzoylperoxy 2-ethylhexyl monocarbonate, 4-isopropyloxybenzoylperoxy decylmonocarbonate, 4-isopropyloxybenzoylperoxy myristyl monocarbonate, 4-isopropyloxybenzoylperoxy myristyl monocarbonate, 4-isopropyloxybenzoylperoxy stearyl monocarbonate, di(4-isopropyloxybenzoylperoxy) ethylene biscarbonate, di(4-isopropyloxybenzoylperoxy) trimethylene biscarbonate, di(4-isopropyloxybenzoylperoxy) tetramethylene biscarbonate, di(4-isopropyloxybenzoylperoxy) pentamethylene biscarbonate, di(4-isopropyloxybenzoylperoxy) hexamethylene biscarbonate, di(4-isopropyloxybenzoylperoxy) decamethylene biscarbonate, di(4-isopropyloxybenzoylperoxy) dodecamethylene biscarbonate, 1,1,1-tris(4-isopropyloxybenzoylperoxycarbonyloxymethyl)ethane, 1,1,1-tris(4-isopropyloxybenzoylperoxycarbonyloxymethyl)propane 1,2,3-tris(4-isopropyloxybenzoylperoxycarbonyloxymethyl)propane, 1,1,1-tris(4-isopropyloxybenzoylperoxycarbonyloxymethyl)butane, 1,1,1,1-tetrakis(4-isopropyloxybenzoylperoxycarbonyloxymethyl)methane, 1,2,3,4-tetrakis(4-isopropyloxybenzoylperoxycarbonyloxymethyl)butane, 1,2,3,4,5-penta(4-isopropyloxybenzoylperoxycarbonyloxymethyl)pentane, 1,2,3,4,5,6-hexa(4-isopropyloxybenzoylperoxycarbonyloxymethyl)hexane,
2,3,4-trimethoxybenzoylperoxy methyl monocarbonate, 2,3,4-trimethoxybenzoylperoxy ethyl monocarbonate, 2,3,4-trimethoxybenzoylperoxy n-propyl monocarbonate, 2,3,4-trimethoxybenzoylperoxy isopropyl monocarbonate, 2,3,4-trimethoxybenzoylperoxy n-butyl monocarbonate, 2,3,4-trimethoxybenzoylperoxy sec-butyl monocarbonate, 2,3,4-trimethoxybenzoylperoxy isobutyl monocarbonate, 2,3,4-trimethoxybenzoylperoxy n-pentyl monocarbonate, 2,3,4-trimethoxybenzoylperoxy neopentyl monocarbonate, 2,3,4-trimethoxybenzoylperoxy 1-methylheptyl monocarbonate, 2,3,4-trimethoxybenzoylperoxy 2-ethylhexyl monocarbonate, 2,3,4-trimethoxybenzoylperoxy decyl monocarbonate, 2,3,4-trimethoxybenzoylperoxy myristyl monocarbonate, 2,3,4-trimethoxybenzoylperoxy myristyl monocarbonate, 2,3,4-trimethoxybenzoylperoxy stearyl monocarbonate, di(2,3,4-trimethoxybenzoylperoxy) ethylene biscarbonate, di(2,3,4-trimethoxybenzoylperoxy) trimethylene biscarbonate, di(2,3,4-trimethoxybenzoylperoxy) tetramethylene biscarbonate, di(2,3,4-trimethoxybenzoylperoxy) pentamethylene biscarbonate, di(2,3,4-trimethoxybenzoylperoxy) hexamethylene biscarbonate, di(2,3,4-trimethoxybenzoylperoxy) decamethylene biscarbonate, di(2,3,4-trimethoxybenzoylperoxy) dodecamethylene biscarbonate, 1,1,1-tris(2,3,4-trimethoxybenzoylperoxycarbonyloxymethyl)ethane, 1,1,1-tris(2,3,4-trimethoxybenzoylperoxycarbonyloxymethyl)propane, 1,2, 3-tris (2,3,4-trimethoxybenzoylperoxycarbonyloxymethyl)propane, 1,1,1-tris(2,3,4-trimethoxybenzoylperoxycarbonyloxymethyl)butane, 1,1,1,1-tetrakis(2,3,4-trimethoxybenzoyperoxycarbonyloxymethyl)methane, 1,2,3,4-tetrakis(2,3,4-trimethoxybenzoylperoxycarbonyloxymethyl)butane, 1,2,3,4,5-penta(2,3,4-trimethoxybenzoylperoxycarbonyloxymethyl)pentane, 1,2,3,4,5,6-hexa(2,3,4-trimethoxybenzoylperoxycarbonyloxymethyl)hexane,
2,4,5-trimethoxybenzoylperoxy methyl monocarbonate, 2,4,5-trimethoxybenzoylperoxy ethyl monocarbonate, 2,4,5-trimethoxybenzoylperoxy n-propyl monocarbonate, 2,4,5-trimethoxybenzoylperoxy isopropyl monocarbonate, 2,4,5-trimethoxybenzoylperoxy n-butyl monocarbonate, 2,4,5-trimethoxybenzoylperoxy sec-butyl monocarbonate, 2,4,5-trimethoxybenzoylperoxy isobutyl monocarbonate, 2,4,5-trimethoxybenzoylperoxy n-pentyl monocarbonate, 2,4,5-trimethoxybenzoylperoxy neopentyl monocarbonate, 2,4,5-trimethoxybenzoylperoxy 1-methylheptyl monocarbonate, 2,4,5-trimethoxybenzoylperoxy 2-ethylhexyl monocarbonate, 2,4,5-trimethoxybenzoylperoxy decyl monocarbonate, 2,4,5-trimethoxybenzoylperoxy myristyl monocarbonate, 2,4,5-trimethoxybenzoylperoxy myristyl monocarbonate, 2,4,5-trimethoxybenzoylperoxy stearyl monocarbonate, di (2,4,5-trimethoxybenzoylperoxy) ethylene biscarbonate, di (2,4,5-trimethoxybenzoylperoxy) trimethylene biscarbonate, di (2,4,5-trimethoxybenzoylperoxy) tetramethylene biscarbonate, di(2,4,5-trimethoxybenzoylperoxy) pentamethylene biscarbonate, di(2,4,5-trimethoxybenzoylperoxy) hexamethylene biscarbonate, di (2,4,5-trimethoxybenzoylperoxy) decamethylene biscarbonate, di(2,4,5-trimethoxybenzoylperoxy) dodecamethylene biscarbonate, 1,1,1-tris(2,4,5-trimethoxybenzoylperoxycarbonyloxymethyl)ethane, 1,1,1-tris(2,4,5-trimethoxybenzoylperoxycarbonyloxymethyl)propane, 1,2,3-tris(2,4,5-trimethoxybenzoylperoxycarbonyloxymethyl)propane, 1,1,1-tris(2,4,5-trimethoxybenzoylperoxycarbonyloxymethyl)butane, 1,1,1,1-tetrakis(2,4,5-trimethoxybenzoylperoxycarbonyloxymethyl)methane, 1,2,3,4-tetrakis(2,4,5-trimethoxybenzoylperoxycarbonyloxymethyl)butane, 1,2,3,4,5-penta(2,4,5-trimethoxybenzoylperoxycarbonyloxymethyl)pentane, 1,2,3,4,5,6-hexa(2,4,5-trimethoxybenzoylperoxycarbonyloxymethyl)hexane,
3,4,5-trimethoxybenzoylperoxy methyl monocarbonate, 3,4,5-trimethoxybenzoylperoxy ethyl monocarbonate, 3,4,5-trimethoxybenzoylperoxy n-propyl monocarbonate, 3,4,5-trimethoxybenzoylperoxy isopropyl monocarbonate, 3,4,5-trimethoxybenzoylperoxy n-butyl monocarbonate, 3,4,5-trimethoxybenzoylperoxy sec-butyl monocarbonate, 3,4,5-trimethoxybenzoylperoxy isobutyl monocarbonate, 3,4,5-trimethoxybenzoylperoxy n-pentyl monocarbonate, 3,4,5-trimethoxybenzoylperoxy neopentyl monocarbonate, 3,4,5-trimethoxybenzoylperoxy 1-methylheptyl monocarbonate, 3,4,5-trimethoxybenzoylperoxy 2-ethylhexyl monocarbonate, 3,4,5-trimethoxybenzoylperoxy decyl monocarbonate, 3,4,5-trimethoxybenzoylperoxy myristyl monocarbonate, 3,4,5-trimethoxybenzoylperoxy myristyl monocarbonate, 3,4,5-trimethoxybenzoylperoxy stearyl monocarbonate, di(3,4,5-trimethoxybenzoylperoxy) ethylene biscarbonate, di(3,4,5-trimethoxybenzoylperoxy) trimethylene biscarbonate, di (3,4,5-trimethoxybenzoylperoxy) tetramethylene biscarbonate, di (3,4,5-trimethoxybenzoylperoxy) pentamethylene biscarbonate, di (3,4,5-trimethoxybenzoylperoxy) hexamethylene biscarbonate, di (3,4,5-trimethoxybenzoylperoxy) decamethylene biscarbonate, di(3,4,5-trimethoxybenzoylperoxy) dodecamethylene biscarbonate, 1,1,1-tris(3,4,5-trimethoxybenzoylperoxycarbonyloxymethyl)ethane, 1,1,1-tris(3,4,5-trimethoxybenzoylperoxycarbonyloxymethyl)propane, 1,2,3-tris(3,4,5-trimethoxybenzoylperoxycarbonyloxymethyl)propane, 1,1,1-tris(3,4,5-trimethoxybenzoylperoxycarbonyloxymethyl)butane, 1,1,1,1-tetrakis(3,4,5-trimethoxybenzoylperoxycarbonyloxymethyl)methane, 1,2,3,4-tetrakis(3,4,5-trimethoxybenzoylperoxycarbonyloxymethyl)butane, 1,2,3,4,5-penta(3,4,5-trimethoxybenzoylperoxycarbonyloxymethyl)pentane, 1,2,3,4,5,6-hexa(3,4,5-trimethoxybenzoylperoxycarbonyloxymethyl)hexane,
4-n-butoxybenzoylperoxy methyl monocarbonate, 4-n-butoxybenzoylperoxy ethyl monocarbonate, 4-n-butoxybenzoylperoxy n-propyl monocarbonate, 4-n-butoxybenzoylperoxy isopropyl monocarbonate, 4-n-butoxybenzoylperoxy n-butyl monocarbonate, 4-n-butoxybenzoylperoxy sec-butyl monocarbonate, 4-n-butoxybenzoylperoxy isobutyl monocarbonate, 4-n-butoxybenzoylperoxy n-pentyl monocarbonate, 4-n-butoxybenzoylperoxy neopentyl monocarbonate, 4-n-butoxybenzoylperoxy 1-methylheptyl monocarbonate, 4-n-butoxybenzoylperoxy 2-ethylhexyl monocarbonate, 4-n-butoxybenzoylperoxy decyl monocarbonate, 4-n-butoxybenzoylperoxy myristyl monocarbonate, 4-n-butoxybenzoylperoxy myristyl monocarbonate, 4-n-butoxybenzoylperoxy stearyl monocarbonate, di(4-n-butoxybenzoylperoxy) ethylene biscarbonate, di(4-n-butoxybenzoylperoxy) trimethylene biscarbonate, di(4-n-butoxybenzoylperoxy) tetramethylene biscarbonate, di(4-n-butoxybenzoylperoxy) pentamethylene biscarbonate, di(4-n-butoxybenzoylperoxy) hexamethylene biscarbonate, di(4-n-butoxybenzoylperoxy) decamethylene biscarbonate, di(4-n-butoxybenzoylperoxy) dodecamethylene biscarbonate, 1,1,1-tris(4-n-butoxybenzoylperoxycarbonyloxymethyl)ethane, 1,1,1-tris(4-n-butoxybenzoylperoxycarbonyloxymethyl)propane, 1,2,3-tris(4-n-butoxybenzoylperoxycarbonyloxymethyl)propane, 1,1,1-tris(4-n-butoxybenzoylperoxycarbonyloxymethyl)butane, 1,1,1,1-tetrakis(4-n-butoxybenzoylperoxycarbonyloxymethyl)methane, 1,2,3,4-tetrakis(4-n-butoxybenzoylperoxycarbonyloxymethyl)butane, 1,2,3,4,5-penta(4-n-butoxybenzoylperoxycarbonyloxymethyl)pentane, 1,2,3,4,5,6-hexa(4-n-butoxybenzoylperoxycarbonyloxymethyl)hexane,
4-n-hexyloxybenzoylperoxymethyl monocarbonate, 4-n-hexyloxybenzoylperoxy ethyl monocarbonate, 4-n-hexyloxybenzoylperoxy n-propyl monocarbonate, 4-n-hexyloxybenzoylperoxy isopropyl monocarbonate, 4-n-hexyloxybenzoylperoxy n-butyl monocarbonate, 4-n-hexyloxybenzoylperoxy sec-butyl monocarbonate, 4-n-hexyloxybenzoylperoxy isobutyl monocarbonate, 4-n-hexyloxybenzoylperoxy n-pentyl monocarbonate, 4-n-hexyloxybenzoylperoxy neopentyl monocarbonate, 4-n-hexyloxybenzoylperoxy 1-methylheptyl monocarbonate, 4-n-hexyloxybenzoylperoxy 2-ethylhexyl monocarbonate, 4-n-hexyloxybenzoylperoxy decyl monocarbonate, 4-n-hexyloxybenzoylperoxy myristyl monocarbonate, 4-n-hexyloxybenzoylperoxy myristyl monocarbonate, 4-n-hexyloxybenzoylperoxy stearyl monocarbonate, di (4-n-hexyloxybenzoylperoxy) ethylene biscarbonate, di(4-n-hexyloxybenzoylperoxy) trimethylene biscarbonate, di(4-n-hexyloxybenzoylperoxy) tetramethylene biscarbonate, di(4-n-hexyloxybenzoylperoxy) pentamethylene biscarbonate, di(4-n-hexyloxybenzoylperoxy) hexamethylene biscarbonate, di(4-n-hexyloxybenzoylperoxy) decamethylene biscarbonate, di(4-n-hexyloxybenzoylperoxy) dodecamethylene biscarbonate, 1,1,1-tris(4-n-hexyloxybenzoylperoxycarbonyloxymethyl)ethane, 1,1,1-tris(4-n-hexyloxybenzoylperoxycarbonyloxymethyl)propane, 1,2,3-tris(4-n-hexyloxybenzoylperoxycarbonyloxymethyl)propane, 1,1,1-tris(4-n-hexyloxybenzoylperoxycarbonyloxymethyl)butane, 1,1,1,1-tetrakis(4-n-hexyloxybenzoylperoxycarbonyloxymethyl)methane, 1,2,3,4-tetrakis(4-n-hexyloxybenzoylperoxycarbonyloxymethyl)butane, 1,2,3,4,5-penta(4-n-hexyloxybenzoylperoxycarbonyloxymethyl)pentane, 1,2,3,4,5,6-hexa(4-n-hexyloxybenzoylperoxycarbonyloxymethyl)hexane,
2-phenylbenzoylperoxy methyl monocarbonate, 2-phenylbenzoylperoxy ethyl monocarbonate, 2-phenylbenzoylperoxy n-propyl monocarbonate, 2-phenylbenzoylperoxy isopropyl monocarbonate, 2-phenylbenzoylperoxy n-butyl monocarbonate, 2-phenylbenzoylperoxy sec-butyl monocarbonate, 2-phenylbenzoylperoxy isobutyl monocarbonate, 2-phenylbenzoylperoxy n-pentyl monocarbonate, 2-phenylbenzoylperoxy neopentyl monocarbonate, 2-phenylbenzoylperoxy 1-methylheptyl monocarbonate, 2-phenylbenzoylperoxy 2-ethylhexyl monocarbonate, 2-phenylbenzoylperoxy decyl monocarbonate, 2-phenylbenzoylperoxy myristyl monocarbonate, 2-phenylbenzoylperoxy myristyl monocarbonate, 2-phenylbenzoylperoxy stearyl monocarbonate, di(2-phenylbenzoylperoxy) ethylene biscarbonate, di(2-phenylbenzoylperoxy) trimethylene biscarbonate, di(2-phenylbenzoylperoxy) tetramethylene biscarbonate, di(2-phenylbenzoylperoxy) pentamethylene biscarbonate, di(2-phenylbenzoylperoxy) hexamethylene biscarbonate, di(2-phenylbenzoylperoxy) decamethylene biscarbonate, di(2-phenylbenzoylperoxy) dodecamethylene biscarbonate, 1,1,1-tris(2-phenylbenzoylperoxycarbonyloxymethyl)ethane, 1,1,1-tris(2-phenylbenzoylperoxycarbonyloxymethyl)propane, 1,2,3-tris(2-phenylbenzoylperoxycarbonyloxymethyl)propane, 1,1,1-tris(2-phenylbenzoylperoxycarbonyloxymethyl)butane, 1,1,1,1-tetrakis(2-phenylbenzoylperoxycarbonyloxymethyl)methane, 1,2,3,4-tetrakis(2-phenylbenzoylperoxycarbonyloxymethyl)butane, 1,2,3,4,5-penta(2-phenylbenzoylperoxycarbonyloxymethyl)pentane, 1,2,3,4,5,6-hexa(2-phenylbenzoylperoxycarbonyloxymethyl)hexane,
3-phenylbenzoylperoxy methyl monocarbonate, 3-phenylbenzoylperoxy ethyl monocarbonate, 3-phenylbenzoylperoxy n-propyl monocarbonate, 3-phenylbenzoylperoxy isopropyl monocarbonate, 3-phenylbenzoylperoxy n-butyl monocarbonate, 3-phenylbenzoylperoxy sec-butyl monocarbonate, 3-phenylbenzoylperoxy isobutyl monocarbonate, 3-phenylbenzoylperoxy n-pentyl monocarbonate, 3-phenylbenzoylperoxy neopentyl monocarbonate, 3-phenylbenzoylperoxy 1-methylheptyl monocarbonate, 3-phenylbenzoylperoxy 2-ethylhexyl monocarbonate, 3-phenylbenzoylperoxy decyl monocarbonate, 3-phenylbenzoylperoxy myristyl monocarbonate, 3-phenylbenzoylperoxy myristyl monocarbonate, 3-phenylbenzoylperoxy stearyl monocarbonate, di(3-phenylbenzoylperoxy) ethylene biscarbonate, di(3-phenylbenzoylperoxy) trimethylene biscarbonate, di(3-phenylbenzoylperoxy) tetramethylene biscarbonate, di(3-phenylbenzoylperoxy) pentamethylene biscarbonate, di(3-phenylbenzoylperoxy) hexamethylene biscarbonate, di(3-phenylbenzoylperoxy) decamethylene biscarbonate, di(3-phenylbenzoylperoxy) dodecamethylene biscarbonate, 1,1,1-tris(3-phenylbenzoylperoxycarbonyloxymethyl)ethane, 1,1,1-tris(3-phenylbenzoylperoxycarbonyloxymethyl)propane, 1,2,3-tris(3-phenylbenzoylperoxycarbonyloxymethyl)propane, 1,1,1-tris(3-phenylbenzoylperoxycarbonyloxymethyl)butane, 1,1,1,1-tetrakis(3-phenylbenzoylperoxycarbonyloxymethyl)methane, 1,2,3,4-tetrakis(3-phenylbenzoylperoxycarbonyloxymethyl)butane, 1,2,3,4,5-penta(3-phenylbenzoylperoxycarbonyloxymethyl)pentane, 1,2,3,4,5,6-hexa(3-phenylbenzoylperoxycarbonyloxymethyl)hexane,
4-phenylbenzoylperoxy methyl monocarbonate, 4-phenylbenzoylperoxy ethyl monocarbonate, 4-phenylbenzoylperoxy n-propyl monocarbonate, 4-phenylbenzoylperoxy isopropyl monocarbonate, 4-phenylbenzoylperoxy n-butyl monocarbonate, 4-phenylbenzoylperoxy sec-butyl monocarbonate, 4-phenylbenzoylperoxy isobutyl monocarbonate, 4-phenylbenzoylperoxy n-pentyl monocarbonate, 4-phenylbenzoylperoxy neopentyl monocarbonate, 4-phenylbenzoylperoxy hexyl monocarbonate, 4-phenylbenzoylperoxy heptyl monocarbonate, 4-phenylbenzoylperoxy octyl monocarbonate, 4-phenylbenzoylperoxy 1-methylheptyl monocarbonate, 4-phenylbenzoylperoxy 2-ethylhexyl monocarbonate, 4-phenylbenzoylperoxy nonyl monocarbonate, benzoylperoxy decyl monocarbonate, 4-phenylbenzoylperoxy myristyl monocarbonate, 4-phenylbenzoylperoxy myristyl monocarbonate, 4-phenylbenzoylperoxy stearyl monocarbonate, di(4-phenylbenzoylperoxy) ethylene biscarbonate, di(4-phenylbenzoylperoxy) trimethylene biscarbonate, di (4-phenylbenzoylperoxy) 2-methyltrimethylene biscarbonate, di(4-phenylbenzoylperoxy) 2,2-dimethyltrimethylene biscarbonate, di(4-phenylbenzoylperoxy) 2,2-diethyltrimethylene biscarbonate, di(4-phenylbenzoylperoxy) tetramethylene biscarbonate, di(4-phenylbenzoylperoxy) pentamethylene biscarbonate, di(4-phenylbenzoylperoxy) 3-methylpentamethylene biscarbonate, di(4-phenylbenzoylperoxy) hexamethylene biscarbonate, di(4-phenylbenzoylperoxy) heptamethylene biscarbonate, di(4-phenylbenzoylperoxy) octamethylene biscarbonate, di(4-phenylbenzoylperoxy) nonamethylene biscarbonate, di(4-phenylbenzoylperoxy) decamethylene biscarbonate, di(4-phenylbenzoylperoxy) dodecamethylene biscarbonate, di(4-phenylbenzoylperoxy) hexadecamethylene biscarbonate, 1,1,1-tris(4-phenylbenzoylperoxycarbonyloxymethyl)ethane, 1,1,1-tris(4-phenylbenzoylperoxycarbonyloxymethyl)propane, 1,2,3-tris(4-phenylbenzoylperoxycarbonyloxymethyl)propane, 1,1,1-tris(4-phenylbenzoylperoxycarbonyloxymethyl)butane, 1,1,1,1-tetrakis(4-phenylbenzoylperoxycarbonyloxymethyl)methane, 1,2,3,4-tetrakis(4-phenylbenzoylperoxycarbonyloxymethyl)butane, 1,2,3,4,5-penta(4-phenylbenzoylperoxycarbonyloxymethyl)pentane, 1,2,3,4,5,6-hexa(4-phenylbenzoylperoxycarbonyloxymethyl)hexane.

In the cases of (a) and (C) including but not limited to the following,
4-trimethylsilylbenzoylperoxy methyl monocarbonate, 4-trimethylsilylbenzoylperoxy ethyl monocarbonate, 4-trimethylsilylbenzoylperoxy n-propyl monocarbonate, 4-trimethylsilylbenzoylperoxy isopropyl monocarbonate, 4-trimethylsilylbenzoylperoxy n-butyl monocarbonate, 4-trimethylsilylbenzoylperoxy sec-butyl monocarbonate, 4-trimethylsilylbenzoylperoxy isobutyl monocarbonate, 4-trimethylsilylbenzoylperoxy n-pentyl monocarbonate, 4-trimethylsilylbenzoylperoxy neopentyl monocarbonate, 4-trimethylsilylbenzoylperoxy hexyl monocarbonate, 4-trimethylsilylbenzoylperoxy heptyl monocarbonate, 4-trimethylsilylbenzoylperoxy octyl monocarbonate, 4-trimethylsilylbenzoylperoxy 1-methylheptyl monocarbonate, 4-trimethylsilylbenzoylperoxy 2-ethylhexyl monocarbonate, 4-trimethylsilylbenzoylperoxy nonyl monocarbonate, 4-trimethylsilylbenzoylperoxy decyl monocarbonate, 4-trimethylsilylbenzoylperoxy myristyl monocarbonate, 4-trimethylsilylbenzoylperoxy myristyl monocarbonate, 4-trimethylsilylbenzoylperoxy stearyl monocarbonate, di(4-trimethylsilylbenzoylperoxy) ethylene biscarbonate, di(4-trimethylsilylbenzoylperoxy) trimethylene biscarbonate, di(4-trimethylsilylbenzoylperoxy) 2-methyltrimethylene biscarbonate, di(4-trimethylsilylbenzoylperoxy) 2,2-dimethyltrimethylene biscarbonate, di(4-trimethylsilylbenzoylperoxy) 2,2-diethyltrimethylene biscarbonate, di(4-trimethylsilylbenzoylperoxy) tetramethylene biscarbonate, di(4-trimethylsilylbenzoylperoxy) pentamethylene biscarbonate, di(4-trimethylsilylbenzoylperoxy) 3-methylpentamethylene biscarbonate, di(4-trimethylsilylbenzoylperoxy) hexamethylene biscarbonate, di(4-trimethylsilylbenzoylperoxy) heptamethylene biscarbonate, di(4-trimethylsilylbenzoylperoxy) octamethylene biscarbonate, di(4-trimethylsilylbenzoylperoxy) nonamethylene biscarbonate, di(4-trimethylsilylbenzoylperoxy) decamethylene biscarbonate, di(4-trimethylsilylbenzoylperoxy) dodecamethylene biscarbonate, di(4-trimethylsilylbenzoylperoxy) hexadecamethylene biscarbonate, 1,1,1-tris(4-trimethylsilylbenzoylperoxycarbonyloxymethyl)ethane, 1,1,1-tris(4-trimethylsilylbenzoylperoxycarbonyloxymethyl)propane, 1,2,3-tris(4-trimethylsilylbenzoylperoxycarbonyloxymethyl)propane, 1,1,1-tris(4-trimethylsilylbenzoylperoxycarbonyloxymethyl)butane, 1,1,1,1-tetrakis(4-trimethylsilylbenzoylperoxycarbonyloxymethyl)methane, 1,2,3,4-tetrakis(4-trimethylsilylbenzoylperoxycarbonyloxymethyl)butane, 1,2,3,4,5-penta(4-trimethylsilylbenzoylperoxycarbonyloxymethyl)pentane, 1,2,3,4,5,6-hexa(4-trimethylsilylbenzoylperoxycarbonyloxymethyl)hexane,
4-dimethylvinylsilylbenzoylperoxy methyl monocarbonate, 4-dimethylvinylsilylbenzoylperoxy ethyl monocarbonate, 4-dimethylvinylsilylbenzoylperoxy n-propyl monocarbonate, 4-dimethylvinylsilylbenzoylperoxy isopropyl monocarbonate, 4-dimethylvinylsilylbenzoylperoxy n-butyl monocarbonate, 4-dimethylvinylsilylbenzoylperoxy sec-butyl monocarbonate, 4-dimethylvinylsilylbenzoylperoxy isobutyl monocarbonate, 4-dimethylvinylsilylbenzoylperoxy n-pentyl monocarbonate, 4-dimethylvinylsilylbenzoylperoxy neopentyl monocarbonate, 4-dimethylvinylsilylbenzoylperoxy hexyl monocarbonate, 4-dimethylvinylsilylbenzoylperoxy heptyl monocarbonate, 4-dimethylvinylsilylbenzoylperoxy octyl monocarbonate, 4-dimethylvinylsilylbenzoylperoxy 1-methylheptyl monocarbonate, 4-dimethylvinylsilylbenzoylperoxy 2-ethylhexyl monocarbonate, 4-dimethylvinylsilylbenzoylperoxy nonyl monocarbonate, 4-dimethylvinylsilylbenzoylperoxy decyl monocarbonate, 4-dimethylvinylsilylbenzoylperoxy myristyl monocarbonate, 4-dimethylvinylsilylbenzoylperoxy myristyl monocarbonate, 4-dimethylvinylsilylbenzoylperoxy stearyl monocarbonate, di(4-dimethylvinylsilylbenzoylperoxy) ethylene biscarbonate, di(4-dimethylvinylsilylbenzoylperoxy) trimethylene biscarbonate, di(4-dimethylvinylsilylbenzoylperoxy) 2-methyltrimethylene biscarbonate, di(4-dimethylvinylsilylbenzoylperoxy) 2,2-dimethyltrimethylene biscarbonate, di(4-dimethylvinylsilylbenzoylperoxy) 2,2-diethyltrimethylene biscarbonate, di(4-dimethylvinylsilylbenzoylperoxy) tetramethylene biscarbonate, di(4-dimethylvinylsilylbenzoylperoxy) pentamethylene biscarbonate, di(4-dimethylvinylsilylbenzoylperoxy) 3-methylpentamethylene biscarbonate, di(4-dimethylvinylsilylbenzoylperoxy) hexamethylene biscarbonate, di(4-dimethylvinylsilylbenzoylperoxy) heptamethylene biscarbonate, di(4-dimethylvinylsilylbenzoylperoxy) octamethylene biscarbonate, di(4-dimethylvinylsilylbenzoylperoxy) nonamethylene biscarbonate, di(4-dimethylvinylsilylbenzoylperoxy) decamethylene biscarbonate, di(4-dimethylvinylsilylbenzoylperoxy) dodecamethylene biscarbonate, di(4-dimethylvinylsilylbenzoylperoxy) hexadecamethylene biscarbonate, 1,1,1-tris(4-dimethylvinylsilylbenzoylperoxycarbonyloxymethyl)ethane, 1,1,1-tris(4-dimethylvinylsilylbenzoylperoxycarbonyloxymethyl)propane, 1,2,3-tris(4-dimethylvinylsilylbenzoylperoxycarbonyloxymethyl)propane, 1,1,1-tris(4-dimethylvinylsilylbenzoylperoxycarbonyloxymethyl)butane, 1,1,1,1-tetrakis(4-dimethylvinylsilylbenzoylperoxycarbonyloxymethyl)methane, 1,2,3,4-tetrakis(4-dimethylvinylsilylbenzoylperoxycarbonyloxymethyl)butane, 1,2,3,4,5-penta(4-dimethylvinylsilylbenzoylperoxycarbonyloxymethyl)pentane, 1,2,3,4,5,6-hexa(4-dimethylvinylsilylbenzoylperoxycarbonyloxymethyl)hexane,
4-diethylvinylsilylbenzoylperoxy methyl monocarbonate, 4-diethylvinylsilylbenzoylperoxy ethyl monocarbonate, 4-diethylvinylsilylbenzoylperoxy n-propyl monocarbonate, 4-diethylvinylsilylbenzoylperoxy isopropyl monocarbonate, 4-diethylvinylsilylbenzoylperoxy n-butyl monocarbonate, 4-diethylvinylsilylbenzoylperoxy sec-butyl monocarbonate, 4-diethylvinylsilylbenzoylperoxy isobutyl monocarbonate, 4-diethylvinylsilylbenzoylperoxy n-pentyl monocarbonate, 4-diethylvinylsilylbenzoylperoxy neopentyl monocarbonate, 4-diethylvinylsilylbenzoylperoxy hexyl monocarbonate, 4-diethylvinylsilylbenzoylperoxy heptyl monocarbonate, 4-diethylvinylsilylbenzoylperoxy octyl monocarbonate, 4-diethylvinylsilylbenzoylperoxy 1-methylheptyl monocarbonate, 4-diethylvinylsilylbenzoylperoxy 2-ethylhexyl monocarbonate, 4-diethylvinylsilylbenzoylperoxy nonyl monocarbonate, 4-diethylvinylsilylbenzoylperoxy decyl monocarbonate, 4-diethylvinylsilylbenzoylperoxy myristyl monocarbonate, 4-diethylvinylsilylbenzoylperoxy myristyl monocarbonate, 4-diethylvinylsilylbenzoylperoxy stearyl monocarbonate, di(4-diethylvinylsilylbenzoylperoxy) ethylene biscarbonate, di(4-diethylvinylsilylbenzoylperoxy) trimethylene biscarbonate, di(4-diethylvinylsilylbenzoylperoxy) 2-methyltrimethylene biscarbonate, di (4-diethylvinylsilylbenzoylperoxy) 2,2-dimethyltrimethylene biscarbonate, di(4-diethylvinylsilylbenzoylperoxy) 2,2-diethyltrimethylene biscarbonate, di(4-diethylvinylsilylbenzoylperoxy) tetramethylenebiscarbonate, di(4-diethylvinylsilylbenzoylperoxy) pentamethylene biscarbonate, di(4-diethylvinylsilylbenzoylperoxy) 3-methylpentamethylenebiscarbonate, di(4-diethylvinylsilylbenzoylperoxy) hexamethylene biscarbonate, di(4-diethylvinylsilylbenzoylperoxy) heptamethylene biscarbonate, di (4-diethylvinylsilylbenzoylperoxy) octamethylene biscarbonate, di(4-diethylvinylsilylbenzoylperoxy) nonamethylene biscarbonate, di(4-diethylvinylsilylbenzoylperoxy) decamethylene biscarbonate, di(4-diethylvinylsilylbenzoylperoxy) dodecamethylene biscarbonate, di(4-diethylvinylsilylbenzoylperoxy) hexadecamethylene biscarbonate, 1,1,1-tris(4-diethylvinylsilylbenzoylperoxycarbonyloxymethyl)ethane, 1,1,1-tris(4-diethylvinylsilylbenzoylperoxycarbonyloxymethyl)propane, 1,2,3-tris(4-diethylvinylsilylbenzoylperoxycarbonyloxymethyl)propane, 1,1,1-tris(4-diethylvinylsilylbenzoylperoxycarbonyloxymethyl)butane, 1,1,1,1-tetrakis(4-diethylvinylsilylbenzoylperoxycarbonyloxymethyl)methane, 1,2,3,4-tetrakis(4-diethylvinylsilylbenzoylperoxycarbonyloxymethyl)butane, 1,2,3,4,5-penta(4-diethylvinylsilylbenzoylperoxycarbonyloxymethyl)pentane, 1,2,3,4,5,6-hexa(4-diethylvinylsilylbenzoylperoxycarbonyloxymethyl)hexane
4-methyldivinylsilylbenzoylperoxy methyl monocarbonate, 4-methyldivinylsilylbenzoylperoxy ethyl monocarbonate, 4-methyldivinylsilylbenzoylperoxy n-propyl monocarbonate, 4-methyldivinylsilylbenzoylperoxy isopropyl monocarbonate, 4-methyldivinylsilylbenzoylperoxy n-butyl monocarbonate, 4-methyldivinylsilylbenzoylperoxy sec-butyl monocarbonate, 4-methyldivinylsilylbenzoylperoxy isobutyl monocarbonate, 4-methyldivinylsilylbenzoylperoxy n-pentyl monocarbonate, 4-methyldivinylsilylbenzoylperoxy neopentyl monocarbonate, 4-methyldivinylsilylbenzoylperoxy hexyl monocarbonate, 4-methyldivinylsilylbenzoylperoxy heptyl monocarbonate, 4-methyldivinylsilylbenzoylperoxy octyl monocarbonate, 4-methyldivinylsilylbenzoylperoxy 1-methylheptyl monocarbonate, 4-methyldivinylsilylbenzoylperoxy 2-ethylhexyl monocarbonate, 4-methyldivinylsilylbenzoylperoxy nonyl monocarbonate, 4-methyldivinylsilylbenzoylperoxy decyl monocarbonate, 4-methyldivinylsilylbenzoylperoxy myristyl monocarbonate, 4-methyldivinylsilylbenzoylperoxy myristyl monocarbonate, 4-methyldivinylsilylbenzoylperoxy stearyl monocarbonate, di(4-methyldivinylsilylbenzoylperoxy) ethylene biscarbonate, di(4-methyldivinylsilylbenzoylperoxy) trimethylene biscarbonate, di (4-methyldivinylsilylbenzoylperoxy) 2-methyltrimethylene biscarbonate, di(4-methyldivinylsilylbenzoylperoxy) 2,2-dimethyltrimethylene biscarbonate, di(4-methyldivinylsilylbenzoylperoxy) 2,2-diethyltrimethylene biscarbonate, di(4-methyldivinylsilylbenzoylperoxy) tetramethylene biscarbonate, di(4-methyldivinylsilylbenzoylperoxy) pentamethylene biscarbonate, di(4-methyldivinylsilylbenzoylperoxy) 3-methylpentamethylene biscarbonate, di(4-methyldivinylsilylbenzoylperoxy) hexamethylene biscarbonate, di(4-methyldivinylsilylbenzoylperoxy) heptamethylene biscarbonate, di(4-methyldivinylsilylbenzoylperoxy) octamethylene biscarbonate, di(4-methyldivinylsilylbenzoylperoxy) nonamethylene biscarbonate, di(4-methyldivinylsilylbenzoylperoxy) decamethylene biscarbonate, di (4-methyldivinylsilylbenzoylperoxy) dodecamethylene biscarbonate, di(4-methyldivinylsilylbenzoylperoxy) hexadecamethylene biscarbonate, 1,1,1-tris(4-methyldivinylsilylbenzoylperoxycarbonyloxymethyl)ethane, 1,1,1-tris(4-methyldivinylsilylbenzoylperoxycarbonyloxymethyl)propane, 1,2,3-tris(4-methyldivinylsilylbenzoylperoxycarbonyloxymethyl)propane, 1,1,1-tris(4-methyldivinylsilylbenzoylperoxycarbonyloxymethyl)butane, 1,1,1,1-tetrakis(4-methyldivinylsilylbenzoylperoxycarbonyloxymethyl)methane, 1,2,3,4-tetrakis(4-methyldivinylsilylbenzoylperoxycarbonyloxymethyl)butane, 1,2,3,4,5-penta(4-methyldivinylsilylbenzoylperoxycarbonyloxymethyl)pentane, 1,2,3,4,5,6-hexa(4-methyldivinylsilylbenzoylperoxycarbonyloxymethyl)hexane,
4-ethyldivinylsilylbenzoylperoxy methyl monocarbonate, 4-ethyldivinylsilylbenzoylperoxy ethyl monocarbonate, 4-ethyldivinylsilylbenzoylperoxy n-propyl monocarbonate, 4-ethyldivinylsilylbenzoylperoxy isopropyl monocarbonate, 4-ethyldivinylsilylbenzoylperoxy n-butyl monocarbonate, 4-ethyldivinylsilylbenzoylperoxy sec-butyl monocarbonate, 4-ethyldivinylsilylbenzoylperoxy isobutyl monocarbonate, 4-ethyldivinylsilylbenzoylperoxy n-pentyl monocarbonate, 4-ethyldivinylsilylbenzoylperoxy neopentyl monocarbonate, 4-ethyldivinylsilylbenzoylperoxy hexyl monocarbonate, 4-ethyldivinylsilylbenzoylperoxy heptyl monocarbonate, 4-ethyldivinylsilylbenzoylperoxy octyl monocarbonate, 4-ethyldivinylsilylbenzoylperoxy 1-methylheptyl monocarbonate, 4-ethyldivinylsilylbenzoylperoxy 2-ethylhexyl monocarbonate, 4-ethyldivinylsilylbenzoylperoxy nonyl monocarbonate, 4-ethyldivinylsilylbenzoylperoxy decyl monocarbonate, 4-ethyldivinylsilylbenzoylperoxy myristyl monocarbonate, 4-ethyldivinylsilylbenzoylperoxy myristyl monocarbonate, 4-ethyldivinylsilylbenzoylperoxy stearyl monocarbonate, di(4-ethyldivinylsilylbenzoylperoxy) ethylene biscarbonate, di(4-ethyldivinylsilylbenzoylperoxy) trimethylene biscarbonate, di(4-ethyldivinylsilylbenzoylperoxy) 2-methyltrimethylene biscarbonate, di (4-ethyldivinylsilylbenzoylperoxy) 2,2-dimethyltrimethylene biscarbonate, di(4-ethyldivinylsilylbenzoylperoxy) 2,2-diethyltrimethylene biscarbonate, di(4-ethyldivinylsilylbenzoylperoxy) tetramethylene biscarbonate, di(4-ethyldivinylsilylbenzoylperoxy) pentamethylene biscarbonate, di(4-ethyldivinylsilylbenzoylperoxy) 3-methylpentamethylene biscarbonate, di(4-ethyldivinylsilylbenzoylperoxy) hexamethylene biscarbonate, di(4-ethyldivinylsilylbenzoylperoxy) heptamethylene biscarbonate, di(4-ethyldivinylsilylbenzoylperoxy) octamethylene biscarbonate, di(4-ethyldivinylsilylbenzoylperoxy) nonamethylene biscarbonate, di(4-ethyldivinylsilylbenzoylperoxy) decamethylene biscarbonate,
di(4-ethyldivinylsilylbenzoylperoxy) dodecamethylene biscarbonate, di(4-ethyldivinylsilylbenzoylperoxy) hexadecamethylene biscarbonate, 1,1,1-tris(4-ethyldivinylsilylbenzoylperoxycarbonyloxymethyl)ethane, 1,1,1-tris(4-ethyldivinylsilylbenzoylperoxycarbonyloxymethyl)propane, 1,2,3-tris(4-ethyldivinylsilylbenzoylperoxycarbonyloxymethyl)propane, 1,1,1-tris(4-ethyldivinylsilylbenzoylperoxycarbonyloxyethyl)butane, 1,1,1,1-tetrakis(4-ethyldivinylsilylbenzoylperoxycarbonyloxymethyl)methane, 1,2,3,4-tetrakis(4-ethyldivinylsilylbenzoylperoxycarbonyloxymethyl)butane, 1,2,3,4,5-penta(4-ethyldivinylsilylbenzoylperoxycarbonyloxymethyl)pentane, 1,2,3,4,5,6-hexa(4-ethyldivinylsilylbenzoylperoxycarbonyloxymethyl)hexane

In the case of (b) and (A) including but not limited to the following,
benzoylperoxy cyclopentyl monocarbonate, benzoylperoxy cyclohexyl monocarbonate, benzoylperoxy 2-methylcyclohexyl monocarbonate, benzoylperoxy 3-methylcyclohexyl monocarbonate, benzoylperoxy 4-methylcyclohexyl monocarbonate, benzoylperoxy 2,3-dimethylcyclohexyl monocarbonate, benzoylperoxy 2,4-dimethylcyclohexyl monocarbonate, benzoylperoxy 2,5-dimethylcyclohexyl monocarbonate, benzoylperoxy 2,6-dimethylcyclohexyl monocarbonate, benzoylperoxy 3,4-dimethylcyclohexyl monocarbonate, bbenzoylperoxy 3,5-dimethylcyclohexyl monocarbonate, benzoylperoxy 2-ethylcyclohexyl monocarbonate, benzoylperoxy 4-ethylcyclohexyl monocarbonate, benzoylperoxy 4-butylcyclohexyl monocarbonate, benzoylperoxy 3,3,5-trimethylcyclohexyl monocarbonate, benzoylperoxy 2-tert-butylcyclohexyl monocarbonate, benzoylperoxy 4-tert-butylcyclohexyl monocarbonate, benzoylperoxy menthyl monocarbonate, benzoylperoxy cyclododecyl monocarbonate, dibenzoylperoxy-1,2-cyclohexanediyldimethylene biscarbonate, dibenzoylperoxy-1,4-cyclohexanediyldimethylene biscarbonate, dibenzoylperoxy-1,2-cyclohexylene biscarbonate, dibenzoylperoxy-1,3-cyclohexylene biscarbonate, dibenzoylperoxy-1,4-cyclohexylene biscarbonate, dibenzoylperoxy-4,4'-bicyclohexylene biscarbonate, bis(4-(benzoylperoxycarbonyloxy)cyclohexyl)methane, 2,2-bis(4-(benzoylperoxycarbonyloxy)cyclohexyl)propane, tris(4-(benzoylperoxycarbonyloxy)cyclohexyl)methane, tetrakis(4-(benzoylperoxycarbonyloxy)cyclohexyl)methane.

In the case of (b) and (B), including but not limited to the following,
2-methylbenzoylperoxy cyclopentyl monocarbonate, 2-methylbenzoylperoxy cyclohexyl monocarbonate, 2-methylbenzoylperoxy 2-methylcyclohexyl monocarbonate, 2-methylbenzoylperoxy 3-methylcyclohexyl monocarbonate, 2-methylbenzoylperoxy 4-methylcyclohexyl monocarbonate, 2-methylbenzoylperoxy 2,3-dimethylcyclohexyl monocarbonate, 2-methylbenzoylperoxy 2,4-dimethylcyclohexyl monocarbonate, 2-methylbenzoylperoxy 2,5-dimethylcyclohexyl monocarbonate, 2-methylbenzoylperoxy 2,6-dimethylcyclohexyl monocarbonate, 2-methylbenzoylperoxy 3,4-dimethylcyclohexyl monocarbonate, 2-methylbbenzoylperoxy 3,5-dimethylcyclohexyl monocarbonate, 2-methylbenzoylperoxy 2-ethylcyclohexyl monocarbonate, 2-methylbenzoylperoxy 4-ethylcyclohexyl monocarbonate, 2-methylbenzoylperoxy 4-butylcyclohexyl monocarbonate, 2-methylbenzoylperoxy 3,3,5-trimethylcyclohexyl monocarbonate, 2-methylbenzoylperoxy 2-tert-butylcyclohexyl monocarbonate, 2-methylbenzoylperoxy 4-tert-butylcyclohexyl monocarbonate, 2-methylbenzoylperoxy menthyl monocarbonate, 2-methylbenzoylperoxy cyclododecyl monocarbonate, di(2-methylbenzoylperoxy) 1,2-cyclohexanediyldimethylene biscarbonate, di(2-methylbenzoylperoxy) 1,4-cyclohexanediyldimethylene biscarbonate, di(2-methylbenzoylperoxy) 1,2-cyclohexylene biscarbonate, di(2-methylbenzoylperoxy) 1,3-cyclohexylene biscarbonate, di(2-methylbenzoylperoxy) 1,4-cyclohexylene biscarbonate, di(2-methylbenzoylperoxy) 4,4'-bicyclohexylene biscarbonate, bis(4-(2-methylbenzoylperoxycarbonyloxy)cyclohexyl)methane, 2,2-bis(4-(2-methylbenzoylperoxycarbonyloxy)cyclohexyl)propane, tris(4-(2-methylbenzoylperoxycarbonyloxy)cyclohexyl)methane, tetrakis(4-(2-methylbenzoylperoxycarbonyloxy)cyclohexyl)methane,
3-methylbenzoylperoxy cyclopentyl monocarbonate, 3-methylbenzoylperoxy cyclohexyl monocarbonate, 3-methylbenzoylperoxy 2-methylcyclohexyl monocarbonate, 3-methylbenzoylperoxy 3-methylcyclohexyl monocarbonate, 3-methylbenzoylperoxy 4-methylcyclohexyl monocarbonate, 3-methylbenzoylperoxy 2,3-dimethylcyclohexyl monocarbonate, 3-methylbenzoylperoxy 2,4-dimethylcyclohexyl monocarbonate, 3-methylbenzoylperoxy 2,5-dimethylcyclohexyl monocarbonate, 3-methylbenzoylperoxy 2,6-dimethylcyclohexyl monocarbonate, 3-methylbenzoylperoxy 3,4-dimethylcyclohexyl monocarbonate, 3-methylbbenzoylperoxy 3,5-dimethylcyclohexyl monocarbonate, 3-methylbenzoylperoxy 2-ethylcyclohexyl monocarbonate, 3-methylbenzoylperoxy 4-ethylcyclohexyl monocarbonate, 3-methylbenzoylperoxy 4-butylcyclohexyl monocarbonate, 3-methylbenzoylperoxy 3,3,5-trimethylcyclohexyl monocarbonate, 3-methylbenzoylperoxy 2-tert-butylcyclohexyl monocarbonate, 3-methylbenzoylperoxy 4-tert-butylcyclohexyl monocarbonate, 3-methylbenzoylperoxy menthyl monocarbonate, 3-methylbenzoylperoxy cyclododecyl monocarbonate, di(3-methylbenzoylperoxy) 1,2-cyclohexanediyldimethylene biscarbonate, di(3-methylbenzoylperoxy) 1,4-cyclohexanediyldimethylene biscarbonate, di(3-methylbenzoylperoxy) 1,2-cyclohexylene biscarbonate, di(3-methylbenzoylperoxy) 1,3-cyclohexylene biscarbonate, di(3-methylbenzoylperoxy) 1,4-cyclohexylene biscarbonate, di(3-methylbenzoylperoxy) 4,4'-bicyclohexylene biscarbonate, bis(4-(3-methylbenzoylperoxycarbonyloxy)cyclohexyl)methane, 2,2-bis(4-(3-methylbenzoylperoxycarbonyloxy)cyclohexyl)propane, tris(4-(3-methylbenzoylperoxycarbonyloxy)cyclohexyl)methane, tetrakis(4-(3-methylbenzoylperoxycarbonyloxy)cyclohexyl)methane, 4-methylbenzoylperoxy cyclopentyl monocarbonate,
4-methylbenzoylperoxy cyclohexyl monocarbonate, 4-methylbenzoylperoxy 2-methylcyclohexyl monocarbonate, 4-methylbenzoylperoxy 3-methylcyclohexyl monocarbonate, 4-methylbenzoylperoxy 4-methylcyclohexyl monocarbonate, 4-methylbenzoylperoxy 2,3-dimethylcyclohexyl monocarbonate, 4-methylbenzoylperoxy 2,4-dimethylcyclohexyl monocarbonate, 4-methylbenzoylperoxy 2,5-dimethylcyclohexyl monocarbonate, 4-methylbenzoylperoxy 2,6-dimethylcyclohexyl monocarbonate, 4-methylbenzoylperoxy 3,4-dimethylcyclohexyl monocarbonate, 4-methylbbenzoylperoxy 3,5-dimethylcyclohexyl monocarbonate, 4-methylbenzoylperoxy 2-ethylcyclohexyl monocarbonate, 4-methylbenzoylperoxy 4-ethylcyclohexyl monocarbonate, 4-methylbenzoylperoxy 4-butylcyclohexyl monocarbonate, 4-methylbenzoylperoxy 3,3,5-trimethylcyclohexyl monocarbonate, 4-methylbenzoylperoxy 2-tert-butylcyclohexyl monocarbonate, 4-methylbenzoylperoxy 4-tert-butylcyclohexyl monocarbonate, 4-methylbenzoylperoxy menthyl monocarbonate, 4-methylbenzoylperoxy cyclododecyl monocarbonate, di(4-methylbenzoylperoxy) 1,2-cyclohexanediyldimethylene biscarbonate, di(4-methylbenzoylperoxy) 1,4-cyclohexanediyldimethylene biscarbonate, di(4-methylbenzoylperoxy) 1,2-cyclohexylene biscarbonate, di(4-methylbenzoylperoxy) 1,3-cyclohexylene biscarbonate, di(4-methylbenzoylperoxy) 1,4-cyclohexylene biscarbonate, di(4-methylbenzoylperoxy) 4,4'-bicyclohexylene biscarbonate, bis(4-(4-methylbenzoylperoxycarbonyloxy)cyclohexyl)methane, 2,2-bis(4-(4-methylbenzoylperoxycarbonyloxy)cyclohexyl)propane, tris(4-(4-methylbenzoylperoxycarbonyloxy)cyclohexyl)methane, tetrakis(4-(4-methylbenzoylperoxycarbonyloxy)cyclohexyl)methane,
4-ethylbenzoylperoxy cyclopentyl monocarbonate, 4-ethylbenzoylperoxy cyclohexyl monocarbonate, 4-ethylbenzoylperoxy 2-methylcyclohexyl monocarbonate, 4-ethylbenzoylperoxy 3-methylcyclohexyl monocarbonate, 4-ethylbenzoylperoxy 4-methylcyclohexyl monocarbonate, 4-ethylbenzoylperoxy 2,3-dimethylcyclohexyl monocarbonate, 4-ethylbenzoylperoxy 2,4-dimethylcyclohexyl monocarbonate, 4-ethylbenzoylperoxy 2,5-dimethylcyclohexyl monocarbonate, 4-ethylbenzoylperoxy 2,6-dimethylcyclohexyl monocarbonate, 4-ethylbenzoylperoxy 3,4-dimethylcyclohexyl monocarbonate, 4-ethylbbenzoylperoxy 3,5-dimethylcyclohexyl monocarbonate, 4-ethylbenzoylperoxy 2-ethylcyclohexyl monocarbonate, 4-ethylbenzoylperoxy 4-ethylcyclohexyl monocarbonate, 4-ethylbenzoylperoxy 4-butylcyclohexyl monocarbonate, 4-ethylbenzoylperoxy 3,3,5-trimethylcyclohexyl monocarbonate, 4-ethylbenzoylperoxy 2-tert-butylcyclohexyl monocarbonate, 4-ethylbenzoylperoxy 4-tert-butylcyclohexyl monocarbonate, 4-ethylbenzoylperoxy menthyl monocarbonate, 4-ethylbenzoylperoxy cyclododecyl monocarbonate, di(4-ethylbenzoylperoxy) 1,2-cyclohexanediyldimethylene biscarbonate, di(4-ethylbenzoylperoxy) 1,4-cyclohexanediyldimethylene biscarbonate, di(4-ethylbenzoylperoxy) 1,2-cyclohexylene biscarbonate, di(4-ethylbenzoylperoxy) 1,3-cyclohexylene biscarbonate, di(4-ethylbenzoylperoxy) 1,4-cyclohexylene biscarbonate, di(4-ethylbenzoylperoxy) 4,4'-bicyclohexylene biscarbonate, bis(4-(4-ethylbenzoylperoxycarbonyloxy)cyclohexyl)methane, 2,2-bis(4-(4-ethylbenzoylperoxycarbonyloxy)cyclohexyl)propane, tris(4-(4-ethylbenzoylperoxycarbonyloxy)cyclohexyl)methane, tetrakis(4-(4-ethylbenzoylperoxycarbonyloxy)cyclohexyl)methane,
2,3-dimethylbenzoylperoxy cyclopentyl monocarbonate, 2,3-dimethylbenzoylperoxy cyclohexyl monocarbonate, 2,3-dimethylbenzoylperoxy 4-tert-butylcyclohexyl monocarbonate, 2,3-dimethylbenzoylperoxy menthyl monocarbonate, 2,3-dimethylbenzoylperoxy cyclododecyl monocarbonate, di(2,3-dimethylbenzoylperoxy) 1,4-cyclohexanediyldimethylene biscarbonate, di(2,3-dimethylbenzoylperoxy) 1,4-cyclohexylene biscarbonate, di(2,3-dimethylbenzoylperoxy) 4,4'-bicyclohexylene biscarbonate, bis(4-(2,3-dimethylbenzoylperoxycarbonyloxy)cyclohexyl)methane, 2,2-bis(4-(2,3-dimethylbenzoylperoxycarbonyloxy)cyclohexyl)propane, tris(4-(2,3-dimethylbenzoylperoxycarbonyloxy)cyclohexyl)methane, tetrakis(4-(2,3-dimethylbenzoylperoxycarbonyloxy)cyclohexyl)methane,
2,4-dimethylbenzoylperoxy cyclopentyl monocarbonate, 2,4-dimethylbenzoylperoxy cyclohexyl monocarbonate, 2,4-dimethylbenzoylperoxy 2-methylcyclohexyl monocarbonate, 2,4-dimethylbenzoylperoxy 3-methylcyclohexyl monocarbonate, 2,4-dimethylbenzoylperoxy 4-methylcyclohexyl monocarbonate, 2,4-dimethylbenzoylperoxy 2,3-dimethylcyclohexyl monocarbonate, 2,4-dimethylbenzoylperoxy 2,4-dimethylcyclohexyl monocarbonate, 2,4-dimethylbenzoylperoxy 2,5-dimethylcyclohexyl monocarbonate, 2,4-dimethylbenzoylperoxy 2,6-dimethylcyclohexyl monocarbonate, 2,4-dimethylbenzoylperoxy 3,4-dimethylcyclohexyl monocarbonate, 2,4-dimethylbbenzoylperoxy 3,5-dimethylcyclohexyl monocarbonate, 2,4-dimethylbenzoylperoxy 2-ethylcyclohexyl monocarbonate, 2,4-dimethylbenzoylperoxy 4-ethylcyclohexyl monocarbonate, 2,4-dimethylbenzoylperoxy 4-butylcyclohexyl monocarbonate, 2,4-dimethylbenzoylperoxy 3,3,5-trimethylcyclohexyl monocarbonate, 2,4-dimethylbenzoylperoxy 2-tert-butylcyclohexyl monocarbonate, 2,4-dimethylbenzoylperoxy 4-tert-butylcyclohexyl monocarbonate, 2,4-dimethylbenzoylperoxy menthyl monocarbonate, 2,4-dimethylbenzoylperoxy cyclododecyl monocarbonate, di(2,4-dimethylbenzoylperoxy) 1,2-cyclohexanediyldimethylene biscarbonate, di(2,4-dimethylbenzoylperoxy) 1,4-cyclohexanediyldimethylene biscarbonate,di(2,4-dimethylbenzoylperoxy) 1,2-cyclohexylene biscarbonate, di(2,4-dimethylbenzoylperoxy) 1,3-cyclohexylene biscarbonate, di(2,4-dimethylbenzoylperoxy) 1,4-cyclohexylene biscarbonate, di(2,4-dimethylbenzoylperoxy) 4,4'-bicyclohexylene biscarbonate, bis(4-(2,4-dimethylbenzoylperoxycarbonyloxy)cyclohexyl)methane, 2,2-bis(4-(2,4-dimethylbenzoylperoxycarbonyloxy)cyclohexyl)propane, tris(4-(2,4-dimethylbenzoylperoxycarbonyloxy)cyclohexyl)methane, tetrakis(4-(2,4-dimethylbenzoylperoxycarbonyloxy)cyclohexyl)methane,
2,5-dimethylbenzoylperoxy cyclopentyl monocarbonate, 2,5-dimethylbenzoylperoxy cyclohexyl monocarbonate, 2,5-dimethylbenzoylperoxy 4-tert-butylcyclohexyl monocarbonate, 2,5-dimethylbenzoylperoxy menthyl monocarbonate, 2,5-dimethylbenzoylperoxy cyclododecyl monocarbonate, di(2,5-dimethylbenzoylperoxy) 1,4-cyclohexanediyldimethylene biscarbonate, di (2,5-dimethylbenzoylperoxy) 1,4-cyclohexylene biscarbonate, di(2,5-dimethylbenzoylperoxy) 4,4'-bicyclohexylene biscarbonate, bis(4-(2,5-dimethylbenzoylperoxycarbonyloxy)cyclohexyl) methane, 2,2-bis(4-(2,5-dimethylbenzoylperoxycarbonyloxy)cyclohexyl)propane, tris(4-(2,5-dimethylbenzoylperoxycarbonyloxy)cyclohexyl)methane, tetrakis(4-(2,5-dimethylbenzoylperoxycarbonyloxy)cyclohexyl)methane,
2,6-dimethylbenzoylperoxy cyclopentyl monocarbonate, 2,6-dimethylbenzoylperoxy cyclohexyl monocarbonate, 2,6-dimethylbenzoylperoxy 4-tert-butylcyclohexyl monocarbonate, 2,6-dimethylbenzoylperoxy menthyl monocarbonate, 2,6-dimethylbenzoylperoxy cyclododecyl monocarbonate, di(2,6-dimethylbenzoylperoxy) 1,4-cyclohexanediyldimethylene biscarbonate, di (2,6-dimethylbenzoylperoxy) 1,4-cyclohexylene biscarbonate, di(2,6-dimethylbenzoylperoxy) 4,4'-bicyclohexylene biscarbonate, bis(4-(2,6-dimethylbenzoylperoxycarbonyloxy)cyclohexyl)methane, 2,2-bis(4-(2,6-dimethylbenzoylperoxycarbonyloxy)cyclohexyl)propane, tris(4-(2,6-dimethylbenzoylperoxycarbonyloxy)cyclohexyl)methane, tetrakis(4-(2,6-dimethylbenzoylperoxycarbonyloxy)cyclohexyl)methane,
3,4-dimethylbenzoylperoxy cyclopentyl monocarbonate, 3,4-dimethylbenzoylperoxy cyclohexyl monocarbonate, 3,4-dimethylbenzoylperoxy 4-tert-butylcyclohexyl monocarbonate, 3,4-dimethylbenzoylperoxy menthyl monocarbonate, 3,4-dimethylbenzoylperoxy cyclododecyl monocarbonate, di(3,4-dimethylbenzoylperoxy) 1,4-cyclohexanediyldimethylene biscarbonate,di(3,4-dimethylbenzoylperoxy) 1,4-cyclohexylene biscarbonate, di(3,4-dimethylbenzoylperoxy) 4,4'-bicyclohexylene biscarbonate, bis(4-(3,4-dimethylbenzoylperoxycarbonyloxy)cyclohexyl)methane, 2,2-bis(4-(3,4-dimethylbenzoylperoxycarbonyloxy)cyclohexyl)propane, tris(4-(3,4-dimethylbenzoylperoxycarbonyloxy)cyclohexyl)methane, tetrakis(4-(3,4-dimethylbenzoylperoxycarbonyloxy)cyclohexyl)methane,
3,5-dimethylbenzoylperoxy cyclopentyl monocarbonate, 3,5-dimethylbenzoylperoxy cyclohexyl monocarbonate, 3,5-dimethylbenzoylperoxy 4-tert-butylcyclohexyl monocarbonate, 3,5-dimethylbenzoylperoxy menthyl monocarbonate, 3,5-dimethylbenzoylperoxy cyclododecyl monocarbonate, di(3,5-dimethylbenzoylperoxy) 1,4-cyclohexanediyldimethylene biscarbonate, di(3,5-dimethylbenzoylperoxy) 1,4-cyclohexylene biscarbonate, di(3,5-dimethylbenzoylperoxy) 4,4'-bicyclohexylene biscarbonate, bis(4-(3,5-dimethylbenzoylperoxycarbonyloxy)cyclohexyl)methane, 2,2-bis(4-(3,5-dimethylbenzoylperoxycarbonyloxy)cyclohexyl)propane, tris(4-(3,5-dimethylbenzoylperoxycarbonyloxy)cyclohexyl)methane, tetrakis(4-(3,5-dimethylbenzoylperoxycarbonyloxy)cyclohexyl)methane,
4-n-propylbenzoylperoxy cyclopentyl monocarbonate, 4-n-propylbenzoylperoxy cyclohexyl monocarbonate, 4-n-propylbenzoylperoxy 2-methylcyclohexyl monocarbonate, 4-n-propylbenzoylperoxy 3-methylcyclohexyl monocarbonate, 4-n-propylbenzoylperoxy 4-methylcyclohexyl monocarbonate, 4-n-propylbenzoylperoxy 2,3-dimethylcyclohexyl monocarbonate, 4-n-propylbenzoylperoxy 2,4-dimethylcyclohexyl monocarbonate, 4-n-propylbenzoylperoxy 2,5-dimethylcyclohexyl monocarbonate, 4-n-propylbenzoylperoxy 2,6-dimethylcyclohexyl monocarbonate, 4-n-propylbenzoylperoxy 3,4-dimethylcyclohexyl monocarbonate, 4-n-propylbbenzoylperoxy 3,5-dimethylcyclohexyl monocarbonate, 4-n-propylbenzoylperoxy 2-ethylcyclohexyl monocarbonate, 4-n-propylbenzoylperoxy 4-ethylcyclohexyl monocarbonate, 4-n-propylbenzoylperoxy 4-butylcyclohexyl monocarbonate, 4-n-propylbenzoylperoxy 3,3,5-trimethylcyclohexyl monocarbonate, 4-n-propylbenzoylperoxy 2-tert-butylcyclohexyl monocarbonate, 4-n-propylbenzoylperoxy 4-tert-butylcyclohexyl monocarbonate, 4-n-propylbenzoylperoxy menthyl monocarbonate, 4-n-propylbenzoylperoxy cyclododecyl monocarbonate, di(4-n-propylbenzoylperoxy) 1,2-cyclohexanediyldimethylene biscarbonate, di(4-n-propylbenzoylperoxy) 1,4-cyclohexanediyldimethylene biscarbonate, di(4-n-propylbenzoylperoxy) 1,2-cyclohexylene biscarbonate, di(4-n-propylbenzoylperoxy) 1,3-cyclohexylene biscarbonate, di (4-n-propylbenzoylperoxy) 1,4-cyclohexylene biscarbonate, di(4-n-propylbenzoylperoxy) 4,4'-bicyclohexylene biscarbonate, bis(4-(4-n-propylbenzoylperoxycarbonyloxy)cyclohexyl)methane, 2,2-bis(4-(4-n-propylbenzoylperoxycarbonyloxy)cyclohexyl)propane, tris(4-(4-n-propylbenzoylperoxycarbonyloxy)cyclohexyl)methane, tetrakis(4-(4-n-propylbenzoylperoxycarbonyloxy)cyclohexyl)methane,
4-isopropylbenzoylperoxy cyclopentyl monocarbonate, 4-isopropylbenzoylperoxy cyclohexyl monocarbonate, 4-isopropylbenzoylperoxy 2-methylcyclohexyl monocarbonate, 4-isopropylbenzoylperoxy 3-methylcyclohexyl monocarbonate, 4-isopropylbenzoylperoxy 4-methylcyclohexyl monocarbonate, 4-isopropylbenzoylperoxy 2,3-dimethylcyclohexyl monocarbonate, 4-isopropylbenzoylperoxy 2,4-dimethylcyclohexyl monocarbonate, 4-isopropylbenzoylperoxy 2,5-dimethylcyclohexyl monocarbonate, 4-isopropylbenzoylperoxy 2,6-dimethylcyclohexyl monocarbonate, 4-isopropylbenzoylperoxy 3,4-dimethylcyclohexyl monocarbonate, 4-isopropylbbenzoylperoxy 3,5-dimethylcyclohexyl monocarbonate,
4-isopropylbenzoylperoxy 2-ethylcyclohexyl monocarbonate, 4-isopropylbenzoylperoxy 4-ethylcyclohexyl monocarbonate, 4-isopropylbenzoylperoxy 4-butylcyclohexyl monocarbonate, 4-isopropylbenzoylperoxy 3,3,5-trimethylcyclohexyl monocarbonate, 4-isopropylbenzoylperoxy 2-tert-butylcyclohexyl monocarbonate, 4-isopropylbenzoylperoxy 4-tert-butylcyclohexyl monocarbonate, 4-isopropylbenzoylperoxy menthyl monocarbonate, 4-isopropylbenzoylperoxy cyclododecyl monocarbonate, di(4-isopropylbenzoylperoxy) 1,2-cyclohexanediyldimethylene biscarbonate, di(4-isopropylbenzoylperoxy) 1,4-cyclohexanediyldimethylene biscarbonate, di(4-isopropylbenzoylperoxy) 1,2-cyclohexylene biscarbonate, di(4-isopropylbenzoylperoxy) 1,3-cyclohexylene biscarbonate, di(4-isopropylbenzoylperoxy) 1,4-cyclohexylene biscarbonate, di(4-isopropylbenzoylperoxy) 4,4'-bicyclohexylene biscarbonate, bis(4-(4-isopropylbenzoylperoxycarbonyloxy)cyclohexyl)methane, 2,2-bis(4-(4-isopropylbenzoylperoxycarbonyloxy)cyclohexyl)propane, tris(4-(4-isopropylbenzoylperoxycarbonyloxy)cyclohexyl)methane, tetrakis(4-(4-isopropylbenzoylperoxycarbonyloxy)cyclohexyl)methane,
2,4,6-trimethylbenzoylperoxy cyclopentyl monocarbonate, 2,4,6-trimethylbenzoylperoxy cyclohexyl monocarbonate, 2,4,6-trimethylbenzoylperoxy 4-tert-butylcyclohexyl monocarbonate, 2,4,6-trimethylbenzoylperoxy menthyl monocarbonate, 2,4,6-trimethylbenzoylperoxy cyclododecyl monocarbonate, di(2,4,6-trimethylbenzoylperoxy) 1,4-cyclohexanediyldimethylene biscarbonate, di(2,4,6-trimethylbenzoylperoxy) 1,4-cyclohexylene biscarbonate, di(2,4,6-trimethylbenzoylperoxy) 4,4'-bicyclohexylene biscarbonate, bis(4-(2,4,6-trimethylbenzoylperoxycarbonyloxy)cyclohexyl)methane, 2,2-bis(4-(2,4,6-trimethylbenzoylperoxycarbonyloxy)cyclohexyl)propane, tris(4-(2,4,6-trimethylbenzoylperoxycarbonyloxy)cyclohexyl) methane, tetrakis(4-(2,4,6-trimethylbenzoylperoxycarbonyloxy)cyclohexyl)methane,
4-n-butylbenzoylperoxy cyclopentyl monocarbonate, 4-n-butylbenzoylperoxy cyclohexyl monocarbonate, 4-n-butylbenzoylperoxy 2-methylcyclohexyl monocarbonate, 4-n-butylbenzoylperoxy 3-methylcyclohexyl monocarbonate, 4-n-butylbenzoylperoxy 4-methylcyclohexyl monocarbonate, 4-n-butylbenzoylperoxy 2,3-dimethylcyclohexyl monocarbonate, 4-n-butylbenzoylperoxy 2,4-dimethylcyclohexyl monocarbonate, 4-n-butylbenzoylperoxy 2,5-dimethylcyclohexyl monocarbonate, 4-n-butylbenzoylperoxy 2,6-dimethylcyclohexyl monocarbonate, 4-n-butylbenzoylperoxy 3,4-dimethylcyclohexyl monocarbonate, 4-n-butylbbenzoylperoxy 3,5-dimethylcyclohexyl monocarbonate, 4-n-butylbenzoylperoxy 2-ethylcyclohexyl monocarbonate, 4-n-butylbenzoylperoxy 4-ethylcyclohexyl monocarbonate, 4-n-butylbenzoylperoxy 4-butylcyclohexyl monocarbonate, 4-n-butylbenzoylperoxy 3,3,5-trimethylcyclohexyl monocarbonate, 4-n-butylbenzoylperoxy 2-tert-butylcyclohexyl monocarbonate, 4-n-butylbenzoylperoxy 4-tert-butylcyclohexyl monocarbonate, 4-n-butylbenzoylperoxy menthyl monocarbonate, 4-n-butylbenzoylperoxy cyclododecyl monocarbonate, di(4-n-butylbenzoylperoxy) 1,2-cyclohexanediyldimethylene biscarbonate, di(4-n-butylbenzoylperoxy) 1,4-cyclohexanediyldimethylene biscarbonate, di(4-n-butylbenzoylperoxy) 1,2-cyclohexylene biscarbonate, di(4-n-butylbenzoylperoxy)1,3-cyclohexylene biscarbonate, di(4-n-butylbenzoylperoxy) 1,4-cyclohexylene biscarbonate, di(4-n-butylbenzoylperoxy) 4,4'-bicyclohexylene biscarbonate, bis(4-(4-n-butylbenzoylperoxycarbonyloxy)cyclohexyl)methane, 2,2-bis(4-(4-n-butylbenzoylperoxycarbonyloxy)cyclohexyl)propane, tris(4-(4-n-butylbenzoylperoxycarbonyloxy)cyclohexyl)methane, tetrakis(4-(4-n-butylbenzoylperoxycarbonyloxy)cyclohexyl)methane,
4-tert-butylbenzoylperoxy cyclopentyl monocarbonate, 4-tert-butylbenzoylperoxy cyclohexyl monocarbonate, 4-tert-butylbenzoylperoxy 2-methylcyclohexyl monocarbonate, 4-tert-butylbenzoylperoxy 3-methylcyclohexyl monocarbonate, 4-tert-butylbenzoylperoxy 4-methylcyclohexyl monocarbonate, 4-tert-butylbenzoylperoxy 2,3-dimethylcyclohexyl monocarbonate, 4-tert-butylbenzoylperoxy 2,4-dimethylcyclohexyl monocarbonate, 4-tert-butylbenzoylperoxy 2,5-dimethylcyclohexyl monocarbonate, 4-tert-butylbenzoylperoxy 2,6-dimethylcyclohexyl monocarbonate, 4-tert-butylbenzoylperoxy 3,4-dimethylcyclohexyl monocarbonate, 4-tert-butylbbenzoylperoxy 3,5-dimethylcyclohexyl monocarbonate, 4-tert-butylbenzoylperoxy 2-ethylcyclohexyl monocarbonate, 4-tert-butylbenzoylperoxy 4-ethylcyclohexyl monocarbonate, 4-tert-butylbenzoylperoxy 4-butylcyclohexyl monocarbonate, 4-tert-butylbenzoylperoxy 3,3,5-trimethylcyclohexyl monocarbonate, 4-tert-butylbenzoy peroxy-2-tert-butylcyclohexyl monocarbonate, 4-tert-butylbenzoylperoxy 4-tert-butylcyclohexyl monocarbonate, 4-tert-butylbenzoylperoxy menthyl monocarbonate, 4-tert-butylbenzoylperoxy cyclododecyl monocarbonate, di(4-tert-butylbenzoylperoxy) 1,2-cyclohexanediyldimethylene biscarbonate, di(4-tert-butylbenzoylperoxy) 1,4-cyclohexanediyldimethylene biscarbonate, di(4-tert-butylbenzoylperoxy) 1,2-cyclohexylene biscarbonate, di(4-tert-butylbenzoylperoxy) 1,3-cyclohexylene biscarbonate, di(4-tert-butylbenzoylperoxy) 1,4-cyclohexylene biscarbonate, di(4-tert-butylbenzoylperoxy) 4,4'-bicyclohexylene biscarbonate, bis(4-(4-tert-butylbenzoylperoxycarbonyloxy)cyclohexyl)methane, 2,2-bis(4-(4-tert-butylbenzoylperoxycarbonyloxy)cyclohexyl)propane, tris(4-(4-tert-butylbenzoylperoxycarbonyloxy)cyclohexyl)methane, tetrakis(4-(4-tert-butylbenzoylperoxycarbonyloxy)cyclohexyl)methane,
4-n-pentylbenzoylperoxy cyclopentyl monocarbonate, 4-n-pentylbenzoylperoxy cyclohexyl monocarbonate, 4-n-pentylbenzoylperoxy 2-methylcyclohexyl monocarbonate, 4-n-pentylbenzoylperoxy 3-methylcyclohexyl monocarbonate, 4-n-pentylbenzoylperoxy 4-methylcyclohexyl monocarbonate, 4-n-pentylbenzoylperoxy 2,3-dimethylcyclohexyl monocarbonate, 4-n-pentylbenzoylperoxy 2,4-dimethylcyclohexyl monocarbonate, 4-n-pentylbenzoylperoxy 2,5-dimethylcyclohexyl monocarbonate, 4-n-pentylbenzoylperoxy 2,6-dimethylcyclohexyl monocarbonate, 4-n-pentylbenzoylperoxy 3,4-dimethylcyclohexyl monocarbonate, 4-n-pentylbbenzoylperoxy 3,5-dimethylcyclohexyl monocarbonate, 4-n-pentylbenzoylperoxy 2-ethylcyclohexyl monocarbonate, 4-n-pentylbenzoylperoxy 4-ethylcyclohexyl monocarbonate, 4-n-pentylbenzoylperoxy 4-butylcyclohexyl monocarbonate, 4-n-pentylbenzoylperoxy 3,3,5-trimethylcyclohexyl monocarbonate, 4-n-pentylbenzoylperoxy 2-tert-butylcyclohexyl monocarbonate, 4-n-pentylbenzoylperoxy 4-tert-butylcyclohexyl monocarbonate, 4-n-pentylbenzoylperoxy menthyl monocarbonate, 4-n-pentylbenzoylperoxy cyclododecyl monocarbonate, di(4-n-penthylbenzoylperoxy) 1,2-cyclohexanediyldimethylene biscarbonate, di(4-n-penthylbenzoylperoxy) 1,4-cyclohexanediyldimethylene biscarbonate, di(4-n-pentylbenzoylperoxy) 1,2-cyclohexylene biscarbonate, di(4-n-pentylbenzoylperoxy) 1,3-cyclohexylene biscarbonate, di(4-n-pentylbenzoylperoxy) 1,4-cyclohexylene biscarbonate, di(4-n-pentylbenzoylperoxy) 4,4'-bicyclohexylene biscarbonate, bis(4-(4-n-pentylbenzoylperoxycarbonyloxy)cyclohexyl)methane, 2,2-bis(4-(4-n-pentylbenzoylperoxycarbonyloxy)cyclohexyl)propane, tris(4-(4-n-pentylbenzoylperoxycarbonyloxy)cyclohexyl)methane, tetrakis(4-(4-n-pentylbenzoylperoxycarbonyloxy)cyclohexyl)methane,
2,3,4,5,6-pentamethylbenzoylperoxy cyclopentyl monocarbonate, 2,3,4,5,6-pentamethylbenzoylperoxy cyclohexyl monocarbonate, 2,3,4,5,6-pentamethylbenzoylperoxy 4-tert-butylcyclohexyl monocarbonate, 2,3,4,5,6-pentamethylbenzoylperoxy menthyl monocarbonate, 2,3,4,5,6-pentamethylbenzoylperoxy cyclododecyl monocarbonate, di(2,3,4,5,6-penthylbenzoylperoxy) 1,4-cyclohexanediyldimethylene biscarbonate, di(2,3,4,5,6-pentamethylbenzoylperoxy) 1,4-cyclohexylene biscarbonate, di(2,3,4,5,6-pentamethylbenzoylperoxy) 4,4'-bicyclohexylene biscarbonate, bis(4-(2,3,4,5,6-pentamethylbenzoylperoxycarbonyloxy)cyclohexyl)methane, 2,2-bis(4-(2,3,4,5,6-pentamethylbenzoylperoxycarbonyloxy)cyclohexyl)propane, tris(4-(2,3,4,5,6-pentamethylbenzoylperoxycarbonyloxy)cyclohexyl)methane, tetrakis(4-(2,3,4,5,6-pentamethylbenzoylperoxycarbonyloxy)cyclohexyl)methane,
4-n-hexylbenzoylperoxy cyclopentyl monocarbonate, 4-n-hexylbenzoylperoxy cyclohexyl monocarbonate, 4-n-hexylbenzoylperoxy 2-methylcyclohexyl monocarbonate, 4-n-hexylbenzoylperoxy 3-methylcyclohexyl monocarbonate, 4-n-hexylbenzoylperoxy 4-methylcyclohexyl monocarbonate, 4-n-hexylbenzoylperoxy 2,3-dimethylcyclohexyl monocarbonate, 4-n-hexylbenzoylperoxy 2,4-dimethylcyclohexyl monocarbonate, 4-n-hexylbenzoylperoxy 2,5-dimethylcyclohexyl monocarbonate, 4-n-hexylbenzoylperoxy 2,6-dimethylcyclohexyl monocarbonate, 4-n-hexylbenzoylperoxy 3,4-dimethylcyclohexyl monocarbonate, 4-n-hexylbbenzoylperoxy 3,5-dimethylcyclohexyl monocarbonate, 4-n-hexylbenzoylperoxy 2-ethylcyclohexyl monocarbonate, 4-n-hexylbenzoylperoxy 4-ethylcyclohexyl monocarbonate, 4-n-hexylbenzoylperoxy 4-butylcyclohexyl monocarbonate, 4-n-hexylbenzoylperoxy 3,3,5-trimethylcyclohexyl monocarbonate, 4-n-hexylbenzoylperoxy 2-tert-butylcyclohexyl monocarbonate, 4-n-hexylbenzoylperoxy 4-tert-butylcyclohexyl monocarbonate, 4-n-hexylbenzoylperoxy menthyl monocarbonate, 4-n-hexylbenzoylperoxy cyclododecyl monocarbonate, di(4-n-hexylbenzoylperoxy) 1,2-cyclohexanediyldimethylene biscarbonate, di(4-n-hexylbenzoylperoxy) 1,4-cyclohexanediyldimethylene biscarbonate, di(4-n-hexylbenzoylperoxy) 1,2-cyclohexylene biscarbonate, di(4-n-hexylbenzoylperoxy) 1,3-cyclohexylene biscarbonate, di(4-n-hexylbenzoylperoxy) 1,4-cyclohexylene biscarbonate, di(4-n-hexylbenzoylperoxy) 4,4'-bicyclohexylene biscarbonate, bis(4-(4-n-hexylbenzoylperoxycarbonyloxy)cyclohexyl)methane, 2,2-bis(4-(4-n-hexylbenzoylperoxycarbonyloxy)cyclohexyl)propane, tris(4-(4-n-hexylbenzoylperoxycarbonyloxy)cyclohexyl)methane, tetrakis(4-(4-n-hexylbenzoylperoxycarbonyloxy)cyclohexyl)methane,
2-methoxybenzoylperoxy cyclopentyl monocarbonate, 2-methoxybenzoylperoxy cyclohexyl monocarbonate, 2-methoxybenzoylperoxy 2-methylcyclohexyl monocarbonate, 2-methoxybenzoylperoxy 3-methylcyclohexyl monocarbonate, 2-methoxybenzoylperoxy 4-methylcyclohexyl monocarbonate, 2-methoxybenzoylperoxy 2,3-dimethylcyclohexyl monocarbonate, 2-methoxybenzoylperoxy 2,4-dimethylcyclohexyl monocarbonate, 2-methoxybenzoylperoxy 2,5-dimethylcyclohexyl monocarbonate, 2-methoxybenzoylperoxy 2,6-dimethylcyclohexyl monocarbonate, 2-methoxybenzoylperoxy 3,4-dimethylcyclohexyl monocarbonate, 2-methoxybbenzoylperoxy 3,5-dimethylcyclohexyl monocarbonate, 2-methoxybenzoylperoxy 2-ethylcyclohexyl monocarbonate, 2-methoxybenzoylperoxy 4-ethylcyclohexyl monocarbonate, 2-methoxybenzoylperoxy 4-butylcyclohexyl monocarbonate, 2-methoxybenzoylperoxy 3,3,5-trimethylcyclohexyl monocarbonate, 2-methoxybenzoylperoxy 2-tert-butylcyclohexyl monocarbonate, 2-methoxybenzoylperoxy 4-tert-butylcyclohexyl monocarbonate, 2-methoxybenzoylperoxy menthyl monocarbonate, 2-methoxybenzoylperoxy cyclododecyl monocarbonate, di(2-methoxybenzoylperoxy) 1,2-cyclohexanediyldimethylene biscarbonate, di(2-methoxybenzoylperoxy) 1,4-cyclohexanediyldimethylene biscarbonate, di(2-methoxybenzoylperoxy) 1,2-cyclohexylene biscarbonate, di(2-methoxybenzoylperoxy)1,3-cyclohexylene biscarbonate, di(2-methoxybenzoylperoxy) 1,4-cyclohexylene biscarbonate, di(2-methoxybenzoylperoxy) 4,4'-bicyclohexylene biscarbonate, bis(4-(2-methoxybenzoylperoxycarbonyloxy)cyclohexyl)methane, 2,2-bis(4-(2-methoxybenzoylperoxycarbonyloxy)cyclohexyl)propane, tris(4-(2-methoxybenzoylperoxycarbonyloxy)cyclohexyl)methane, tetrakis(4-(2-methoxybenzoylperoxycarbonyloxy)cyclohexyl) ethane,
3-methoxybenzoylperoxy cyclopentyl monocarbonate, 3-methoxybenzoylperoxy cyclohexyl monocarbonate, 3-methoxybenzoylperoxy 4-tert-butylcyclohexyl monocarbonate, 3-methoxybenzoylperoxy menthyl monocarbonate, 3-methoxybenzoylperoxy cyclododecyl monocarbonate, di (3-methoxybenzoylperoxy) 1,4-cyclohexanediyldimethylene biscarbonate, di(3-methoxybenzoylperoxy) 1,4-cyclohexylene biscarbonate, di(3-methoxybenzoylperoxy) 4,4'-bicyclohexylene biscarbonate, bis(4-(3-methoxybenzoylperoxycarbonyloxy)cyclohexyl)methane, 2,2-bis(4-(3-methoxybenzoylperoxycarbonyloxy)cyclohexyl)propane, tris(4-(3-methoxybenzoylperoxycarbonyloxy)cyclohexyl)methane, tetrakis(4-(3-methoxybenzoylperoxycarbonyloxy)cyclohexyl)methane, 4-methoxybenzoylperoxy cyclopentyl monocarbonate,
4-methoxybenzoylperoxy cyclohexyl monocarbonate, 4-methoxybenzoylperoxy 2-methylcyclohexyl monocarbonate, 4-methoxybenzoylperoxy 3-methylcyclohexyl monocarbonate, 4-methoxybenzoylperoxy 4-methylcyclohexyl monocarbonate, 4-methoxybenzoylperoxy 2,3-dimethylcyclohexyl monocarbonate, 4-methoxybenzoylperoxy 2,4-dimethylcyclohexyl monocarbonate, 4-methoxybenzoylperoxy 2,5-dimethylcyclohexyl monocarbonate, 4-methoxybenzoylperoxy 2,6-dimethylcyclohexyl monocarbonate, 4-methoxybenzoylperoxy 3,4-dimethylcyclohexyl monocarbonate, 4-methoxybbenzoylperoxy 3,5-dimethylcyclohexyl monocarbonate, 4-methoxybenzoylperoxy 2-ethylcyclohexyl monocarbonate, 4-methoxybenzoylperoxy 4-ethylcyclohexyl monocarbonate, 4-methoxybenzoylperoxy 4-butylcyclohexyl monocarbonate, 4-methoxybenzoylperoxy 3,3,5-trimethylcyclohexyl monocarbonate, 4-methoxybenzoylperoxy 2-tert-butylcyclohexyl monocarbonate, 4-methoxybenzoylperoxy 4-tert-butylcyclohexyl monocarbonate, 4-methoxybenzoylperoxy menthyl monocarbonate, 4-methoxybenzoylperoxy cyclododecyl monocarbonate, di(2-methoxybenzoylperoxy) 1,2-cyclohexanediyldimethylene biscarbonate, di(2-methoxybenzoylperoxy) 1,4-cyclohexanediyldimethylene biscarbonate, di(4-methoxybenzoylperoxy) 1,2-cyclohexylene biscarbonate, di(4-methoxybenzoylperoxy) 1,3-cyclohexylene biscarbonate, di(4-methoxybenzoylperoxy) 1,4-cyclohexylene biscarbonate, di(4-methoxybenzoylperoxy) 4,4'-bicyclohexylene biscarbonate, bis(4-(4-methoxybenzoylperoxycarbonyloxy)cyclohexyl)methane, 2,2-bis(4-(4-methoxybenzoylperoxycarbonyloxy)cyclohexyl)propane, tris(4-(4-methoxybenzoylperoxycarbonyloxy)cyclohexyl)methane, tetrakis(4-(4-methoxybenzoylperoxycarbonyloxy)cyclohexyl)methane,
2-ethoxybenzoylperoxy cyclopentyl monocarbonate, 2-ethoxybenzoylperoxy cyclohexyl monocarbonate, 2-ethoxybenzoylperoxy 4-tert-butylcyclohexyl monocarbonate, 2-ethoxybenzoylperoxy menthyl monocarbonate, 2-ethoxybenzoylperoxy cyclododecyl monocarbonate, di (2-ethoxybenzoylperoxy) 1,4-cyclohexanediyldimethylene biscarbonate, di(2-ethoxybenzoylperoxy) 1,4-cyclohexylene biscarbonate, di(2-ethoxybenzoylperoxy) 4,4'-bicyclohexylene biscarbonate, bis(4-(2-ethoxybenzoylperoxycarbonyloxy)cyclohexyl)methane, 2,2-bis(4-(2-ethoxybenzoylperoxycarbonyloxy)cyclohexyl)propane, tris(4-(2-ethoxybenzoylperoxycarbonyloxy)cyclohexyl)methane, tetrakis(4-(2-ethoxybenzoylperoxycarbonyloxy)cyclohexyl)methane,
3-ethoxybenzoylperoxy cyclopentyl monocarbonate, 3-ethoxybenzoylperoxy cyclohexyl monocarbonate, 3-ethoxybenzoylperoxy 4-tert-butylcyclohexyl monocarbonate, 3-ethoxybenzoylperoxy menthyl monocarbonate, 3-ethoxybenzoylperoxy cyclododecyl monocarbonate, di(3-ethoxybenzoylperoxy) 1,4-cyclohexanediyldimethylene biscarbonate, di(3-ethoxybenzoylperoxy) 1,4-cyclohexylene biscarbonate, di(3-ethoxybenzoylperoxy) 4,4'-bicyclohexylene biscarbonate, bis(4-(3-ethoxybenzoylperoxycarbonyloxy)cyclohexyl)methane, 2,2-bis(4-(3-ethoxybenzoylperoxycarbonyloxy)cyclohexyl)propane, tris(4-(3-ethoxybenzoylperoxycarbonyl oxy)cyclohexyl)methane, tetrakis(4-(3-ethoxybenzoylperoxycarbonyloxy)cyclohexyl)methane,
4-ethoxybenzoylperoxy cyclopentyl monocarbonate, 4-ethoxybenzoylperoxy cyclohexyl monocarbonate, 4-ethoxybenzoylperoxy 4-tert-butylcyclohexyl monocarbonate, 4-ethoxybenzoylperoxy menthyl monocarbonate, 4-ethoxybenzoylperoxy cyclododecyl monocarbonate, di (4-ethoxybenzoylperoxy) 1,4-cyclohexanediyldimethylene biscarbonate, di(4-ethoxybenzoylperoxy) 1,4-cyclohexylene biscarbonate, di(4-ethoxybenzoylperoxy) 4,4'-bicyclohexylene biscarbonate, bis(4-(4-ethoxybenzoylperoxycarbonyloxy)cyclohexyl)methane, 2,2-bis(4-(4-ethoxybenzoylperoxycarbonyloxy)cyclohexyl)propane, tris(4-(4-ethoxybenzoylperoxycarbonyloxy)cyclohexyl)methane, tetrakis(4-(4-ethoxybenzoylperoxycarbonyloxy)cyclohexyl)methane,
2,3-dimethoxybenzoylperoxy cyclopentyl monocarbonate, 2,3-dimethoxybenzoylperoxy cyclohexyl monocarbonate, 2,3-dimethoxybenzoylperoxy 4-tert-butylcyclohexyl monocarbonate, 2,3-dimethoxybenzoylperoxymenthyl monocarbonate, 2,3-dimethoxybenzoylperoxy cyclododecyl monocarbonate, di(2,3-dimethoxybenzoylperoxy) 1,4-cyclohexanediyldimethylene biscarbonate, di(2,3-dimethoxybenzoylperoxy) 1,4-cyclohexylene biscarbonate, di(2,3-dimethoxybenzoylperoxy) 4,4'-bicyclohexylene biscarbonate, bis(4-(2,3-dimethoxybenzoylperoxycarbonyloxy)cyclohexyl)methane, 2,2-bis(4-(2,3-dimethoxybenzoylperoxycarbonyloxy)cyclohexyl)propane, tris(4-(2,3-dimethoxybenzoylperoxycarbonyloxy)cyclohexyl)methane, tetrakis(4-(2,3-dimethoxybenzoylperoxycarbonyloxy)cyclohexyl)methane,
2,4-dimethoxybenzoylperoxy cyclopentyl monocarbonate, 2,4-dimethoxybenzoylperoxy cyclohexyl monocarbonate, 2,4-dimethoxybenzoylperoxy 4-tert-butylcyclohexyl monocarbonate, 2,4-dimethoxybenzoylperoxymenthylmonocarbonate, 2,4-dimethoxybenzoylperoxy cyclododecyl monocarbonate, di(2,4-dimethoxybenzoylperoxy) 1,4-cyclohexanediyldimethylene biscarbonate, di(2,4-dimethoxybenzoylperoxy) 1,4-cyclohexylene biscarbonate, di(2,4-dimethoxybenzoylperoxy) 4,4'-bicyclohexylene biscarbonate, bis(4-(2,4-dimethoxybenzoylperoxycarbonyloxy)cyclohexyl)methane, 2,2-bis(4-(2,4-dimethoxybenzoylperoxy arbonyloxy)cyclohexyl)propane, tris(4-(2,4-dimethoxybenzoylperoxycarbonyloxy)cyclohexyl)methane, tetrakis(4-(2,4-dimethoxybenzoylperoxycarbonyloxy)cyclohexyl)methane,
2,5-dimethoxybenzoylperoxy cyclopentyl monocarbonate, 2,5-dimethoxybenzoylperoxy cyclohexyl monocarbonate, 2,5-dimethoxybenzoylperoxy 4-tert-butylcyclohexyl monocarbonate, 2,5-dimethoxybenzoylperoxymenthylmonocarbonate,2,5-dimethoxybenzoylperoxy cyclododecyl monocarbonate, di(2,5-dimethoxybenzoylperoxy) 1,4-cyclohexanediyldimethylene biscarbonate, di(2,5-dimethoxybenzoylperoxy) 1,4-cyclohexylene biscarbonate, di(2,5-dimethoxybenzoylperoxy) 4,4'-bicyclohexylene biscarbonate, bis(4-(2,5-dimethoxybenzoylperoxycarbonyloxy)cyclohexyl)methane, 2,2-bis(4-(2,5-dimethoxybenzoylperoxycarbonyloxy)cyclohexyl)propane, tris(4-(2,5-dimethoxybenzoylperoxycarbonyloxy)cyclohexyl)methane, tetrakis(4-(2,5-dimethoxybenzoylperoxycarbonyloxy)cyclohexyl)methane,
2,6-dimethoxybenzoylperoxy cyclopentyl monocarbonate, 2,6-dimethoxybenzoylperoxy cyclohexyl monocarbonate, 2,6-dimethoxybenzoylperoxy 4-tert-butylcyclohexyl monocarbonate, 2,6-dimethoxybenzoylperoxy menthyl monocarbonate, 2,6-dimethoxybenzoylperoxy cyclododecyl monocarbonate, di(2,6-dimethoxybenzoylperoxy) 1,4-cyclohexanediyldimethylene biscarbonate, di(2,6-dimethoxybenzoylperoxy) 1,4-cyclohexylene biscarbonate, di(2,6-dimethoxybenzoylperoxy) 4,4'-bicyclohexylene biscarbonate, bis(4-(2,6-dimethoxybenzoylperoxycarbonyloxy)cyclohexyl)methane, 2,2-bis(4-(2,6-dimethoxybenzoylperoxycarbonyloxy)cyclohexyl)propane, tris (4-(2,6-dimethoxybenzoylperoxycarbonyloxy)cyclohexyl)methane, tetrakis(4-(2,6-dimethoxybenzoylperoxycarbonyloxy)cyclohexyl)methane,
3,4-dimethoxybenzoylperoxy cyclopentyl monocarbonate, 3,4-dimethoxybenzoylperoxy cyclohexyl monocarbonate, 3,4-dimethoxybenzoylperoxy 4-tert-butylcyclohexyl monocarbonate, 3,4-dimethoxybenzoylperoxy menthyl monocarbonate, 3,4-dimethoxybenzoylperoxy cyclododecyl monocarbonate, di(3,4-dimethoxybenzoylperoxy) 1,4-cyclohexanediyldimethylene biscarbonate, di(3,4-dimethoxybenzoylperoxy) 1,4-cyclohexylene biscarbonate, di(3,4-dimethoxybenzoylperoxy) 4,4'-bicyclohexylene biscarbonate, bis(4-(3,4-dimethoxybenzoylperoxycarbonyloxy)cyclohexyl)methane, 2,2-bis(4-(3,4-dimethoxybenzoylperoxycarbonyloxy)cyclohexyl)propane, tris(4-(3,4-dimethoxybenzoylperoxycarbonyloxy)cyclohexyl)methane, tetrakis(4-(3,4-dimethoxybenzoylperoxycarbonyloxy)cyclohexyl)methane,
3,5-dimethoxybenzoylperoxy cyclopentyl monocarbonate, 3,5-dimethoxybenzoylperoxy cyclohexyl monocarbonate, 3,5-dimethoxybenzoylperoxy 4-tert-butylcyclohexyl monocarbonate, 3,5-dimethoxybenzoylperoxymenthyl monocarbonate, 3,5-dimethoxybenzoylperoxy cyclododecyl monocarbonate, di(3,5-dimethoxybenzoylperoxy) 1,4-cyclohexanediyldimethylene biscarbonate, di(3,5-dimethoxybenzoylperoxy) 1,4-cyclohexylene biscarbonate, di(3,5-dimethoxybenzoylperoxy) 4,4'-bicyclohexylene biscarbonate, bis(4-(3,5-dimethoxybenzoylperoxycarbonyloxy)cyclohexyl)methane, 2,2-bis(4-(3,5-dimethoxybenzoylperoxycarbonyloxy)cyclohexyl)propane, tris(4-(3,5-dimethoxybenzoylperoxycarbonyloxy)cyclohexyl)methane, tetrakis(4-(3,5-dimethoxybenzoylperoxycarbonyloxy)cyclohexyl)methane,
4-n-propyloxybenzoylperoxy cyclopentyl monocarbonate, 4-n-propyloxybenzoylperoxy cyclohexyl monocarbonate, 4-n-propyloxybenzoylperoxy 4-tert-butylcyclohexyl monocarbonate, 4-n-propyloxybenzoylperoxymenthyl monocarbonate, 4-n-propyloxybenzoylperoxy cyclododecyl monocarbonate, di(4-n-propyloxybenzoylperoxy) 1,4-cyclohexanediyldimethylene biscarbonate, di(4-n-propyloxybenzoylperoxy) 1,4-cyclohexylene biscarbonate, di(4-n-propyloxybenzoylperoxy) 4,4'-bicyclohexylene biscarbonate, bis(4-(4-n-propyloxybenzoylperoxycarbonyloxy)cyclohexyl)methane, 2,2-bis(4-(4-n-propyloxybenzoylperoxycarbonyloxy)cyclohexyl)propane, tris(4-(4-n-propyloxybenzoylperoxycarbonyloxy)cyclohexyl)methane, tetrakis(4-(4-n-propyloxybenzoylperoxycarbonyloxy)cyclohexyl)methane,
4-isopropyloxybenzoylperoxy cyclopentyl monocarbonate, 4-isopropyloxybenzoylperoxy cyclohexyl monocarbonate, 4-isopropyloxybenzoylperoxy 4-tert-butylcyclohexyl monocarbonate, 4-isopropyloxybenzoylperoxy menthyl monocarbonate, 4-isopropyloxybenzoylperoxy cyclododecyl monocarbonate, di(4-isopropyloxybenzoylperoxy) 1,4-cyclohexanediyldimethylene biscarbonate, di(4-isopropyloxybenzoylperoxy) 1,4-cyclohexylene biscarbonate, di(4-isopropyloxybenzoylperoxy) 4,4'-bicyclohexylene biscarbonate, bis(4-(4-isopropyloxybenzoylperoxycarbonyloxy)cyclohexyl)methane, 2,2-bis(4-(4-isopropyloxybenzoylperoxycarbonyloxy)cyclohexyl)propane, tris(4-(4-isopropyloxybenzoylperoxycarbonyloxy)cyclohexyl)methane, tetrakis(4-(4-isopropyloxybenzoylperoxycarbonyloxy)cyclohexyl)methane,
2,3,4-trimethoxybenzoylperoxy cyclopentyl monocarbonate, 2,3,4-trimethoxybenzoylperoxy cyclohexyl monocarbonate, 2,3,4-trimethoxybenzoylperoxy 4-tert-butylcyclohexyl monocarbonate, 2,3,4-trimethoxybenzoylperoxy menthyl monocarbonate, 2,3,4-trimethoxybenzoylperoxy cyclododecyl monocarbonate, di (2,3,4-trimethoxybenzoylperoxy) 1,4-cyclohexanediyldimethylene biscarbonate, di(2,3,4-trimethoxybenzoylperoxy) 1,4-cyclohexylene biscarbonate, di(2,3,4-trimethoxybenzoylperoxy) 4,4'-bicyclohexylene biscarbonate, bis(4-(2,3,4-trimethoxybenzoylperoxycarbonyloxy)cyclohexyl)methane, 2,2-bis(4-(2,3,4-trimethoxybenzoylperoxycarbonyloxy)cyclohexyl)propane, tris(4-(2,3,4-trimethoxybenzoylperoxycarbonyloxy)cyclohexyl)methane, tetrakis(4-(2,3,4-trimethoxybenzoylperoxycarbonyloxy)cyclohexyl)methane,
2,4,5-trimethoxybenzoylperoxy cyclopentyl monocarbonate, 2,4,5-trimethoxybenzoylperoxy cyclohexyl monocarbonate, 2,4,5-trimethoxybenzoylperoxy 4-tert-butylcyclohexyl monocarbonate, 2,4,5-trimethoxybenzoylperoxy menthyl monocarbonate, 2,4,5-trimethoxybenzoylperoxy cyclododecyl monocarbonate, di (2,4,5-trimethoxybenzoylperoxy) 1,4-cyclohexanediyldimethylene biscarbonate, di(2,4,5-trimethoxybenzoylperoxy) 1,4-cyclohexylene biscarbonate, di(2,4,5-trimethoxybenzoylperoxy) 4,4'-bicyclohexylene biscarbonate, bis (4- (2,4, 5-trimethoxybenzoylperoxycarbonyloxy) cyclohexyl)methane, 2,2-bis(4-(2,4,5-trimethoxybenzoylperoxycarbonyloxy)cyclohexyl)propane, tris(4-(2,4,5-trimethoxybenzoylperoxycarbonyloxy)cyclohexyl)methane, tetrakis(4-(2,4,5-trimethoxybenzoylperoxycarbonyloxy)cyclohexyl)methane,
3,4,5-trimethoxybenzoylperoxy cyclopentyl monocarbonate, 3,4,5-trimethoxybenzoylperoxy cyclohexyl monocarbonate, 3,4,5-trimethoxybenzoylperoxy 4-tert-butylcyclohexyl monocarbonate, 3,4,5-trimethoxybenzoylperoxy menthyl monocarbonate, 3,4,5-trimethoxybenzoylperoxy cyclododecyl monocarbonate, di(3,4,5-trimethoxybenzoylperoxy) 1,4-cyclohexanediyldimethylene biscarbonate, di(3,4,5-trimethoxybenzoylperoxy) 1,4-cyclohexylene biscarbonate, di(3,4,5-trimethoxybenzoylperoxy) 4,4'-bicyclohexylene biscarbonate, bis(4-(3,4,5-trimethoxybenzoylperoxycarbonyloxy)cyclohexyl) methane, 2,2-bis(4-(3,4,5-trimethoxybenzoylperoxycarbonyl oxy)cyclohexyl)propane, tris(4-(3,4,5-trimethoxybenzoylperoxycarbonyloxy)cyclohexyl)methane, tetrakis(4-(3,4,5-trimethoxybenzoylperoxycarbonyloxy)cyclohexyl)methane,
4-n-butoxybenzoylperoxy cyclopentyl monocarbonate, 4-n-butoxybenzoylperoxy cyclohexyl monocarbonate, 4-n-butoxybenzoylperoxy 4-tert-butylcyclohexyl monocarbonate, 4-n-butoxybenzoylperoxy menthyl monocarbonate, 4-n-butoxybenzoylperoxy cyclododecyl monocarbonate, di(4-n-butoxybenzoylperoxy) 1,4-cyclohexanediyldimethylene biscarbonate, di(4-n-butoxybenzoylperoxy) 1,4-cyclohexylene biscarbonate, di(4-n-butoxybenzoylperoxy) 4,4'-bicyclohexylene biscarbonate, bis(4-(4-n-butoxybenzoylperoxycarbonyloxy)cyclohexyl)methane, 2,2-bis(4-(4-n-butoxybenzoylperoxycarbonyloxy)cyclohexyl)propane, tris(4-(4-n-butoxybenzoylperoxycarbonyloxy)cyclohexyl)methane, tetrakis(4-(4-n-butoxybenzoylperoxycarbonyloxy)cyclohexyl)methane,
4-n-hexyloxybenzoylperoxy cyclopentyl monocarbonate, 4-n-hexyloxybenzoylperoxy cyclohexyl monocarbonate, 4-n-hexyloxybenzoylperoxy 4-tert-butylcyclohexyl monocarbonate, 4-n-hexyloxybenzoylperoxy menthyl monocarbonate, 4-n-hexyloxybenzoylperoxy cyclododecyl monocarbonate, di(4-n-hexyloxybenzoylperoxy) 1,4-cyclohexanediyldimethylene biscarbonate, di(4-n-hexyloxybenzoylperoxy) 1,4-cyclohexylene biscarbonate, di(4-n-hexyloxybenzoylperoxy) 4,4'-bicyclohexylene biscarbonate, bis(4-(4-n-hexyloxybenzoylperoxycarbonyloxy)cyclohexyl)methane, 2,2-bis(4-(4-n-hexyloxybenzoylperoxycarbonyloxy)cyclohexyl)propane, tris(4-(4-n-hexyloxybenzoylperoxycarbonyloxy)cyclohexyl)methane, tetrakis(4-(4-n-hexyloxybenzoylperoxycarbonyloxy)cyclohexyl)methane,
2-phenylbenzoylperoxy cyclopentyl monocarbonate, 2-phenylbenzoylperoxy cyclohexyl monocarbonate, 2-phenylbenzoylperoxy 4-tert-butylcyclohexyl monocarbonate, 2-phenylbenzoylperoxy menthyl monocarbonate, 2-phenylbenzoylperoxy cyclododecyl monocarbonate, di(2-phenylbenzoylperoxy) 1,4-cyclohexanediyldimethylene biscarbonate, di(2-phenylbenzoylperoxy) 1,4-cyclohexylene biscarbonate, di(2-phenylbenzoylperoxy) 4,4'-bicyclohexylene biscarbonate, bis (4- (2-phenylbenzoylperoxycarbonyloxy)cyclohexyl)methane, 2,2-bis(4-(2-phenylbenzoylperoxycarbonyloxy)cyclohexyl)propane, tris(4-(2-phenylbenzoyl peroxycarbonyloxy)cyclohexyl)methane, tetrakis(4-(2-phenylbenzoylperoxycarbonyloxy)cyclohexyl)methane,
3-phenylbenzoylperoxy cyclopentyl monocarbonate, 3-phenylbenzoylperoxy cyclohexyl monocarbonate, 3-phenylbenzoylperoxy 4-tert-butylcyclohexyl monocarbonate, 3-phenylbenzoylperoxy menthyl monocarbonate, 3-phenylbenzoylperoxy cyclododecyl monocarbonate, di(3-phenylbenzoylperoxy) 1,4-cyclohexanediyldimethylene biscarbonate, di(3-phenylbenzoylperoxy) 1,4-cyclohexylene biscarbonate, di(3-phenylbenzoylperoxy) 4,4'-bicyclohexylene biscarbonate, bis(4-(3-phenylbenzoylperoxycarbonyloxy)cyclohexyl)methane, 2,2-bis(4-(3-phenylbenzoylperoxycarbonyloxy)cyclohexyl)propane, tris(4-(3-phenylbenzoylperoxycarbonyloxy)cyclohexyl)methane, tetrakis(4-(3-phenylbenzoylperoxycarbonyloxy)cyclohexyl)methane,
4-phenylbenzoylperoxy cyclopentyl monocarbonate, 4-phenylbenzoylperoxy cyclohexyl monocarbonate, 4-phenylbenzoylperoxy 2-methylcyclohexyl monocarbonate, 4-phenylbenzoylperoxy 3-methylcyclohexyl monocarbonate, 4-phenylbenzoylperoxy 4-methylcyclohexyl monocarbonate,
4-phenylbenzoylperoxy 2,3-dimethylcyclohexyl monocarbonate, 4-phenylbenzoylperoxy 2,4-dimethylcyclohexyl monocarbonate, 4-phenylbenzoylperoxy 2,5-dimethylcyclohexyl monocarbonate, 4-phenylbenzoylperoxy 2,6-dimethylcyclohexyl monocarbonate, 4-phenylbenzoylperoxy 3,4-dimethylcyclohexyl monocarbonate, 4-phenylbbenzoylperoxy 3,5-dimethylcyclohexyl monocarbonate, 4-phenylbenzoylperoxy 2-ethylcyclohexyl monocarbonate, 4-phenylbenzoylperoxy 4-ethylcyclohexyl monocarbonate, 4-phenylbenzoylperoxy 4-butylcyclohexyl monocarbonate,
4-phenylbenzoylperoxy 3,3,5-trimethylcyclohexyl monocarbonate, 4-phenylbenzoylperoxy 2-tert-butylcyclohexyl monocarbonate, 4-phenylbenzoylperoxy 4-tert-butylcyclohexyl monocarbonate, 4-phenylbenzoylperoxy menthyl monocarbonate, 4-phenylbenzoylperoxy cyclododecyl monocarbonate, di(4-phenylbenzoylperoxy) 1,2-cyclohexanediyldimethylene biscarbonate, di(4-phenylbenzoylperoxy) 1,4-cyclohexanediyldimethylene biscarbonate, di(4-phenylbenzoylperoxy) 1,2-cyclohexylene biscarbonate, di(4-phenylbenzoylperoxy) 1,3-cyclohexylene biscarbonate, di(4-phenylbenzoylperoxy) 1,4-cyclohexylene biscarbonate, di(4-phenylbenzoylperoxy) 4,4'-bicyclohexylene biscarbonate, bis(4-(4-phenylbenzoylperoxycarbonyloxy)cyclohexyl)methane, 2,2-bis(4-(4-phenylbenzoylperoxycarbonyloxy)cyclohexyl)propane, tris(4-(4-phenylbenzoylperoxycarbonyloxy)cyclohexyl)methane, tetrakis(4-(4phenylbenzoylperoxycarbonyloxy)cyclohexyl)methane.

In the cases of (b) and (C) including but not limited to the following, 4-trimethylsilylbenzoylperoxy cyclopentyl monocarbonate, 4-trimethylsilylbenzoylperoxy cyclohexyl monocarbonate, 4-trimethylsilylbenzoylperoxy 2-methylcyclohexyl monocarbonate, 4-trimethylsilylbenzoylperoxy 3-methylcyclohexyl monocarbonate, 4-trimethylsilylbenzoylperoxy 4-methylcyclohexyl monocarbonate, 4-trimethylsilylbenzoylperoxy 2,3-dimethylcyclohexyl monocarbonate, 4-trimethylsilylbenzoylperoxy 2,4-dimethylcyclohexyl monocarbonate, 4-trimethylsilylbenzoylperoxy 2,5-dimethylcyclohexyl monocarbonate, 4-trimethylsilylbenzoylperoxy 2,6-dimethylcyclohexyl monocarbonate,
4-trimethylsilylbenzoylperoxy 3,4-dimethylcyclohexyl monocarbonate, 4-trimethylsilylbbenzoylperoxy 3,5-dimethylcyclohexyl monocarbonate, 4-trimethylsilylbenzoylperoxy 2-ethylcyclohexyl monocarbonate, 4-trimethylsilylbenzoylperoxy 4-ethylcyclohexyl monocarbonate, 4-trimethylsilylbenzoylperoxy 4-butylcyclohexyl monocarbonate, 4-trimethylsilylbenzoylperoxy 3,3,5-trimethylcyclohexyl monocarbonate, 4-trimethylsilylbenzoylperoxy 2-tert-butylcyclohexyl monocarbonate, 4-trimethylsilylbenzoylperoxy 4-tert-butylcyclohexyl monocarbonate, 4-trimethylsilylbenzoylperoxy menthyl monocarbonate, 4-trimethylsilylbenzoylperoxy cyclododecyl monocarbonate, di(4-trimethylsilylbenzoylperoxy) 1,2-cyclohexylene biscarbonate, di(4-trimethylsilylbenzoylperoxy) 1,2-cyclohexanediyldimethylene biscarbonate, di(4-trimethylsilylbenzoylperoxy) 1,4-cyclohexanediyldimethylene biscarbonate, di(4-trimethylsilylbenzoylperoxy) 1,3-cyclohexylene biscarbonate, di(4-trimethylsilylbenzoylperoxy) 1,4-cyclohexylene biscarbonate, di(4-trimethylsilylbenzoylperoxy) 4,4'-bicyclohexylene biscarbonate, bis(4-(4-trimethylsilylbenzoylperoxycarbonyloxy)cyclohexyl)methane, 2,2-bis(4-(4-trimethylsilylbenzoylperoxycarbonyloxy)cyclohexyl)propane, tris(4-(4-trimethylsilylbenzoylperoxycarbonyloxy)cyclohexyl)methane, tetrakis(4-(4-trimethylsilylbenzoylperoxycarbonyloxy)cyclohexyl)methane,
4-dimethylvinylsilylbenzoylperoxy cyclopentyl monocarbonate, 4-dimethylvinylsilylbenzoylperoxy cyclohexyl monocarbonate, 4-dimethylvinylsilylbenzoylperoxy 2-methylcyclohexyl monocarbonate, 4-dimethylvinylsilylbenzoylperoxy 3-methylcyclohexyl monocarbonate, 4-dimethylvinylsilylbenzoylperoxy 4-methylcyclohexyl monocarbonate, 4-dimethylvinylsilylbenzoylperoxy 2,3-dimethylcyclohexyl monocarbonate, 4-dimethylvinylsilylbenzoylperoxy 2,4-dimethylcyclohexyl monocarbonate,

4-dimethylvinylsilylbenzoylperoxy 2,5-dimethylcyclohexyl monocarbonate, 4-dimethylvinylsilylbenzoylperoxy 2,6-dimethylcyclohexyl monocarbonate, 4-dimethylvinylsilylbenzoylperoxy 3,4-dimethylcyclohexyl monocarbonate, 4-dimethylvinylsilylbbenzoylperoxy 3,5-dimethylcyclohexyl monocarbonate, 4-dimethylvinylsilylbenzoylperoxy 2-ethylcyclohexyl monocarbonate, 4-dimethylvinylsilylbenzoylperoxy 4-ethylcyclohexyl monocarbonate, 4-dimethylvinylsilylbenzoylperoxy 4-butylcyclohexyl monocarbonate, 4-dimethylvinylsilylbenzoylperoxy 3,3,5-trimethylcyclohexyl monocarbonate, 4-dimethylvinylsilylbenzoylperoxy 2-tert-butylcyclohexyl monocarbonate, 4-dimethylvinylsilylbenzoylperoxy 4-tert-butylcyclohexyl monocarbonate, 4-dimethylvinylsilylbenzoylperoxy menthyl monocarbonate, 4-dimethylvinylsilylbenzoylperoxy cyclododecyl monocarbonate, di(4-dimethylvinylsilylbenzoylperoxy) 1,2-cyclohexanediyldimethylene biscarbonate, di(4-dimethylvinylsilylbenzoylperoxy) 1,4-cyclohexanediyldimethylene biscarbonate, di(4-dimethylvinylsilylbenzoylperoxy) 1,2-cyclohexylene biscarbonate, di(4-dimethylvinylsilylbenzoylperoxy) 1,3-cyclohexylene biscarbonate, di(4-dimethylvinylsilylbenzoylperoxy) 1,4-cyclohexylene biscarbonate, di(4-dimethylvinylsilylbenzoylperoxy) 4,4'-bicyclohexylene biscarbonate, bis(4-(4-dimethylvinylsilylbenzoylperoxycarbonyloxy)cyclohexyl)methane, 2,2-bis(4-(4-dimethylvinylsilylbenzoylperoxycarbonyloxy)cyclohexyl)propan e, tris(4-(4-dimethylvinylsilylbenzoylperoxycarbonyloxy)cyclohexyl)methane, tetrakis(4-(4-dimethylvinylsilylbenzoylperoxycarbonyloxy)cyclohexyl)metha ne,
4-diethylvinylsilylbenzoylperoxy cyclopentyl monocarbonate, 4-diethylvinylsilylbenzoylperoxy cyclohexyl monocarbonate, 4-diethylvinylsilylbenzoylperoxy 2-methylcyclohexyl monocarbonate, 4-diethylvinylsilylbenzoylperoxy 3-methylcyclohexyl monocarbonate, 4-diethylvinylsilylbenzoylperoxy 4-methylcyclohexyl monocarbonate, 4-diethylvinylsilylbenzoylperoxy 2,3-dimethylcyclohexyl monocarbonate,
4-diethylvinylsilylbenzoylperoxy 2,4-dimethylcyclohexyl monocarbonate, 4-diethylvinylsilylbenzoylperoxy 2,5-dimethylcyclohexyl monocarbonate, 4-diethylvinylsilylbenzoylperoxy 2,6-dimethylcyclohexyl monocarbonate, 4-diethylvinylsilylbenzoylperoxy 3,4-dimethylcyclohexyl monocarbonate, 4-diethylvinylsilylbbenzoylperoxy 3,5-dimethylcyclohexyl monocarbonate, 4-diethylvinylsilylbenzoylperoxy 2-ethylcyclohexyl monocarbonate, 4-diethylvinylsilylbenzoylperoxy 4-ethylcyclohexyl monocarbonate, 4-diethylvinylsilylbenzoylperoxy 4-butylcyclohexyl monocarbonate, 4-diethylvinylsilylbenzoylperoxy 3,3,5-trimethylcyclohexyl monocarbonate, 4-diethylvinylsilylbenzoylperoxy 2-tert-butylcyclohexyl monocarbonate,
4-diethylvinylsilylbenzoylperoxy 4-tert-butylcyclohexyl monocarbonate, 4-diethylvinylsilylbenzoylperoxy menthyl monocarbonate, 4-diethylvinylsilylbenzoylperoxy cyclododecyl monocarbonate, di(4-diethylvinylsilylbenzoylperoxy) 1,2-cyclohexanediyldimethylenebiscarbonate, di(4-diethylvinylsilylbenzoylperoxy) 1,4-cyclohexanediyldimethylene biscarbonate, di(4-diethylvinylsilylbenzoylperoxy) 1,2-cyclohexylene biscarbonate, di(4-diethylvinylsilylbenzoylperoxy) 1,3-cyclohexylene biscarbonate, di(4-diethylvinylsilylbenzoylperoxy) 1,4-cyclohexylene biscarbonate, di(4-diethylvinylsilylbenzoylperoxy) 4,4'-bicyclohexylene biscarbonate, bis(4-(4-diethylvinylsilylbenzoylperoxycarbonyloxy)cyclohexyl)methane, 2,2-bis(4-(4-diethylvinylsilylbenzoylperoxycarbonyloxy)cyclohexyl)propane, tris(4-(4-diethylvinylsilylbenzoylperoxycarbonyloxy)cyclohexyl)methane, tetrakis(4-(4-diethylvinylsilylbenzoylperoxycarbonyloxy)cyclohexyl)methane,
4-methyldivinylsilylbenzoylperoxy cyclopentyl monocarbonate, 4-methyldivinylsilylbenzoylperoxy cyclohexyl monocarbonate, 4-methyldivinylsilylbenzoylperoxy 2-methylcyclohexyl monocarbonate, 4-methyldivinylsilylbenzoylperoxy 3-methylcyclohexyl monocarbonate, 4-methyldivinylsilylbenzoylperoxy 4-methylcyclohexyl monocarbonate, 4-methyldivinylsilylbenzoylperoxy 2, 3-dimethylcyclohexyl monocarbonate, 4-methyldivinylsilylbenzoylperoxy 2,4-dimethylcyclohexyl monocarbonate, 4-methyldivinylsilylbenzoylperoxy 2,5-dimethylcyclohexyl monocarbonate, 4-methyldivinylsilylbenzoylperoxy 2,6-dimethylcyclohexyl monocarbonate, 4-methyldivinylsilylbenzoylperoxy 3,4-dimethylcyclohexyl monocarbonate, 4-methyldivinylsilylbbenzoylperoxy 3,5-dimethylcyclohexyl monocarbonate, 4-methyldivinylsilylbenzoylperoxy 2-ethylcyclohexyl monocarbonate, 4-methyldivinylsilylbenzoylperoxy 4-ethylcyclohexyl monocarbonate, 4-methyldivinylsilylbenzoylperoxy 4-butylcyclohexyl monocarbonate, 4-methyldivinylsilylbenzoylperoxy 3,3,5-trimethylcyclohexyl monocarbonate, 4-methyldivinylsilylbenzoylperoxy 2-tert-butylcyclohexyl monocarbonate, 4-methyldivinylsilylbenzoylperoxy 4-tert-butylcyclohexyl monocarbonate, 4-methyldivinylsilylbenzoylperoxy menthyl monocarbonate, 4-methyldivinylsilylbenzoylperoxy cyclododecyl monocarbonate, di(4-methyldivinylsilylbenzoylperoxy) 1,2-cyclohexanediyldimethylene biscarbonate, di(4-methyldivinylsilylbenzoylperoxy) 1,4-cyclohexanediyldimethylene biscarbonate, di(4-methyldivinylsilylbenzoylperoxy) 1,2-cyclohexylene biscarbonate, di(4-methyldivinylsilylbenzoylperoxy) 1,3-cyclohexylene biscarbonate, di(4-methyldivinylsilylbenzoylperoxy) 1,4-cyclohexylene biscarbonate, di(4-methyldivinylsilylbenzoylperoxy) 4,4'-bicyclohexylene biscarbonate, bis(4-(4-methyldivinylsilylbenzoylperoxycarbonyloxy)cyclohexyl)methane, 2,2-bis(4-(4-methyldivinylsilylbenzoylperoxycarbonyloxy)cyclohexyl)propan e, tris(4-(4-methyldivinylsilylbenzoylperoxycarbonyloxy)cyclohexyl)methane, tetrakis(4-(4-methyldivinylsilylbenzoylperoxycarbonyloxy)cyclohexyl)methane, 4-ethyldivinylsilylbenzoylperoxy cyclopentyl monocarbonate, 4-ethyldivinylsilylbenzoylperoxy cyclohexyl monocarbonate, 4-ethyldivinylsilylbenzoylperoxy 2-methylcyclohexyl monocarbonate, 4-ethyldivinylsilylbenzoylperoxy 3-methylcyclohexyl monocarbonate, 4-ethyldivinylsilylbenzoylperoxy 4-methylcyclohexyl monocarbonate, 4-ethyldivinylsilylbenzoylperoxy 2,3-dimethylcyclohexyl monocarbonate, 4-ethyldivinylsilylbenzoylperoxy 2,4-dimethylcyclohexyl monocarbonate, 4-ethyldivinylsilylbenzoylperoxy 2,5-dimethylcyclohexyl monocarbonate, 4-ethyldivinylsilylbenzoylperoxy 2,6-dimethylcyclohexyl monocarbonate, 4-ethyldivinylsilylbenzoylperoxy 3,4-dimethylcyclohexyl monocarbonate, 4-ethyldivinylsilylbbenzoylperoxy 3,5-dimethylcyclohexyl monocarbonate, 4-ethyldivinylsilylbenzoylperoxy 2-ethylcyclohexyl monocarbonate, 4-ethyldivinylsilylbenzoylperoxy 4-ethylcyclohexyl monocarbonate, 4-ethyldivinylsilylbenzoylperoxy 4-butylcyclohexyl monocarbonate, 4-ethyldivinylsilylbenzoylperoxy 3,3,5-trimethylcyclohexyl monocarbonate, 4-ethyldivinylsilylbenzoylperoxy 2-tert-butylcyclohexyl monocarbonate, 4-ethyldivinylsilylbenzoylperoxy 4-tert-butylcyclohexyl monocarbonate, 4-ethyldivinylsilylbenzoylperoxy menthyl monocarbonate, 4-ethyldivinylsilylbenzoylperoxy cyclododecyl monocarbonate, di(4-ethyldivinylsilylbenzoylperoxy) 1,2-cyclohexanediyldimethylene biscarbonate, di(4-ethyldivinylsilylbenzoylperoxy) 1,4-cyclohexanediyldimethylene biscarbonate, di(4-ethyldivinylsilylbenzoylperoxy) 1,2-cyclohexylene biscarbonate, di(4-ethyldivinylsilylbenzoylperoxy) 1,3-cyclohexylene biscarbonate, di (4-ethyldivinylsilylbenzoylperoxy) 1,4-cyclohexylene biscarbonate, di(4-ethyldivinylsilylbenzoylperoxy) 4,4'-bicyclohexylene biscarbonate, bis(4-(4-ethyldivinylsilylbenzoylperoxycarbonyloxy)cyclohexyl)methane, 2,2-bis(4-(4-ethyldivinylsilylbenzoylperoxycarbonyloxy)cyclohexyl)propane, tris(4-(4-ethyldivinylsilylbenzoylperoxycarbonyloxy)cyclohexyl)methane, tetrakis(4-(4-ethyldivinylsilylbenzoylperoxycarbonyloxy)cyclohexyl)methane.

In the case of (c) and (A) including but not limited to the following,
benzoylperoxy 2-methoxyethyl monocarbonate, benzoylperoxy 2-ethoxyethyl monocarbonate, benzoylperoxy 2-propyloxyethyl monocarbonate, benzoylperoxy 2-butoxyethyl monocarbonate, benzoylperoxy 1-methyl-2-methoxyethyl monocarbonate, benzoylperoxy 3-methoxybutyl monocarbonate, benzoylperoxy 3-methoxy-3-methylbutyl monocarbonate, benzoylperoxy 2-tert-butoxy-1-methylethyl monocarbonate, benzoylperoxy 2- (2-ethylhexyloxy)ethyl monocarbonate, dibenzoylperoxy 3-oxapentamethylene biscarbonate, dibenzoylperoxy 3,6-dioxaoctamethylene biscarbonate, dibenzoylperoxy 4-oxaheptamethylene biscarbonate, dibenzoylperoxy 4,8-dioxaundecamethylene biscarbonate.

In the cases of (c) and (B) including but not limited to the following,
2-methylbenzoylperoxy 2-methoxyethyl monocarbonate, 2-methylbenzoylperoxy 2-ethoxyethyl monocarbonate, 2-methylbenzoylperoxy 2-propyloxyethyl monocarbonate, 2-methylbenzoylperoxy 2-butoxyethyl monocarbonate, 2-methylbenzoylperoxy 1-methyl-2-methoxyethyl monocarbonate, 2-methylbenzoylperoxy 3-methoxybutyl monocarbonate, 2-methylbenzoylperoxy 3-methoxy-3-methylbutyl monocarbonate, 2-methylbenzoylperoxy 2-tert-butoxy-1-methylethyl monocarbonate, 2-methylbenzoylperoxy 2-(2-ethylhexyloxy)ethyl monocarbonate, di(2-methylbenzoylperoxy) 3-oxapentamethylene biscarbonate, di(2-methylbenzoylperoxy) 3,6-dioxaoctamethylene biscarbonate, di(2-methylbenzoylperoxy) 4-oxaheptamethylene biscarbonate, di(2-methylbenzoylperoxy) 4,8-dioxaundecamethylene biscarbonate,
3-methylbenzoylperoxy 2-methoxyethyl monocarbonate, 3-methylbenzoylperoxy 2-ethoxyethyl monocarbonate, 3-methylbenzoylperoxy 2-propyloxyethyl monocarbonate, 3-methylbenzoylperoxy 2-butoxyethyl monocarbonate, 3-methylbenzoylperoxy 1-methyl-2-methoxyethyl monocarbonate, 3-methylbenzoylperoxy 3-methoxybutyl monocarbonate, 3-methylbenzoylperoxy 3-methoxy-3-methylbutyl monocarbonate, 3-methylbenzoylperoxy 2-tert-butoxy-1-methylethyl monocarbonate, 3-methylbenzoylperoxy 2-(2-ethylhexyloxy)ethyl monocarbonate, di(3-methylbenzoylperoxy) 3-oxapentamethylene biscarbonate, di(3-methylbenzoylperoxy) 3,6-dioxaoctamethylene biscarbonate, di(3-methylbenzoylperoxy) 4-oxaheptamethylene biscarbonate, di(3-methylbenzoylperoxy) 4,8-dioxaundecamethylene biscarbonate,
4-methylbenzoylperoxy 2-methoxyethyl monocarbonate, 4-methylbenzoylperoxy 2-ethoxyethyl monocarbonate, 4-methylbenzoylperoxy 2-propyloxyethyl monocarbonate, 4-methylbenzoylperoxy 2-butoxyethyl monocarbonate, 4-methylbenzoylperoxy 1-methyl-2-methoxyethyl monocarbonate, 4-methylbenzoylperoxy 3-methoxybutyl monocarbonate, 4-methylbenzoylperoxy 3-methoxy-3-methylbutyl monocarbonate, 4-methylbenzoylperoxy 2-tert-butoxy-1-methylethyl monocarbonate, 4-methylbenzoylperoxy 2-(2-ethylhexyloxy)ethyl monocarbonate, di(4-methylbenzoylperoxy) 3-oxapentamethylene biscarbonate, di(4-methylbenzoylperoxy) 3,6-dioxaoctamethylene biscarbonate, di(4-methylbenzoylperoxy) 4-oxaheptamethylene biscarbonate, di(4-methylbenzoylperoxy) 4,8-dioxaundecamethylene biscarbonate,
4-ethylbenzoylperoxy 2-methoxyethyl monocarbonate, 4-ethylbenzoylperoxy 2-ethoxyethyl monocarbonate, 4-ethylbenzoylperoxy 2-propyloxyethyl monocarbonate, 4-ethylbenzoylperoxy 2-butoxyethyl monocarbonate, 4-ethylbenzoylperoxy 1-methyl-2-methoxyethyl monocarbonate, 4-ethylbenzoylperoxy 3-methoxybutyl monocarbonate, 4-ethylbenzoylperoxy 3-methoxy-3-methylbutyl monocarbonate, 4-ethylbenzoylperoxy 2-tert-butoxy-1-methylethyl monocarbonate, 4-ethylbenzoylperoxy 2-(2-ethylhexyloxy)ethyl monocarbonate, di(4-ethylbenzoylperoxy) 3-oxapentamethylene biscarbonate, di(4-ethylbenzoylperoxy) 3,6-dioxaoctamethylene biscarbonate, di(4-ethylbenzoylperoxy) 4-oxaheptamethylene biscarbonate, di(4-ethylbenzoylperoxy) 4,8-dioxaundecamethylene biscarbonate,
2,3-dimethylbenzoylperoxy 2-ethoxyethyl monocarbonate, 2,3-dimethylbenzoylperoxy 2-propyloxyethyl monocarbonate, 2,3-dimethylbenzoylperoxy 2-butoxyethyl monocarbonate, 2,3-dimethylbenzoylperoxy 1-methyl-2-methoxyethyl monocarbonate, 2,3-dimethylbenzoylperoxy 3-methoxybutyl monocarbonate, 2,3-dimethylbenzoylperoxy 3-methoxy-3-methylbutyl monocarbonate, di(2,3-dimethylbenzoylperoxy) 3-oxapentamethylene biscarbonate, di(2,3-dimethylbenzoylperoxy) 3,6-dioxaoctamethylenebiscarbonate,
2,4-dimethylbenzoylperoxy 2-methoxyethyl monocarbonate, 2,4-dimethylbenzoylperoxy 2-ethoxyethyl monocarbonate, 2,4-dimethylbenzoylperoxy 2-propyloxyethyl monocarbonate, 2,4-dimethylbenzoylperoxy 2-butoxyethyl monocarbonate, 2,4-dimethylbenzoylperoxy 1-methyl-2-methoxyethyl monocarbonate, 2,4-dimethylbenzoylperoxy 3-methoxybutyl monocarbonate, 2,4-dimethylbenzoylperoxy 3-methoxy-3-methylbutyl monocarbonate, 2,4-dimethylbenzoylperoxy 2-tert-butoxy-1-methylethyl monocarbonate, 2,4-dimethylbenzoylperoxy 2-(2-ethylhexyloxy)ethyl monocarbonate, di (2,4-dimethylbenzoylperoxy) 3-oxapentamethylene biscarbonate, di(2,4-dimethylbenzoylperoxy) 3,6-dioxaoctamethylenebiscarbonate, di(2,4-dimethylbenzoylperoxy) 4-oxaheptamethylene biscarbonate, di(2,4-dimethylbenzoylperoxy) 4,8-dioxaundecamethylene biscarbonate,
2,5-dimethylbenzoylperoxy 2-ethoxyethyl monocarbonate, 2,5-dimethylbenzoylperoxy 2-propyloxyethyl monocarbonate, 2,5-dimethylbenzoylperoxy 2-butoxyethyl monocarbonate, 2,5-dimethylbenzoylperoxy 1-methyl-2-methoxyethyl monocarbonate, 2,5-dimethylbenzoylperoxy 3-methoxybutyl monocarbonate, 2,5-dimethylbenzoylperoxy 3-methoxy-3-methylbutyl monocarbonate, di(2,5-dimethylbenzoylperoxy) 3-oxapentamethylene biscarbonate, di(2,5-dimethylbenzoylperoxy) 3,6-dioxaoctamethylenebiscarbonate,
2,6-dimethylbenzoylperoxy 2-ethoxyethyl monocarbonate, 2,6-dimethylbenzoylperoxy 2-propyloxyethyl monocarbonate, 2,6-dimethylbenzoylperoxy 2-butoxyethyl monocarbonate, 2,6-dimethylbenzoylperoxy 1-methyl-2-methoxyethyl monocarbonate, 2,6-dimethylbenzoylperoxy 3-methoxybutyl monocarbonate, 2,6-dimethylbenzoylperoxy 3-methoxy-3-methylbutyl monocarbonate, di(2,6-dimethylbenzoylperoxy) 3-oxapentamethylene biscarbonate, di(2,6-dimethylbenzoylperoxy) 3,6-dioxaoctamethylenebiscarbonate,
3,4-dimethylbenzoylperoxy 2-ethoxyethyl monocarbonate, 3,4-dimethylbenzoylperoxy 2-propyloxyethyl monocarbonate, 3,4-dimethylbenzoylperoxy 2-butoxyethyl monocarbonate, 3,4-dimethylbenzoylperoxy 1-methyl-2-methoxyethyl monocarbonate, 3,4-dimethylbenzoylperoxy 3-methoxybutyl monocarbonate, 3,4-dimethylbenzoylperoxy 3-methoxy-3-methylbutyl monocarbonate, di(3,4-dimethylbenzoylperoxy) 3-oxapentamethylene biscarbonate, di(3,4-dimethylbenzoylperoxy) 3,6-dioxaoctamethylenebiscarbonate,
3,5-dimethylbenzoylperoxy 2-ethoxyethyl monocarbonate, 3,5-dimethylbenzoylperoxy 2-propyloxyethyl monocarbonate, 3,5-dimethylbenzoylperoxy 2-butoxyethyl monocarbonate, 3,5-dimethylbenzoylperoxy 1-methyl-2-methoxyethyl monocarbonate, 3,5-dimethylbenzoylperoxy 3-methoxybutyl monocarbonate, 3,5-dimethylbenzoylperoxy 3-methoxy-3-methylbutyl monocarbonate, di(3,5-dimethylbenzoylperoxy) 3-oxapentamethylene biscarbonate, di(3,5-dimethylbenzoylperoxy) 3,6-dioxaoctamethylenebiscarbonate,
4-n-propylbenzoylperoxy 2-methoxyethyl monocarbonate, 4-n-propylbenzoylperoxy 2-ethoxyethyl monocarbonate, 4-n-propylbenzoylperoxy 2-propyloxyethyl monocarbonate, 4-n-propylbenzoylperoxy 2-butoxyethyl monocarbonate, 4-n-propylbenzoylperoxy 1-methyl-2-methoxyethyl monocarbonate, 4-n-propylbenzoylperoxy 3-methoxybutyl monocarbonate, 4-n-propylbenzoylperoxy 3-methoxy-3-methylbutyl monocarbonate, 4-n-propylbenzoylperoxy 2-tert-butoxy-1-methylethyl monocarbonate, 4-n-propylbenzoylperoxy 2-(2-ethylhexyloxy)ethyl monocarbonate, di(4-n-propylbenzoylperoxy) 3-oxapentamethylene biscarbonate, di(4-n-propylbenzoylperoxy) 3,6-dioxaoctamethylenebiscarbonate, di(4-n-propylbenzoylperoxy) 4-oxaheptamethylene biscarbonate, di(4-n-propylbenzoylperoxy) 4,8-dioxaundecamethylene biscarbonate,
4-isopropylbenzoylperoxy 2-methoxyethyl monocarbonate, 4-isopropylbenzoylperoxy 2-ethoxyethyl monocarbonate, 4-isopropylbenzoylperoxy 2-propyloxyethyl monocarbonate, 4-isopropylbenzoylperoxy 2-butoxyethyl monocarbonate, 4-isopropylbenzoylperoxy 1-methyl-2-methoxyethyl monocarbonate, 4-isopropylbenzoylperoxy 3-methoxybutyl monocarbonate, 4-isopropylbenzoylperoxy 3-methoxy-3-methylbutyl monocarbonate, 4-isopropylbenzoylperoxy 2-tert-butoxy-1-methylethyl monocarbonate, 4-isopropylbenzoylperoxy 2-(2-ethylhexyloxy)ethyl monocarbonate, di(4-isopropylbenzoylperoxy) 3-oxapentamethylene biscarbonate, di(4-isopropylbenzoylperoxy) 3,6-dioxaoctamethylene biscarbonate, di(4-isopropylbenzoylperoxy) 4-oxaheptamethylene biscarbonate, di(4-isopropylbenzoylperoxy) 4,8-dioxaundecamethylene biscarbonate,
2,4,6-trimethylbenzoylperoxy 2-ethoxyethyl monocarbonate, 2,4,6-trimethylbenzoylperoxy 2-propyloxyethyl monocarbonate, 2,4,6-trimethylbenzoylperoxy 2-butoxyethyl monocarbonate, 2,4,6-trimethylbenzoylperoxy 1-methyl-2-methoxyethyl monocarbonate, 2,4,6-trimethylbenzoylperoxy 3-methoxybutyl monocarbonate, 2,4,6-trimethylbenzoylperoxy 3-methoxy-3-methylbutyl monocarbonate, di(2,4,6-trimethylbenzoylperoxy) 3-oxapentamethylene biscarbonate, di(2,4,6-trimethylbenzoylperoxy) 3,6-dioxaoctamethylene biscarbonate,
4-n-butylbenzoylperoxy 2-methoxyethyl monocarbonate, 4-n-butylbenzoylperoxy 2-ethoxyethyl monocarbonate, 4-n-butylbenzoylperoxy 2-propyloxyethyl monocarbonate, 4-n-butylbenzoylperoxy 2-butoxyethyl monocarbonate, 4-n-butylbenzoylperoxy 1-methyl-2-methoxyethyl monocarbonate, 4-n-butylbenzoylperoxy 3-methoxybutyl monocarbonate, 4-n-butylbenzoylperoxy 3-methoxy-3-methylbutyl monocarbonate, 4-n-butylbenzoylperoxy 2-tert-butoxy-1-methylethyl monocarbonate, 4-n-butylbenzoylperoxy 2-(2-ethylhexyloxy)ethyl monocarbonate, di(4-n-butylbenzoylperoxy) 3-oxapentamethylene biscarbonate, di(4-n-butylbenzoylperoxy) 3,6-dioxaoctamethylene biscarbonate, di(4-n-butylbenzoylperoxy) 4-oxaheptamethylene biscarbonate, di(4-n-butylbenzoylperoxy) 4,8-dioxaundecamethylene biscarbonate,
4-tert-butylbenzoylperoxy 2-methoxyethyl monocarbonate, 4-tert-butylbenzoylperoxy 2-ethoxyethyl monocarbonate, 4-tert-butylbenzoylperoxy 2-propyloxyethyl monocarbonate, 4-tert-butylbenzoylperoxy 2-butoxyethyl monocarbonate, 4-tert-butylbenzoylperoxy 1-methyl-2-methoxyethyl monocarbonate, 4-tert-butylbenzoylperoxy 3-methoxybutyl monocarbonate, 4-tert-butylbenzoylperoxy 3-methoxy-3-methylbutyl monocarbonate, 4-tert-butylbenzoylperoxy 2-tert-butoxy-1-methylethyl monocarbonate, 4-tert-butylbenzoylperoxy 2-(2-ethylhexyloxy)ethyl monocarbonate, di(4-tert-butylbenzoylperoxy) 3-oxapentamethylene biscarbonate, di(4-tert-butylbenzoylperoxy) 3,6-dioxaoctamethylene biscarbonate, di(4-tert-butylbenzoylperoxy) 4-oxaheptamethylene biscarbonate, di(4-tert-butylbenzoylperoxy) 4,8-dioxaundecamethylene biscarbonate,
4-n-pentylbenzoylperoxy 2-methoxyethyl monocarbonate, 4-n-pentylbenzoylperoxy 2-ethoxyethyl monocarbonate, 4-n-pentylbenzoylperoxy 2-propyloxyethyl monocarbonate, 4-n-pentylbenzoylperoxy 2-butoxyethyl monocarbonate, 4-n-pentylbenzoylperoxyl-methyl-2-methoxyethylmonocarbonate, 4-n-pentylbenzoylperoxy 3-methoxybutyl monocarbonate, 4-n-pentylbenzoylperoxy 3-methoxy-3-methylbutyl monocarbonate, 4-n-pentylbenzoylperoxy 2-tert-butoxy-1-methylethyl monocarbonate, 4-n-pentylbenzoylperoxy 2-(2-ethylhexyloxy)ethyl monocarbonate, di(4-n-pentylbenzoylperoxy) 3-oxapentamethylene biscarbonate, di(4-n-pentylbenzoylperoxy) 3,6-dioxaoctamethylenebiscarbonate, di(4-n-pentylbenzoylperoxy) 4-oxaheptamethylene biscarbonate, di(4-n-pentylbenzoylperoxy) 4,8-dioxaundecamethylene biscarbonate,
2,3,4,5,6-pentamethylbenzoylperoxy 2-ethoxyethyl monocarbonate, 2,3,4,5,6-pentamethylbenzoylperoxy 2-propyloxyethyl monocarbonate, 2,3,4,5,6-pentamethylbenzoylperoxy 2-butoxyethyl monocarbonate, 2,3,4,5,6-pentamethylbenzoylperoxy 1-methyl-2-methoxyethyl monocarbonate, 2,3,4,5,6-pentamethylbenzoylperoxy 3-methoxybutyl monocarbonate, 2,3,4,5,6-pentamethylbenzoylperoxy 3-methoxy-3-methylbutyl monocarbonate, di(2,3,4,5,6-pentamethylbenzoylperoxy) 3-oxapentamethylene biscarbonate, di(2,3,4,5,6-penta-methylbenzoylperoxy) 3,6-dioxaoctamethylene biscarbonate,
4-n-hexylbenzoylperoxy 2-methoxyethyl monocarbonate, 4-n-hexylbenzoylperoxy 2-ethoxyethyl monocarbonate, 4-n-hexylbenzoylperoxy 2-propyloxyethyl monocarbonate, 4-n-hexylbenzoylperoxy 2-butoxyethyl monocarbonate, 4-n-hexylbenzoylperoxyl-methyl-2-methoxyethyl monocarbonate, 4-n-hexylbenzoylperoxy 3-methoxybutyl monocarbonate, 4-n-hexylbenzoylperoxy 3-methoxy-3-methylbutyl monocarbonate, 4-n-hexylbenzoylperoxy 2-tert-butoxy-1-methylethyl monocarbonate, 4-n-hexylbenzoylperoxy 2-(2-ethylhexyloxy)ethyl monocarbonate, di(4-n-hexylbenzoylperoxy) 3-oxapentamethylene biscarbonate, di(4-n-hexylbenzoylperoxy) 3,6-dioxaoctamethylene biscarbonate, di(4-n-hexylbenzoylperoxy) 4-oxaheptamethylene biscarbonate, di(4-n-hexylbenzoylperoxy) 4,8-dioxaundecamethylene biscarbonate,
2-methoxybenzoylperoxy 2-methoxyethyl monocarbonate, 2-methoxybenzoylperoxy 2-ethoxyethyl monocarbonate, 2-methoxybenzoylperoxy 2-propyloxyethyl monocarbonate, 2-methoxybenzoylperoxy 2-butoxyethyl monocarbonate, 2-methoxybenzoylperoxy1-methyl-2-methoxyethylmonocarbonate, 2-methoxybenzoylperoxy 3-methoxybutyl monocarbonate, 2-methoxybenzoylperoxy 3-methoxy-3-methylbutyl monocarbonate, 2-methoxybenzoylperoxy 2-tert-butoxy-1-methylethyl monocarbonate, 2-methoxybenzoylperoxy 2-(2-ethylhexyloxy)ethyl monocarbonate, di(2-methoxybenzoylperoxy) 3-oxapentamethylene biscarbonate, di(2-methoxybenzoylperoxy) 3,6-dioxaoctamethylene biscarbonate, di(2-methoxybenzoylperoxy) 4-oxaheptamethylene biscarbonate, di(2-methoxybenzoylperoxy) 4,8-dioxaundecamethylene biscarbonate,
3-methoxybenzoylperoxy 2-ethoxyethyl monocarbonate, 3-methoxybenzoylperoxy 2-propyloxyethyl monocarbonate, 3-methoxybenzoylperoxy 2-butoxyethyl monocarbonate, 3-methoxybenzoylperoxy 1-methyl-2-methoxyethyl monocarbonate, 3-methoxybenzoylperoxy 3-methoxybutyl monocarbonate, 3-methoxybenzoylperoxy 3-methoxy-3-methylbutyl monocarbonate, di(3-methoxybenzoylperoxy) 3-oxapentamethylene biscarbonate, di(3-methoxybenzoylperoxy) 3,6-dioxaoctamethylene biscarbonate,
4-methoxybenzoylperoxy 2-methoxyethyl monocarbonate, 4-methoxybenzoylperoxy 2-ethoxyethyl monocarbonate, 4-methoxybenzoylperoxy 2-propyloxyethyl monocarbonate, 4-methoxybenzoylperoxy 2-butoxyethyl monocarbonate, 4-methoxybenzoylperoxy 1-methyl-2-methoxyethyl monocarbonate, 4-methoxybenzoylperoxy 3-methoxybutyl monocarbonate, 4-methoxybenzoylperoxy 3-methoxy-3-methylbutyl monocarbonate, 4-methoxybenzoylperoxy 2-tert-butoxy-1-methylethyl monocarbonate, 4-methoxybenzoylperoxy 2-(2-ethylhexyloxy)ethyl monocarbonate, di(4-methoxybenzoylperoxy) 3-oxapentamethylene biscarbonate, di(4-methoxybenzoylperoxy) 3,6-dioxaoctamethylene biscarbonate, di(4-methoxybenzoylperoxy) 4-oxaheptamethylene biscarbonate, di(4-methoxybenzoylperoxy)-4,8-dioxaundecanmethylene biscarbonate,
2-ethoxybenzoylperoxy 2-ethoxyethyl monocarbonate, 2-ethoxybenzoylperoxy 2-propyloxyethyl monocarbonate, 2-ethoxybenzoylperoxy 2-butoxyethyl monocarbonate, 2-ethoxybenzoylperoxy 1-methyl-2-methoxyethyl monocarbonate, 2-ethoxybenzoylperoxy 3-methoxybutyl monocarbonate, 2-ethoxybenzoylperoxy 3-methoxy-3-methylbutyl monocarbonate, di(2-ethoxybenzoylperoxy) 3-oxapentamethylene biscarbonate, di(2-ethoxybenzoylperoxy) 3,6-dioxaoctamethylene biscarbonate,
3-ethoxybenzoylperoxy 2-ethoxyethyl monocarbonate, 3-ethoxybenzoylperoxy 2-propyloxyethyl monocarbonate, 3-ethoxybenzoylperoxy 2-butoxyethyl monocarbonate, 3-ethoxybenzoylperoxy 1-methyl-2-methoxyethyl monocarbonate, 3-ethoxybenzoylperoxy 3-methoxybutyl monocarbonate, 3-ethoxybenzoylperoxy 3-methoxy-3-methylbutyl monocarbonate, di(3-ethoxybenzoylperoxy) 3-oxapentamethylene biscarbonate, di(3-ethoxybenzoylperoxy) 3,6-dioxaoctamethylene biscarbonate, 4-ethoxybenzoylperoxy 2-ethoxyethyl monocarbonate, 4-ethoxybenzoylperoxy 2-propyloxyethyl monocarbonate, 4-ethoxybenzoylperoxy 2-butoxyethyl monocarbonate, 4-ethoxybenzoylperoxy 1-methyl-2-methoxyethyl monocarbonate, 4-ethoxybenzoylperoxy 3-methoxybutyl monocarbonate, 4-ethoxybenzoylperoxy 3-methoxy-3-methylbutyl monocarbonate, di(4-ethoxybenzoylperoxy) 3-oxapentamethylene biscarbonate, di(4-ethoxybenzoylperoxy) 3,6-dioxaoctamethylene biscarbonate,
2,3-dimethoxybenzoylperoxy 2-ethoxyethyl monocarbonate, 2,3-dimethoxybenzoylperoxy 2-propyloxyethyl monocarbonate, 2,3-dimethoxybenzoylperoxy 2-butoxyethyl monocarbonate, 2,3-dimethoxybenzoylperoxy 1-methyl-2-methoxyethyl monocarbonate, 2,3-dimethoxybenzoylperoxy 3-methoxybutyl monocarbonate, 2,3-dimethoxybenzoylperoxy 3-methoxy-3-methylbutyl monocarbonate, di(2,3-dimethoxybenzoylperoxy) 3-oxapentamethylene biscarbonate, di(2,3-dimethoxybenzoylperoxy) 3,6-dioxaoctamethylene biscarbonate,
2,4-dimethoxybenzoylperoxy 2-ethoxyethyl monocarbonate, 2,4-dimethoxybenzoylperoxy 2-propyloxyethyl monocarbonate, 2,4-dimethoxybenzoylperoxy 2-butoxyethyl monocarbonate, 2,4-dimethoxybenzoylperoxy 1-methyl-2-methoxyethyl monocarbonate, 2,4-dimethoxybenzoylperoxy 3-methoxybutyl monocarbonate, 2,4-dimethoxybenzoylperoxy 3-methoxy-3-methylbutyl monocarbonate, di(2,4-dimethoxybenzoylperoxy) 3-oxapentamethylene biscarbonate, di(2,4-dimethoxybenzoylperoxy) 3,6-dioxaoctamethylene biscarbonate,
2,5-dimethoxybenzoylperoxy 2-ethoxyethyl monocarbonate, 2,5-dimethoxybenzoylperoxy 2-propyloxyethyl monocarbonate, 2,5-dimethoxybenzoylperoxy 2-butoxyethyl monocarbonate, 2,5-dimethoxybenzoylperoxy 1-methyl-2-methoxyethyl monocarbonate, 2,5-dimethoxybenzoylperoxy 3-methoxybutyl monocarbonate, 2,5-dimethoxybenzoylperoxy 3-methoxy-3-methylbutyl monocarbonate, di(2,5-dimethoxybenzoylperoxy) 3-oxapentamethylene biscarbonate, di(2,5-dimethoxybenzoylperoxy) 3,6-dioxaoctamethylene biscarbonate,
2,6-dimethoxybenzoylperoxy 2-ethoxyethyl monocarbonate, 2,6-dimethoxybenzoylperoxy 2-propyloxyethyl monocarbonate, 2,6-dimethoxybenzoylperoxy 2-butoxyethyl monocarbonate, 2,6-dimethoxybenzoylperoxy 1-methyl-2-methoxyethyl monocarbonate, 2,6-dimethoxybenzoylperoxy 3-methoxybutyl monocarbonate, 2,6-dimethoxybenzoylperoxy 3-methoxy-3-methylbutyl monocarbonate, di(2,6-dimethoxybenzoylperoxy) 3-oxapentamethylene biscarbonate, di(2,6-dimethoxybenzoylperoxy) 3,6-dioxaoctamethylene biscarbonate,
3,4-dimethoxybenzoylperoxy 2-ethoxyethyl monocarbonate, 3,4-dimethoxybenzoylperoxy 2-propyloxyethyl monocarbonate, 3,4-dimethoxybenzoylperoxy 2-butoxyethyl monocarbonate, 3,4-dimethoxybenzoylperoxy 1-methyl-2-methoxyethyl monocarbonate, 3,4-dimethoxybenzoylperoxy 3-methoxybutyl monocarbonate, 3,4-dimethoxybenzoylperoxy 3-methoxy-3-methylbutyl monocarbonate, di(3,4-dimethoxybenzoylperoxy) 3-oxapentamethylene biscarbonate, di(3,4-dimethoxybenzoylperoxy) 3,6-dioxaoctamethylene biscarbonate,
3,5-dimethoxybenzoylperoxy-2-etoxyethyl monocarbonate, 3,5-dimethoxybenzoylperoxy 2-propyloxyethyl monocarbonate, 3,5-dimethoxybenzoylperoxy 2-butoxyethyl monocarbonate, 3,5-dimethoxybenzoylperoxy 1-methyl-2-methoxyethyl monocarbonate, 3,5-dimethoxybenzoylperoxy 3-methoxybutyl monocarbonate, 3,5-dimethoxybenzoylperoxy 3-methoxy-3-methylbutyl monocarbonate, di(3,5-dimethoxybenzoylperoxy) 3-oxapentamethylene biscarbonate, di(3,5-dimethoxybenzoylperoxy) 3,6-dioxaoctamethylene biscarbonate,
4-n-propyloxybenzoylperoxy 2-ethoxyethyl monocarbonate, 4-n-propyloxybenzoylperoxy 2-propyloxyethyl monocarbonate, 4-n-propyloxybenzoylperoxy 2-butoxyethyl monocarbonate, 4-n-propyloxybenzoylperoxy 1-methyl-2-methoxyethyl monocarbonate, 4-n-propyloxybenzoylperoxy 3-methoxybutyl monocarbonate, 4-n-propyloxybenzoylperoxy 3-methoxy-3-methylbutyl monocarbonate, di(4-n-propyloxybenzoylperoxy) 3-oxapentamethylene biscarbonate, di(4-n-propyloxybenzoylperoxy) 3,6-dioxaoctamethylene biscarbonate,
4-isopropyloxybenzoylperoxy 2-ethoxyethyl monocarbonate, 4-isopropyloxybenzoylperoxy 2-propyloxyethyl monocarbonate, 4-isopropyloxybenzoylperoxy 2-butoxyethyl monocarbonate, 4-isopropyloxybenzoylperoxy 1-methyl-2-methoxyethyl monocarbonate, 4-isopropyloxybenzoylperoxy 3-methoxybutyl monocarbonate, 4-isopropyloxybenzoylperoxy 3-methoxy-3-methylbutyl monocarbonate, di(4-isopropyloxybenzoylperoxy) 3-oxapentamethylene biscarbonate, di(4-isopropyloxybenzoylperoxy) 3,6-dioxaoctamethylene biscarbonate,
2,3,4-trimethoxybenzoylperoxy 2-ethoxyethyl monocarbonate, 2,3,4-trimethoxybenzoylperoxy 2-propyloxyethyl monocarbonate, 2,3,4-trimethoxybenzoylperoxy 2-butoxyethyl monocarbonate, 2,3,4-trimethoxybenzoylperoxy 1-methyl-2-methoxyethyl monocarbonate, 2,3,4-trimethoxybenzoylperoxy 3-methoxybutyl monocarbonate, 2,3,4-trimethoxybenzoylperoxy 3-methoxy-3-methylbutyl monocarbonate, di(2,3,4-trimethoxybenzoylperoxy) 3-oxapentamethylene biscarbonate, di(2,3,4-trimethoxybenzoylperoxy) 3,6-dioxaoctamethylene biscarbonate,
2,4,5-trimethoxybenzoylperoxy 2-ethoxyethyl monocarbonate, 2,4,5-trimethoxybenzoylperoxy 2-propyloxyethyl monocarbonate, 2,4,5-trimethoxybenzoylperoxy 2-butoxyethyl monocarbonate, 2,4,5-trimethoxybenzoylperoxy 1-methyl-2-methoxyethyl monocarbonate, 2,4,5-trimethoxybenzoylperoxy 3-methoxybutyl monocarbonate, 2,4,5-trimethoxybenzoylperoxy 3-methoxy-3-methylbutyl monocarbonate, di(2,4,5-trimethoxybenzoylperoxy) 3-oxapentamethylene biscarbonate, di(2,4,5-trimethoxybenzoylperoxy) 3,6-dioxaoctamethylene biscarbonate,
3,4,5-trimethoxybenzoylperoxy 2-ethoxyethyl monocarbonate, 3,4,5-trimethoxybenzoylperoxy 2-propyloxyethyl monocarbonate, 3,4,5-trimethoxybenzoylperoxy 2-butoxyethyl monocarbonate, 3,4,5-trimethoxybenzoylperoxy 1-methyl-2-methoxyethyl monocarbonate, 3,4,5-tri methoxybenzoylperoxy 3-methoxybutyl monocarbonate, 3,4,5-trimethoxybenzoylperoxy 3-methoxy-3-methylbutyl monocarbonate, di(3,4,5-trimethoxybenzoylperoxy) 3-oxapentamethylene biscarbonate, di(3,4,5-trimethoxybenzoylperoxy) 3,6-dioxaoctamethylene biscarbonate,
4-n-butoxybenzoylperoxy 2-ethoxyethyl monocarbonate, 4-n-butoxybenzoylperoxy 2-propyloxyethyl monocarbonate, 4-n-butoxybenzoylperoxy 2-butoxyethyl monocarbonate, 4-n-butoxybenzoylperoxy 1-methyl-2-methoxyethyl monocarbonate, 4-n-butoxybenzoylperoxy 3-methoxybutyl monocarbonate, 4-n-butoxybenzoylperoxy 3-methoxy-3-methylbutyl monocarbonate, di(4-n-butoxybenzoylperoxy) 3-oxapentamethylene biscarbonate, di(4-n-butoxybenzoylperoxy) 3,6-dioxaoctamethylene biscarbonate,
4-n-hexyloxybenzoylperoxy 2-ethoxyethyl monocarbonate, 4-n-hexyloxybenzoylperoxy 2-propyloxyethyl monocarbonate, 4-n-hexyloxybenzoylperoxy 2-butoxyethyl monocarbonate, 4-n-hexyloxybenzoylperoxy 1-methyl-2-methoxyethyl monocarbonate, 4-n-hexyloxybenzoylperoxy 3-methoxybutyl monocarbonate, 4-n-hexyloxybenzoylperoxy 3-methoxy-3-methylbutyl monocarbonate, di(4-n-hexyloxybenzoylperoxy) 3-oxapentamethylene biscarbonate, di(4-n-hexyloxybenzoylperoxy) 3,6-dioxaoctamethylene biscarbonate,
2-phenylbenzoylperoxy 2-ethoxyethyl monocarbonate, 2-phenylbenzoylperoxy 2-propyloxyethyl monocarbonate, 2-phenylbenzoylperoxy 2-butoxyethyl monocarbonate, 2-phenylbenzoylperoxy 1-methyl-2-methoxyethyl monocarbonate, 2-phenylbenzoylperoxy 3-methoxybutyl monocarbonate, 2-phenylbenzoylperoxy 3-methoxy-3-methylbutyl monocarbonate, di(2-phenylbenzoylperoxy) 3-oxapentamethylene biscarbonate, di(2-phenylbenzoylperoxy) 3,6-dioxaoctamethylene biscarbonate,
3-phenylbenzoylperoxy 2-ethoxyethyl monocarbonate, 3-phenylbenzoylperoxy 2-propyloxyethyl monocarbonate, 3-phenylbenzoylperoxy 2-butoxyethyl monocarbonate, 3-phenylbenzoylperoxy 1-methyl-2-methoxyethyl monocarbonate, 3-phenylbenzoylperoxy 3-methoxybutyl monocarbonate, 3-phenylbenzoylperoxy 3-methoxy-3-methylbutyl monocarbonate, di(3-phenylbenzoylperoxy) 3-oxapentamethylene biscarbonate, di(3-phenylbenzoylperoxy) 3,6-dioxaoctamethylene biscarbonate,
4-phenylbenzoylperoxy 2-methoxyethyl monocarbonate, 4-phenylbenzoylperoxy 2-ethoxyethyl monocarbonate, 4-phenylbenzoylperoxy 2-propyloxyethyl monocarbonate, 4-phenylbenzoylperoxy 2-butoxyethyl monocarbonate, 4-phenylbenzoylperoxy 1-methyl-2-methoxyethyl monocarbonate, 4-phenylbenzoylperoxy 3-methoxybutyl monocarbonate, 4-phenylbenzoylperoxy 3-methoxy-3-methylbutyl monocarbonate, 4-phenylbenzoylperoxy 2-tert-butoxy-1-methylethyl monocarbonate, 4-phenylbenzoylperoxy 2-(2-ethylhexyloxy)ethyl monocarbonate, di(4-phenylbenzoylperoxy) 3-oxapentamethylene biscarbonate, di(4-phenylbenzoylperoxy) 3,6-dioxaoctamethylene biscarbonate, di(4-phenylbenzoylperoxy) 4-oxaheptamethylene biscarbonate, di(4-phenylbenzoylperoxy) 4,8-dioxaundecamethylene biscarbonate.

In the case of (c) and (C) including but not limited to the following, 4-trimethylsilylbenzoylperoxy 2-methoxyethyl monocarbonate, 4-trimethylsilylbenzoylperoxy 2-ethoxyethyl monocarbonate, 4-trimethylsilylbenzoylperoxy 2-propyloxyethyl monocarbonate, 4-trimethylsilylbenzoylperoxy 2-butoxyethyl monocarbonate, 4-trimethylsilylbenzoylperoxy 1-methyl-2-methoxyethyl monocarbonate, 4-trimethylsilylbenzoylperoxy 3-methoxybutyl monocarbonate, 4-trimethylsilylbenzoylperoxy 3-methoxy-3-methylbutyl monocarbonate, 4-trimethylsilylbenzoylperoxy 2-tert-butoxy-1-methylethyl monocarbonate, 4-trimethylsilylbenzoylperoxy 2-(2-ethylhexyloxy)ethyl monocarbonate, di(4-trimethylsilylbenzoylperoxy) 3-oxapentamethylene biscarbonate, di(4-trimethylsilylbenzoylperoxy) 3,6-dioxaoctamethylene biscarbonate, di(4-trimethylsilylbenzoylperoxy) 4-oxaheptamethylene biscarbonate, di(4-trimethylsilylbenzoylperoxy) 4,8-dioxaundecamethylene biscarbonate,
4-dimethylvinylsilylbenzoylperoxy 2-methoxyethyl monocarbonate, 4-dimethylvinylsilylbenzoylperoxy 2-ethoxyethyl monocarbonate, 4-dimethylvinylsilylbenzoylperoxy 2-propyloxyethyl monocarbonate, 4-dimethylvinylsilylbenzoylperoxy 2-butoxyethyl monocarbonate, 4-dimethylvinylsilylbenzoylperoxy 1-methyl-2-methoxyethyl monocarbonate, 4-dimethylvinylsilylbenzoylperoxy 3-methoxybutyl monocarbonate, 4-dimethylvinylsilylbenzoylperoxy 3-methoxy-3-methylbutyl monocarbonate, 4-dimethylvinylsilylbenzoylperoxy 2-tert-butoxy-1-methylethyl monocarbonate, 4-dimethylvinylsilylbenzoylperoxy 2-(2-ethylhexyloxy)ethyl monocarbonate, di(4-dimethylvinylsilylbenzoylperoxy) 3-oxapentamethylene biscarbonate, di(4-dimethylvinylsilylbenzoylperoxy) 3,6-dioxaoctamethylene biscarbonate, di(4-dimethylvinylsilylbenzoylperoxy) 4-oxaheptamethylene biscarbonate, di(4-dimethylvinylsilylbenzoylperoxy) 4,8-dioxaundecamethylene biscarbonate,
4-diethylvinylsilylbenzoylperoxy 2-methoxyethyl monocarbonate, 4-diethylvinylsilylbenzoylperoxy 2-ethoxyethyl monocarbonate, 4-diethylvinylsilylbenzoylperoxy 2-propyloxyethyl monocarbonate, 4-diethylvinylsilylbenzoylperoxy 2-butoxyethyl monocarbonate, 4-diethylvinylsilylbenzoylperoxy 1-methyl-2-methoxyethyl monocarbonate, 4-diethylvinylsilylbenzoylperoxy 3-methoxybutyl monocarbonate, 4-diethylvinylsilylbenzoylperoxy 3-methoxy-3-methylbutyl monocarbonate, 4-diethylvinylsilylbenzoylperoxy 2-tert-butoxy-1-methylethyl monocarbonate, 4-diethylvinylsilylbenzoylperoxy 2-(2-ethylhexyloxy)ethyl monocarbonate, di(4-diethylvinylsilylbenzoylperoxy) 3-oxapentamethylene biscarbonate, di(4-diethylvinylsilylbenzoylperoxy) 3,6-dioxaoctamethylene biscarbonate, di(4-diethylvinylsilylbenzoylperoxy) 4-oxaheptamethylene biscarbonate, di(4-diethylvinylsilylbenzoylperoxy) 4,8-dioxaundecamethylene biscarbonate,
4-methyldivinylsilylbenzoylperoxy 2-methoxyethyl monocarbonate, 4-methyldivinylsilylbenzoylperoxy 2-ethoxyethyl monocarbonate, 4-methyldivinylsilylbenzoylperoxy 2-propyloxyethyl monocarbonate, 4-methyldivinylsilylbenzoylperoxy 2-butoxyethyl monocarbonate, 4-methyldivinylsilylbenzoylperoxy 1-methyl-2-methoxyethyl monocarbonate, 4-methyldivinylsilylbenzoylperoxy 3-methoxybutyl monocarbonate, 4-methyldivinylsilylbenzoylperoxy 3-methoxy-3-methylbutyl monocarbonate, 4-methyldivinylsilylbenzoylperoxy 2-tert-butoxy-1-methylethyl monocarbonate, 4-methyldivinylsilylbenzoylperoxy 2-(2-ethylhexyloxy)ethyl monocarbonate, di(4-methyldivinylsilylbenzoylperoxy) 3-oxapentamethylene biscarbonate, di(4-methyldivinylsilylbenzoylperoxy) 3,6-dioxaoctamethylene biscarbonate, di(4-methyldivinylsilylbenzoylperoxy) 4-oxaheptamethylene biscarbonate, di(4-methyldivinylsilylbenzoylperoxy) 4,8-dioxaundecamethylene biscarbonate,
4-ethyldivinylsilylbenzoylperoxy 2-methoxyethyl monocarbonate, 4-ethyldivinylsilylbenzoylperoxy 2-ethoxyethyl monocarbonate, 4-ethyldivinylsilylbenzoylperoxy 2-propyloxyethyl monocarbonate, 4-ethyldivinylsilylbenzoylperoxy 2-butoxyethyl monocarbonate, 4-ethyldivinylsilylbenzoylperoxy 1-methyl-2-methoxyethyl monocarbonate, 4-ethyldivinylsilylbenzoylperoxy 3-methoxybutyl monocarbonate, 4-ethyldivinylsilylbenzoylperoxy 3-methoxy-3-methylbutyl monocarbonate, 4-ethyldivinylsilylbenzoylperoxy 2-tert-butoxy-1-methylethyl monocarbonate, 4-ethyldivinylsilylbenzoylperoxy 2-(2-ethylhexyloxy)ethyl monocarbonate, di(4-ethyldivinylsilylbenzoylperoxy)-3-oxapentamethylen biscarbonate, di(4-ethyldivinylsilylbenzoylperoxy) 3,6-dioxaoctamethylene biscarbonate, di(4-ethyldivinylsilylbenzoylperoxy)-4-oxaheptamethylen biscarbonate, di(4-ethyldivinylsilylbenzoylperoxy) 4,8-dioxaundecamethylene biscarbonate.

The present invention isn't limited by the above illustration.

The above asymmetry organic peroxide having the specific structure of the present invention can be obtained by any well-known method.

For example, the reaction of (substituted)benzoyl chloride and alkyl chloroformate with sodium peroxide, the reaction of aldehyde and alkyl chloroformate in the oxygen under the existence of basic compounds, the reaction of alkali salt of (substituted)perbenzoic acid and alkyl chloroformate, the reaction of (substituted)perbenzoic acid and alkyl chloroformate under the existence of basic compounds, and so on are applicable. (Substituted) perbenzoic acid used in the above several methods can be obtained easily by the methods reported in L.S.Silbert, E.Siegel, D.Swern, *J.Org.Chem.,* **27,** 1336 (1962), or in J.R.Moyer, N.C.Manley, *J.Org.Chem.,* **29,** 2099 (1964), and so on.

The method of synthesizing the asymmetry organic peroxide of the present invention isn't limited to the above.

The asymmetry organic peroxide of the present invention may be used singly or as a mixture of two or more kinds of the asymmetry organic peroxides. The mixture of two or more kinds of the asymmetry organic peroxides can be obtained by mixing asymmetry organic peroxideds after preparing each of them independently, or by preparing two or more kinds of the asymmetry organic peroxides simultaneously, for example, by the reaction using a mixture of two or more kinds of (substituted) benzoic peracids or aldehydes, or by the reaction using two or more kinds of alkyl chloroformates.

In the case where an alkyl chloroformate having two or more chloroformate group in one molecule thereof is allowed to react with the mixture of two or more kinds of (substituted)perbenzoic acids, the asymmetry organic peroxide having same (substituted) benzoyl groups in each molecule thereof can be obtained at the same time with the one having different (substituted)benzoyl groups in one molecule thereof. For example, the mixture consisting of dibenzoylperoxy hexamethylene biscarbonate, di(4-methylbenzoylperoxy) hexamethylene biscarnate, and 1-(benzoylperoxy)-6-(4-methylbenzoylperoxy) hexamethylene biscarbonate was obtained when perbenzoic acid and 4-methylperbenzoic acid were reacted with 1, 6-hexamethylene dichloroformate, and the mixture consisting of 1,1,1-tris(benzoylperoxycarbonyloxymethyl)propane, 1,1,1-tris(4-methylbenzoylperoxycarbonyloxymethyl)propane, 1,1-bis(benzoylperoxycarbonyloxymethyl)-1-(4-methylbenzoylperoxycarbonylo xymethyl)propane, and 1-(benzoylperoxycarbonyloxymethyl)-1,1-bis(4-methylbenzoylperoxycarbonylo xymethyl) propane was obtained when perbenzoic acid and 4-methylperbenzoic acid were reacted with trimethylolpropane trischloroformate. The asymmetry organic peroxide having different (substituted)benzoyl groups in one molecule thereof is not limited by the above examples.

The asymmetry organic peroxide of the present invention can be also used as a mixture with the other conventional organic peroxide.

The other conventional organic peroxide that can be used with the asymmetory organic peroxide simultaneously including but not limited to the following, ketone peroxides such as acetylacetone peroxide, methylacetoacetate peroxide, methylethylketone peroxide, methylisobutylketone peroxide, cyclohexanon peroxide, methylcyclohexanone peroxide, 3,3,5-trimethylcyclohexanon peroxide; peroxy ketals such as 1,1-bis(tert-butylperoxy)cyclohexane, 1,1-bis(tert-hexylperoxy)cyclohexane, 1,1-bis(tert-butylperoxy)-2-methylcyclohexane, 1,1-bis(tert-hexylperoxy)-2-methylcyclohexane, 1,1-bis(tert-butylperoxy)-3,3,5-trimethylcyclohexane, 1,1-bis(tert-hexylperoxy)-3,3,5-trimethylcyclohexane, 1,1-bis(tert-butylperoxy)cyclododecane, 2,2-bis(4,4-di-tert-butylperoxycyclohexyl)propane, 2,2-bis(tert-butylperoxy)butane, 2,2-bis(tert-hexylperoxy)butane, 2,2-bis(tert-butylperoxy)octane, 2,2-bis(tert-hexylperoxy)octane, ethyl-3,3'-bis(tert-butylperoxy)butyrate, n-butyl-4,4'-bis(tert-butylperoxy)valerate, 3,3,6,6,9,9-hexamethyl-1,2,4,5-tetraoxacyclohexane, hydroperoxide such as tert-butyl hydroperoxide, tert-amyl hydroperoxide, tert-hexyl hydroperoxide, 1,1,3,3-tetra-methylbutyl hydroperoxide, cumene hydroperoxide, diisopropylbenzene hydroperoxide, 2,5-dimethyl-2,5-bis(hydroperoxy)hexane, 2,5-dimethyl-2,5-bis(hydroperoxy)hexyne, para-menthane hydroperoxide and pinene hydroperoxide, dialkyl peroxide such as di-tert-butyl peroxide, di-tert-amyl peroxide, di-tert-hexyl peroxide, tert-butyl cumyl peroxide, dicumyl peroxide, 2,5-dimethyl-2,5-bis(tert-butylperoxy)hexane, 2,5-dimethyl-2,5-bis(tert-butylperoxy)hexyne, α,α -bis(tert-butyl peroxy)diisopropylbenzene and tris(tert-butylperoxy)triazine; diacyl peroxides such as acetyl peroxide, isobutyryl peroxide, octanoyl peroxide, isononanoyl peroxide, decanoyl peroxide, 3,5,5-trimethylhexanoyl peroxide, lauroyl peroxide, stearoyl peroxide, benzoyl peroxide, 4-methylbenzoyl peroxide, 3-methylbenzoyl peroxide, 2-methylbenzoyl peroxide, 2-methoxybenzoyl peroxide, succinic acid peroxide and caprylyl peroxide, peroxy dicarbonates such as di-n-propyl peroxy dicarbonate, diisopropyl peroxy dicarbonate, di-sec-butyl peroxy dicarbonate, di-2-ethylhexyl peroxy dicarbonate, dimyristyl peroxy dicarbonate, dicetyl peroxy dicarbonate, dicyclohexyl peroxy dicarbonate, bis(4-tert-butylcyclohexyl) peroxy dicarbonate, dicyclododecyl peroxy dicarbonate, di(2-ethoxyethyl) peroxy dicarbonate, di(methoxyisopropyl) peroxy dicarbonate, di(3-methoxybutyl) peroxy dicarbonate, di(3-methyl-3-methoxybutyl) peroxy dicarbonate and di(2-phenoxylethyl)peroxy dicarbonate; peroxy esters such as tert-amyl peroxy neodecanoate, tert-hexyl peroxyneodecanoate, tert-butylperoxyneodecanoate, cumylperoxyneodecanoate, 1,1,3,3-tetra-methylbutyl peroxy neodecanoate, 1-cyclohexyl-1-methylethyl peroxy neodecanoate, tert-amyl peroxy pivalate, tert-hexyl peroxy pivalate, tert-butyl peroxy pivalate, tert-butyl peroxy 2-ethylhexanoate, tert-amyl peroxy 2-ethylhexanoate, tert-hexyl peroxy 2-ethylhexanoate, 1,1,3,3-tetra-methylbutyl peroxy 2-ethylhexanoate, 1-cyclohexyl-1-methylethyl peroxy 2-ethylhexanoate, *α,* α-bis(neodecanoyl peroxy)diisopropylbenzene, tert-butyl peroxy isobutyrate, tert-butyl peroxy maleic acid, tert-butyl peroxy 3,5,5-trimethylhexanoate, tert-butyl peroxy laurate, 2,5-dimethyl-2,5-bis(benzoylperoxy)hexane, 2,5-dimethyl-2,5-bis(3-toluoylperoxy)hexane, 2,5-dimethyl-2, 5-bis (2-ethylhexanoylperoxy) hexane, tert-butyl peroxy acetate, tert-butyl peroxy benzoate, tert-amyl peroxy benzoate, tert-hexyl peroxy benzoate, tert-octyl peroxy benzoate, tert-butyl peroxy 4-methylbenzoate, tert-hexyl peroxy 4-methylbenzoate, di-tert-butyl peroxy isophthalate, cumyl peroxy octoate, tert-butyl peroxy neohexanoate, tert-hexyl peroxy neohexanoate, cumyl peroxy neohexanoate, di-tert-butyl peroxy hexahydroterephthalate, bis(tert-butylperoxy)trimethyl adipate, tert-butyl peroxy neoheptanoate, tert-hexyl peroxy 2-methylbenzoate; peroxy alkyl carbonates such as tert-butyl peroxy isopropyl monocarbonate, tert-hexylperoxy isopropyl monocarbonate, tert-butylperoxy 2-ethylhexyl monocarbonate, tert-butylperoxy myristyl monocarbonate, tert-butylperoxy octadecyl monocarbonate, tert-amylperoxy octadecyl carbonate, tert-butylperoxy allyl monocarbonate, 1,6-bis(tert-butylperoxycarbonyloxy)hexane, 1,5-bis(tert-butylperoxycarbonyloxy)-3-oxapentane, tert-amylperoxy 2,6,8-trimethyl-4-nonyl carbonate, tert-butyl trimethylsilyl peroxide, 1,6-bis(tert-butylperoxycarbonyloxy)hexane, 1,3-(tert-butylperoxycarbonyloxy)-2,2-dimethylpropane, 1,5-(tert-butylperoxycarbonyloxy)-3-oxapentane, 3,3',4,4'-tetra(tert-amylperoxycarbonyl)benzophenone, acetylcyclohexyl sulfonyl peroxide.

(Substituted) benzoyl peroxides and peroxydicarbonates, having been produced as by-products in the above-mentioned synthesizing methods of the asymmetry organic peroxide in certain cases, can be used as the mixture with the asymmetry ortganic peroxide. Theasymmetry organic peroxide and a (substituted)benzoyl peroxide might have been synthesized independently and mixed thereafter, or the mixture containing a (substituted) benzoyl peroxide formed as a by-product in synthesizing the asymmetry organic peroxide may be used as the mixture of the asymmetry organic peroxide and a (substituted)benzoyl peroxide such as 4-methylbenzoyl peroxide.

The other conventional organic peroxides such as a (substituted)benzoyl peroxide can be used with the asymmetry organic peroxide at any desired concentration.
When high crosslinking speed is required, the pure content of the asymmetry organic peroxide in the mixtures of the crosslinking agent should be preferably 50 % by weight or more. A crosslinking speed would decrease when the asymmetry organic peroxide content becomes less than 50 % by weight.

The asymmetry organic peroxide of the present invention can be used as a pure material being solid or liquid. In addition, it can be used as a paste diluted with an oil or plasticizers, a solution diluted with an organic solvent, or a masterbatch diluted with an inorganic or an organic solid substance, to keep safety and good workability as a crosslinking agent.

The content of the asymmetry organic peroxide in these paste and so on, which depends on the kind of the asymmetry organic peroxide or the form, generally falls in a range of 10-90 % by weight.

When the asymmetry organic peroxide of the present invention is used as a paste and so on, all diluents, plasticizers, inorganic fillers, organic compounds, polymers or the other available additives can be used as long as that does not affect to the crosslinking characters and the properties of crosslinked rubber moldings. These diluent and so on can be added to the organic peroxide while or after manufacturing it.

As mentioned above, the asymmetry organic peroxide of the present invention can be used as a paste diluted with oil or plasticizer. For example, the paste, which was dispersed the asymmetry organic peroxide to silicone oil or a plasticizers such as phthalate esters, can be used so as to make it easier to mix with a silicone rubber base compound.

A silicone oil including organopolysiloxanes being generally added to silicone rubber such as dimethylsilicone oil, methylhydrogensilicone oil, and methylphenylsilicone oil, , and a phthalate ester including dimethylphthalate, dibutylphthalate, and di-2-ethylhexylphthalate can be used.

A silica, which is generally used as a filler of silicone rubber composition, can be contained into the paste.
The paste of the present invention can be obtained by synthesizing and drying the asymmetry organic peroxide before mixing it with silicone oil, or by synthesizing the asymmetry organic peroxide with using silicone oil or plasticizer as a solvent before the obtained paste is passed through a two-roll mill to disperse the particles of asymmetry organic peroxide uniformly.

The asymmetry organic peroxide of the present invention can be also used as a product diluted with an organic solvent.

The above diluent is specifically exemplified but not limited including aliphatic hydrocarbons such as pentane, heptane, dodecane, isododecane, "Shellsol 71 (shell Japan)", "Solvesso 100 (exon chemical)", "Isoper L (exon chemical)", "IP solvent 1620, 2028 (Idemitsu petrochemical)", and inorganic spirit; aromatic hydrocarbons such as toluene, xylene, ethylbenzene, and cumene; halogenated hydrocarbons such as carbontetrachloride, dichloromethylene, dichlorobenzene; acetate esters such as methyl 2-ethylhexanoate, isoamyl propionate, heptyl propionate, benzyl propionate, propyl caprylate, methyl decanoate dimethyl succinate, diethyl succinate, pentyl acetate, hexyl acetate, 2-ethylhexyl acetate, cyclohexyl acetate, benzyl acetate; pivalates such as butyl pivalate, isoamyl pivalate,and hexyl pivalate; adipic acid esters such as dioctyl adipate, diisodecyl adipate; neodecanoic acid esters such as methyl neodecanoate, and butylneodecanoate; glutaric acid esters such as dimethyl glutarate, dibutyl glutarate anddi-2-ethylhexyl glutarate; succinic acidesters such as dimethyl succinate, dibutyl succinate and di-2-ethylhexyl succinate; adipic acid esters such as dimethyl adipate, dibutyl adipate and di-2-ethylhexyl adipate; maleic acid esters such as dimethyl maleate, dibutyl maleate and di-2-ethylhexyl maleate; fumaric acid esters such as dimethyl fumarate, dibutyl fumarate and di-2-ethylhexyl fumarate; benzoic acid esters such as methyl benzoate, ethyl benzoate and octyl benzoate; phthalate esters such as dimethylphthalate, dibutylphthalate and di-2-ethylhexylphthalate; phosphates such as triethyl phosphate, tributyl phosphate, tri(2-ethylhexyl) phosphate and tricresyl phosphate; alkyleneglycol diacetates such as ethyleneglycol diacetate, propyleneglycol diacetate and ethyleneglycol dipropionate; ketones such as acetone, methylethylketone, methylisobutylketone, methylamylketone, cyclohexanone and acetophenone; alcohols such as n-butanol and cyclohexanol; alkyleneglycols such as ethyleneglycol, propyleneglycol; polyalkyleneglycols such as polyethyleneglycol, polypropyleneglycol; alkyleneglycol monoalkylethers such as cellosolve, 2-methoxyethanol, propyleneglycol monoethylether and propyleneglycol monomethylether; dialkyleneglycol monoalkylethers such as diethyleneglycol monoethylether, diethyleneglycol monobutylether, dipropyleneglycol monomethylether; alkyleneglycol monoalkylether acetates such as ethyleneglycol monoethylether acetate, propyleneglycol monoethyl ether acetate, propyleneglycol monomethylether acetate, 3-methoxybutyl acetate and 3-methyl-3-methoxybutyl acetate, styreneoxide, dimethylformamide and N-methyl pyrolidone.

The asymmetry organic peroxide of the present invention can be also used as a masterbatch diluted with solid of inorganic or organic compound.

The above diluent is specifically exemplified but not limited including organic compound, which has comparatively low melting point but which is solid at ordinaly temperature, such as dicyclohexylphtharate, dimethylfumarate, triphenylphosphate, paraffin wax; inorganic compound such as silica, clay, calcium carbonate, molecular sieve; polymers or rubbers such as chlorinated polyethylene, chlorosulfonated polyethylene, ethylene-propylene rubber, ethylene-propylene-diene rubber, fluororubber, polyethylene, polypropylene, butylene rubber, isobutylene rubber, polycyclopentadiene, polymethylcyclopentadiene, polynorbornene, polymethylpentene, polyethylene terephthalate, polybutylene terephthalate, polyamide, acrylic rubber, poly(meta)acrylamide, polysulfide, hydrogenated nitrilerubber, ethylene-vinyl acetate copolymer, olefine-vinylalcohol copolymer, propylene-diene-monomer copolymer, brominatedisoprene-isobutylene copolymer, isoprene-styrene copolymer, allylglycidylether-epichlorohydrin copolymer, ethyleneoxide-epichlorohydrin copolymer, copolymer of acrylic ester with the monomer of a small quantity because of the crosslinking, butadiene rubber, chloroprene rubber, isoprene rubber, natural rubber, acrylonitrile-butadiene rubber, hydrogenated acrylonitrile-butadiene rubber, styrene butadiene rubber, acrylonitrile-butadiene-styrene copolymer, urethane rubber.

The asymmetry organic peroxide of the present invention and the mixture comprising the asymmetry organic peroxide can be used as a crosslinking agent for silicone rubbers such as dimethylsilicone rubber, methylphenylsilicone rubber and fluorosilicone rubber.

A silicone rubber is obtained by mixing a silicone rubber base compound, which consists of organopolysiloxane gum having mainly continuous Ra₂SiO unit, a reinforce filler and other additives to give other functions and characters to the objected silicone rubber, with a crosslinking agent, which is comprising the asymmetry organic peroxide of the present invention, and then, the mixture is allowed to crosslinking by heating or other methods.

The manufactured silicone rubber can be used for the various use such as electrical wire and cable coatings, optical fiber coatings, high voltage electrical insulators, sponges, gaskets for building, waterproof seals for building, medical supplys, sound medium, sealants for food container, fixation rubbers for roller, electrically conductive rubbers for roller, paper loading rubbers for roller, rubber contacts, keyboards, diaphragms, wiper blades, preventions of electrification, insulating radiation seats, potting materials, protection seats for solar battery module, trilaminar resist intermediate layers, extreme infrared rediation radiators, oil bleeds, parts for swimming articles, and so on.

The crosslinking process of silicone rubber using the crosslinking agent, comprising the asymmetry organic peroxide of the present invention or the mixture of the asymmetry organic peroxide, will be described in the following. The amounts of the reinforce fillar, additives and the crosslinking agents will be shown as the amount added to 100 parts by weight of organopolysiloxane gum unless otherwise indicated.

The above organopolysiloxane gum used for the manufacture of silicone rubber is organopolysiloxane represented by the average component formula Ra₂SiO.

R^{a} in the formula is specifically exemplified by linear or branched alkyl groups such as methyl, ethyl, propyl, butyl, isobutyl, tert-butyl, pentyl, 2-methylbutyl, amyl, hexyl, 2,3-dimetybutyl, 3-methylpentyl, octyl, 2-ethylhexyl, decyl, dodecyl and octadecyl; cycloalkyl groups such as cyclopentyl, cyclohexyl, cycloheptyl and cyclooctyl; aryl groups or substituted-aryl groups such as phenyl, naphthyl, tolyl and mesityl; aralkyl groups such as benzyl, 2-phenylethyl and 2-phenylpropyl; and halogenated-alkyl groups, a part or all of the carbon-bonded hydrogen atoms of the above substituent is substituted by halogen atoms (for example, chlorine atom, fluorine atom and bromine atom) such as chloromethyl, bromoethyl, 3-chloropropyl, trifluoropropyl, 3,3,3-trifluoropropyl, 3,3,4,4,4-pentafluorobutyl, 3,3,4,4,5,5,6,6,6-nonafluorohexyl; halogenated aryl groups such as chlorophenyl and dibromophenyl; silyl group-containing groups substituted by trimethylsilyl groups such as 2-trimethylsilylethyl and 3-trimethylsilylpropyl.

R^{a}, which has addition reactivity, condensation reactivity and radical reactivity including hydrogen atom, in the formula is specifically exemplified by the groups having carbon-carbon double bond such as vinyl, allyl, isopropenyl, butenyl, cyclopentadienyl, cyclohexenyl, styryl, α-methylstyryl; the groups having 3 or more carbon atoms and having carbon-carbon double bond at the silicone-bonded carbon atom such as 1-hexenyl, 1-ethyl-1-butenyl, 1-octenyl, 1-methylene-2-propenyl; the groups having 5 or more carbon atoms and having carbon-carbon double bond at the carbon atom apart through at least one carbon atom from the silicone-bonded carbon atom such as ethylidenenorbornyl, ethylenenorbornyl, dicyclopentenyl, 4-pentenyl, 4-hexenyl and cyclooctenyl; (meth) acrylic group-containing groups such as 3- (meth) acryloxypropyl; hydroxyl groups; alkoxy group-containing groups such as methoxy, ethoxy, propoxy and methoxyethoxy; acetoxy groupcontaining groups; ketoxime group-containing groups; alkenyloxygroup-containing groups; α-alkoxycarbonylgroup-containing groups; alkoxysilylgroup-containing groups such as 2-trimethoxysilylethyl, 2-triethoxysilylethyl, 2-tripropoxysilylpropyl, 3-trimethoxysilylpropyl, 3-triethoxysilylpropyl, 3-tripropoxysilylpropyl, 2-methyldimethoxysilylethyl, 2-methyldiethoxysilylethyl, 3-methyldimethoxysilylpropyl and 3-methyldiethoxysilylpropyl; epoxy group-containing groups such as 3-glycidoxypropyl and 2-(3,4-epoxycyclohexyl)ethyl; amino group-containing groups such as 3-aminopropyl; amino alkyl-substisuted amino group-containing groups such as N-(2-aminoethyl)-3-aminopropyl; aminoxy group-containing groups; amide group-containing groups; azido group-containing groups such as 4-azido-benzoyloxypropyl, 4-azido-3-methylbenzoyloxypropyl, 4-azido-3-ethylbenzoyloxypropyl, 4-azido-3-nitrobenzoyloxypropyl, 4-azido-3-methoxybenzoyloxypropyl, 4-azido-3-chlorobenzoyloxypropyl, 3-azidobenzoyloxypropyl and 2-azidobenzoyloxypropyl; mercapto group-containing groups such as mercaptomethyl, mercaptoethyl, mercaptopropyl and mercaptobutyl, cyclic hydrocarbon group having mercapto group; cyano group-containing groups such as cyanomethyl, cyanoethyl and cyanopropyl. The above R^{a} may be employed either singly or in suitable combination of two or more.

The terminal of the molecule chain of the above organopolysiloxane can be capped with a groups such as triorganosilyl groups (substituted by methyl, ethyl, propyl, buthyl, phenyl such as trimethylsilyl group, dimethylvinylsilyl group, dimethylallylsilyl group, dimethylpropenylsilyl group, dimethylphenylsilyl group, dimethylhydroxysilyl group, dimethyl-3,3,3-trifluoropropylsilyl group, methyldiphenylsilyl group, methylphenylvinylsilyl group, methylvinylhydroxysilyl group, methylvinyl-3,3,3-trifluoropropylsilyl group, methyldivinylsilyl group, ethyldivinylsilyl group, phenyldivinylsilyl group, trivinylsilyl group); alkoxy groups such as methoxy, ethoxy, propoxy and butoxy; lower ethylenically unsaturated groups such as vinyl, allyl and butenyl.

Specifically such a organopolysiloxane, including siloxane having both molecular terminals capped with siloxy groups, can be, trimethylsiloxy-capped dimethylpolysiloxane, trimethylsiloxy-capped methylvinylpolysiloxane, trimethylsiloxy-capped methylphenylpolysiloxane, trimethylsiloxy-capped methyl(3,3,3-trifluoropropyl)polysiloxane, trimethylsiloxy-capped dimethylsiloxane-methylvinylsiloxane copolymer, trimethylsiloxy-capped dimethylsiloxane-methylphenylsiloxane copolymer, trimethylsiloxy-capped dimethylsiloxane-methyl(3,3,3-trifluoropropyl)siloxane copolymer, trimethylsiloxy-capped dimethylsiloxane-methylvinylsiloxane-methylphenylsiloxane copolymer, trimethylsiloxy-capped methyl(3,3,3-trifluoropropyl)siloxane-methylvinylsiloxane copolymer, trimethylsiloxy-capped dimethylsiloxane-diphenylsiloxane-methylvinylsiloxane copolymer, dimethylvinylsiloxy-capped dimethylpolysiloxane, dimethylvinylsiloxy-capped methylvinylpolysiloxane, dimethylvinylsiloxy-capped methylphenylpolysiloxane, dimethylvinylsiloxy-capped methyl(3,3,3-trifluoropropyl)polysiloxane, dimethylvinylsiloxy-capped dimethylsiloxane-methylvinylysiloxane copolymer, dimethylvinylsiloxy-capped dimethylsiloxane-methylphenylsiloxane copolymer, dimethylvinylsiloxy-capped dimethylsiloxane-methyl(3,3,3-trifluoropropyl)siloxane copolymer, dimethylvinylsiloxy-capped methyl(3,3,3-trifluoropropyl)siloxane-methylvinylsiloxane copolymer, dimethylvinylsiloxy-capped dimethylsiloxane-methylphenylsiloxane-methylvinylsiloxane copolymer, dimethylvinylsiloxy-capped dimethylsiloxane-diphenylsiloxane-methylvinylsiloxane copolymer, methylvinylphenylsiloxy-capped dimethylpolysiloxane, methylvinylphenylsiloxy-capped methylvinylpolysiloxane, methylvinylphenylsiloxy-capped methylphenylpolysiloxane, methylvinylphenylsiloxy-capped methyl(3,3,3-trifluoropropyl)polysiloxane, methylvinylphenylsiloxy-capped dimethylsiloxane-methylvinylsiloxane copolymer, methylvinylphenylsiloxy-capped dimethylsiloxane-methylphenylsiloxane copolymer, methylvinylphenylsiloxy-capped dimethylsiloxane-methyl(3,3,3-trifluoropropyl)siloxane copolymer, methylvinylphenylsiloxy-capped methyl(3,3,3-trifluoropropyl)siloxane-methylvinylsiloxane copolymer, methylvinylphenylsiloxy-capped dimethylsiloxane-methylphenylsiloxane-methylvinylsiloxane copolymer, methylvinylphenylsiloxy-capped dimethylsiloxane-methylphenylsiloxane-methylvinylsiloxane copolymer, methylvinylphenylsiloxy-capped dimethylsiloxane-diphenylsiloxane-methylvinylsiloxane copolymer, methylvinylhydroxysiloxy-capped dimethylpolysiloxane, methylvinylhydroxysiloxy-capped methylvinylpolysiloxane, methylvinylhydroxysiloxy-capped methylphenylpolysiloxane, methylvinylhydroxysiloxy-capped methyl(3,3,3-trifluoropropyl)polysiloxane, methylvinylhydroxysiloxy-capped dimethylsiloxane-methylvinylsiloxane copolymer, methylvinylhydroxysiloxy-capped dimethylsiloxane-methylphenylsiloxane copolymer, methylvinylhydroxysiloxy-capped dimethylsiloxane-methyl(3,3,3-trifluoropropyl)siloxane copolymer, methylvinylhydroxysiloxy-capped methyl(3,3,3-trifluoropropyl)siloxane-methylvinylsiloxane copolymer, methylvinylhydroxysiloxy-capped dimethylsiloxane-methylphenylsiloxane-methylvinylsiloxane copolymer, methylvinylhydroxysiloxy-capped dimethylsiloxane-diphenylsiloxane-methylvinylsiloxane copolymer, silanol-cappeddimethylpolysiloxane, silanol-cappedmethylvinylpolysiloxane, silanol-capped methylphenylpolysiloxane, silanol-capped methyl(3,3,3-trifluoropropyl)polysiloxane, silanol-capped dimethylsiloxane-methylvinylsiloxane copolymer, silanol-capped dimethylsiloxane-methylphenylsiloxane copolymer, silanol-capped dimethylsiloxane-methyl(3,3,3-trifluoropropyl)siloxane copolymer, silanol-capped methyl(3,3,3-trifluoropropyl)siloxane-methylvinylsiloxane copolymer, silanol-capped dimethylsiloxane-methylphenylsiloxane-methylvinylsiloxane copolymer, silanol-capped dimethylsiloxane-diphenylsiloxane-methylvinylsiloxane copolymer, dimethylhydroxysiloxy-capped dimethylpolysiloxane, dimethylhydroxysiloxy-capped methylvinylpolysiloxane, dimethylhydroxysiloxy-capped methylphenylpolysiloxane, dimethylhydroxysiloxy-capped methyl(3,3,3-trifluoropropyl)polysiloxane, dimethylhydroxysiloxy-capped dimethylsiloxane-methylvinylsiloxane copolymer, dimethylhydroxysiloxy-capped dimethylsiloxane-methylphenylsiloxane copolymer, dimethylhydroxysiloxy-capped dimethylsiloxane-methyl(3,3,3-trifluoropropyl)siloxane copolymer, dimethylhydroxysiloxy-capped methyl(3,3,3-trifluoropropyl)siloxane-methylvinylsiloxane copolymer, dimethylhydroxysiloxy-capped dimethylsiloxane-methylphenylsiloxane-methylvinylsiloxane copolymer, dimethylhydroxysiloxy-capped dimethylsiloxane-diphenylsiloxane-methylvinylsiloxane copolymer, trimethoxysiloxy-capped dimethylpolysiloxane, trimethoxysiloxy-capped methylvinylpolysiloxane, trimethoxysiloxy-capped methylphenylpolysiloxane, trimethoxysiloxy-capped methyl(3,3,3-trifluoropropyl)polysiloxane, trimethoxysiloxy-capped dimethylsiloxane-methylvinylsiloxane copolymer, trimethoxysiloxy-capped dimethylsiloxane-methylphenylsiloxane copolymer, trimethoxysiloxy-capped dimethylsiloxane-methyl(3,3,3-trifluoropropyl)siloxane copolymer, trimethoxysiloxy-capped methyl(3,3,3-trifluoropropyl)siloxane-methylvinylsiloxane copolymer, trimethoxysiloxy-capped dimethylsiloxane-methylphenylsiloxane-methylvinylsiloxane copolymer, trimethoxysiloxy-capped dimethylsiloxane-diphenylsiloxane-methylvinylsiloxane copolymer, but not limited to the above.

The molecular structure of the organopolysiloxane can be a straight, branched, cyclic, or three-dimensional network polymer, a random copolymer, or a block copolymer, and the organopolysiloxane gum can be a mixture of two or more kinds of structures including all what is called organopolysiloxane gum in the art. An organopolysiloxane gum having ethylenically unsaturated group in the range of 0-30 % by mole in R^{a} and having the degree of polymerization in the range of 10-1,000,000 and the viscosity in the range of 10-10,000,000 cst (25°C) can be used, but the present invention isn't limited to the above.

As the above-mentioned organopolysiloxane gum, the one of suitable structure can be selected in accordance with the intended use and the required nature.

For example, the above R^{a} of the organopolysiloxane gum can be used 3,3,3-trifluoropropyl group or cyanoethyl group to give oil resistance, solvent resistance and chemical resistance; perfluoroalkyl group or phenylmethylsiloxane unit to give low-temperature resistance; phenylmethylsiloxane unit to give radiation resistance; and silanol group-containing group to raise adhesive property.

The above R^{a} of the organopolysiloxane gum, containing 10-50 % by mol of siloxane unit having perfluoroalkyl group such as 3-trifluoromethyl group and 0.02-0.5 % by mole of vinyl group to improve durability and hysteresis character, can be used.

The above R^{a} of the solid organopolysiloxane gum, having methyl group in the range of 0.40-0.80 % by mole and phenyl group in the range of 0.20-0.80 % by mole and vinyl group in the range of 10-40 % by mole, and the total number of methyl, phenyl and vinyl group bonded with a silicone atom in the range of 1.0-1.8, and the ratio (the methyl group : vinyl group) in the range of 1.0-4.0 : 1.0, and the amount of hydroxyl group in the range of 0.2-4.5 % by weight, can be used for injection molding.

The azido group-containing organopolysiloxane gum or azido group-containing cyclic organopolysiloxane gum can be used as a photosensitive composition.

The organopolysiloxane gum, having the number of the above R^{a} bonded per one silicone atom in the range of 1.98-2.01, the degree of polymerization in the range of 1,000-10,000, and molecular weight distribution index (Mw/Mn) of Mw/Mn<5, can be used as a coating agent for air bag.

The organopolysiloxane gum, having polysiloxane unit, which has perfluoroalkyl group of 1 to 3 carbon atoms, in the range of 5-40 % by mole and vinyl group in the range of 0.02-0.5 % by mole as the above R^{a}, and average degree of polymerization in the range of 1,000-10,000, can be used for liquid enclosure damper.

Two or more kinds of organopolysiloxane gum can be used in accordance with the required nature and the aimed use as the above gum.

For example, two or more kinds of organopolysiloxane gum having different content of vinyl group can be used together, or organopolysiloxane gum having lower content of vinyl group and higher content of vinyl group can be used together to improve tear strength.

50-90 parts by weight of organopolysiloxane gum, having the vinyl group content in the range of 0.2-2 % by mole in the above R^{a} and the average degree of polymerization of 3,000 or more, and 10-50 parts by weight of dimethylpolysiloxane gum, having molecular terminal capped with trimethylsilyl group and the average degree of polymerization in the range of 20-500, can be used together to have high friction factor and a high durability for a loading paper roller.

100 parts by weight of cyclic organopolysiloxane gum having azide group and 20-1000 parts by weight of organopolysiloxane gum, having the average degree of polymerization in the range of 1,000 or more and the content of vinyl group in the range of 0.05-20 % by mole in the above R^{a}, can be used together as a photoactive composition.

5-95 parts by weight of the organopolysiloxane gum, having aliphatic unsaturated group-containing ratio in the range of 0.1-10 % by mole in the above R^{a} and the viscosity of 10,000 centistokes (25°C) or more, and 95-5 parts by weight of the organopolysiloxane gum, having aliphatic unsaturated group-containing ratio of 0-5 % by mole in the above R^{a}, can be used together as a medium to absorb impact force such as a damper.

The above reinforce filler, which is added to the above organopolysiloxane gum while manufacturing silicone rubber, includes dry-process silicas such as fumed silica, wet-process silicas such as precipitated silica, silica ealogel, silica hydrogel, fired silica, quartz powder and diatomaceous earth and so on.
A silica filler having the specific surface area of 1m²/g or more and the average grain size in the range of 1-50nm can be exemplified.
Additionally, various conventional microgranular or fiber shaped fillers used in conjunction with known silicone rubber compositions including carbon black, magnesium oxide, calcium carbonate, mica, clay, hollow glassspheres, glass powder, fiberglass, carbon fiber, polyester fiber and organopolysiloxane resin powder can be added to silicone rubber base compound.

The reinforce filler can be used either singly or in combination of two or more kinds, and the sum amount of them is generally in the range of 0-600 parts by weight based on 100 parts by weight of the organopolysiloxane gum. The reinforce filler can be used with no limitation so far as it can achieve the aimed materiality.

An excellent extrusion character and an excellent mechanical strength of the crosslinked silicone rubber will be achieved by adding silica filler as a reinforce filler to silicone rubber base compound.

It is desirable to choose a suitable silica fillers in accordance with the aimed use and the required nature.

For example, silica filler having average diameter of the primary particles of 18nm or more and specific surface area of 95m²/g or less can be used to improve interlamellar exfoliation of silicone rubber composition wounded around a roller.

A calcined silica having refractive index of more than 1.446 and ignition loss of less than 3% can be used to improve electric characters such as voltage resistance for electrical electrical wire and cable coatings, tracking resistance, arc resistance and erosion resistants.

A dispersant so-called "softener" or "wetter", which is adsorbed on the surface of silica fillers and promotes the dispersion of them to the organopolysiloxane gum, such as low molecular weight polysiloxanes, silanol group-containing silanes, silanol-capped low molecular weight siloxanes, diphenylsilanediols, alkoxy group-containing silanes, and carbon functional silanes can be used, if necessary at mixing the above silica fillers with the organopolysiloxane gum.

A silane or an organopolysiloxane having organic groups such as methyl, phenyl, vinyl, and 3,3,3-trifluoropropyl group bonded to a silicone atom and polar groups such as hydroxyl and alkoxy group at terminal of molecule chain can be used as such a dispersant.
The above organopolysiloxane used as a dispersant includes hexamethyltripolysiloxane-1,5-diol; silanol group-containing organopolysiloxanes such as diphenylsilanediol, α,ω-dimethylsiloxanediol, organooligosiloxane having diphenyl siloxane unit and one molecular terminal capped with silanol group; organopolysiloxanes such as dimethyldimethoxysilanes having a lower alkoxy group including methoxy, ethoxy, propoxy and butoxy groups; 3,3,3-trifluoropropyl group-containing alkoxysilanes and a partially hydrolyzed condensated products thereof; and linear or cyclic organosilazanized compounds such as 3,3,3-trifluoropropyl group-containing siloxane oligomers, hexamethyldisilazane, hexaorganodisilazane.

The hydrophobic surface-treated microparticulate silica may also be used as the above silica filler.

The above microparticulate silica can be treated by silanol groups such as diphenylsilanediol, 1,3-dipolysiloxanediol, methylvinylpolysiloxane oligomer having molecular terminal capped with silanol, diorganopolysiloxane having both molecular terminals capped with silanol group; organosiloxane oligomer having ethylenically unsaturated group or alkoxy group; organosilicone compound having character of resin; chain organopolysiloxanes such as methylhydrogenpolysiloxane, and dimethylpolysiloxane; cyclic organopolysiloxanes such as hexamethylcyclotrisiloxane and octamethylcyclotetrapolysiloxane; silane compounds such as chlorosilane, alkoxysilane and hexamethyldisilazane; reactive silanes such as silane coupling agent; silanizing agents such as silazane having vinyl group, alkylaminosilane, alkoxysilane. These may be used only a single or two or more kinds of compound in combination.

A method of surface treatment of silica filler to hydrophobic treatment include the method of causing the above silica filler to adsorb liquid phase containing the above hydrophobic treatment agent and inert solvent such as n-hexane at normal temperature or high temperature and thereafter heating, or the method of causing the surface of microparticulate silica filler to react with silane compounds such as halosilane, alkoxysilane and silazane; or low molecular weight siloxanes such as 1,1,3,3,5,5,7,7-octamethylcyclotetrasiloxane at a temperature higher than 300°C, or the method of mixing silica filler and organopolysiloxane gum with organosilicone compounds such as hexamethyldisilazane while heating.

Otherwise, the silica fillar, of which surface was treated by hot-kneading with a organosilicon compound such as hexamethyldisilazane after having been mixed with the organopolysiloxane gum, may also be used. The above hydrophobic treatment of the surface of the silica filler can be done either in gas phase or in liquid phase, and it is effective to use Fe or Sn salt of fatty acid as the catalyst if necessary.

The characters of silicone rubber, like mechanical strength, dynamic fatigue character, oil resistance, steam resistance or LLC resistance, may be improved by using the above surface-treated silica filler.

For example, a tearing strength of silicone rubber can be improved by using silica filler treated with a silane or a siloxane, or particularly with a silane or a siloxane having alkoxy or silanol group, and a discoloration of silicone rubber can be prevented by using silica filler treated with an organo silazane.

The silica filler, treated with the methyldiphenylsilyl group-containing silane compound on the surface, provides damping depression, and the silica filler, having the specific surface area of 50m2/g or more treated with diphenylsilanediol on the surface, provides big loss factor and small temperature dependency of the elastic modulus, and hydrophobic silica filler having the specific surface area in the range of 50-600m²/g treated with silane compound represented by CH₃SiO_{3/2} provides oil resistance, steam resistance and machine strength to the crosslinked silicone rubber moldings.

The hydrophobic silica filler treated with agent chosen from halosilane, alkoxysilane, silazane and low molecular weight siloxane at a temperature higher than 300°C(the specific surface area is 50m²/g or more hydrophobic group on the surface of silica filler consists of CH₃SiO_{3/2} group and (CH₃)₂SiO group, the weight ratio of CH₃SiO_{3/2} group to (CH₃)₂SiO group is in the range of 20/80-40/60, and the total weight of carbon contained into the above CH₃SiO_{3/2} group and (CH₃)₂SiO group is 0.5 % by weight or more based on said silica filler) provides steam resistance and LLC resistance to the crosslinked silicone rubber moldings.

The aminosilane treated silica fillers having a positive electrostatic charge such as fumed silica having specific surface area of 10m²/g or more can control the electrification on the surface of roller for use in loading paper roller or developing roller.

The silica filler (having at least one organopolysiloxane units selected from the group consisting of Ra₃SiO_{1/2} units, Ra₂SiO_{2/2} units and RaSiO_{3/2} units, and SiO_{4/2} units (wherein the ratio of number of moles of organopolysiloxane unit to the number of moles of the SiO_{4/2} unit is in the range of 0.08-2.0.), and the specific surface area of 200m²/g or more) provides excellent character to the crosslinked silicone rubber moldings like toner releasability, low hardness, abrasion resistance, heat conductivity and heat-resistance for fixation roller.

The above additives, being added to the organopolysiloxane gum together with the above reinforce filler while manufacturing silicone rubber, such as low compression set agent, flame retardant, conductive agent, heat conductive agent, adhesive agent, adhesion accelerator, self-fusion agent, tear strength improver, elasticmodulus improver, damping depression improver, friction factor improver, radiation shielding agent, ultraviolet absorber, magnetic agent, humidity conditioning agent, coloring agent, fungicides, anti-fungus, germicide, blooming inhibitor, and surface non-adhesive agent, provide various functions, and the above additives such as fatigue resistance improver, abrasion resistance improver, radiation resistance agent, oil resistance agent, solvent resistance agent, chemical resistance agent, water resistance agent, steam resistance agent, heat-resistant agent, low-temperature resistance agent, antioxidant, age resistor improve the durability to the crosslinked silicone rubber moldings. The above additives can be used properly in accordance with the intended use, and the required nature.

These concrete instances will be given in the following but the present invention isn't specially limited. The above additives may be used either singly or in combination of two or more.

The organohydrogenpolysiloxane having hydrogen atom in the above R^{a} and 0.1-10 parts by weight of calcium compounds such as calcium hydride, calcium hydroxide, calcium oxide and calcium peroxide can be added together to 100 parts by weight of organopolysiloxane gum to improve low compression set of the crosslinked silicone rubber moldings.

An inorganic flame retardant, an organic halogen flame retardant, a platinum compound or a platinum compound combinated with other compounds can be used as a flame retardant.

A rare earth metal oxide or hydroxide and microparticulate copper can be used as an inorganic flame retardant.

A chlorinated flame retardants such as chlorinated paraffin, chlorinated polyethylene, chlorinated polyphenyl, chlorinated diphenyl, vinylchloroacetate, diallyl chloroendate, head acid, anhydrous head acid and tetrachlorophthalic anhydride; brominated flame retardants such as brominated polyphenyl, tetrabromoethane, tetrabromobutane, tetrabromobenzene, hexabromobenzene, hexabromocyclododecane, decabromodiphenyloxide, tetrabromophthalic anhydride and 2,2-bis(4-hydroxy-3,5-dibromophenyl)propane can be used as organic halogen flame retardant.

The microparticulated silicone-titanium compound (obtained by low boiling point silicone compounds such as tetramethoxysilane to react with low boiling point titanium compounds such as propyl titanate in the ratio of the number of silicone atom to the number of titane atom of 1 : 1-1 : 20) can also be used.

The above platinum compound which is ordinally used for silicone rubber composition as platinum catalyst, include pigment platinum, chloroplatinic acid, and platinumolefine complexes.

The subject compound used together with the above platinum compound include carbon black, fumed titanium dioxide, rutile type titanium dioxide, titanium dioxide treated with organosilane or organosiloxane, metal oxide, iron oxide, antimony oxide, a palladium compound, ceric oxide, cerium hydroxide, cerium carbonate, organic phosphite, microparticulate manganese carbonate, manganese carbonate treated with organosilane or organosiloxane, -NHNH2 group-containing compound, azo compound, carboxylic acid amide, chlorinated benzoic acids such as 4-chlorobenzoic acid and 2,4-dichlorobenzoic acid, aromatic azido compound, triazole compound, chain or cyclic organopolysiloxane.

The mixture consisting of carbon black,titanium dioxide, aromatic acid and the compound (selected from the group consisting metal oxide belong to secondary group in perodic table and rare earth metal oxide), the mixture consisting of carbon black, fumed titanium dioxide and triazole compound, the mixture consisting of fumed titanium dioxide and γ-Fe₂O₃, the mixture consisting of titanium dioxide and zirconium dioxide and zinc oxide, the mixture consisting of fumed titanium dioxide and cerium hydroxide and basic zinc carbonate, the mixture consisting of titanium dioxide and substituted benzoic acid having at least one halogen substituted group, the mixture consisting of zinc carbonate and ceric oxide, the mixture consisting of hydration alumina and magnesium oxide, the mixture consisting of aluminum hydroxide and ceric oxide, the miture consisting of rare earth metal oxide or water and fatty acid and/or metal salt of fatty acid, or the mixture consisting of heterosiloxane containing cerium atom and titanate coupling agent can be used together with platinum compound.

The amount of Platinum compound is in the range of 0.01-5, 000 ppm as platinum atom based on the organopolysiloxane gum and the other additive is in the range of 0.01-200 parts by weight based on 100 parts by weight of organopolysiloxane gum can be used as flameretargdant.

10-50 parts by weight of organic halogen flame retardant and a 10-100 parts by weight of microparticulate aluminum hydroxide based on 100 parts by weight of organopolysiloxane gum can be added to organopolysiloxane gum to improve flame resistance and an electric insulation characters such as tracking resistance and arc resistance.

A mixture obtained by platinum compound reacted with the compound, having alkynyl group and alcoholic hydroxyl group in the molecule, such as 3, 5-dimethyl -1-hexyne-3-ol, 2-methyl-3-butyne-2-ol, 2-methyl-3-butyne-2-ol, 1-ethynyl-1-cyclohexanol, or a mixture obtained by platinum compound reacted with the compound having carbon-carbon double bond and carbon-carbon triple bond such as 3,5-dimethyl-3-hexene-1-yne, or a mixture obtained by platinum compound reacted with the alkynyl group-containing organosilicic compounds such as methyl-tris(3-methyl-1-butyne-3-oxy)silane can be added to organopolysiloxane gum in the amount of 1-1,000 ppm as platinum metal based on organopolysiloxane gum.

The mixture, obtained by the above platinum compound reacted with alkynyl group-containing organosilicone compound, and 1.0-50 parts by weight of hydroxide aluminum powder and 0.01-100 parts by weight of triazole compound can be added to organopolysiloxane gum based on 100 parts by weight of.

A conductive carbon black, a conductive metal oxide, nonmetallic conductive filler and metal conductive filler can be added as the above conductive agent.

The above conductive carbon black include acetylene black, furnace black, conductive furnace black, super conductive furnace black, extra conductive furnace black, channel black, conductive channel black, thermal black, carbon black having surface area of 900m²/g or more and hollow shell-shaped particles.

Cyclic or straight chain organopolysiloxanes, having degree of polymerization of 10 or less, such as octamethylcyclotetrasiloxane and decamethylcyclopentasiloxane as a diluent is preferably used to adding carbon black to organopolysiloxane gum in the large quantities. The above diluent can be removed by heating at the temperrature from room temperature to 400°C under ordinary or reduced pressure within or after molding.

The above conductive metal oxide include metal oxides, the surface of which is treated with metal conductive materials such as silver and stibium, such as titanium dioxide, tin oxide and antimony oxide, solid solution of iron oxide and titanium oxide, solid solution of magnesium oxide and aluminum oxide, solid solution of tin oxide and antimony oxide, solid solution of metal oxides such as aluminum, gallium, tin, indium and titanium and zinc oxide, and the compounds covered with these above solid solution on the surface of metals such as magnesium, zinc, calcium, strontium, barium and titane, metal oxides, metal hydroxides, metal sulfides, or metal nitroxides.

The above nonmetallic conductive filler include a conductive materials such as graphite and carbon fiber, nonmetallic inorganic powders such as silica, activated carbon, mica, titanium oxide, potassium titanate whisker, silicone carbide whisker, zinc oxide whisker, boric acid aluminum whisker and titania whisker, and powder, flake or fiber formed nonconductive inorganic materials such as glass, mica, alumina or carbon, covered on the surface with a metal conductive materials such as silver and stibium or nickel.

The above metal conductive filler include powder, flake, or fiber formed alloys or metals such as silver, nickel, aluminum, copper, zinc, iron, gold, silicon, titanium, gallium, indium, magnesium, calcium, strontium, barium, tin, and stibium.

These above conductive agent may be used singly or in the form of two or more combination, for example, the combination of acetylene black and furnace black, or the combination of carbon black and conductive fiber.

The amount of these conductive agent is generally in the range of 1-500 parts by weight to 100 parts by weight of organopolysiloxane gum, but in variable in accordance with the desired conductive level, required materiality or conditions.

The conductive silicone rubber having the above conductive agent can be added to the following additives to prevent from crosslinking inhibition, change the rate of volume resistivity and improve preservation stability.

5-150 parts by weight of carbon black having specific surface area of 80m²/g or more and 1.0-20 parts by weight of organosilicone compounds having silicone-bonded hydrogen atom such as (CH₃)₃Si(OSi(CH₃)H)₁₀OSi(CH₃)₃, (CH₃)₃Si(OSi(CH₃)H)₃₀OSi(CH₃)₃ can be added to 100 parts by weight of organopolysiloxane gum, or a fatty acids such as formic acid, acetic acid, propionic acid, lauric acid, stearic acid, ricinoleic acid, naphthenic acid and 2-ethylhexanoic acid; an acid anhydride, obtained by intermolecular condensation, such as acetic anhydride; and an acid anhydrides such as maleic anhydride and phthalic anhydride can be added in the range of 0.01-5 parts by weight to 100 parts by weight of organopolysiloxane gum, or 1-150 parts by weight of carbon black, having the amount of iodine adsorption in the range of 1-50mg/g, and 1-150 parts by weight of acetylene black, having the amount of iodine adsorption in the range of 1-50mg/g, the absorbed amount of hydrochloric acid in the range of 1-12ml/5g and the specific surface area of 20-40m²/g, to give conductivity effectively can be added to 100 parts by weight of organopolysiloxane gum to prevent from crosslinking inhibition in the system using carbon black.

Ethylene oxide adducts, having the amount of ethylene oxide adduct in the range of 5-15 mole, such as polyoxyethylene alkylphenyl ether, polyoxyethylene alkyl ether, polyoxyethylene sorbitan monooleate, polyoxyethylene sorbitan trioleate, polyoxyethylene glycerin monooleate and polyoxyethylene glycerin mono-stearate can be added 0.1-10 parts by weight to 100 parts by weight of organopolysiloxane gum to change the rate of volume resistivity of conductive silicone rubber.

Inorganic filler treated by surface active agent, 0.1-100 parts by weight of surfactants such as cationic, anionic, amphoteric or nonionic surfactants, 0.1-10 parts by weight of phthalocyanine compound, or 5-200 parts by weight of iron oxide powder represented by (FeO)a(Fe₂O₃)_{b} (wherein a+b=1, 0.5≦a≦1 and 0≦b≦0.5) can be added to 100 parts by weight of organopolysiloxane gum to minimize the voltage dependence of volume resistivity and yield stable electric response even if voltage or electric current to conductive silicone rubber was changed.

Non-formable azo cmpounds such as benzotriazole, benzotriazole-5-carboxylic acid, 1,2,3-benzotriazine-4(3H)-on, N-(1H)-benzotriazole-1-ylmethyl-formamide, lH-benzotriazole-1-ylmethyl-isocyanide can be added in the range of 0.01-5 parts by weight to 100 parts by weight of organopolysiloxane gum to improve preservation stability of conductive silicone rubber.

An acetylene black, having the absorbed amount of hydrochloric acid of 12ml/5g or less and the amount of iodine adsorption of 50mg/g or less, can be added in the range of 1-500 parts by weight to 100 parts by weight of organopolysiloxane gum to extend the pot life of the compound to use for wire and cable coatings having conductive silicone rubber layer on the surroundings of a conductor.

5-100 parts by weight of conductive carbon black and 0.1-50 parts by weight of boric acid or boric acid compound can be added to 100 parts by weight of organopolysiloxane gum to have self-fusion-adherence.

The above heat conductive agent include heat conductive inorganic compounds of silicone carbide,metal oxide and metal powders such as alumina, magnesium oxide, chinese white, aluminum oxide, aluminum hydroxide, aluminum nitride, quartz, magnesium oxide, boron nitride, silicone nitride, zinc oxide and zinc oxide having the average particle size of 2-50µm. The above heat conductive agent is added at 100-1000 parts by weight to 100 parts by weight of organopolysiloxane gum.

Boron nitride treated by silane coupling agent, having unsaturated double bond group bonded to silicone atom through divalent organic group in the organic functional group, or 10-500 parts by weight of heat conductive inorganic additive and 0.01-50 parts by weight of organosilicone compound, having at least three hydrogen atoms bonded to silicone atoms in one molecule, can be added to 100 parts by weight of organopolysiloxane gum to provide electrical insulation.

The above adhesive and adhesion accelerator include alkoxy silanes such as tetramethoxysilane and tetraethoxysilane, one or more kind of silane coupling agent and partially hydrolyzed and condensated product, alkylperoxysilanes such as vinyltris(tert-butylperoxy)silane, triphenyl(tert-butylperoxy)silane, trimethyl(tert-butylperoxy)silane, and methylvinyldi(tert-butylperoxy)silane;
cyclic siloxanes such as 3-glycidoxypropyl group-containing cyclic methylhydrogentetrasiloxane, 3-methacryloxypropyl group-containing cyclic methylhydrogentetrasiloxane, 3-glycidoxypropyl group-containing cyclic methylmethoxysiloxane, cyclic methylhydrogentetrasiloxane having 3-glycidoxypropyl group and 2-(trimethoxysilyl)ethyl group, cyclic methylhydrogentetrasiloxane having 3-methacryloxypropyl and 2-(trimethoxysilyl)ethyl group; 2-((3-trimethoxysilyl)propyloxycarbonyl)propyl group-containing cyclic methylhydrogensiloxane,
straight chain siloxanes such as 3-glycidoxypropyl group-containing methylmethoxysiloxane, methylhydrogensiloxane having 3-glycidoxy and vinyl group, methyl siloxane having 3-glycidoxy and 2-(trimethoxysilyl)ethyl group, cyanurate ring-containing organosilicone compounds such as tris(3-trimethoxysilylpropyl)isocyanurate.

A metal salt of carboxylic acid and condensation reaction catalyst such as titanic acid ester can be added as adhesion accelerator.

The above self-adhesive is boron compounds, which is exemplified by boric acids such as boric acid, boric acid anhydride, pyroboric acid, orthoboric acid; derivatives of boric acid or boric acid anhydride such as trimethyl borate, triethyl borate, trimethoxy boroxine; polyorganoborosiloxane having boroxane-bond in polysiloxane chain (obtained by heating boric acid anhydrous and organoalkoxysilanes such as dimethyldimethoxysilane and dimethyldiethoxysilane to condensate).

0.1-30 parts by weight of the above boron compound and 0.01-10 parts by weight of tin compound used as catalyst for condensation reaction can also be added to 100 parts by weight of organopolysiloxane gum.

0.1-10 parts by weight of organopolysiloxane having the number of the above R³ per one silicone atom of 0 or more and smaller than 2 and the number of vinyl group of more than 1 and less than 4, and the degree of polymerization in the range of 1-1,000 can be added to 100 parts by weight of organopolysiloxane gum.

0.01-30 parts by weight of the organosilicone compound (the above R^{a} has the group represented by the fomura -C(R^{1b}) (R^{1c}) (CR₂)_{c}COOR^{1d} (R^{1d} represents substituted or unsubstituted monovalent hydrocarbon group, R^{1b} and R^{1c} are the same or different and stands for any group selected from hydrogen atom, methyl group , and ethyl group, c stands for an integer selected from 0, land 2) can be added to 100 parts by weight of organopolysiloxane gum to provide self-adhesive property to the crosslinked silicone rubber moldings.

The above tear strength improver includes polytetrafluoroethylene and asbestos fiber.

0.1-5 parts by weight of ethylene glycol, propylene glycol, glycerin or polyalcohol derived from the above glycol or glycerine and 5-20 parts by weight of organohydrogenpolysiloxane having vinyl group content of 0.2-2.5%, and the degree of polymerization of 30-3, 000 to 100 parts by weight of organopolysiloxane gum provides the crosslinked silicone rubber moldings having low modulus and high elongation.

0.01-3 parts by weight of nonionic surface active agent to 100 parts by weight of organopolysiloxane gum provides the crosslinked silicone rubber moldings having excellent characters such as modulus, fatigue durability, resilience and high tear strength.

0.1-50 parts by weight of organopolysiloxane, having at least one hydrogen atom and hydroxyl group or alkyl group in the molecular terminal and the degree of polymerization of 3-100, to 100 parts by weight of organopolysiloxane gum provides the crosslinked silicone rubber moldings having lower hardness arbitrarily with higher mechanical strength.

The composition consisting of one or more kind of vinyl group-containing organopolysiloxane and organohydrogenpolysiloxane, or the composition consisting of vinyl group-containing organopolysiloxane having higher degree of polymerization, vinyl group-containing organopolysiloxane having lower degree of polymerization, silica filler, organohydrogenpolysiloxane and platinum catalyst, or the composition consisting of organopolysiloxane having vinyl group in molecular terminal and high viscosity, vinyl group-containing organopolysiloxane having low viscosity, silica filler, silane having silicone-bonded hydrogen atom, polysiloxane having silicone-bonded hydrogen atom, and platinum catalyst, or the composition consisting of 1-20 parts by weight of liquid organopolysiloxane resin (consisting of 20-60 % by mole of C₆H₅SiO_{1.5} unit, 0-20 % by mole of CH₂CH₂=CHSiO_{1.5} unit, 15-30 % by mole of (CH₃)(CH₂=CH)SiO unit and 20-50 % by mole of (CH₃)₂SiO₂ unit, and having the total amount of C₆H₅SiO_{1.5} unit and the CH₂=CHSiO_{1.5} unit in the range of 20-60 % by mole per all siloxane units. ) and 10-100 parts by weight of polyester fiber (having softening point of 200°C or higher, the degree of fiber at least one denier and the length= of 0.5-5mm) and 0.5-10 parts by weight of organosilicone compound (having hydroxyl group or hydrolyzable group and the group selected from NH₂-, NC-, HOCOCH₂NH-, NH₂CONH-, -(CH₂CH₂)O(CH₂CH₂)-N-, C₆H₅NH- and HS-), or the composition consisting of 100 parts by weight of organopolysiloxane having both molecular terminals capped with dimethylvinyl group (having 0-0.15% of vinyl group and degree of polymerization of 3,000 or more) and 5-50 parts by weight of organopolysiloxane having molecular terminal capped with trimethyl group, dimethylvinyl group or dimethylhydroxy group (having 0.2-2.5% of vinyl group and degree of polymerization of 1,000 or more) and 0.1-20 parts by weight of organohydrogenpolysiloxane having two or more silicone-bonded hydrogen atom in each molecule provides the crosslinked silicone rubber moldings of tear strength.

The above elastic modulus improver include 0.5-300 parts by weight of polymethylsilsesquioxane powder having average particle size of 0.1-100m and 5-200 parts by weight of polyorganosilsesquioxane powder, treated on the surface by organosilicone compound having triorganosilyl group, hexamethyldisilazane or disilazane having polyfluoroalkyl group, and having specific surface area of 100m²/g or more to 100 parts by weight of organopolysiloxane gum. These can also improve lower compression set and higher transparency.

The above damping depression improvor include polyols such as ethylene glycol, propylene glycol and glycerin, phenyl group containing α , w -dihydroxyorganopolysiloxane having low degree of polymerization, the mixture consisting of 0.1-10 parts by weight of crosslinking aids and 0.1-20 parts by weight of polytetrafluoroethylene powder, or a dispersion containing polytetrafluoroethylene prepared by emulsion polymerization.

The above friction factor improver include glass bead, silicone powder (having 80-3 % by mole of R^{a}₃SiOSiO_{3/2} unit and 20-97 % by mole of R^{a}SiO_{3/2} unit) having average particle size of 0.05-20µm, spherical or right spherical shaped titanium dioxide having average particle size of 0.1-10µm, or 0.01-500 parts by weight of low molecular weight polyethylene having the density of 0.95 or smaller at 20°C to 100 parts by weight of organopolysiloxane gum.

The above radiation shielding agent include lead compounds such as lead, lead carbonate and lead hydroxide, the above ultraviolet absorber and impact resistance improver include 3-20 parts by weight of organopolysiloxane represented by the formula HO-((CH₃) (C₆H₅)SiO)_{d}-H to 100 parts by weight of organopolysiloxane gum, the above magnetic agent include barium titanate and ferrite, and the above humidity conditioning agent include natural zeolite, respectively.

The above coloring agent include inorganic pigments such as red iron oxide, yellow iron oxide, chromium oxide, chromium hydroxide, cobalt oxide, carbon black, titane black and black iron oxide, organic pigments such as coal-tar color, safflower pigment, β-carotene , cochenille and chlorophyll, anatase type or rutile type titanium oxide, white pigments such as chinese white, white lead, lithopone, talc, kaolin, calcium carbonate, magnesium carbonate, magnesium silicate, silicic anhydride and zinc oxide, blue pigments such as ultramarine, cobalt blue, copper phthalocyanine blue, ultramarine blue and procion blue, pearl pigments such as color phosphorus foil, bismuth oxychloride, mica titane and mica, dye pigments such as basic dye, acidic dye, vat dye and mordant color, azo pigments such as soluble azo, insoluble azo, condensated azo, azo complex salt and benzimidazolone azo, phthalocyanine pigment, fused-ring polycyclic pigment, nitro pigment, nitrosopigment, fluorescent pigment and oxazole brightener. The above coloring agent can be used with necessary amount.

The above fungicides include triazolyl group (such as 1,2,4-triazolyl-1-yl, 1,2,3-triazolyl-1-yl, 1,2,3-triazolyl-2-yl, 1,2,4-triazolyl-4-yl and 1,3,4-triazolyl-1-yl group) containing compounds, such as tebuconazole, hexaconazole, myclobutanil, bitertanol, and 0.05-5 parts by weight of them can be used to 100 parts by weight of organopolysiloxane gum.

The above antibacterial agent or germicide include inorganic antibacterial agent supported a metals having antibacterial action such as silver, copper, lead, zinc and nickel on powdery or microparticulate inorganic ion-exchanger or porous materials such as zeolite, ziriconium phosphate and calcium phosphate; or 0.1-30 parts by weight of organicsilicone-quaternary ammonium salts such as octadecyldimethyl(3-(trimethoxysilyl)propyl) ammonium chloride or partially hydrolyzed compound thereof to 100 parts by weight of organopolysiloxane gum.

The above blooming inhibitor in hot water include the combination of 0.01-30 parts by weight of compound, having little compatibility with organopolysiloxane and little solubility for water such as flame retardants (aromatic halogenated compounds such as pentabromobenzene and decabromodiphenyl ether) and mold-releasable agents (higher fatty acid or metal salts thereof such as stearic acid or zinc stearate), and 0.01-20 parts by weight of oxide or hydroxide of alkaline earth metals such as beryllium oxide, magnesium oxide, calcium oxide, strontium oxide, barium oxide, calcium hydroxide, strontium hydroxide and barium hydroxide to 100 parts by weight of organopolysiloxane gum.

The above surface non-adhesive agents include 0.5-300 parts by weight of polymethylsilsesquioxane powder having average particle size of 0.1-100 µm to 100 parts by weight of crude rubbe, and epoxy resin non-adhesive agents include, 0.05-1 patrs by weight of phenyl group and methyl group-containing organopolysiloxane having average degree of polymerization of 10-20 to 100 parts by weight of organopolysiloxane gum,
and deterioration inhibitor of silicone rubber under coexistence of synthetic rubber or plastic include supermicroparticulate calcium carbonate having virtually untreated surface and average particle size of 0.3µm or smaller and specific surface area of 3m²/g or more.

The above fatigue resistance improver include phosphorus-containing organic compound, fluorine-containing polymer having the low degree of polymerization, the mixture of 10-100 parts by weight of silica filler (adsorbed water quantity is 0.50 % by weight or less and specific surface area is 250m²/g or more) and 1-50 parts by weight of organopolysiloxane having both molecular terminals capped with hydroxyl group containing group (having vinyl group of 0-75 % by mole in the above Ra and degree of polymerization of 1-100), and 0.1-20 parts by weight of silanol group-containing organooligosiloxane having at least one ethylenically unsaturated group bonded to silicone atom and hydrolyzable group in the molecule and degree of polymerization of 2-50 can be added to 100 parts by weight of organopolysiloxane gum.

The composition consisting of 100 parts by weight of organopolysiloxane gum having both molecular terminals capped with vinyl group containing group and degree of polymerization is 3,000 or more, 50-150 parts by weight of organopolysiloxane gum (having degree of polymerization of 3,000 or more and vinyl group in the range of 0.1-2.0 % by mole), 0.5-10 parts by weight of organohydrogenpolysiloxane having at least two hydrogen atoms in the above Ra, 20-50 parts by weight of silica filler having the specific surface area of at least 250m²/g, and 5-50 parts by weight of organopolysiloxane oil having molecular terminals capped with hydroxyl group containing group (having degree of polymerization in the range of 5-50 and vinyl group in the range of 10-75 % by mole) can be used for fatigue resistanced silicone rubber moldings.

The above abrasion resistance improver include magnesium oxide, the mixture containing magnesium oxide, mica powder, scale minerals such as talc powder, 5-100 parts by weight of hydrohobic treatment lamellar clay mineral powder using siloxane obtained by mixing with organopolysiloxane (the molecular terminal is substituted by the same or different functional group selected from the group consisting of hydrogen atom, hydrolyzable groups such as hydroxy, alkoxy, acetoxy and acyloxy group or the group having =NH or -NH group,) and then heated under reduced pressure, and 5-40 parts by weight of fluorinated plastic powder, having particle size of 0.1-100 µ m, such as polytetrafluoroethylene, polychlorotrifluoroethylene, polyfluorinated vinylidene, polytetrafluoroallene, tetrafluoroethylene and hexafluoropropene to 100 parts by weight of organopolysiloxane gum.

The radiation resistance silicone rubber can be the composition consisting of 100 parts by weight of organopolysiloxane having (halogenated)phenyl group of 0.1-0.80 and vinyl group of 0.001-0.05 per one silicone atom, 0.5-2 parts by weight of cyclopolysiloxane having (halogenated) phenyl group and vinyl group and 10-20 parts by weight of silica filler, treated on the surface by organosilicone having at least one (halogenated) phenyl, hydroxyl or hydrolyzable group, having the specific surface area of 50m²/g or more.

The subject component, which imparts oil resistance, solvent resistance and chemical resistance to silicone rubber moldings, include silica filler with 0.2-3.5 parts by weight of organopolysiloxane oil, which has no ethylenically unsaturated group in the molecule, having the viscosity in the range of 1-1,000 centipoise (25°C) to organopolysiloxane gum, or 0.5-50 parts by weight of metal powder selected from the group consisting of magnesium (such as magnesium oxide and magnesium carbonate), calcium, barium, manganese and aluminum, or 0.5-50 parts by weight in term of metal content of inorganic compounds powder 10-200 parts by weight of polyurethane having softening point of 100°C or higher, or the mixture consisting of 10-50 parts by weight of silicone oil having average degree of polymerization of 20-300, 0.1-5 parts by weight in terms of polytetrafluoroethylene content of dispersion of polytetrafluoroethylene obtained by emulsion polymerization and 1-100 parts by weight of silicic acid or calcium silicate having oil absorption ability of 150cc/100g or more to 100 parts by weight of organopolysiloxane gum. And also, the composition consisting of 100 parts by weight of organopolysiloxane, which is capped at either or both of molecular terminal with vinyldiorganosiloxy group, represented by the following formula ((CH₂=CH-(R^{2b})₂SiO((R^{2b})₂SiO)ₑ)- ((CH₃) (CF₃CH₂CH₂) SiO)_{f}-X² or (CH₂=CH- (R^{2b})₂SiO((R^{2b})₂SiO)ₑ)-((CH₃) (CF₃CH₂CH₂)SiO)_{f}) _{g}-SiR^{2c} (_{4-g}) (wherein R^{2b} stands for non-substituted monovalent hydrocarbon group having 1 to 8 carbon atoms, R^{2c} stands for non-substituted or substituted saturated monovalent hydrocarbon group having 1to 8 carbon atoms, X stands for group or atom selected from the group consisting of hydrogen atom, saturated monovalent hydrocarbon group of the same with R^{2c} or silyl group represented by the following formula -Si(R^{2b})₂CH=CH₂ or -Si(R^{2c})₃, e=1-30, f≧500, g stands for an integer of 2 to 4)) , 10-100 parts by weight of silica filler, and 0.5-20 parts by weight of siloxaneoligomer having both molecular terminals capped with 3-trifluorpropyl and hydroxyl group and viscosity in the range of 15-50cst (25°C), or the composition consisting of 100 parts by weight of dimethylsiloxane-methyl-3,3,3-trifluoropropylsiloxane-methylvinylsiloxane copolymer gum (wherein the content of methylvinylsiloxane unit is in the range of 0.001-5 % by mole in the above polymer), 10-100 parts by weight of silica filler and 1-80 parts by weight of organopolysiloxane oil having less than two functional group or silicone-bonded hydrogen atom to react with organopolysiloxane gum and silica filler can be used.

The above water resistance agent include 0.1-200 parts by weight of calcium oxide manufactured in wet-process, or 0.1-10 parts by weight of polyethylene glycol having melting point of 25°C or higher and hydroxyl value of 10-100 to 100 parts by weight of organopolysiloxane gum.

The above steam resistance agent include calcium compounds such as calcium hydride, calcium hydroxide, calcium oxideand calcium peroxide, or the combination of 0.1-20 parts by weight of organopolysiloxane having hydroxyl or alkoxy group and degree of polymerization of 2-50 and 10-100 parts by weight of reinforce filler treated on the surface with organosiloxy group-containing organosiloxane represented by (CH₃)₂SiO, (CH₃)(CH₂=CH)SiO, (CH₃)(C₆H₅)SiO, CH₃SiO_{3/2} or (CH₂=CH)SiO_{3/2} to 100 parts by weight of organopolysiloxane gum.

The above heat-resistant agent include iron oxides called colcothar red, colcothar yellow and colcothar black, aerosol titanium oxide, rareearth fatty acid salt, iron octylate, cerium zirconate, cerium silanolate, cerium siloxanate, cerium fatty acid salt, iron oxide, red iron oxide, red iron oxide, caesium oxide, cerium oxide, cerium hydroxide, barium zirconate, barium oxide, the combination of barium oxide and iron oxide and 0.001-10 parts by weight of iron oxide ferrites, which is solid solution in the range of 1-50 % by the weight of metal oxide such as beryllium oxide, magnesium oxide, calcium oxide, strontium oxide, barium oxide, cadmium oxide, mercury oxide, lead oxide, indium oxidize, chromium oxide, molybdenum oxide, tungsten oxide, cobalt oxide, nickel oxide, zinc oxide and copper oxide to 100 parts by weight of organopolysiloxane gum.

The subject composition, which imparts low-temperature resistance to silicone rubber moldings, include the composition consisting of 100 parts by weight of organopolysiloxane having 20-90 % by mole of methyl group, 8-80 % by mole of substituted or unsubstituted monovalent hydrocarbon group having 2 to 4 carbon atoms and 0-7 % by mole of aromatic monovalent hydrocarbon group in all silicone-bonded substituted group, 1-20 parts by weight of organopolysiloxane having viscosity (at 25°C) of 200cst or less and 20-100 parts by weight of silica filler.

The above antioxidants include acid acceptabe agents such as chinese white and calcium carbonate; or amine compounds such as naphthylamine, diphenylamine and p-phenylene diamine; quinones such as 1,4-benzoquinone, naphthoquinone, hydroquinone monomethylether and 2,5-di-tert-butylhydroquinone; catechols such as catechol and 4-tert-butyl catechol; pyrogallols such as 4,6-diphenylpyrogallol; monophenol compounds such as (3,5-tert-butyl-4-hydroxyphenyl)propionate and 2,6-di-tert-butyl-p-cresol; polyphenol compounds such as bisphenols or trisphenols; sulfur compounds such as 4,4-thiobis(3-methyl-6-tert-butylphenol), bis(2-methyl-4-(3-n-alkylthiopropionyloxy)-5-tert-butylphenyl)sulfide, 2,2-thiodiethylenebis(3-(3,5-tert-butyl-4-hydroxyphenyl) propionate and dilaurylthiopropionate; and benzoic acid derivatives such as benzoic acid, 4-chlorobenzoic acid, 2,4-dichlorobenzoic acid, sodium benzoate, ammonium benzoate and benzoyl peroxide, which can form benzoic acid by thermal decomposition.

The above age resistor include naphthylamine compounds such as phenyl-2-naphthylamine; diphenylamine compounds such as N, N' -diphenylethylene diamine; p-phenylene diamine compounds such as N,N'-diphenyl-p-phenylene diamine; quinoline compounds; hydroquinone derivatives such as 6-ethoxy-2,2,4-trimethyl-1,2-dihydroquinone; monophenol compounds such as 2,6-di-tert-butyl-4-methylphenol; bis, tris or polyphenol compounds such as 2,2'-methylene-bis-(4-ethyl-6-tert-butylphenol); thiobisphenol compounds such as 4,4'-thiobis-(6-tert-butyl-3-methylphenol); and 2-mercaptobenzimidazole, which can be used in the range of 0.1-10 parts by weight to 100 parts by weight of organopolysiloxane gum.

The additives used while manufacturing silicone rubber in this invention further include such additives that improve processability, productivity or stability.
They are exemplified by the additives which provide the silicone rubber suitable characters for extrusion molding or injection molding; which prevent foaming, blooming or discoloration; which improve releasability, processability, moldability, productivity and preservation stability. Still further, useful additives as processing auxiliary, plasticizer, reversal inhibitor, stabilizer, false crosslinking inhibitor or sagging inhibitor can be used.

The additives are illustrated in the following.

Silicone rubber base compound for extrusion molding is desirable to have higher green strength and lower surface tackiness.

The subject component, which improve green strength to silicone rubber moldings, include polytetrafluoroethylene, the paste obtained by impregnating 10-50% by weight of tetrafluoroethylene powder produced by emulsion polymerization into wet-process silica-water suspension, or 0.001-10 parts by weight of boric compounds such as boric acid, boric acid anhydride or alkyl borate to 100 parts by weight of organopolysiloxane gum In addition, 5-50 parts by weight of organopolysiloxane copolymer (the mole ratio of R^{a}₃SiO_{1/2} unit to SiO₂ unit (wherein 0.003-0.4 of R^{a} is vinyl group) is 0.6-1.2 : 1.0), or the mixture consisting of the above organopolysiloxane copolymer and 5-50 parts by weight of organopolysiloxane oil having degree of polymerization of 1,000 or less and vinyl group of 0.01-30 % by mole to 100 parts by weight of organopolysiloxane gum provides crosslinked silicone rubber moldings having higher hardness and higher modulus.

The subject component, which decreases tackiness on the surface of silicone rubber moldings, include 3-100 parts by weight of silicone elastomer particles having the ratio of hydrogen atom to vinyl group in the range of 0.8-5 : 1.0 in the above Ra and average particle size in the range of 0.1-250µm, or 0-10 parts by weight of organohydrogenpolysiloxane having 5% by mole of (CH₃) SiO unit, 50% by mole of (CH₃)SiO unit and 45% by mole of (CH₃)₂SiO unit and the viscosity of 18cst (25°C) to 100 parts by weight of organopolysiloxane gum.

The subject component, which imparts non-tacky surface, non-foaming and non-yellowing within crosslinking and also crosslinked effectively in deep part of thick moldings or surroundance of cable and wire coatings of silicone rubber moldings, include the combination of 0.1-10 parts by weight of organohydrogenpolysiloxane having at least two hydrogen atom in the above R^{a} in each molecular with platinum catalyst can be added together, or the combination of organohydrogenpolysiloxane having at least two hydrogen atom in the above R^{a} in each molecular with 0.05-10 parts by weight of dialkylperoxide to 100 parts by weight of organopolysiloxane gum.

A silicone rubber base compound for injection molding is desirable to gelling free and have excellent discharge (flow characteristics) with maintaining suitable viscosity before it's crosslinking.

The subject component include 0.05-20 parts by weight of methyloligosiloxane having one molecular terminal capped with silanol group and the other molecular terminal capped with trimethylsiloxy group to 100 parts by weight of organopolysiloxane gum and the composition consisting of 100 parts by weight of vinyl group-containing block silicone resin having 0.3-1.0 parts by weight of vinyl, phenyl or residual hydroxyl group, 250-400 parts by weight of silica filler and condensation catalyst (which is either of powder, grain or fiber solid at normal temperature, soften at 50°C or higher and has flowability at further high temperature.

The subject component, which imparts prevent foaming in crosslinking of silicone rubber, include calcium oxide, strontium oxide or barium oxide, which imparts prevent blooming in crosslinking of silicone rubber include calcium compounds, and also which prevents discoloration such as yellowing of silicone rubber moldings include organohydrogenpolysiloxane, 0.01-10 parts by weight of organohydrogenpolysiloxane having one or more hydroxyl or alkoxyl group in each molecule and the viscosity of 5-1,000 cst (25°C) or 0.001-10 parts by weight of blue or black pigments or organic pigments to 100 parts by weight of organopolysiloxane gum.

The subject component, which improve releasability of silicone rubber moldings from roller or metallic mold and improve workability, include
organic releasable compounds such as higher fatty acid, metal salt of higher fatty acid, fatty acid ester, fatty alcohol ester, fatty acid amide and paraffin. The above higher fatty acid include lauric acid, stearic acid, palmitic acid, oleic acid, archic acid and caprylic acid.

The above metal salt of higher fatty acid include magnesium stearate, calcium stearate, zinc stearate, cobalt stearate, aluminum stearate, barium stearate, lithiumstearate, strontium stearate, lead stearate, cadmium stearate, calcium chlorostearate, barium chlorostearate, cadmium chlorostearate, zinc laurate, barium laurate, magnesium laurate, calcium laurate, cadmium laurate, zincoleate, magnesiumoleate, manganese oleate, zinc myristate, aluminum myristate, barium ricinoleate, zinc ricinoleate and cadmium ricinoleate.

The above fatty acid ester include the ester of fatty acids such as saturated fatty acid of 4 to 9 carbon atoms, saturated fatty acid of 10 to 20 carbon atoms, unsaturated fatty acid of 12 to 20 carbon atoms, with alcohols such as methanol, ethanol, butanol, octanol, lauryl alcohol and cetyl alcohol or polyols such as ethylene glycol, sorbitol, glycerin, methylcellosolve.

The above fatty alcohol ester include, esters of dibasic acid with fatty alcohols such as ester of glutaric acid and ester of suberic acid, and esters of tribasic acid with fatty alcohols such as ester of citric acid with fatty alcohol such as saturated or unsaturated alcohol of 8 to 20 carbon atoms.

The above fatty acid amides include lauramide, stearamide, palmitamide, oleamide, N-methylstearamide, N-ethyllauramide, N,N-diethylpalmitamide and bis(lauric acid)ethylene amide.

The above paraffin include solid paraffin, paraffin wax, paraffin oil, liquid paraffin and polyethylene wax.

The subject component, which improve releasability of silicone rubber moldings from roller or metallic mold and improve workability, is usually added at 0.01-10 parts by weight to 100 parts by weight of organopolysiloxane gum, and may be used singly or as a suitable combination of two or more.

For example, the composition consisting of 0.025-5 parts by weight of higher fatty acid or metal salt thereof and 0.05-5 parts by weight of water, the composition consisting of 0.05-5 parts by weight of higher fatty acid or metal salt thereof and 0.1-10 parts by weight of organohydrogenpolysiloxane supported on reinforce filler, or the composition consisting of 1-20 parts by weight of organopolysiloxane oils such as dimethlsiloxane oil having molecular terminal capped with trimethylsiloxy group, dimethylsiloxane-methylphenylsiloxane copolymer oil having molecular terminal capped with trimethylsiloxy group, dimethylsiloxane-diphenylsiloxane copolymer oil having molecular terminal capped with trimethylsiloxy group and 0.05-5 parts by weight of water, the composition consisting of organic releasable compounds and polytetrafluoroethylene, the composition consisting of 0.03-5 parts by weight of organic releasable compounds and 0.01-5 parts by weight of polytetrafluorinated ethylene resin powder, the composition consisting of 0.025-5 parts by weight of organic releasable compounds and 0.1-10 parts by weight of organohydrogenpolysiloxane and 0.05-5 parts by weight of water, or the composition consisting of 0.01-5 parts by weight of organic releasable compounds and 0.1-10 parts by weight of organohydrogenpolysiloxane having two or more hydrogen atom in the above Ra in each molecule can also be added to 100 parts by weight of organopolysiloxane gum.

Sphere microparticulate catalysts consisting of 0.1-10 parts by weight of organohydrogenpolysiloxane having two or more hydrogen atoms in the above R^{a} in each molecule and silicone resin containing 0.01 or more parts by weight of platinum catalyst in term of platinum atom, 0.01-20 parts by weight of low molecular weight polyethylene having density of 0.95 or lower at 20°C, 0.01-0.1 parts by weight of organic azo compounds such as 2,2 '-azobis(2,4,4-trimethylpentane) and 1,1'-azobis(cyclohexane-1-carbonitrile), or 0.2-20 parts by weight of liquid polyisobutylene having average moecular weight of 250-4,000 and viscosity of 8-500,000 cst (at 25°C) can be added to 100 parts by weight of organopolysiloxane gum.

The subject component, which improve releasability of fluorosilicone rubber moldings, include the combination of perfluoroalkylsubstisuted-organosilicic compound having hydroxyl group with organosilicone compound, which is inert for fluorosilicone base polymer, soluble in benzene and having boiling point of higher than 100°C, or with polydiorganosiloxane having vinyl group.

The composition consisting of 100 parts by weight of organopolysiloxane having degree of polymerization of 1000 or more (the number of the above R^{a} per one silicone atom is in the range of 1.98-2.002, 5-50 % by mole of 3,3,3-trifluoropropyl group and 0.01-2 % by mole of vinyl group is contained in organic group thereof), 10-150 parts by weight of silica filler, 1-20 parts by weight of organopolysiloxane having degree of polymerization of 100 or more (the above R³ is monovalent hydrocarbon group selected from the group consistng of alkyl, phenyl and vinyl group, the number of R^{a} per one silicone atom is in the range of 1.98-2.02 and 2-25 % by mole of vinyl group is contained in each molecule), and 0.5-10 parts by weight of 3,3,3-trifluoropropyl group-containing silane represented by the formula CF₃CH₂CH₂R^{3b}ₕSi (OR^{3c})₍₃₋ₕ₎ (R^{3b} stands for substituted or unsubstituted monovalent hydrocarbon group, R^{3c} stands for alkyl or alkoxy group, h stands for 0, 1 or 2) or partially hydrolyzed and condensated product thereof can also be used.

The subject component, which improve processability and moldability of silicone rubber moldings, include 0.01-50 parts by weight of polytetrafluoroethylene or fluororesin powder (tetrafluoroethylene, hexafluoropropylene, chlorotrifluoroethylene, perfluoro(alkylvinylether), perfluoro(alkoxyalkylvinylether), trifluoroethylene, perfluoroalkylethylene or copolymer with the other monomers)
or in addition, 0.01-40 parts by weight of linear or cyclic organopolysiloxane having the unit represented by the formula F(CF₂)ᵢCH₂CH₂(CH₃)ⱼSiO_{(3-j)/2} (wherein i stands for an integer of 4 or more, j stands for 0, 1 or 2) for compatilizer of organopolysiloxane with fluororesin powder, to 100 parts by weight of organopolysiloxane gum.

The subject component, which increases crosslinking speed of silicone rubber and improve productivity, include the mixture of 0.01-5 parts by weight of silicone compound (having at least two hydrogen atoms in the above Ra in each molecule) selected from the group of organopolysiloxane, polysilalkylenesiloxane and polyphenylenesiloxane or the mixture thereof and 0.001-1parts by weight of iron salts of fatty acid such as iron acetate, ferrous oxalate and iron octylate, or 0.01-5 parts by weight of polysilalkylene siloxane, polysilane, polycarbosilane or the mixture thereof, which has at least two hydrogen atoms in the above Ra in each molecule, or the mixture of organohydrogenpolysiloxane and calcium compound to 100 parts by weight of organopolysiloxane gum.

The subject component, which improve productivity without needs for aftercrosslinking and yields silicone rubber moldings having excellent characters of water resistance, humidity resistance, electric character and mechanical strength by only precure or presscure, include the combination of 2-70 parts by weight of organoalkoxy group-containing silane or organoalkoxy group-containing siloxane, 1-60 parts by weight of organoamino group-containing silane or organoamino group-containing siloxane, 5-100 parts by weight of hydrophilic organic solvent, and solvents such as benzene, toluene and xylene for vinyl group-containing organopolysiloxane to 100 parts by weight of organopolysiloxane gum.

The subject component, which improve preservation stability by prevent from crosslinking inhibition responsible for the existence of microparticulate transition metals such as iron, cobalt, nickel, copper, molybdenum, silver and tungsten, include the combination of 0.1-1,000 ppm of chlorine atom free platinum complex in term of platinum to 100 parts by weight of organopolysiloxane gum and 2-1, 000 equivalent of the asymmetry organic peroxide to platinum complex.

The above plasticizer include phthalate, adipate, maleate, fumarate and phosphate.

The above stabilizer include organophosphorus compound and organic amine.

The subject component, which minimize plasticization reversion of silicone rubber, is

0.2-20 parts by weight of organohydrogenpolysiloxane having at least two HR^{a}₂SiO_{1/2} units in each molecule to 100 parts by weight of organopolysiloxane gum,
the subject component, which improve reversion (softening of rubber), is magnesium oxide
the subject component, which prevents from false-crosslinking, is alcohols such as methanol, ethanol, isopropanol, propylene glycol and glycerin, and sagging inhibitors as well can be added properly.

In addition to the above additives, the additives such as surface-active agent, crosslinking auxiliary agent, silane compound, silane coupling agent, organic solvent, silane, siloxane and water or the like can be added while manufacturing silicone rubber.

Such additives are illustrated in the following.

The above surface-active agent includes all of various surface-active agents having ability of surface-activie such as nonionic, cationic, anionic and amphoteric surfactants.

The above nonionic surfactant is exemplified by sucrose fatty acid esters, glycerin fatty acid esters, decaglycerin fatty acid esters, polyglyceryl fatty acid esters, sorbitan fatty acid esters, propyleneglycol fatty acid esters, propyleneglycol pentaerythritol fatty acid esters, propyleneglycol pentaerythritol fatty acid esters, polyoxyethylene glycerin fatty acid esters, polyoxyethylene sorbitan fatty acid esters, polyoxyethylene sorbitol fatty acid esters, alkyl glyceryl ethers, polyoxyethylene alkyl ethers, polyoxyethylene alkenyl ethers, polyoxyethylene alkyl phenyl ethers, polyoxyethylene polystyryl phenyl ethers, polyoxyethylene polyoxy propylene ethers, polyoxyethylene polyoxypropylene alkyl ethers, polyethyleneglycol fatty acid esters, polyoxyethylene fatty acid esters, polyglyceryl fatty acid esters, crosslinked polyoxyethylene castor oils, fatty acid diethanolamides, polyoxyethylene alkylamines, triethanolamine fatty acids esterifiedpartially, trialkylamine oxides, alkylalkanolamides, ethyleneglycol adducts of acetyleneglycol, fluoroalkyl group-containing nonionic surfactants such as polyoxyethylene perfluoroalkylether, fluoroalkylsiloxane group-containing nonionic surfactant, and the analogue of nonionic sarfactant.

The above cationic surfactant is exemplified by alkylamine salt, alkyl trimethyl ammonium salt, dialkyl dimethyl ammonium salt, tetra-alkyl ammonium salt, trialkyl benzyl ammonium salt, alkyl pyridinium salt, N, N'-dialkyl morpholinium salt, polyethylene polyamine fatty acid amide salt.

The above anionic surfactant is exemplified by salt of fatty acids, alkylbenzene sulfonates, alkylnaphthalene sulfonates, alkyl sulfonates, α -olefine sulfonates, dialkyl sulfosuccinates, salt of α-sulfonated fatty acids, N-acyl-N-methyl taurates, alkyl sulfates, sulfonated fats, polyoxyethylene alkyl phenyl ether sulfates, polyoxyethylene styrenatedphenyl ether sulfates, alkyl phosphates, polyoxyethylene alkyl ether phosphates, polyoxyethylene alkylphenyl ether phosphates, naphthalene sulfonate formaldehyde polycondensation

The above Amphoteric surfactants is exemplified by N, N-dimethyl-N-alkyl-N-carboxy methyl ammonium betaines, N,N-dialkyl amino alkylene carboxylates, N,N,N-trialkyl-N-sulfoalkylene ammonium betaines, N,N-dialkyl-N,N-bispolyoxyethylene ammonium sulfuric ester betaines, 2-alkyl-1-carboxymethyl-1-hydroxyethylimidazolinium betain.

The above crosslinking aids having doublebond or triplebond of carbon atom in each molecule include (meth)acrylic group-containing compounds such as trimethylolethane tri(meth)acrylate, trimethylolpropane tri(meth)acrylate, glycerin tri(meth)acrylate, tetramethylolmethane tri(meth)acrylate, pentaerythritol tri(meth)acrylate, pentaerythritol tetra(meth)acrylate, ethylenedi(meth)acrylate, 1,3-propylene di(meth)acrylate, neopentylglycol di(meth)acrylate, 1,4-butylene di(meth)acrylate, 1,6-hexanediol di(meth)acrylate, ethyleneglycol di(meth)acrylate, butyleneglycol di(meth)acrylate, 1,3-butyleneglycol di(meth)acrylate, triethyleneglycol di (meta) acrylate, tetraethyleneglycol di (meth) acrylate, polyethyleneglycol di (meth) acrylate, benzyl(meth)acrylate, N,N'-methylenebis(meth)acrylamide, diallylphthalate, vinyl (meth) acrylate and allyl (meth) acrylate, divinylbenzene, polybutadiene, diallylphthalate, vinyl crotonate, 2,4-diphenyl-4-methyl-1-pentene, N,N-m-phenylenebismaleimido, triallylisocyanurate, triallylcyanurate, triallyltrimellitate, dibenzoylquinonedioxime, 3-methacryloxypropyltrimethoxysilane, vinyltriethoxysilane, polyalkylsiloxane having vinyl group of two or more in each molecule, or a compound obtained by reaction of the compuonds having triplebond of carbon atom in each molecule such as 3,5-dimethyl-1-hexyne-3-of with organicsilicone compound.

These may be employed singly or as a suitable combination of two or more.

The above silane compound, which is a silicone compound such as chlorosilane, alkoxy silane, silazane and specific silane used as silicone monomer, include organochlorosilanes such as trimethylchlorosilane, dimethylbutylchlorosilane, dimethyloctadecylchlorosilane, tert-butyldimethylchlorosilane, methyldichlorosilane, dimethyldichlorosilane, methylphenyldichlorosilane, diphenyldichlorosilane, methyltrichlorosilane and phenyltrichlorosilane; organoalkoxysilanes such as tetramethoxysilane, tetraethoxysilane, tetrapropoxysilane, methyltrimethoxysilane, methyltriethoxysilane, methyltripropoxysilane, ethyltrimethoxysilane, ethyltriethoxysilane, n-propyltrimethoxysilane, chloropropyltrimethoxysilane, isobutyltrimethoxysilane, hexyltrimethoxysilane, decyltrimethoxysilane, phenyltrimethoxysilane, dimethyldimethoxysilane, dimethyldiethoxysilane, diethyldiethoxysilane, diphenyldimethoxysilane, diphenyldiethoxysilane, trimethylmethoxysilane and trimethylethoxysilane; organosilazanes such as hexamethyldisilazane, divinyltetramethyldisilazane, diphenyltetramethyldisilazane, 1,3-bis(3,3,3-trifluoropropyl)tetramethyldisilazane, octamethyltrisilazane and 1,1,3,3,5,5,7,7-octamethylcyclotetrasilazane, and N,N-bis(trimethylsilyl)urea.

The above silane coupling agent, which has one or more different reactive group selected from both reactive groups (one of the reactive group, such as methoxy, ethoxy, 2-methoxyethoxy and 2-ethoxyethoxy group, is hydrolyzable and possible to bond with inorganic material, and the other reactive group, such as vinyl, epoxy, (meth) acrylic, (meth) acrylamide, mercapto and amino group, is possible to bond with organic material) respectively, effects as a binder of organic material with inorganic material.

The above silane coupling agent include vinyl group-containing alkoxysilanes such as vinyltrimethoxysilane, vinyltriethoxysilane, vinyltripropoxysilane, vinyltris(2-methoxyethoxy)silane, vinylmethyldimethoxysilane, vinylmethyldiethoxysilane, vinyldimethylmethoxysilane, vinyldimethylethoxysilane, vinyldimethylpropoxysilane, allyltrimethoxysilane, allyltriethoxysilane, allylmethyldiethoxysilane, butynyltrimethoxysilane, hexenyltrimethoxysilane and divinyldimethoxysilane; epoxy group-containing alkoxysilanes such as 3-glycidoxypropyltrimethoxysilane, 3-glycidoxypropyltriethoxysilane, 3-glycidoxypropyl(methyl)dimethoxysilane, 2-(3,4-epoxycyclohexyl)ethyltrimethoxysilane, 2-(3,4-epoxycyclohexyl)ethyltriethoxysilane and 2-(3,4-epoxycyclohexyl)ethyl(methyl)dimethoxysilane; (meth)acrylic group-containing alkoxysilanes such as 3-(meth)acryloxymethyltrimethoxysilane, 3-(meth)acryloxypropyltrimethoxysilane, 3-(meth)acryloxypropyltriethoxysilane, 3-(meth)acryloxypropylmethyldimethoxysilane and 3- (meth)acryloxypropylmethyldiethoxysilane; (meth)acrylamide group-containing alkoxysilanes such as N-methylol(meth)acrylamide, N,N'-dimethyl(meth)acrylamide, diacetone(meth)acrylamide, N,N'-methylenebis(meth)acrylamide and (N-(meth)acrylic)3-aminopropyltrimethoxysilane; mercapto group-containing alkoxysilanes such as 3-mercaptopropyltrimethoxysilane, 3-mercaptopropylmethyldimethoxysilane, 3-mercaptopropylmethyldimethoxysilane and 3-mercaptopropylmethyldiethoxysilane; amino group-containing alkoxysilanes such as 3-aminopropyltrimethoxysilane, 3-aminopropyltriethoxysilane, N-(2-aminoethyl)-3-aminopropyltrimethoxysilane, N-(2-aminoethyl)-3-aminopropylmethyldimethoxysilane and N-2-(N-vinylbenzylaminoethyl) -3-aminopropyltrimethoxysilane; amino group and vinyl group-containing silanes such as dimethylvinyl(aminoethyl)silane, dimethylvinyl (aminopropyl) silane and dimethylvinyl (aminobutyl) silane; vinyl group-containing alkoxysilazanes such as 1,1,3,3-tetramethyl-1,3-divinyldisilazane and 2,4,6-trimethyl-2,4,6-trivinylcyclosilazane, trimethoxysilylethylvinylether, triethoxysilylethylvinylether, methyldimethoxysilylethylvinylether, methyldiethoxysilylethylvinylether, trimethoxysilylpropylvinylether, triethoxysilylpropylvinylether, methyldimethoxysilylpropylvinylether, and methyldiethoxysilylpropylvinylether.

The above organic solvent include hydrocarbons such as benzene, toluene, xylene, n-hexane, cyclohexane, n-heptane, petroleum benzine and gasoline; halogenated hydrocarbons such as 1,1,1-trichloroethane; alcohols such as methanol, ethanol, propanol, isopropanol and tert-butanol; ketones such as acetone, methylethylketone, methylisobutylketone and diethyl ketone; esters such as ethyl acetate, butyl acetate, propyleneglycolmonomethylether acetate; ethers such as diethyl ether, dioxane and tetrahydrofuran; polyols such as ethyleneglycolmonomethylether, ethyleneglycolmonoethylether, ethyleneglycolmonobutylether, diethyleneglycolmonomethylether, diethyleneglycolmonoethylether, diethyleneglycolmonobutylether, diethyleneglycoldiethylether, propyleneglycolmonomethylether, propyleneglycoldimethylether, propyleneglycolmonoethylether, dipropyleneglycoldimethylether, tripropyleneglycoldimethylether, aceticacid 2-methoxyethanol, acetic acid diethylene glycol monomethylether, acetic acid diethyleneglycolmonoethylether and ether threof.

The above silane include tetraethylsilane, dimethyldiethylsilane, trimethylbutylsilane, and the above siloxanes include linear siloxanes such as hexamethyldisiloxane, octamethyltrisiloxane, decamethyltetrasiloxane and dodecamethylpentasiloxane; branched siloxanes such as 3-trimethylsiloxy-1,1,1,3,7,7,7-heptamethyltrisiloxane; and cyclic siloxanes such as hexamethylcyclotetrasiloxane, octamethylcyclotetrasiloxane, decamethylcyclopentasiloxane and 1-ethyl-3,3,5,5,7,7-hexamethylcyclotetrasiloxane.

Water can also be used as mentioned above.

Suitable combinations of the above organopolysiloxane gum, reinforce fillers and other additives would provide silicone rubber compositions used for electrical wire and cable coatings, optical fiber coatings, high voltage electrical insulators, sponges, gaskets for building, waterproof seals for building, medical supplys, sound mediums, sealants for food container, applications to fixing roller, electrically conductive rubber application to electrically conductive rollers, loading paper rollers, rubber contacts, keyboards, coating agents for airbag cloth, diaphragms, wiper blades, antistatic materials, seats for insulation and releasing heat, potting materials, protection seats for solar battery module, intermediate layers for trilaminar resist, extreme infrared rediation radiators, oil bleeds and parts for swimming articles and so on.

The suitable combination for each use will be mentioned in the following.

For example, the following additives can be added to silicone rubber for electrical wire and cable coatings to improve electric characters, cut-strip property and flame resistance of crosslinked silicone rubber moldings, and to prevent them from discoloration.

The subject aditives, which improve electric characters such as voltage resistance, tracking resistance, arc resistance and erosion resistance of silicone rubber moldings for electrical wire and cable coatings, include boron nitride powder, especially high purity boron nitride powder having average particle size of 20µm or less, hydrotalcites and aluminum hydroxide powder.

The subject aditives, which improve the cut-strip property, in other words, the nature that the edge of silicone rubber of silicone-rubber-covered electrical wire can be striped off easily due to no joining or no adhesion of silicone rubber to a conductor (the adhesion of silicone rubber to the core wire) in use, and to improve electrical properties such as insulating property, include diatomaceous earth; quartz powder; magnesium oxide; powder inorganic zinc compounds such as zinc oxide, zinc carbonate, zinc hydroxide, zinc sulfide and selenium-zinc; zinc compounds of fatty acid such as zinc stearate, zinc palmitate, zinc caprylate, zinc laurate and zinc oleate; fatty acid metal salts such as calcium stearate; fluororesin oil or wax; esters of monocarboxylic acid of 6 or more carbon atoms with alcohol; esters of monocarboxylic acid of 6 ormore carbon atoms with polyol; ester waxes such as esters of polycarboxylic acid of 6 or more carbon atoms with monoalcohol of 6 or more carbon atoms having melting point of 180°C or higher; fatty acid-modified organopolysiloxanes having groups such as -CH₂OCOC₁₇H₃₅, -(CH₂)₂OCOC₁₅R₃₁ and- (CH₂)₄OCOC₁₇H₃₅ or the combinations of the above fatty acid-modified organopolysiloxanes with diatomaceous earth or quartz powder; the combinations of fatty acid metal salt of 10-18 carbon atoms with magnesium oxide; or 0.01-20 parts by weight of the combinations of fatty acid or fatty acid metal salt with zinc oxide powder to 100 parts by weight of organopolysiloxane gum.
The subject composition consisting 100 parts by weight of organopolysiloxane gum having both molecular terminals capped with ethylenically unsaturated groups and 5-200 parts by weight of diatomaceous earth and/or quartz powder can also be used.

The subject aditives, which improve flame resistance of silicone rubber moldings for electrical wire and cable coatings, include 0.1-10 parts by weight of molybdenum compounds consisting zinc molybdate and powdery eutectic compound of zinc molybdate with oxide of the metals belonging to secondary group in the periodic table towards 100 parts by weight of organopolysiloxane gum. The composition consisting 100 parts by weight of organopolysiloxane gum, 1-10 parts by weight of fumed titanium dioxide, 1-50 ppm of platinum compound expressed in terms of the platinum atom content and 0.1-2 parts by weight of fatty acid amide such as lauric acid, palmitic acid, stearic acid and oleic acid having 10 or more carbon atoms can also be used.
The subject additives for silicone rubber moldings for electrical wire and cable coatings composed by glass cloth, which is interpenetrated with varnish such as acrylate, polyester and silicone, include the composition of 0.1-1,000 ppm of platinum compound expressed in terms of the platinum atom content per organopolysiloxane gum and 0.1-200 parts by weight of rutile-type titanium dioxide towards 100 parts by weight of organopolysiloxane gum.

The composition consisting 100 parts by weight of polydiorganosiloxane gum having the degree of polymerization of 2,000 or more (having 0-10 % by mole of phenyl group-containing siloxane unit and 0-2 % by mole of vinyl group-containing siloxane unit), 10-100 parts by weight of silica filler, 1-10 parts by weight of fumed titanium dioxide, 1-50 ppm of platinum compound expressed in terms of the platinum atom content per organopolysiloxane gum, 0.1-5 parts by weight of cerium hydroxide and 0.1-10 parts by weight of molybdenum compound consisting zink compounds such as zinc ammonium and zinc molybdate and powdery eutectic compounds of the above zink compounds with oxide of metals belonging to secondary group in the periodic table can also be used.
The subject aditives, which prevent discoloration of silicone rubber moldings for electrical wire and cable coatings, include 0.1-5 parts by weight of crosslinking auxiliary agent having double bond or triple bond of carbon atom in each molecule and molecular weight of 80-1,000 towards 100 parts by weight of organopolysiloxane gum.

The subject aditive, which improve adherence to glass fiber with silicone rubber moldings for optical fiber coatings, can be the composition consisting of organopolysiloxane having vinyl group in the above R^{a} and organohydrogenpolysiloxane having hydrogen atom in the above R^{a}, or the composition consisting of organopolysiloxane (the number of the above R^{a} per one silicone atom is more than 1 and 3 or less and containing 0.0004-3 % by mole of aliphatic unsaturated group), organopolysiloxane (the number of the above R^{a} is more than 1 and 3 or less per one silicone atom and containing 0.0004-3 % by mole of mercaptoalkyl group), mercapto group-containing alkoxy silane and sensitizer can be added to.

High voltage electrical insulators, is made of the composition consisting of organopolysiloxane elastomer and crosslinkable resin, or the material obtained by coating organopolysiloxane composition on outside surface of electrical insulator, and the like.
A silicone rubber molding for high voltage electrical insulator is required for resistance to weather, to pollution, to voltage, to tracking, to arc, and to erosion, 50-200 parts by weight of zinc carbonate powder or basic zinc carbonate powder, or 1-100 parts by weight of titanium dioxide powder for photocatalyst having photocatalyst function, which can keep resistance characteristics to voltage for a long time, and average particle size of 0.3µm or smaller and crystal unit lattice of anatase type can be added to 100 parts by weight of organopolysiloxane gum.

15-900 parts by weight of aluminum hydroxide represented by the formula Al₂O₃(H₂O)₃, especially, aluminum hydroxide consisting of 10-95 parts by weight of aluminum hydroxide having average particle size of 7-50µm and 90-5 parts by weight of aluminum hydroxide having average particle size of 7µm or less, the combination of 30-300 parts by weight of aluminum hydroxide and 0.1-20 parts by weight of metal oxide containing transition metal selected from the group consisting of Co₂O₃, Co₃O₄, MoO₃, CeO₃, FeO · Fe₂O₃, Fe₂O₃, TiO₂, V₂O₅, NiO₃, ZrO₂, Cr₂O₃, CrO₃, PdO₂, MnO₂, ZnO, RuO₂, Ni₃O₄, Nb₂O₅, La₂O₃, WO₂, or the combination of 30-300 parts by weight of aluminum hydroxide and 0.1-50 parts by weight of antimony trioxide can be added to 100 parts by weight of organopolysiloxane gum.

Aluminum hydroxide, especially, hydrophobic treatmented aluminum hydroxide having the content of water soluble-sodium ion of 0.01 % by weight or less, ph value of 30% slurry in water of in the range of 6.5≦pH≦8.0 and electric conductivity of 50µs/cm or less,
aluminum hydroxide surface-treated to have vinyl group of 1.0×10⁻⁶-2.0 ×10⁻⁴ mole per 1g, or 10-500 parts by weight of aluminum hydroxide powder surface-treated with silane or siloxane oligomer having ethylenically unsaturated group and alkoxy or hydroxyl group can be added to 100 parts by weight of organopolysiloxane gum.

The combination of 10-300 parts by weight of aluminum hydroxide powder and 0.1-30 parts by weight of silane coupling agent having ethylenically unsaturated group and alkoxy or hydroxyl group, or the combination of 10-300 parts by weight of aluminum hydroxide and dimethlpolysiloxane having molecular terminal of silicone-bonded alkoxy group of 1 to 3 carbon atoms and average degree of polymerization of 5-500 can be added to 100 parts by weight of organopolysiloxane gum.

1-20 parts by weight of organosilane or organosiloxane oligomer having phenyl group and having both molecular terminals capped with hydroxyl group, or polymethylalkylpolysiloxane oil having both molecular terminals capped with trialkylsiloxy group, or polymethylalkylpolysiloxane oil having both molecular terminals capped with silanol group, or 0.5-50 parts by weight of one or more polyoxyalkylene-containing compounds selected from the group consisting of polyoxyalkylene derivatives represented by the formula (R^{4b}-O-(C₂H₄O)ₚ-(C₃H₆O)_{q}-R^{4c} (wherein R^{4b} and R^{4c} stand for hydrogen atom or monovalent hydrocarbon group having 1 to 18 carbon atoms, p stand for 1-50, q stand for 0-50)) and polyoxyalkylene-modified silicone oil represented by the formula R^{4d}₍₃₋ᵣ₎(A⁴)ᵣSiO-( (R^{4d})₂SiO)ₛ-( (R^{4d}) (A⁴)SiO)ₜ-Si(A⁴)ᵣR^{4d}₍₃₋ᵣ₎ (wherein R^{4d} stand for substituted or unsubstituted monovalent hydrocarbon group having 1 to 18 carbon atoms and R^{4d} can be the same or different, A⁴ stand for -(CH₂)ᵤ-O-(C₂H₄O)ₚ-(C₃H₆O)_{q}-R^{4c}, u stand for an integer in the range of 0-5, r stand for an integer in the range of 0-3, s stand for an integer in the range of 0-100, t stand for an integer in the range of 0-100, r+t stand for an integer of 1 or more), or 1-100 parts by weight of liquid organopolysiloxane having both molecular terminals capped with (CH₃)₂(OH)SiO_{1/2} unit (silicone-bonded organic groups are monovalent saturated hydrocarbon group, and have viscosity in the range of 100-100,000 centipoise at 25°C), or 1-20 parts by weight of organopolysiloxane represented by the formula R^{5b}ᵥ(OX⁵)_{w}SiO_{(4-v-w)/2} (wherein, R^{5b} stand for substituted or unsubstituted monovalent hydrocarbon group, R^{5b} can be the same or different and R^{5b} has at least 10 % by mole of phenyl group, X⁵ stand for hydrogen atoms or substituted or unsubstituted monovalent hydrocarbon group, which can be the same or different, v and w stand for an integer satisfiing 1.0≦v≦2.0, 1<v+w≦3, and 0.001≦w/ (v+w) ≦0.8 respectively), or 1-60 parts by weight of phenyl silane having hydrolyzable groups such as phenyltrimethoxysilane, phenyltriethoxysilane, phenyltrimethylethylketooxysilane, phenyltriacetoxysilane, phenyltrivinyloxysilane and phenyltri(diethylamino)silane can be added to 100 parts by weight of organopolysiloxane gum.

1-100 parts by weight of non-sphere silica filler and 100-900 parts by weight of sphere and meltable silica filler consisting of 50-95 parts by weight of sphere and meltable silica having average particle size of 7-40 µm and 50-5 parts by weight of sphere and meltable silica having average particle size of 0.1-6.5µm can be added to 100 parts by weight of organopolysiloxane gum to improve filling property of filler.

A silicone rubber composition for high voltage electrical insulator is, for example, exemplified by the composition consisting of 100 parts by weight of organopolysiloxane (comprises 70 % by weight or more of organopolysiloxane having both molecular terminals capped with trivinylsilyl, divinylmethylsilyl or vinyldimethylsilyl group having viscosity of 400-100, 000 centipoise at 25°C, 1-100 parts by weight of silica filler, 50-300 parts by weight of aluminum hydroxide represented by the formula Al₂O₃(H₂O)₃ and 0-20 parts by weight of organopolysiloxane (both molecular terminals capped with (CH₃)₃SiO_{1/2} unit) having viscosity of 30-100,000 centipoise at 25°Ccan be added to 100 parts by weight of organopolysiloxane gum.

Silicone rubber sponge is sponge-shaped crosslinked silicone rubber molding obtained by adding organic forming agents and foaming auxiliaries to organopolysiloxane gum.

The above organic forming agenst, which is stable at room temperature but which is decompose at decomposition temperature or higher to release N₂ gas or CO₂ gas, include organic azo compounds such as azobisisobutyronitrile, azobisformamide, diazoaminobenzene, azodicarvoneamide, barium azodicarboxylate, 2,2'-azobis-2-methylbutyronitrile, 2,2'-azobis(2,4,4-trimethylpentane), 2,2'-azobis-2,4-dimethylvaleronitrile, 2,2'-azobis(4-methoxy-2,4-dimethylvaleronitrile), 1,1'-azobis(cyclohexane-1-carbonitrile), 1,1'-azobis(1-acetoxy-1-phenylethane) and terephthalamideazodicarbonamide; nitroso compounds such as dinitrilopentamethylenetetramine and N,N-dimethyl-N,N-dinitrosoterephthalamide; hydrazine derivatives such as benzenesulfonylhydrazide, 4,4'-oxybis(benzenesulfonylhydrazide), paratoluenesulfonylhydrazine and trihydrazinetriazine; tert-alkylhydrazinium salts such as 5-phenyltetrazole, tert-butylhydrazinium chloride and di-tert-butylhydrazinium sulfate; carbonyl hydrazines such as 2-propenate hydrazide and acetyl hydrazine; inorganic foaming agents having decomposition temperature in the range of 40-280°C such as ammonium carbonate and sodium bicarbonate; foaming agents of plastic hollow microsphere packed volatile compound into plastic shell, which swells by heating.

The above organic forming agents usually added 1-10 parts by weight to 100 parts by weight of organopolysiloxane gum.

The above foaming auxiliarie include urea, stearic acid and zinc oxide. Organic peroxides such as dialkyl peroxide or peroxy ester can be added together to raise foaming magnification and yields uniform cell of silicone rubber moldings.

The method to obtain silicone rubber sponge without using the above foaming agents include the method using condensation reaction of hydroxyorganopolysiloxane with organohydrogenpolysiloxane under existence of heavy metal salt of organic acid and quaternary ammonium salt; the method using addition reaction of vinylorganopolysiloxane with organohydrogenpolysiloxane under existence of platinum catalyst and then obtained silicone rubber composition is foamed by decomposed gas of thermal decomposable foaming agent; and the method using addition reaction of organohydrogenpolysiloxane under existence of platinum catalyst to crosslinking and foaming.

In preparing silicone rubber sponge, the following additives can be added alone or in combination of two or more into silicone rubber base compound to yield sponge-shaped silicone rubber moldings having high foaming magnification, uniform and microstructural cell, no problem of surface smothness and surface tack, excellent property in heat-resistance, high strength, tear strength and compression set, no necessity of long postcure, and no causing depolymerization.

The above additive, for example, 0.01-5 parts by weight of polytetrafluoroethylene; the combination of organopolysiloxane having no fluorine atom in the above R^{a} and 0.1-5 parts by weight of organopolysiloxane having 10-50 % by mole of fluorine atom in the above R^{a}; the combination of 0.1-20 parts by weight of 1,1'-azobis(1-acetoxy-1-phenylethane) and 0.1-20 parts by weight of organosilicone compound having hydrogen atom in the above R^{a}, the combination of 0.5-5 parts by weight of organopolysiloxane-polyoxyalkylene copolymer and 1-20 parts by weight of silane or siloxane having viscosity of 20cst at 25°C, the combination of 0.3-10 parts by weight of foaming agents selected from the group consisting of p-toluenesulfonylhydrazide and derivatives thereof and 0.01-30 parts by weight of oxide or hydroxide of alkaline earth metal, the combination of 0.1-10 parts by weight of organohydrogenpolysiloxane having two or more hydrogen atom in the above R^{a} and 0-5 parts by weight of silicone regularizing bubble agent and 0.1-10 parts by weight of elastic recovery agents such as silicone resin having melting point of 150°Cor lower and organopolysiloxane copolymer having at least one silicone-bonded hydroxyl group in each molecule can be added to 100 parts by weight of organopolysiloxane gum.

The combination of 0.1-20 parts by weight of 1,1'-azobis(1-acetoxy-1-phenylethane) and 0.01-10 parts by weight of oxides, hydroxides, carbonates or hydrotalcite compounds of metal (which belongs to first group or secondary group of the perodic table such as zinc oxide, calcium oxide, magnesium oxide, barium oxide, barium peroxide, lithium hydroxide, potassium hydroxide, sodium hydroxide, zinc hydroxide, calcium hydroxide, magnesium hydroxide, barium hydroxide, zinc carbonate, calcium carbonate, magnesium carbonate, barium carbonate, Mg_{4.5}Al₂(OH)₁₃CO₃ · (H₂O)_{3.5}, and Mg_{4.5}Al₂(OH)₁₃CO₃), or the combination of 0.01-50 parts by weight of dimethyl-2,2'-azobisisobutyrate and 1-1,000 ppm of platinum catalyst in term of platinum atom and 0.01-10 parts by weight of organohydrogenpolysiloxane having two or more hydrogen atom in the above Ra can be used to 100 parts by weight of organopolysiloxane gum.

The subject aditive, which makes low temperature crosslinking of silicone rubber moldings for sponge, include the combination of 0.01-5 parts by weight of silicone compound, having two or more hydrogen atom in the above Ra, selected from the group consisting of organopolysiloxane, polysilalkylenesiloxane and polyphenylenesiloxane and 0.001-1 parts by weight of iron salt of fatty acid to 100 parts by weight of organopolysiloxane gum.

The subject aditive, which prevents aging by heat of silicone rubber sponge, include the combination of 4,4'-oxybis(benzenesulfonylhydrazide) as foaming agenst and alkaline earth metal salts such as beryllium oxide, magnesium oxide, calcium oxide, strontium oxide, barium oxide, magnesium hydroxide, calcium hydroxide, strontium hydroxide, and barium hydroxide.

The subject aditive, which prevents corrosion of copper silicone rubber sponge, include the combination of 0.5-20 parts by weight of azobisisobutyronitrile or azodicarvone amide, and 0.1-10 parts by weight of metals, belong to secondary group of the perodic table, such as zinc oxide, calcium oxide, magnesium oxide, barium oxide, barium peroxide, zinc hydroxide, calcium hydroxide, magnesium hydroxide, barium hydroxide, zinc carbonate, calcium carbonate, magnesium carbonate and barium carbonate to 100 parts by weight of organopolysiloxane gum.

The subject aditive, which provide character of adjust elastic recovery and return gradually to original form after transforming under compression to silicone rubber sponge, include 3-20 parts by weight of α , ω -dihydroxyorganopolysiloxane having phenyl group or alkyl group having 2 to 10 carbon atoms to 100 parts by weight of organopolysiloxane gum.

The subject aditive, which raise smothness of silicone rubber sponge surface, include 2-20 parts by weight of acrylic rubber to 100 parts by weight of organopolysiloxane gum.

The subject aditive, which prevent formation of high toxic decomposition product silicone rubber moldings for sponge, include azo compounds selected from the group consisting of 2,2'-azobis(4-methoxy-2,4-dimethylvaleronitrile), 2,2'-azobis(dimethylvaleronitrile) and 2,2'-azobis(2-methylbutyronitrile), and then obtained base compound crosslinked at 80-100°C.

The subject aditive, which provide conductivity to silicone rubber sponge, include the combination of hydroxyl or alkoxy group-capped organosilicone compounds having phenyl group and average degree of polymerization of 1,000 or less, 3-300 parts by weight of conductive agents, and 0.1-20 parts by weight of organic forming agents to 100 parts by weight of organopolysiloxane gum.

In the case of using 2-50 parts by weight of 1,1'-azobis(1-acetoxy-1-phenylethane) as foaming agents, 100 parts by weight of organopolysiloxane having 2-50 % by mole of phenyl group in the above Ra and 0.5-40 parts by weight of organosilicone compound having phenyl group and average degree of polymerization of 1,000 or less can be added together to 100 parts by weight of organopolysiloxane gum to prevent blooming.

A fireproof sponge gasket can be obtained by adhibit crosslinking using double extrusion molding of the composition, consisting of 1-20 parts by weight of cerium hydroxide, 10-50 parts by weight of tri-iron tetroxide powder, 5-45 parts by weight of laminal mineral clay powder having average primary particle size of 20µm or less, 1-3,000 ppm of platinum or platinum compound in term of platinum atom, foaming agents in required quantity and 0.01-10 parts by weight of tetrazole compound, and silicone-modified EPDM.

The above laminal mineral clay powder include muscovite, paragonite, fuloppite, biotite, lepidolite, zinnwaldite, fluorine phlogopite, kaolinite, talc, sericite, montmorillonite, chlorite, halloysite, and the above tetrazole compound include 5-phenyltetrazole, 5-aminotetrazole, 5,5-bistetrazole-2-ammonium salt, 5,5-bistetrazole-2-aminoguanidine salt, 5,5-bistetrazolepiperazine salt, and azobistetrazole-2-aminoguanidine salt.

To prepare wether-strip for automobile made of silicone rubber sponges having excellent character in compression set, low-temperature resistance and tearing strength, 0.01-5 parts by weight of polytetrafluoroethylene can be added to 100 parts by weight of organopolysiloxane gum, or to prepare fixed form of seal having excellent character in fireproof, 1-2,000 ppm of platinum catalyst in term of platinum atom and 5-45 parts by weight of ceramics agent can be added to 100 parts by weight of organopolysiloxane gum.

The above ceramics agents include clay minerals such as glass, asbesto, kaolin and montmorillonite; mica, talc, aluminium silicate, magnesium silicate, zincsilicate, zirconium silicate, titanium silicate, alumina, magnesium oxide, zirconia, tungsten carbide, titane carbide, molybdenum carbide, silicone carbide, aluminum calcium silicate, aluminium magnesium silicate and aluminium lithium silicate.

Silicone rubber for crosslinked sponge is obtained from the composition consisting of 100 parts by weight of polymer (consisting of 50-95 parts by weight of ethylene-α-olefine copolymer rubber and 50-5 parts by weight of organopolysiloxane having vinyl group in the range of 0-3.0 % by mole in the above Ra), 15-100 parts by weight of silica filler and 1-50 parts by weight of fluororesin powder having melting point of 200°C or more.

5-50 parts by weight of additives (10-500 parts by weight of cyclic or linear dimethylsiloxane having siloxane unit of 3-10 is supported on 100 parts by weight of inorganic micropowder having average grain size of 1-50µm and oil absorption of 300cc/100g or more and minute pore), or 0.5-10 parts by weight of porous filler having ability of oil absorption of 300cc/100g or more can be added to 100 parts by weight of organopolysiloxane gum to yield crosslinked silicone rubber moldings used for silicone gasket for building having excellent character in wetherability, low compression set, molding processability, creep resistance and pollution resistance.

Silicone rubber zipper gasket for building, which has excellent wetherability, creep resistance, high glass retention, wind resistance, temperature stability, and coloration free, can be obtained by the composition consisting of 100 parts by weight of ethylene-propylene polymer and/or ethylene-propylene-diene polymer, 10-500 parts by weight of organopolysiloxane and 10-150 parts by weight of silica fillers silicone rubber zipper gasket for building, can also be used of crosslinked silicone rubber moldings having crosslinked membrane consist of ethylene-propylene-diene copolymer, organohydrogenpolysiloxane and platinum on the surface.

Silicone adhesives, which has self-adhesive property used for waterproof building seal, can be obtained by using the composition consisting of 100 parts by weight of dimethylpolysiloxane having no ethylenically unsaturated group in the above R^{a} and average degree of polymerization of 5,000-9,000 and 50-150 parts by weight of organopolysiloxane having two or more ethylenically unsaturated group in the above R^{a} in each molecule and average degree of polymerization of the range of 3,000-7,000 (which has lower degree of polymerization than the above dimethylpolysiloxane),
or the composition consisting of organopolysiloxane copolymer (consisting of R^{a}₃SiO_{1/2} unit and SiO₂ unit, and the ratio of R^{a}₃SiO_{1/2} unit to SiO₂ unit is in the range of 0.6-0.9) and organopolysiloxane (the number of the above R^{a} per one silicone atom is in the range of 1.95-2.05, the content of ethylenically unsaturated group in each molecule is 0.0001-0.5% by mole, and the degree of polymerization is 3,000 or more) having the weight ratio of organopolysiloxane copolymer to organopolysiloxane of the range of 30/70-70/30 with 0.1-20 parts by weight of carbon black as catalyst for promoting crosslinking speed under anaerobic condition.

Silicone rubber for medical supply, which is desirable to have slightly change at pH of extracted water, potassium permanganate consumption and deterioration in the quality of material by γ -ray irradiation for sterilization, can be added 0.1-100 parts by weight of precipitated silicic acid showing pH8-12 or 0.1-2.0 parts by weight of oxide, hydroxide or carbonate of calciumormagnesium to 100 parts by weight of organopolysiloxane gum.

Crosslinked silicone rubber moldings for medical supply having excellent character in covering for x-rays, ctheter for contrastradiography, intravascular contrast medium, and anti-thrombus (anti-blood clotting) can be obtained by adding 1-300 parts by weight of micropowder of tungsten or tungsten carbide to 100 parts by weight of organopolysiloxane gum.

Silicone rubber for sound medium, which is used as acoustic lens for supersonic probe of ultrasonic diagnostic equipment, can be obtained by the composition consisting of 100 parts by weight of organopolysiloxane having halogen-substituted alkyl group and 10-200 parts by weight of inorganic metal oxide powder and/or metal powder having average particle size of 0.1-1.0µm and density of 2.0g/cm² or more.

Silicone rubber packing for food container, which needs to have higher contamination resistance and mechanical strength, can be obtained by the composition consisting of 5-350 parts by weight of titanium dioxide powder and 0.01-10 parts by weight of carboxyl-modified silicone oil, which has at least one carboxyl group or carboxyl group-containing group in organic group bonded per one silicone atom of organopolysiloxane, or 0.1-4 parts by weight of anatase type titanium dioxide micropowder can be added to 100 parts by weight of organopolysiloxane gum.

Silione rubber molding for fixing roller used in printer or facsimile, which needs to have toner releasability within low hardness, abrasion resistance, heat conductivity and heat resistance, can be obtained by using polymethylsilsesquioxane or 20-500 parts by weight of alumina having sodium content of 50 ppm or less and particle size of 5-50µm to 100 parts by weight of organopolysiloxane gum.

Conductive materials such as carbon black, silver and copper; electron removers such as polyoxyalkylene and polyoxyalkylene adducts; fluorine compound; or 0.05-5 parts by weight of fluorine-containing organosilicone compounds such as C₈F₁₇CH₂CH₂Si (CH₃) (OSi (CH₃)₂C₁₀H₂₁)₃, C₈F₁₇CH₂CH₂Si (OSi (CH₃)₂(CH₂)₃O (CH₂CH₂O)₃H)₃, F(CF(CF₃)CF₂O)₂CF(CF₃)CH₂O(CH₂)₃Si(OSi(CH₃)₂C₈H₁₇)₃ can be added to 100 parts by weight of organopolysiloxane gum as antistat.

Silicone rubber composition for fixing roller can be obtained by the composition consisting of two kinds of methylvinylpolysiloxane having different viscosity respectively, organohydrogenpolysiloxane and platinum catalysts, the composition consisting of organohydrogenpolysiloxane, non-crosslinkable organopolysiloxane and platinum catalysts, or the composition consisting of dimethylvinylsiloxy-capped dimethlpolysiloxane and organopolysiloxane resin (consisting of resin segments having 4 functional group and/or 3 functional group of two or more vinyl groups and oil segments having 2 functional group).

Fluororesin covered elastic roller can be manufactured by forming silicone elastic layer on roller-shaped core such as aluminum, stainless steel and iron and placing adhesion layer made from self-adhesive silicone rubber of addition reaction type thereon and then covering with fluororesin consisting of tetrafluoroethylene-hexafluoropropylene copolymer having melt flow rate of 2.0 or less to form extreme external layer, or forming layer of crosslinked organopolysiloxane composition on the surface of roller substrate and then covering with the fluororesin layer of fluororesin such as tetrafluoroethylene resin-perfluoroalkoxyethylene resin copolymer, tetrafluoroethylene resin and tetrafluoroethylene-hexafluoropropylene copolymer thereon.

Electrically conductive silicone sponge, which has microcell for conductive roller, can be obtained by the composition of 5-100 parts by weight of furnace black, 0-50 parts by weight of acetylene black and 2-30 parts by weight of organic forming agent to 100 parts by weight of organopolysiloxane gum, the composition of 5-100 parts by weight of reinforcing material consisting of silica fillers obtained by subjecting surface-treated with using 0.05-10 parts by weight of cationic surfactants such as chlorinatedstearyltrimethyl ammonium and 5-50 parts by weight of conductive carbon black and 15-200 parts by weight of sphere powder of silicone rubber having average particle size of 0.5-30 µm to 100 parts by weight of silica filler, or the composition of 5-100 parts by weight of conductive carbon black, 0.05-10 parts by weight of organohydrogenpolysiloxane, 0.1-1,000 ppm of platinum catalyst in term of platinum atom and 1-10 parts by weight of foaming agent to 100 parts by weight of organopolysiloxane gum.

Electrically conductive silicone sponge can be modified by 5-200 parts by weight of silicone elastomer particles having average particle size of 0.1-250 µm obtained by reaction organohydrogenpolysiloxane with vinyl group-containing organopolysiloxane (the molar ratio of silicone-bonded hydrogen atom to silicone-bonded vinyl group is in the range of 1.2-5 : 1.0) to 100 parts by weight of organopolysiloxane gum for lower hardness and surface condition.

Resistance value of electrically conductive roller can be stabilized by using conductive silicone rubber composition mixed the composition consisting of 100 parts by weight of organopolysiloxane (methyl group in the above Ra is 95% by mole or less, phenyl group is in the range of 5-20 % by mole and vinyl group is in the range of 0.001-10 % by mole) and 1-50 parts by weight of carbon black with the composition consisting of organopolysiloxane (methyl group in the above Ra is 98 % by mole or more and vinyl group is in the range of 0.001-2% by mole) at the weight ratio (the former composition : the latter composition) of the range of 5 : 95-95 : 5.

Silicone rubber molding for loading paper roller used in printer or facsimile can be added to platinum or platinum compound as antistatic agent, or amorphous silicone rubber powder (grounded silicone rubber) or diphenylsilanediol to provide high friction factor.

Rubber contact made of silicone rubber moldings used for office work machine, which is desirable to have excellent character in dynamic fatigue, low compression set and low hysteresis loss, 10-150 parts by weight of solvent-soluble polymer (organopolysiloxane resin consist of R^{a}₃SiO_{1/2} unit and R^{a}₂SiO_{2/2} unit and SiO_{4/2} unit, and have at least two ethylenically unsaturated group in each molecule, and the ratio of the total of R^{a}₃SiO_{1/2} unit and R^{a}₂SiO_{2/2} unit to SiO_{4/2} unit is in the range of 0.4-1.5:1.0 and the ratio of R^{a}₃SiO_{1/2} to R^{a}₂SiO_{2/2} is in the range of 1 or more : 1.0) can be added to 100 parts by weight of organopolysiloxane gum instead of inorganic filler.

Silicone rubber moldings for keyboard, which has small amount of oozing constituent frommolded silicone rubber, can be obtained by using the composition consisting of 3-100 parts by weight of microparticulate silica filler having the amount of oil absorption of 200cc/100g or more, 0.03-5 parts by weight of aliphatic metal salt having 10 carbon atoms or more and 0.5-30 parts by weight of processing aid agents to 100 parts by weight of organopolysiloxane gum.

Silicone rubber coating agent for air bag cloth is desirable to have adhesive property to synthetic fiber cloth, flame resistance and excellent pliability.

An adhesive property to synthetic fiber cloth is improved by using dimethylpolysiloxane having epoxy group and silicone-bonded methoxy group, methylvinylpolysiloxane having epoxy group and silicone-bonded methoxy group, dimethylsiloxane-methylvinylsiloxane copolymer having epoxy group and silicone-bonded methoxy group, or methylhydrogensiloxane-dimethylsiloxane copolymer having epoxy group and silicone-bonded methoxy group as organopolysiloxane gum, in addition, flame resistance can be improved by adding the combination of platinum compound and microparticulate manganese carbonate or zinc carbonate thereto.

Surface tackiness of silicone rubber-coated air bag is improved by adding 0.1-20 parts by weight of organosilane having isocyanate group and hydrolysable group in the molecule or partially hydrolyzed compound thereof and 0.1-10 parts by weight of organohydrogenpolysiloxane having at least three hydrogen atoms in the above R^{a} to 100 parts by weight of organopolysiloxane gum.

Pliability and flame resistance of silicone rubber-coated air bag is improved by adding 1-1,000 ppm of platinum or platinum compound in term of platinum atom per organopolysiloxane gum and 0.5-100 parts by weight of micriparticulate manganese carbonate or zinc carbonate to 100 parts by weight of organopolysiloxane gum, or 1-1,000 ppm of platinum or platinum compound in term of platinum atom per organopolysiloxane gum and 1-100 parts by weight of organopolysiloxane resin having siloxane unit represented by the formula R^{a}SiO_{3/2} (wherein R^{a} stands for substituted or unsubstituted monovalent hydrocarbon group ) and/or siloxane unit represented by the formula SiO_{4/2} in each molecule to 100 parts by weight of organopolysiloxane gum.

Air bag having excellent adhesive property, airtightness and mechanical strength is obtained by the method that, silicone elastomer composition is coated on cloth and silicone resin composition (the type crosslinking by addition reaction, the average mole ratio of organic group to silicone atom is in the range of 0.8-1.8) is coated on the above material and then crosslinked by heat simultaneously, or the method emulsifyed silicone composition consisting of 100 parts by weight of colloidal silica-silicone core shell (consisting of 80-5 % by weight of core of colloidal silica and 20-95 % by weight of shell of organopolysiloxane having 1 to 8 carbon atoms in the above R^{a}, the number of the above R^{a} per one silicone atom in the range of 1.80-2.20 and 0.01-25 % by mole of ethylenically unsaturated group), 1-20 parts by weight of emulsifier and 50-1,000 parts by weight of water is coated thinly on cloth. Silicone rubber moldings for diaphragm, which is useful for diaphragm of automobile having excellent character in swelling resistance, oil resistance and dynamic fatigue resistance, and coloration free, can be obtained by the composition consisting of 100 parts by weight of organopolysiloxane having average degree of polymerization of 1,000-10,000 (containing 5-40 % by mole of siloxane unit having perfluoroalkyl group of 1 to 3 carbon atoms and 0.02-0.5 % by mole of vinyl group) and 0.05-50 parts by weight of carbonate, hydroxides or oxide of iron, cerium, manganese, zinc, magnesium, aluminum or calcium.

The following additives, 10-50 parts by weight of wetprocess silica, 10-50 parts by weight of diatomaceous earth, 0-50 parts by weight of mineral powders able to be divided in parallel cleavage such as ferrosilicate salt powder and graphite powder (mica minerals such as selfmica, paragonite, biotite and lepidolite; stilpnomelane; pyrophyllite; talc; snake coat of arms stone; chlorite; prehnite; clay minerals such as montmorillonite and illite; flokite), and 0-10 parts by weight of fluororesin powder such as polytetrafluoroethylene powder to 100 parts by weight of organopolysiloxane gum can be added to silicone rubber moldings for wiper blade to prevent occurrence of chatter.

Silicone rubber moldings for antistatic material can be obtained by adding the above conductive agent, or 0.1-100 parts by weight of conductive materials such as carbon black, silver and copper; electron remover materials such as polyoxyalkylene and polyoxyalkylene adduct; or organopolysiloxane having at least one lower ethylenically unsaturated group and organic group represented by the formula R^{6b}-(OR^{6c})ₓOR^{6d}- (wherein R^{6b} stands for alkyl group, R^{6c} and R^{6d} stands for same or different divalent organic group, x stands for an integer in the range of 2 to 52.) in the above R^{a} to 100 parts by weight of organopolysiloxane gum.

Silicone rubber seatmoldings for insulation and releasing heat can be obtained by the composition consisting of polyolefine rubber (ethylene-propylene-diene copolymer, ethylene-propylene copolymer, polyisoprene-isobutylene copolymer) and organopolysiloxane (the ratio of polyolefine rubber to organopolysiloxane is in the range of from 100/0 to 60/40).

Silicone rubber for potting material, which is desiable to crosslink with strongly adhering to base material such as plastic at low temperature, can be obtained by the composition consisting of 100 parts by weight of vinyl group-capped organopolysiloxane gum having viscosity of 100-50,000 cst (at 25°C) , 0.5-10 parts by weight of siloxanes such as isopropenoxysilane or partially hydrolyzed and condensed compound thereof, a quantity enough to react with a vinyl group of organopolysiloxane gum (hydrogen atom of 0.5-1.2 in the above R^{a} per one vinyl group of the above organopolysiloxane gum) of organohydrogenpolysiloxane having at least two hydrogen atom in the above R^{a}, and platinum or platinum compound as catalyst.

Silicone rubber composition having excellent flowability and low permeability for moisture can be obtained by the composition consisting of organopolysiloxane or polyorganosilsesquioxane having silphenylene-bond and straight-chain organopolysiloxane having at least one cyclic siloxane unit.

Protective seat for solar battery module, which is desiable to have excellent character in transparency, flame resistance, light resistance and processability can be obtained by adding 40-130 parts by weight of microparticulate silica surface-treated by hexamethyldisilazane and having primary particle size in the range of 0.1-10nm to 100 parts by weight of organopolysiloxane gum, or can be manufactured by the method of laminating powder or membrane of thermoplasticity silicone resin having softening temperature of 30-200°Cto either side or both sides of un-crosslinked silicone rubber seat.

The silicone rubber for intermediate layer in trilaminar resist is an intermediate layer material for use in trilaminar resist lithography technology to form precisely minute resist pattern on board within manufacturing solid elements such as semiconductor integrated circuit.

The above intermediate layer material can be the composition using organic solvent soluble organopolysiloxane having softening point of higher than normal temperature.

Silicone rubber for extreme infrared rediation radiator can be used the composition consisting of 20-200 parts by weight of boron nitride having grain size in the range of 1-10 µm, 3-300 parts by weight of ceramics composed for use in this aim having grain size in the range of 1-10µm and 100 parts by weight of organopolysiloxane gum can be used.

Silicone rubber for oil bleeding can be added phenylmethylsilicone oil having small degree of polymerization as a bleed element. Fluorosilicone rubber composition consisting of 100 parts by weight of organopolysiloxane (the number of the above R^{a} per one silicone atom is in the range of 1.98-2.02 and 25-50 % by mole of R^{a} is containing the group represented by the formula -CH₂CH₂CFR^{7b} (wherein R^{7b} stands for perfluoroalkyl group having 1 to 3 carbon atoms), another 75-50 % by mole of R^{a} is saturated or unsaturated monovalent hydrocarbon group and viscosity is 1,000 centipoise (25°C) or more), and 3-15 parts by weight of organopolysiloxane oil having the viscosity in the range of 20-10,000 centipoise (at 25°C) (the number of the above R^{a} per one silicone atom is in the range of 1.98-2.02 and the content of the group represented by the formula -CH₂CH₂CFR^{7b} (wherein R^{7b} stands for perfluoroalkyl group having 1 to 3 carbon atoms) is 5-30 % by mole or less, wherein -CH₂CH₂CFR^{7b} group content is 20-45 % by mole less than the content of the above organopolysiloxane, and another 95-75 % by mole of the R^{a} is selected from methyl and phenyl group) can also be used.

Silicone rubber for swimming or diving articles can be obtained by the composition consisting of 0.1-50 parts by weight of white coloring agent, 0-0.02 parts by weight of blue coloring agent and 0.005-0.5 parts by weight of oxazole brightener to 100 parts by weight of organopolysiloxane gum to be pure white and no-yellowing.

Silicone-based composite rubber called as hybrid type rubber can be obtained by mixing silicone rubber with another elastomer.

The above elastomer mixed with silicone rubber include polyolefine synthetic rubbers such as butyl rubber, butadiene rubber, ethylene-propylene copolymer, ethylene-propylene-diene copolymer, ethylene-vinyl acetate copolymer, ethylene-methyl acrylate copolymer, ethylene-ethyl acrylate copolymer, acrylonitrile-butadiene copolymer and isobutene-isobutylene copolymer; nitrile rubber; epichlorohydrin rubber; acrylic rubber and fluororubber or the like.

The above acrylic rubber is obtained by copolymerization of one or more principal constituent selected from the group consisting of alkyl acrylates such as ethyl acrylate and butyl acrylate; alkoxy acrylates such as ethoxyethyl acrylate and methoxyethyl acrylate; alkylthioalkyl acrylate; and cyanoalkyl acrylate with one or more diene monomers selected from the group consisting of butadiene, methylbutadiene, isoprene, piperylene, pentadiene, acetoxybutadiene, cyanobutadiene, ethylidenenorbornene, dicyclopentadienyl acrylate, vinylidenenorbornene, cyclopentadiene and propenylnorbornane as crosslinking constituent.

The above fluororubber is obtained by copolymerization of fluorinated vinylidene homopolymer, fluorinated vinylidene-tetrafluoroethylene copolymer, fluorinated vinylidene-hexafluoro propylene copolymer, fluorinated vinylidene-tetrafluoroethylene-hexafluoropropylene copolymer, tetrafluoroethylene-propylene copolymer, tetrafluoroethylene-perfluoroalkylvinylether copolymer.

Composite rubber of silicone rubber and another elastomer can be obtained by mechanically mixing silicone rubber with another elastomer or by bonding organopolysiloxane gum with carbon-carbon double bond of elastomer.

Composite rubber can be obtained by the method of combining the rubber composition obtained by reacting the asymmetry organic peroxide, under shear deformation, with the composition consisting of 3-70 parts by weight of silicone rubber, 30-97 parts by weight of saturated elastomer, that substantially fail to be crosslinked with the asymmetry organic peroxide when it is alone, such as fluororubber, epichlorohydrin rubber, acrylic rubber and ethylene-α-olefine copolymerized rubber, and 0-20 parts by weight of another elastomer, that is co-crosslinkable with silicone rubber in the presence of the asymmetry organic peroxide and which is also co-crosslinkable or highly miscible with saturated elastomer, such as fluororubber, unsaturated group-containing epichlorohydrin rubber, acrylic rubber and ethylene-α-olefine-diene terpolymerizated rubber with the crosslinking agent for saturated elastomer.

Silicone-polyolefine composite rubber of the above composite rubber can be obtained by the composition of 0.1-10 parts by weight of age resistor and 1-30 parts by weight of calcium oxide to 100 parts by weight of the mixture consisting of 100 parts by weight of synthetic polyolefine rubber and 1-70 parts by weight of organopolysiloxane.

Crosslinkable polybutadiene composition consisting of 100 parts by weight of polybutadiene derivatives, which has organic silicone group bonded to principal chain of polybutadiene or copolymer consists of polymerized butadiene as main component through carbon atom-silicone atom, and 1-200 parts by weight of organopolysiloxane having ethylenically unsaturated group and 0-900 parts by weight of silica filler is suitable for casting so that they have a little dependency of the viscosity for temperature.

A silicone-acrylic composite rubber as the above composite rubber can be the mixture added siloxane-acrylate ester copolymer or aliphatic unsaturated group-containing siloxane-acrylate ester copolymer to uncrsslinked organopolysiloxane and uncrsslinked acrylic rubber, or the composition added 0.5-30 parts by weight of fluorine atom-containing polysiloxane having 15-50 % by mole of fluorine atom in the above R^{a} and average degree of polymerization of 5-10,000 as compatibilizer to 100 parts by weight of the mixture consisting of 90-10 parts by weight of acrylicpolymer, which be omes elastmer by crosslinking, and 10-90 parts by weight of organopolysiloxane as basepolymer, or the composition consisting of 90-10 parts by weight of acrylic polymer and 10-90 parts by weight of organopolysiloxane having degree of polymerization of 500-10,000 (having 15-50 % by mole of fluorine atom-containing organic group and 0.02-5 % by mole of vinyl group in the above Ra).

Foamed material with microcell, which has excellent character in heat-resistance, oil resistance, low-temperature resistance and resilience, can be manufactured from the siloxane-modified acrylic rubber foaming composition consisting of siloxane modified acrylic polymer (obtained by mixing emulsified organopolysiloxane having 0.025-10 % by mole of mercapto group in the above R^{a} with the mixture of acrylic monomer and 0.1-10 % by mole of monomer having two ethylenically unsaturated group in the molecule and then polymerized under existence of polymerization initiator) and foaming agents.

A silicone-acrylic composite rubber can be obtained by the method using hydrosilanization, under shear deformation, of 0.005-50 parts by weight of organohydrogenpolysiloxane and 0.00001-1 parts by weight of transition metal compounds belonging to the eighth group of the periodic table to 100 parts by weight of rubber-forming polymer (consist of 3-70 parts by weight of organopolysiloxane and 97-30 parts by weight of elastomer such as acrylic rubber), or the method obtained by compounding 100 parts by weight of rubber-forming polymer (containing as main components of 3-70 parts by weight of organopolysiloxane having 1.900-2.004 of the above R^{a} per one silicone atom and 0.005-3% by mole of hydroxyl group and degree of polymerization of 50-10,000 and 97-30 parts by weight of elastomer) with 0.005-50 parts by weight of a silicone compound having at least two hydrolyzable groups (which are able to undergo hydrolysis and condensation reaction with silanols) per molecule and 0.0001-10 parts by weight of heavy metal compounds, amine or quaternary ammonium salt, which catalyzes said hydrolysis and condensation reactions, and allowing the resulting formulation to undergo hydrolysis and condensation reactions under shear deformation.

A silicone-nitrile composite rubber as the above composite rubber can be the composition, which has excellent character in roller workability and processability before crosslinking and has excellent mechanical strength after crosslinking, consisting of 100 parts by weight of nitrile rubber, 2-100 parts by weight of organopolysiloxane gum, 0.1-20 parts by weight of chloroalkyl group-containing organosilicone compound and 5-200 parts by weight of reinforce filler.

The above chloroalkyl group-containing organosilicone compounds include alkoxy silanes such as 3-chloropropyltrimethoxysilane, 3-chloropropylmethyldimethoxysilane and 3-chloropropyltriethoxysilane and partially hydrolyzed and condensated product thereof; organopolysiloxane represented by the formula ClCH₂CH₂CH₂Si(OSiCH₃)₃; dimethyl(3-chloropropyl)siloxy-capped dimethylpolysiloxane; dimethyl(3-chloropropyl)siloxy-capped dimethylpolysiloxane-methyl(3-chloropropyl)siloxane copolymer; and trimethylsiloxy-capped dimethylsiloxane-methyl(3-chloropropyl)siloxane copolymer.

Silicone-fluorine composite rubber as the above composite rubber can be the composition, which has excellent workability before crosslinking and has slightly declination of mechanical character, consisting of 100 parts by weight of fluoro-rubbers synthesized from vinylidene fluoride such as chlorotrifluoro ethylene-vinylidene fluoride copolymer, pentafluoropropene-vinylidene fluoride copolymer and hexafluoropropene-vinylidene fluoride copolymer, 1-60 parts by weight of organopolysiloxane, 0.1-30 parts by weight of epoxy group-containing organoalkoxysilane or partially hydrolyzed compound thereof, and reinforce fillers, or the composition, which has slightly declination of mechanical character at lower temperature, consisting of 100 parts by weight of fluororubber, 2-200 parts by weight of fluoro-silicone gum having siloxane unit represented by the formula CF₃CH₂CH₂(CH₃)SiO, 0.01-40 parts by weight of fluorosilicone oil having siloxane units represented by the formula F(CF₂)₃CH₂CH₂(CH₃)_{y}SiO_{(3-y)/2} and the formula CF₃CH₂CH₂(CH₃)SiO as compatibilizer and 1-100 parts by weight of silica fillers, or-heat-shrinkable fluororubber composition, which has hot-air-crosslinkable property and has excellent mechanical strength, retention and residual shrinkage, obtained by adding 5-50 parts by weight of thermoplastic resin for heat-shrinkable property and 1-40 parts by weight of reinforce filler to the mixture of 95-60 parts by weight of fluoro-rubber polymer and 5-40 parts by weight of fluoro-silicone rubber.

A silicone rubber can be used as composit with various plastics such as addition type-polyimide resin and thermosetting polyester resin.

The above composition consisting of organopolysiloxane and addition type-polyimide resin, which canbe used for thermally stable electronicmaterial, especially for semiconductor sealant, include the composition consisting of 100 parts by weight of reaction product obtained by polymerizing additionally N,N'-substitutedbismaleinimide with diamine in the range of the mole ratio of N,N'-substituted bismaleinimide to diamine =0.5-2 : 1.0 and 100-600 parts by weight of crystal or non-crystal quartz (98% or more in purity, average particle size in the range of 5-50µm and 0.2% or less of the particles having grain size of 200µm or more are contained) and 0.3-10 parts by weight of silicone compound having silicone-bonded hydroxyl group or alkoxy group, and the composition consisting of 100 parts by weight of maleinimide resin selected from addition type-polyimide resin obtained by the reaction of N,N'-substitutedbismaleinimide compound with diamine; bismaleinimide-triazine resin obtained by the reaction of N,N'-substitutedbismaleinimide compound with dicyanate; and N,N'-substitutedbismaleinimide compound and 1-300 parts by weight of vinyl group-containing silicone resin.

The above compositions consisting of organopolysiloxane and thermosetting polyester resin, which also has excellent molding processability, include the composition consisting of 100 parts by weight of polyester resin having radically reactable group and 0.1-10 parts by weight of organopolysiloxane represented by the average compositional formula R^{8b}(R^{8c}R^{8d}SiO)_{z}SiR^{8b}R^{8c}R^{8d} (wherein R^{8b} stands for hydroxide group, or substituted or unsubstituted monovalent hydrocarbon group of 1-6 carbon atoms, z stands for weight-average molecular weight, which is converted to polystyrene molecular weight, of 100000 or more).

Silicone rubbers can be used for primer, self-bonding silicone insulation in the form of sheets or tapes and adhesives or the like as well. These are mentioned in the following.

Silicone rubbers for primer include the composition consisting of silicone gum, vinyl group-containing silicone oil, and metal salt of fatty acid, or the composition consisting of 100 parts by weight of organopolysiloxane having the viscosity of 100,000 cs (at 25°C), 5-100 parts by weight of organopolysiloxane having vinyl group of 0.05-1 in the above R^{a} and viscosity of 5-10,000 cs (25°C), 0.5-5 parts by weight of vinyl group-containing silane coupling agent and 0.5-5 parts by weight of phosphoric acid, or the composition obtained by adding 0.1-20 parts by weight of iron salt of fatty acid and 1-100 parts by weight of (meth) acrylic group-containing silane coupling agent based on the total amount to the mixture of 1-100 parts by weight of organopolysiloxane (having vinyl group of 0.5 % by mole or more and the viscosity of 5 cst (at 25°C) or more) and 0-99 parts by weight of organopolysiloxane (having vinyl group of 0.5 % by mole or less and the viscosity of 100,000 cst (at 25°C) or more), or the composition having film-formable ability obtained by adding 1-100 parts by weight of (meta)acrylic group-containing silane coupling agent and 0.1-20 parts by weight of iron salt of fatty acid to 100 parts by weight of vinyl group-containing polyorganosiloxane (consisting of 0-70 parts by weight of organopolysiloxane and 30-100 parts by weight of branched silicone resin (having the number of the above R^{a} per one silicone atom in the range of 1.0-1.7 and having at least one vinyl group in the molecule)), or the composition consisting of organopolysiloxane, organopolysiloxane resin, organoalkoxysilane or siloxane, organic titanate ester and organohydrogensiloxane, or the composition consisting of silane coupling agent, titanate ester, metal salt of organic fatty acid and organosilicone compound having silicone-bonded hydrogen atom, or the composition consisting of 100 parts by weight of ethylenically unsaturated group-containing organopolysiloxane (having the number of the above R^{a} per one silicone atom in the range of 1.9-2.3 and at least 0.15 % by mole of ethylenically unsaturated group in the molecule) having the viscosity of 5,000 cst (25°C) or more, 5-200 parts by weight of organopolysiloxane resin having the number of silicone-bonded substituted or unsubstituted monovalent hydrocarbon group per one silicone atom in the range of 0.5-1.8, 5-500 parts by weight of silane coupling agent, 5-500 parts by weight of organohydrogenpolysiloxane (the number of hydrogen atoms of the above R^{a} per one silicone atom is an integer of 2 or less and at least two hydrogen atoms in the molecule), 1-100 ppm of platinum catalyst in term of platinum atom and 0.5-50 parts by weight of organic titanate ester, or the composition consisting of 100 parts by weight of alkoxysiloxane having at least one remained alkoxy group in the molecular (obtained by silane coupling agent and partially hydrolyzed compound thereof), alkoxysilyl group-containing organohydrogensiloxane represented by the formula H_{a'}R^{9b}_{b'} (XSiR^{9c}_{d'} (OR^{9d})_{e'})_{c'}SiO _{(4-a'-b'-c')/2} (wherein R^{9b} and R^{9c} stand for unsubstituted or substituted monovalent hydrocarbon group respectively, R^{9d} stand for unsubstituted or alkoxy group substituted alkyl group, X stand for unsubstituted or substituted divalent organic group or oxygen atom, a', b', c' stand for the number satisfiing each 0<a' ≦ 2, 0 ≦ b' ≦ 3, 0<c' ≦ 3 and 0<a'+b'+c' ≦ 4 respectively, d' stand for 0, 1 or 2, e' stand for 1, 2 or 3, and, d'+e'=3) and organic titanate ester, or the composition consisting of 5-100 parts by weight of organopolysiloxane having ethylenically unsaturated group in the above R^{a} per one silicone atom of 0-0.005 and the viscosity of 10,000 cst (at 25°C) or more and/or vinyl group-containing organopolysiloxane having the number of ethylenically unsaturated group in the above R^{a} per one silicone atom of 0.05-1 and the viscosity of 5-10,000 cst or more (at 25°C), 0-100 parts by weight of organopolysiloxane resin, 0.5-200 parts by weight of vinyl group-containing silane coupling agent, 0-100 parts by weight of silica filler having specific surface area of 100m²/g or more and 0.5-50 parts by weight of catalyst for condensation reaction, or the composition consisting of 100 parts by weight of organopolysiloxane having viscosity of 5,000 cst or more (at 25°C) (having the number of substituted or unsubstituted monovalent silicone-bonded hydrocarbon group per one silicone atom in the range of 1.9-2.3 and at least 0.15 % by mole of ethylenically unsaturated group in the molecule), 5-200 parts by weight of organopolysiloxane resin (having the number of silicone-bonded substituted or unsubstituted monovalent hydrocarbon group per one silicone atom in the range of 0.5-1.8), 5-500 parts by weight of alkoxy siloxane obtained by silane coupling agent or partially hydrolyzed compound thereof, 1-1,000 ppm of platinum catalyst in term of platinum atom per organopolysiloxane gum and 0.5-50 parts by weight of organic titanate ester.

Organosiloxane (monovalent organic group having unsaturated bond are more than 1 and less than 4, substituted or unsubstituted monovalent hydrocarbon group are 0 or more and less than 2, the total number of these group are less than 4, and the degree of polymerization is in the range of 1-1,000), or red iron oxide, ceric oxide, titanium dioxide or the like, which improve adhesive durability for long time, can also be added to the composition.

Use of silicone rubber primer causes adhesion of silicone rubber to aluminum treated by black sulfuric acid alumite, which was difficult so far.

Apply the primer composition consisting of organoalkoxysilane or siloxane, platinum compound and organic titanate ester to the surface of metal causes adhesion of thermally crosslinkable silicone rubber containing organohydrogenpolysiloxane to metal, in addition, adhesion heat resistance will be improved.

Primers for adhering fluoro-silicone rubber (fluorine-containing group-ccontaining organopolysiloxane) to various base materials include the composition consisting of 100 parts by weight of organopolysiloxane having the viscosity of 1,000,000 cst (at 25°C) or more (having the number of the above R^{a} per one silicone atom in the range of 1.98-2.01 and perfluoroalkyl group of 25-50 % by mole and vinyl group of 0.01-10 % by mole in the moleecule), 5-100 parts by weight of silica filler, 0.5-50 parts by weight of isocyanurate group-containing organosilicone compound represented by the formula -(-C(=O)-N(-(CH₂)_{f}, Si(OR^{10b})₃)-)₃- (wherein R^{10b} stand for alkyl group of 1-5 carbon atoms, fluoroalkyl group or alkoxyalkyl group, f' stand for an integer in the range of 1 to 5) and organic solvent, or the composition consisting of 100 parts by weight of organopolysiloxane having the viscosity of 1,000,000 cst or more (at 25°C) (having the number of the above R^{a} per one silicone atom in the range of 1.98-2.01 and perfluoroalkyl group of 10-50 % by mole and vinyl group of 0.01-10 % by mole in the molecule), 5-100 parts by weight of silica filler, 0.5-50 parts by weight of (meth)acrylamide group-containing silane coupling agent or partially condensated product of these alkoxysilane and organic solvent.

Tightly adhesion of fluorosilicone rubber to metal or plastic and so on can be yield by contacting the composition consisting of 100 parts by weight of organopolysiloxane having the viscosity of 1,000 cst or more (at 25°C) (having the number of the above R^{a} per one silicone atom in the range of 1.95-2.05 and 3,3,3-trifluoropropyl group of 20-50 % by mole), 5-100 parts by weight of silica filler and 0.01-1 parts by weight of organohydrogenpolysiloxane having silicone-bonded hydrogen atom of 0.8 % by weight or more with base material treated the said composition with the primer (having ethylenically unsaturated group-containing silane coupling agent as main ingredient) and is crosslinked by heat.

The above self-bonding silicone insulation in the form of sheets or tapes include silicone varnish obtained by mixing vinyl group-containing organopolysiloxane (obtained by hydrolyzing the composition consisting of 2-25 % by mole of vinyltrichlorosilane, 20-60 % by mole of phenyltrichlorosilane (wherein at least 16 % by mole of phenyl group is in organic group) and methyl trichlorosilane, and 30-70 % by mole of dichlorodimethyl-capped polysiloxane) and the asymmetry organic peroxide is apply to insulating base material in the form of sheets or tapes and then crosslinked by heat and adhered.

The above adhesives include roll-shaped adhesives formed by tape-shaped laminates consisting differ layer of silicone rubber adhesives and base layer having releasability for the said silicone rubber adhesives wounded to cylinder-shaped core, or film-shaped silicone adhesives using hydrophobic-treated wet-process silica or seat-shaped adhesives formed by clay-shaped silicone composition consisting of organopolysiloxane gum, reinforcement filler and the above isocyanurate compound or silane coupling agent enlarged in thickness of 0.1-5mm.

Pressure-sensitive silicone adhesives, for example, include adhesives obtained by adhering the composition consisting of 30-70 parts by weight of organopolysiloxane represented by the formula R^{11c}R^{11b}₂SiO(R^{11b}₂SiO)_{g},SiR^{11b}₂R^{11c} (wherein R^{11b} stand for monovalent hydrocarbon, each R^{11c} stand for R^{11b} group or OH group, g' stand for the number of more than 0) and 70-30 parts by weight of organopolysiloxane (having the ratio of R^{11d}SiO_{1/2} unit to (R^{11e}O) ₕ,SiO_{(4-h')} _{/2} unit in the range of 0.6 ; 1-0.9 : 1, at least 50 % by mole of methyl group in all R^{11b} group, R^{11e} stand for H or R^{11b}, and h' stand for 0-0.3) with roller exfoliatable liner, which is exfoliatable and protective seat or tape, and they can be adhered easily by pressure due to have good tackiness and adhesion in separating from liner, and can be removed if necessary.

Adhesion or lamination of silicone rubber with various substrates is mentioned in the following.

The method of adhesion for silicone rubber to metals include the method of thermal crosslinking in contact during its crosslinking of organopolysiloxane composition containing organohydrogenpolysiloxane and having crosslinkable property of organic peroxide with the metal treated on the surface by platinum compound chemically, and the method of adhesion for silicone rubber to metals or another substrates include the method of adherence of silicone rubber with substrates after apply the solution containing platinum compound on the surface of previously crosslinked organopolysiloxane composition and/or of metals or another substrates.

The composition for adhering silicone rubber to thermoplastic resin include the composition consisting of 100 parts by weight of ethylene-propylene copolymer and 5-500 parts by weight of organopolysiloxane having at least two ethylenically unsaturated group in the above R^{a} in the molecular and the average degree of polymerization in the range of 10-10,000, (the value multiplying the amount of the said organopolysiloxane by the mole ratio of ethylenically unsaturated group to organic group in the molecule of the said organopolysiloxane is 0.1 or more), and the composition consisting of 100 parts by weight of organopolysiloxane gum (consisting of organopolysiloxane having at least two ethylenically unsaturated group in the above R^{a} in the molecular and organohydrogenpolysiloxane having at least two hydrogen atom in the above R^{a} in the molecular, and the amount of silicone-bonded hydrogen atom in the said organohydrogenpolysiloxane to the total amount of silicone-bonded group in organopolysiloxane gum is in the range of 1-4 % by mole) and 500 parts by weight of silica filler.

Silicone rubber can be adhered to thermoplastic resins such as ethylene-propylene rubber and ethylene-propylene-diene rubber in excellent bond strength by these adhesives.

The method of adhering silicone rubber to thermoplastic resin include the method of forming silane coupling layer on the surface of crosslinked silicone rubber and forming silicone-polyolefine copolymer thereon and adhering while melting thereafter, or the method of forming two adhesive layer of different compositions such as adhesive layer of organopolysiloxane containing polyorganohydrogensiloxane on the surface of silicone rubber and adhesive layer consisting of ethylene-propylene copolymer and organopolysiloxane on the surface of thermoplastic resin, and then crosslinked together.

Adhesives to adhesive silicone rubber with EPDM include the composition containing 0.1-10 parts by weight of products obtained by addition reaction of particular carboxylates represented by the formula (R^{12b}OCO)_{j'}C₆H_{(6-j'-k')}(COOR^{12e}(R^{12c}) (OR^{12d})_{I'} Si_{(3-I')})ₖ, (wherein R^{12b} stnds for ethylenically unsaturated group, R^{12c} stnds for monovalent hydrocarbon group not having aliphatic unsaturated bond, R^{12d} stnds for alkyl group, R^{12e} studs for alkylene group, I' stnds for the number of 1-3, j' stnds for the number of 0-5, k' stnds for the number of 1-6, j'+k' stnds for the number of 1-6) with alkoxysilane or reaction mixture thereof, or the composition containing 0.1-20 parts by weight of the compound having the group having at least two phenyl skeletons in the molecule such as -C₆H₄-O-C₆H₄-, -C₆H₄-S(=O)-C₆H₄-, -C₆H₄-C(=O) -C₆H₄- and -C₆H₄-S-C₆H₄- group, and the group having at least one (meth)acrylic group in chain terminal of molecule, and uncrosslinked rubber allow to crosslink with organic peroxide, or the composition consisting of 100 parts by weight of organopolysiloxane having at least two silanol group in the above R^{a}, 0.5-20 parts by weight of organosilane (containing at least two alkoxysilyl group in the above R^{a} and at least one sulfur atom bonded with silicone atom through alkylene group) or partially hydrolyzed compound thereof, 0.1-10 parts by weight of amino group-containing silanes such as C₆H₅CH₂NHC₃H₆Si (OCH₃)₃ or partially hydrolyzed compound thereof and 0.01-10 parts by weight of catalyst for condensation reaction, or the composition consisting of 100 parts by weight of organopolysiloxane (the average number of the above R^{a} is in the range of 1-3, the average number of alkyl group or aryl group is in the range of 0-1.0, the average number of ethylenically unsaturated group is in the range of 0.1-2.5 and the average number of hydroxy group or alkoxy group is in the range of 0.1-2.5 per one silicone atom respectively) and 0.01-200 parts by weight of ester of organic titanate as catalyst for condensation reaction.

The method of adhesion for silicone rubber to unsaturated polyester resin include the adhesion method of coating primer on the surface of molding resin and then coating uncrosslinking silicone rubber thereon and crosslinking together, or the crosslinking method of self-bonding silicone rubber on a molding resin, or the crosslinking method of crosslinking either silicone rubber or unsaturated polyester resin previously and then adherence with the other uncrosslinked composition thereafter, or the unifying method by physically adhere of silicon e rubber to organic resin, or the unifying method of adhering silicone rubber to olefine resin grafted with the compound having aliphatic unsaturated group and silicone-bonded hydrolyzable group, or the adhesion method of organic resin containing organopolysiloxane having silicone-bonded hydrogen atom of 30 % by mole or more to addition reaction crosslinking-type silicone rubber. The method of adhesion for silicone rubber to epoxy polyester resin include the crosslinking method while adhering directly of crosslinkable silicone rubber composition by organic peroxide to crosslinkable epoxy resin composition or crosslinkable silicone-epoxy resin composition (crosslinking the other composition after crosslinking or half-crosslinking either of the above compositions), or the crosslinking method =while adhering to silicone rubber composition after crosslinking or half-crosslinking silicone resin containing organopolysiloxane (having ethylenically unsaturated group of 0.1-50 % by mole in the above R^{a}, the total number of hydrocarbon group of 1 or more and less than 2, and the number of phenyl group of hydrocarbon group of 5% or more and less than 80%. The above crosslinking aids, reactable vinyl monomers such as hydroxyethyl (meth)acrylate, N-vinyl-2-pyrolidone, styrene, 2-chlorostyrene, methyl (meth)acrylate, ethyl (meth)acrylate and diallyl benzene phosphonate or oligomer can be added to adjust a hardness or a viscosity of the composition.

The composition to laminate silicone rubber and elastmer include the composition consisting of 0.1-20 parts by weight of organopolysiloxane having the degree of polymerization of 3,000 or more (having the number of R^{a} per one silicone atom of more than 0 and less than 4, and containing vinyl group of 0-5 % by mole in each molecule), 0.1-20 parts by weight of organohydrogenpolysiloxane having average hydrogen atom in the above R^{a} of more than 2 in each molecule and 0.1-5,000 ppm of platinum catalysts to organopolysiloxane gum.

The method of laminating silicone rubber and elastomer include the method of inserting plural materials blended elastomer with silicone rubber in the intermediate layer, for example, the method of forming silicone rubber layer (the composition consisting of 100 parts by weight of the copolymer having ethylene unit and propylene unit as main unit, 5-50 parts by weight of the ethylenically unsaturated group-containing organopolysiloxane having degree of polymerization of 10 or more and the amount of ethylenically unsaturated group in the above R^{a} in the range of 0.3-50 % by mole and 10-200 parts by weight of filler) on adjacency of uncrosslinked ethylene-propylene rubber layer, thereafter forming layer of the composition consisting of 100 parts by weight of the copolymer of ethylene unit and propylene unit as main unit, 40-150 parts by weight of organopolysiloxane (either or both of the conditions (having at least one ethylenically unsaturated group in the above R^{a} and average degree of polymerization of 2,000 or more) is satisfied, and containing 5-80 parts by weight of ethylenically unsaturated group-containing organopolysiloxane having the degree of polymerization of 10 or more and the amount of ethylenically unsaturated group in all organic group is in the range of 0.3-50 % by mole, and 10-120 parts by weight of organopolysiloxane having the average degree of polymerization of 2,000 or more) and 15-300 parts by weight of filler on adjacency of the above uncrosslinked silicone rubber layer, or the method of mixing 100 parts by weight of organopolysiloxane composition (consisting of 100 parts by weight of organopolysiloxane and 5-500 parts by weight of silica filler) and/or polyolefine-organic polymer composition (consisting of 100 parts by weight of ethylene-propylene-diene copolymer and/or ethylene-propylene copolymer, 1-100 parts by weight of organopolysiloxane and 10-150 parts by weight of silica filler) with 1-10 parts by weight of silica filler having specific surface area of at least 50m²/g at a temperature of 80°C or lower, thereafter crosslinking the above organopolysiloxane composition and polyolefine-organic polymer composition as laminated structure.

The method of laminating conductive silicone rubber to insulating silicone rubber include of molding crosslinked conductive silicone rubber obtained by adding the asymmetry organic peroxide to carbon black containing uncrosslinked silicone rubber by heat-compression molding with insulating silicone rubber of essentially carbon black free uncrosslinked silicone rubber containing the asymmetry organic peroxide by heat-compression molding together, or the thermal crosslinking method of adherence uncrosslinked essentially carbon black free-insulating silicone rubber composition containing the asymmetry organic peroxide with carbon black containing conductive silicone rubber moldings having addition-reactivity together, or the method of molding by heating with the application of pressure while sticking conductive silicone rubber to uncrosslinked insulating organopolysiloxane (consisting of ethylenically unsaturated group-containing organopolysiloxane, organohydrogenpolysiloxane, platinum catalyst and the asymmetry organic peroxide), or the method of molding while adherence of conductive silicone rubber (consisting of 100 parts by weight of one ormore kinds of organopolysiloxane having average degree of polymerization in the range of 500-12,000 and at least two vinyl group in the above R^{a}, 5-75 parts by weight of carbon black, 0.1-5 parts by weight of organohydrogenpolysiloxane having average hydrogen atom in the above R^{a} in the molecule of more than 2, and 0.2-300 ppm per its composition of platinum atom as the platinum or platinum compound) to essentially carbon black free uncrosslinked silicone rubber (consisting 100 parts by weight of one or more kinds of organopolysiloxane having average degree of polymerization in the range of 500-12,000 and at least two vinyl group in the above R^{a}, 10-200 parts by weight of reinforcement or unreinforcement filler, 0.1-10 parts by weight of the asymmetry organic peroxide, and 0.1-5 parts by weight of organohydrogenpolysiloxane having at least one hydrogen atom in the above R^{a}).

Insulation layer can be adhered to conductive layer by these methods.

Silicone rubber laminated board can be obtained by the thermal crosslinking method with the application of pressure while adhering fabrics impregnating semicrosslinked resin (obtained by coating silicone resin composition represented by the average compositional formula (CH₂=CHSiO_{1.5})_{1'}(R^{13b}SiO_{1.5})_{m'}((CH₃)₂SiO)_{n'}(R^{13c}R^{13d}SiO)_{o'} (wherein R^{13b} stands for methyl or phenyl group, having at least 16 % by mole of phenyl group, R^{13c} and R^{13d} stands for methyl, phenyl or vinyl group, 1'=0.02-0.25, m'=0.3-0.7, n'+o'=0.2-0.6 wherein l'+m'+n'+o'=1) to insulation base material having heat-resistant, and then drying) to silicone rubber composition.

Laminating rubber of silicone resin and glass cloth as main component,can be usd a silicone resin composition for laminating consisting of 100 parts by weight of vinyl group-containing organopolysiloxane (represented by the average compositional formula (R^{14b}SiO_{1.5})_{p'}(R^{14b}₂SiO)_{q'} (R^{14b}₃SiO_{0.5})_{r'} (SiO₂)_{s'} (wherein R^{14b} stands for same or different group selected from substituted or unsubstituted monovalent hydrocarbon group of 1-10 carbon atoms, p'=0.5-0.9, q'=0.1-0.5, r'=0-0.05 and s'=0-0.005), and having vinyl group in the above R^{14b} per one silicone atom of 0.05-0.3), 10-150 parts by weight of organohydrogenpolysiloxane (represented by the average compositional formula (R^{15b}SiO_{1.5})_{t'}(R^{15b}₂SiO)_{u'}(R^{15b}₃SiO_{0.5})_{v'}(wherein R^{15b} stands for hydrogen atom, or same or different group selected from substituted or unsubstituted monovalent hydrocarbon group of 1-10 carbon atoms, t'=0.1-0.7, u'=0.2-0.5 and v'=0.1-0.7), and having hydrogen atom of 0.05-0.3 a silicone atom in the above R^{15b}), and platinum compounds. Laminating rubber of silicone resin and glass cloth as main component, can be obtained by laminating acrylic rubber composition consisting of acrylic rubber containing unsaturated vinyl group-containing organo silicone (obtained by copolymerizing (meth) acrylate with ethylenically unsaturated monomer having vinyl group-containing organo silicone) and 10-200 parts by weight of reinforcement fillers on the silicone rubber composition consisting of organopolysiloxane gum and microparticulate silica filler and the asymmetry organic peroxide, and then crosslinking together by heat.

Silicone can also be used as aqueous emulsion.

The silicone aqueous emulsion include, for example, the composition consisting of organopolysiloxane having both molecular terminals capped with hydroxyl group, polyorganohydrogensiloxane, polyalkyl silicate and tin salt of fatty acid, or the composition consisting of organopolysiloxane having both terminals capped with hydroxyl group, silane having 3 or more functional group and tin salt of fatty acid, or the composition obtained by adding tin salt of fatty acid and alkaline colloidal silica to the emulsion of polydiorganosiloxane having both terminals capped with hydroxyl group, or the composition obtained by adding alkaline colloidal silica to the silicone emulsion obtained by emulsion polymerization of hydroxyl group-capped diorganosiloxane having low degree of polymerization and alkoxy silane having 3 or more functional group, or the composition containing organopolysiloxane having silicone-bonded hydroxyl group, or the composition consisting of polydiorganosiloxane having both molecular terminals capped with vinyl group, polyorganohydrogensiloxane and platinum compound, or the composition obtained by emulsion polymerization of cyclic organo siloxane and functional group-containing organoalkoxy silane, or the composition obtained by adding 1-20 parts by weight of emulsifier and 50-1,000 parts by weight of water to 100 parts by weight of colloidal silica-silicone core shell (consisting of 80-5 % by weight of core of colloidal silica and 20-95 % by weight of shell of organopolysiloxane (the above R^{a} containing 1-8 carbon atoms, having the above R^{a} per one silicone atom of 1.80-2.20 and 0.01-25 % by mole of ethylenically unsaturated group)).

The above silicone aqueous emulsion can be obtained by conventional method of manufacturing emulsion, or the method of polymerizing diorganosiloxane (having low degree of polymerization and both molecular terminals capped with hydroxyl group) with alkoxy silane having 3 or more functional group under presence of acidic colloidal silica by emulsion polymerization.

Sphere crosslinked silicone rubber moldings can be obtained by using such silicone aqueous emulsion. In other words, sphere crosslinkeded silicone rubber can be obtained by the method of converting liquid silicone rubber composition into emulsion or suspension and thereafter contacting with liquid or gas at high temperature.

Sphere crosslinked silicone rubber can be also obtained by the method of atomizing liquid silicone rubber composition in hot air and crosslinking under atomized condition.

Crosslinking systems such as ultraviolet rays crosslinking, anaerobic crosslinking, high-frequency dielectric crosslinking, crosslinking by addition reaction, and crosslinking by condensation reaction can be used together with the crosslinking process of the present invention.

These will be mentioned in the following.

The above ultraviolet rays-crosslinkable silicone rubber containing photosensitizer, which cause free radical by absorption of ultraviolet rays and subsequent encouraging decomposition of the asymmetry organic peroxide and forming free radical, can be provide crosslinked silicone rubber moldings by ultraviolet rays effectively.

The above photosensitizers include, for example, acetophenone, propiophenone, benzophenone, benzoin, benzoin ether, aromatic ketone, 2-benzoylbenzoate, quinone compounds, xanthone, thioxanthone, amino or nitro or phenol compounds, aromatic hydrocarbons, azo compounds, peroxides, disulfide compounds, halogenated hydrocarbons, amines and derivative thereof.

The above photosensitizers can be Acetophenones such as acetophenone, 3-methylacetophenone, 4-methylacetophenone, 3-isopropylacetophenone, 4-isopropylacetophenone, 3-pentylacetophenone, 4-pentylacetophenone, 3-methoxyacetophenone, 4-methoxyacetophenone, 2,2-diethoxyacetophenone, 3-chloroacetophenone, 4-chloroacetophenone, 3-bromoacetophenone, 4-bromoacetophenone, 4-allylacetophenone, 4-dimethylaminoacetophenone, 4-tert-butyltrichloroacetophenone, 4-t-butyldichloroacetophenone, benzyl methoxy ketal and benzyl dimethyl ketal, and the derivatives thereof; propiophenones such as propiophenone, 2-methyl-2-hydroxypropiophenone and 4'-isopropyl-2-hydroxy-2-methylpropiophenone, and the derivatives thereof; benzophenones such as benzophenone, 3-methylbenzophenone, 4-methylbenzophenone, 2,4-dimethylbenzophenone, 3-methoxybenzophenone, 4-methoxybenzophenone, 3-chlorobenzophenone, 4-chlorobenzophenone, 2,4-dichlorobenzophenone, 4-fluorobenzophenone, 4,4'-dichlorobenzophenone, 4,4'-dimethoxybenzophenone, 4-chloro-4'-benzylbenzophenone, 4,4'-bis(dimethylamino)benzophenone and 4,4'-tetra-methyldiaminobenzophenone, and the derivatives thereof; benzoins and benzoin ether such as benzoin, benzoin methyl ether, benzoin ethyl ether, benzoin propyl ether, benzoin isopropyl ether, benzoin butyl ether, benzoin isobutyl ether andbenzoin triphenyl silyl ether, and these derivatives; aromatic ketones such as benzyl, benzaldehyde, 2-hydroxy-2-methyl-1-phenylpropane-1-on, 1-(4-isopropylphenyl)-2-hydroxy-2-methylpropanone, bis(4-dimethylaminophenol) ketone, benzoyl ethyl ether, benzoyl isopropyl ether, benzoyl butyl ether and 4-diacetylbenzene; 2-benzoylbenzoates such as methyl 2-benzoylbenzoate, ethyl 2-benzoylbenzoate and phenyl 2-benzoylbenzoate; quinone compounds such as benzoquinone, anthraquinone, 1,2-naphthoquinone, anthraquinone, 5,12-naphthacenedione and 2,7-pyrene dione; xanthones such as xanthone, 2,4-dimethylxanthone, 2,4-dichloroxanthone, 2-chlorothioxanthone, 3-chloroxanthone, 3,9-dichloroxanthone and 3-chloro-8-nonylxanthone, and the derivatives thereof; thioxanthones such as thioxanthone, 2,4-dimethylthioxanthone and 2,4-dichlorothioxanthone, and the derivatives thereof; amino or nitro or phenol compounds such as nitrobenzene, 4-nitrodiphenyl, 4-nitroaniline, 2-chloro-4-nitroaniline, 4-nitrophenol and 2,4,6-trinitrophenol; aromatic hydrocarbons such as benzene, naphthalene, biphenyl, fluorene, triphenylene, phenanthorene, azulene, pyrene, anthracene, tetracene and pentacene; azo compounds such as azobisbutyronitrile; peroxides such as benzoyl peroxide and tert-butyl peroxide; disulfide compounds; halogenated hydrocarbons such as carbon tetrachloride, hexachloroethane and carbon tetrabromide; amines such as diphenylamine, carbazole and triphenylamine.

The above photosensitizer is usually used in an amount of 0.1-20 parts by weight for every 100 parts by weight of organopolysiloxane gum.

The effective crosslinking method of silicone rubber having fast crosslinking speed and oxygen inhibition free to provide excellent characters such as electric character, heat-resistance, low-temperature resistance and wetherability for silicone rubber moldings can be the method of adding various radiosensitizers to vinyl group-containing organopolysiloxane and then irradiating strong ultraviolet rays, or the method of combining inorganic filler treated on the surface by vinyl group-containing silanizing agent or phenon optical initiator are effective.

The composition consisting of 100 parts by weight of organopolysiloxane having ethylenically unsaturated group in the molelular terminal and 0.1-5 parts by weight of aromatic ketone photosensitizer, or the composition consisting of vinyl group-containing organopolysiloxane, organohydrogenpolysiloxane and sensitizer, or the composition consisting of acrylic unsaturated group-containing organopolysiloxane and sensitizer, or the composition consisting of photosensitizer and acrylic-dialkoxy silyl or acrylic-diallyloxysilyl group-capped organopolysiloxane containing moisture-crosslinking catalyst, or the composition consists of vinyl group and hydrogen atom-containing organohydrogenpolysiloxane and polyvinylmethylsiloxane having low molecular weight, or the composition consisting of 100 parts by weight of organopolysiloxane having at least two hydroxyl group or hydrolysable group in the above R^{a} in each molecule and viscosity of 10-1,000,000 cst (at 25°C), 0-2,000 parts by weight of organopolysiloxane having at least two ethylenically unsaturated group in the above R^{a} in each molecule and viscosity of 10-1,000,000 cst (at 25°C), 0.5-20 parts by weight of organosilicone compound having the average number of silicone-bonded hydrolysable group in each molecule of more than 2 and 0-10 parts by weight of catalysts for condensation reaction, or the composition consisting of hydroxyl group-capped organopolysiloxane, hydrolysable group-containing vinyl silane, mercapto group-containing organosiloxane and sensitizer, or the composition consisting of 100 parts by weight of organopolysiloxane having azido group-containing group in the above R^{a} and 20-1,000 parts by weight of vinyl group-containing organopolysiloxane, or the composition consisting of mercapto group-containing organopolysiloxane and methylvinylpolysiloxane or the composition combined antigelling agent thereto, or the composition consisting of mercapto group-containing organopolysiloxane and dihydric phenol or alkyl derivatives thereof as antigelling agent, or the composition consisting of dialkyl siloxane-alkyl epoxy siloxane copolymer having molecular terminal of epoxy group-containing group and bis (aryl) iodonium salt, or the composition consisting of 80-98 % by weight of organopolysiloxane having both of condensation reactivity and ultraviolet rays-crosslinking reactivity in each molecule due to double bond and 2-20 % by weight of crosslinking aids can also be used.

Ultraviolet rays curable silicone emulsion composition consisting of 100 parts by weight of organopolysiloxane obtained by emulsion polymerization of one or more organopolysiloxane having low molecular weight and photoreactive group by using organic sulfonic acid and 0.1-20 parts by weight of photoreaction initiator can also be used.

Anaerobic-crosslinkable silicone rubber composition can be crosslinked anaerobicly inside of jointing while no contact with air.

The anaerobic crosslinking accelerator include organic sulfone imidos such as 2-benzoic sulfone imido, 1,2,3,4-tetrahydroquinoline salt of 2-benzoic sulfone imido; amines such as diethylamine, triethylamine, triethylenediamine, N,N-dimethyl p-toluidine, N,N,N',N'-tetramethyl 1,2-propanediamine; copper salts represented by CuCl₂(H₂O)_{w'}; halides such as hypochlorous acid, bromic acid potassium or hydrogen chloride; thiocyanic acid and halogenated acetic acid.

The anaerobic-crosslinkable silicone rubber composition can be the composition containing of straight-chain organopolysiloxane having both molecular terminals of acryloyl group and/or methacryloyl group and hydrolysable group as main ingredient.

The composition consisting of 100 parts by weight of organopolysiloxane consisting of siloxane unit represented by the following formula CH₂=C(R^{16b})COOR^{16c}Si(R^{16d})_{x'}O_{(3-x')/2} (wherein R^{16b} stand for hydrogen atom or methyl group, R^{16c} stand for alkylene group of 1-4 carbon atoms, R^{16d} stand for substituted or unsubstituted monovalent hydrocarbon group containing no aliphatic unsaturated group respectively, x' stand for 0, 1 or 2 ) and siloxane unit represented by the following formula R^{17b}_{y'}SiO_{(4-y')/2} (wherein R^{17b} stand for substituted or unsubstituted monovalent hydrocarbon group containing no aliphatic unsaturated group respectively, y' stand for 0, 1 or 2 ), and 10-500 parts by weight of the mixture of organopolysiloxane (consisting of SiO₂ unit, the unit represented by the following formula R^{18b}SiO_{1/2} (R^{18b} stand for substituted or unsubstituted hydrocarbon group respectively), and hydroxyl group at the content of 0.0004-1 per silicone atom in one mole of said SiO₂ unit) and polydiorganosiloxane (having both molecular terminals capped with silanol group and having viscosity in the range of 30-2,000,000 cst (at 25°C)) or condensated reaction product thereof (the amount of the above polydiorganosiloxane based on the above organopolysiloxane is in the range of 10-1,000 parts by weight), and 0.5-50 parts by weight of organosilicone compound having average silicone-bonded hydrolyzable group in each molecule of more than 2, or the composition combined additionally 0.1-20 parts by weight of anaerobic crosslinking accelerator and 0.5-20 parts by weight of photopolymerization initiator into the above composition can also be illustrated.

The composition, which comprises anaerobic-crosslinkable and ultraviolet rays-crosslinkable organopolysiloxane, consisting of organopolysiloxane having at least two hydroxyl group in the above R^{a} in each molecule, organosilicone compound represented by the following formula (R^{19b})_{a"}Si(OC(CH₃)=CH₂)_{b"}(OR^{19b})_{c"} (wherein R^{19b} stand for substituted or unsubstituted monovalent hydrocarbon group of 1-8 carbon atoms and having no aliphatic unsaturated bond, a'' stand for 0 or 1, b'' stand for 2 or 3, c'' stand for an integer in the range of 0 to 2, a''+b''+c' =4) and partially hydrolyzed and condensated product thereof, photosensitizer and carbon black can also be illustrated.

Such compositions can be crosslinked anaerobicly and promptly inside of jointing while having no contact with air and crosslinked promptly by ultraviolet irradiation for extruded part of the composition as well.

The method of crosslinking by high-frequency dielectric heating can be used for obtaining thick molded silicone rubbers such as sponge.

A high-frequency dielectric heating method can be applicable of adding 0.1-5 parts by weight of carbon fiber, preferably minute carbon fiber having diameter in the range of 3.0×10⁻³-1.0µm and aspect ratio in the range of 5-100,000, or ferrite powder represented by the following formula M²⁺Fe³⁺₂O₄ or MO(Fe₂O₃) (wherein M stand for metal atoms such as manganese, zinc, nickel and copper) having the particle size of 1-50µm, or the mixture of 10-200 parts by weight of ferrite powder, 0.5-10 parts by weight of foaming agent and 1-10 parts by weight of silanol group-capped polydiorganosiloxane having viscosity in the range of 500-10,000 cst (at 25°C) to 100 parts by weight of organopolysiloxane gum.

Furthermore, flame resistance can be given by addition of platinum compound of 1-1,000 ppm in term of platinum.

The crosslinking system by addition reaction, for example, the crosslinking system obtaining crosslinked silicone rubber by causing the vinyl group of organopolysiloxane having vinyl group to react additively with hydrogen atom of organohydrogenpolysiloxane having silicone-bonded hydrogen atom under existence of addition catalysts such as platinum and platinum compound can be used together with the crosslinking system of the present invention.

The addition reaction crosslinking system is the crosslinking system obtaining by the addition reaction of vinyl group of organopolysiloxane having vinyl group with hydrogen atom of organohydrogenpolysiloxane having silicone-bonded hydrogen atom under existence of addition reaction catalysts such as platinum and platinum compound, that system can be used together with crosslinking system of the present invention.

The above organohydrogenpolysiloxane have at least two or more hydrogen atom, preferably have three or more hydrogen atom, which may be at molecular terminal or at middle unit in each molecule. The substituted group bonded to silicone atom except for hydrogen atom can be the same as the above R^{a} in the above organopolysiloxane. Organohydrogenpolysiloxane can be any of straight-chain, cyclic or three dimensional structure.

The above organohydrogenpolysiloxane include trimethylsiloxy-capped methylhydrogenpolysiloxane, trimethylsiloxy-capped dimethylsiloxane-methylhydrogensiloxane copolymer, trimethylsiloxy-capped diphenylsiloxane-methylhydrogensiloxane copolymer, trimethylsiloxy-capped dimethylsiloxane-methylhydrogensiloxane-diphenylsiloxane copolymer, dimethylphenylsiloxy-capped dimethylsiloxane-methylhydrogensiloxane copolymer, dimethylphenylsiloxy-capped methylphenylsiloxane-methylhydrogensiloxane copolymer, dimethylhydrogensiloxy-capped dimethylpolysiloxane, dimethylhydrogensiloxy-capped methylvinylpolysiloxane, dimethylhydrogensiloxy-capped dimethylsiloxane-methylvinylsiloxane copolymer, dimethylhydrogensiloxy-capped dimethylsiloxane-methylhydrogensiloxane copolymer, dimethylhydrogensiloxy-capped methylphenylsiloxane-methylhydrogenpolysiloxane copolymer, dimethylhydrogensiloxy-capped methylvinylsiloxane-methyl(3,3,3-trifluoropropyl)siloxane copolymer, dimethylhydrogensiloxy-capped dimethylsiloxane-methylvinylsiloxane-methylphenylsiloxane copolymer, dimethylsiloxane-methylhydrogensiloxane cyclic copolymer, cyclic methylhydrogenpolysiloxanes such as tetramethyltetrahydrogencyclotetrasiloxane, cyclic methylhydrogenpolysiloxane tetramer, 1,3,5,7-tetrahydrogen-1,3,5,7-tetra-methylcyclotetrasiloxane, 1-propyl-3,5,7-trihydrogen-1,3,5,7-tetramethylcyclotetrasiloxane, 1,5-dihydrogen-3,7-dihexyl-1,3,5,7-tetramethylcyclotetra-siloxane, dimethylsiloxane-methylhydrogensiloxane copolymer having methyl(trimethoxysilylpropyl)siloxane unit and 3-glycidoxypropylmethylsiloxane unit, copolymer having dimethylhydrogensiloxane units and SiO₂ units, copolymer having (CH₃)₂HSiO_{1/2} units and SiO_{4/2} units, copolymer having (CH₃)₂HSiO_{1/2} units, SiO_{4/2} units and (CH₃)₃SiO_{1/2} units, copolymer having (CH₃)₂HSiO_{1/2} units, SiO_{4/2} units and (C₆H₅)SiO_{3/2} units, copolymer having R^{a}HSiO_{2/2} units and SiO_{3/2} units, copolymer having R^{a}HSiO_{2/2} units and HSiO_{3/2} units, pentasiloxane represented by the following formula Si(OSi(CH₃)₂H)₄, polysilalkylenesiloxane having silicone-bonded hydrogen atom, polysilane, polycarbosilane.

For example, a microparticle of platinum, palladium or rhodium which was adsorbed on a supports such as silica and alumina or silicagel, carbon black and carbonpowder can be added to organopolysiloxane gum in the range of 0.01-5,000 ppm as the above catalyst for addition reaction.

The above addition reaction catalyst include platinium catalyst, palladium catalyst or rhodium catalyst known as an addition reaction catalysts such as microparticulate platinum, palladium or rhodium compound adsorbed on supports such as silica, alumina, silicagel, carbon black and carbon powder. The above addition reaction catalyst can be used in the range of 0.01-5,000 ppm to 100 parts by weight of organopolysiloxane gum.

The above platinum compounds include platinium compounds such as microparticulate platinum catalyst; chloroplatinic acid; mixture obtained by react platinum compound with alcohol; platinum-hydrocarbon complexs obtained by react platinum compound with olefines such as ethylene, propylene, butadiene and cyclohexene or with cyclic diene; platinum-aldehyde complex; platinum-diketone complex; platinum-alkenylsiloxane complexs obtained by react platinum compound with 3-divinyltetramethyldisiloxane or 1,1,3,3-tetramethyl-1,3-divinyldisiloxane; reactants obtained by react platinum compound with (alkyl/aryl) tris(3-(alkyl/aryl)-1-butyne-3-oxy)silanes such as methyltris(3-methyl-1-butyne-3-oxy)silane, platinum-acetyl acetonate complex; platinum-phosphorus complexs such as tetrakis(triethylphosphine)platinum, tetrakis(tributylphosphine)platinum, tetrakis(triphenylphosphine)platinum, tetrakis(trimethylphosphite)platinum, tetrakis(triethylphosphite)platinum, tetrakis(tributylphosphite)platinum, tetrakis(triphenylphosphite)platinum; platinum-vinyl siloxane complex such as 2-platinum 1-unsaturated group-containing siloxane complex; platinum complex with divinyltetramethyldisiloxane or divinyldimethylsilicone, palladium catalysts such as tetrakis(triphenylphosphine)palladium, mixture of palladium black with trihenylphosphine and palladium-phosphine complex, rhodium catalysts such as rhodium-phosphine complex and cobalt carbonyl or the like.

Thermoplastic resins such as polycarbonate resin, polymethyl methacrylate resin, polystyrene resin, nylon resin and silicone resin having softening point in the range of 50-200°C, which contains the above platinum catalysts, can also be used as sphere microparticlate catalyst.

The above platinum compounds can be used as solution of saturated concentration or lower.

The above solvent for platinum compound include liquid and inert low molecular weight organopolysiloxanes of straight-chain or cyclic dimethylsiloxane oligomer of 1to 10 silicone atoms such as hexamethyldisiloxane, octamethyltrisiloxane, decamethyltetrasiloxane, dodecamethylpentasiloxane, octamethylcyclotetrasiloxane and decamethylcyclopentasiloxane; liquid silanes such as dimethyldimethoxysilane; hydrocarbons such as n-hexane, n-heptane, cyclohexane, toluene and hexylene; alcohols; ketones; esters; ethers; halogenated hydrocarbon; and water on can be used, and these may be used singly or as combination of two or more.

Silicone rubber composition of addition reaction system using platinium catalyst can be used with the platinium catalyst such as divalent platinum-phosphorus complex, zerovalent platinum-phosphorus complex, mixtures or reaction producsts of platinum-phosphorus complex with the asymmetry organic peroxide to restrain crosslinking reaction occurred at about normal temperature or, if necessary, with crosslinking retardants to restrain addition reaction properly.

The above crosslinking retardant include diallyl maleate; triallyl isocyanurate; organic compound having acetylenic bonding and having no elements such as nitrogen and phosphorus or sulfur in α-position thereof; en-yne compounds such as 3-methyl-3-pentene-1-yne and 3,5-dimethyl-3-hexene-1-yne; acetylene compounds such as ethynylcyclohexanol; acetylene alcohols such as 3-methyl-1-butyn-3-ol, 3-methyl-3-butyne-2-ol, 2-methyl-1-pentyn-3-ol, 3-methyl-1-pentene-3-ol, 3,5-dimethyl-1-hexyne-3-ol, 3,5-dimethyl-1-hexyne-2,5-diol, 3,6-dimethyl-4-octyne-3,6-diol, 2,4,7,9-tetra-methyl-5-decyne-4,7-diol and phenylbutynol or condensated product of acetylenealcohol with siloxane unit; azo compound; triazoles such as benzotriazole, 1-methyltriazole, 5,6-dimethylbenzotriazole, 2-phenylbenzotriazole, 1,2,3-triazole, 1-methyl-1,2,3-triazole, 1-phenyl-1,2,3-triazole, 4-methyl-2-phenyl-1,2,3-triazole, 1,2,4-triazole, 1-methyl-1,2,4-triazole and 1, 3-diphenyl-1,2,4-triazole; amine compounds such as 2,2-biquinoline, 2-picoline, 4-picoline, 2,6-lutidine, 2,2'-piperidine, pyridine, N,N-dimethylformamide, N,N-diethylformamide, ethylene thiourea and N,N'-diethylthiourea; nitrile compounds; hydrazine compounds represented in general formula RNH(NH₂) to improve flame resistance such as methylhydrazine, ethylhydrazine and phenylhydrazine; sulfonyl compounds; sulfur compounds; mercaptan compounds; phosphorus compounds such as triethyl phosphate, tributyl phosphate, triamyl phosphate, triphenyl phosphate, tritolyl phosphate, trimethyl phosphite, triethyl phosphite, tributyl phosphite, triphenyl phosphite and tribenzyl phosphite;
crosslinking inhibitor having -COOH group in the molecule; organic peroxides having at least one hydroperoxy group in the molecule thereof such as methyl ethyl ketone peroxide, tert-butyl hydroperoxide, cumene hydroperoxide, 1,1,3,3-tetramethylbutyl hydroperoxide, 2,5-dimethyl-2,5-dihydroperoxide, 1-hydroxycyclohexyl hydroperoxide, dekalin hydroperoxide, 1,1,2,2-tetra-methylpropyl hydroperoxide, p-menthane hydroperoxide and pinane hydroperoxide; cyclic vinyl group-containing siloxanes such as methyltris(methylisobutynoxy) silane, methylvinylcyclotetrasiloxane, for example, 1,3,5,7-tetramethyl-1,3,5,7-tetravinylcyclotetrasiloxane, 1,3,5,7-tetramethyl-1,3,5,7-tetrahexenylcyclotetrasiloxane; tin salt; ion compounds of heavy metal.

The condensation crosslinking system is the method of condensating reaction of the crosslinking agent (having at least two silicone-bonded hydroxyl group or hydrolyzable group in one molecule) under existence of condensation reaction catalyst, that system can be used together with crosslinking system of the present invention.

It is necessary that the condensation reaction-crosslinking agent have at least two silicone-bonded hydroxyl group or hydrolysable group in one molecule to crosslink by condensation reaction.

The above hydrolysable group include alkoxy groups such as methoxy, ethoxy, propoxy and butoxy group; alkoxyalkoxy groups such as 2-methoxyethoxy and 2-ethoxyethoxy group; alkenyloxy groups such as propenoxy group; acyloxy groups such as acetoxy and octanoyloxy group; ketoxime groups such as acetone oxime and methylethylketoxime group; organo amino groups such as 1-methylvinyloxy, diethylamino, butylamino, hexylamino and cyclohexylamino group; diorganoaminoxy groups such as dimethylaminoxy and diethylaminoxy group; organoamide groups such as N-methylacetamide group; imino group;and isocyanate group, and organosilane or organopolysiloxane having these group can be used for condensation reaction-crosslinking agent.

The above condensation reaction-crosslinking agent include the above silane coupling agents, alkoxysilanes such as ethyl orthosilicate, ethyl polysilicate, propyl orthosilicate and silane compound; and partially hydrolyzed and condensated product of the above alkoxysilanes; alkenyloxysilanes such as methyltris(methoxyethoxy)silane, vinyltris(methoxyethoxy)silane, methyltripropenoxysilane, vinylpropenoxysilane and tetrapropenoxysilane; and partially hydrolyzed and condensated product of the above alkenyloxysilanes; acyloxysilanes such as methyltriacetoxysilane, vinyltriacetoxysilane and diacetoxydibutoxysilane; and partially hydrolyzed and condensated product of the above acyloxysilanes; acyloxysiloxanes such as (CH₃COO) (CH₃)₂SiOSi (CH₃)₂(OCOCH₃) ; ketoximesilanes such as methyltris(acetoneoxime)silane, methyltris (methylethylketoxime)silane, vinyltri(acetoneoxime)silane and vinyltri(methylethylketoxime)silane; and partially hydrolyzed and condensated product of the above ketoximesilanes; organoaminosilanes such as methyltris(dimethylamino)silane, methyltris (diethylamino)silane and methyltris(cyclohexylamino)silane; and partially hydrolyzed and condensated product of the above organoaminosilanes; organoaminosiloxanes such as (CH₃)₂N ((CH₃)₂SiO) (CH₃)₂SiN(CH₃)₂; diorganoaminoxysilanes such as methyltris(diethylaminoxy)silane; and partially hydrolyzed and condensated product of the above diorganoaminoxysilanes; organoamidsilanes such as methyltris (N-methylacetamide) silane; and partially hydrolyzed and condensated product of the above organoamidsilanes; cyclic polysiloxanes such as vinyltris(1-methylvinyloxy)silane, hexamethylbis(diethylaminoxy)cyclotetrasiloxane, tetramethyldibutylbis (diethylaminoxy)cyclotetrasiloxane and heptamethyl(diethylaminoxy)cyclotetrasiloxane, pentamethyltris(diethylaminoxy)cyclotetrasiloxane and hexamethylbis (methylethylaminoxy) cyclotetrasiloxane, tetramethyl-bis (diethylaminoxy) -mono (methylethylaminoxy) cyclotetrasiloxane.

The above condensation reaction catalyst include tin oxides, organic carboxylate metal salt, titanate coupling agent, quaternary ammonium salt and guanidine group containing compound.

The above tin oxides include stannic oxide; the above organic carboxylate metal salt is consisting of organic carboxylic acids such as acetic acid, octylic acid, lauric acid, stearic acid, oleic acid, linoleic acid, benzoic acid and naphthoeic acid and metals such as tin, iron, lead, zirconium, stibium, cadmium, cobalt andmanganese, especially exemplified by dimethyl tin dilaurate, dimethyl tin dioleate, dibutyl tin di-2-ethylhexanoate, dibutyl tin dilaurate, dibutyl tin diacetate, dibutyl tin dimethylate, dibutyl tin dioctoate, dibutyl tin dilaurate, dibutyl tin dioleate, dibutylbis(triethoxysiloxy) tin, dibutyl tin dibenzoylmaleate, dioctyl tin dilaurate, diphenyl tin diacetate, tin lactate, tin octenoate, tin octanoate, tin decanoate, tin naphthenate, tin caprylate, tin oleate, dibutyl tin dioxide, dibutyl tin dimethoxide, zinc octanoate, iron stearate, iron octoate, cobalt octoate, manganese octoate and lead octylate.

The above titanate coupling agent include organic titanates, organic titanate polymer, titanium complex and titanium chelate compounds such as tetramethyl titanate, tetraethyl titanate, tetrapropyl titanate, tetraisopropyl titanate, tetrabutyl titanate, tetraoctyl titanate, tetra-2-ethylhexyl titanate, tetrastearyl titanate, dibutyl diisopropyl titanate, (2-methacryloxyethyl) triisopropyl titanate, isopropyl tristearoyl titanate, isopropyl tris(dioctylpyrophosphate) titanate, bis(dioctylpyrophosphate)ethylene titanate, butyl titanate dimer, polytitanium acetylacetonato, titanium acetylacetate, titanium ethylacetoacetate, dipropoxy bis(acetylacetonato) titane, diisopropoxy bis(acetylacetonato) titane, dipropoxy bis(ethylacetoacetonate) titane, diisopropoxy bis(ethylacetoacetonate) titane, dibutoxy bis(acetylacetonato) titane, 1,3-dioxypropane bis(acetylacetonato) titane, 1,3-dioxypropane bis(ethylacetoacetonate) titane, titane tetrakis(bis-2,2(allyloxymethyl)butoxide), tetrakis(trimethylsiloxy)titane, (triisopropyltitanoxy)tri-n-butyl tin, titane diisopropoxide(bis-2,4-pentanedionate), titane di-n-butoxidebis-(ethylacetoacetate), titane lactate, titane lactateethyl ester, isopropyldodecylbenzenesulfonyl titanate, titane methacrylate triisopropoxide, titane allylacetate triisopropoxide, titane methacryloxyethylacetoacetate triisopropoxide, titanocene diphenoxide, triethanolamine titanate, ethyleneglycol titanate and octyleneglycol titanate.

The above quaternary ammonium salt include amine compounds such as dimethylhexylamine, di-n-butylamine, diethylhydroxylamine and tetramethylguanidine; and quaternary ammonium salts such as tetramethylammonium chloride and trimethylhexyl ammonium chloride.

The method of preparing silicone rubber compound comprising the asymmetry organic peroxide of the present invention will be mentioned in the following.

In preparing silicone rubber compound, for example, the asymmetry organic peroxide having a (substituted) benzoylperoxycarbonyloxy group of the present invention can be added as the crosslinking agent to the silicone rubber base compound obtained by mixing the prescribed quantity of organopolysiloxane gum, silica filler and various additives uniformly.

The amount of the crosslinking agent to be added is usually in the range of 0.1-10 mmole, more preferably 0.2-5 mmole expressed in terms of the peroxy bond content based on 100g of silicone rubber base compound. The present crosslinking agent provides insufficient crosslinking at additions below 0.1 mmole, while resulting excess decomposition residue provides undesiable influence to moldings and costs rises at additions more than 10mmole.

In kneading of raw materials to obtain silicone rubber base compound, it can be heated if necessary.
For example, lower compression set can be attained by mixing organopolysiloxane, silica filler and organohydrogenpolysiloxane while heating at 100°C or higher. Green strength can be improved by mixing 0.005-5.0 parts by weight of orthoboric acid or metaboric acid, 0.5-20 pars by weight of hydroxyl group-containing organosilicone compound represented by the formula HO(R₂SiO)_{d''}H (where in R stands for substituted or unsubstituted monovalent hydrocarbon group, d''=1-20), and 100 parts by weight of organopolysiloxane gum while heating at 80°C or higher, and releasability can be improved by mixing small quantity of metal salt of higher fatty acid with silicone rubber base compound while heating, and electric character of high voltage electrical insulators can be improved by mixing organopolysiloxane gum and aluminum hydrate while heating for thirty minutes or longer at 100°C or higher.
Fatigue resistance can be improved by mixing 1-30 parts by weight of the same organopolysiloxane to the prekneaded mixture of 100 parts by weight of organopolysiloxane gum, silica filler and dispersant.
Silicone-polyolefine composite rubbermoldings, which have excellent characters in roller processability, heat-resistance and mechanical strength, can be obtained by mixing organopolysiloxane and microparticulatesilica before mixing ethylene-α-olefine copolymerization rubber, and optionally, microparticulate silica.

Usual kneader machines , for example, a two-roll mill, a kneader, a banbury mixer, an inter-blender, amixmaler, a henshell-mixer, a lossblender, aplanetary mixer, a hovert-mixer, a Shinagawa mixer, a twin screw continuous kneader-extruder, can be used in this invention.

Crosslinked silicone rubber moldings can be obtained from uncrosslinked silicone rubber moldings such as tube-shaped moldings, rod-shaped moldings and seat-shaped moldings obtained by using a kneader machine such as two-rolls, a calendar roller or an extruder, by thermal crosslinking at a crosslinkable temperature of the silicone rubber composition or higher under ordinary pressure.

The molding method of the present invention includes pressure molding methods such as an extrusion molding, in which the moldings are crosslinked by hot air, steam or the other heat medium under normal pressure, press molding methods such as a compression molding, an injection molding, a liquid injection molding and a transfer molding, and other methods of processing such as a calendar processing, a hollow molding, and an impregnating or appliing of rubber solution. Large size rollers or packings are subject to primary molding before finished by crosslinking in a steam pot.
Flowability and crosslinking character of compounds are properly adjusted for each process.
The crosslinking temperature can be in the range from room temperature to 550°C, and crosslinking time can be in the range from 5 seconds to 10 hours, they are suitably selected in accordance with the molding method.

The heating method in crosslinking silicone rubber of the present invention includes a direct exposusre to an electric heater or an infrared rays heater, and a hot air-circulation, or a combination of a high-frequency (UHF) crosslinking furnace or a radiologic crosslinking furnace such as an electron beam-crosslinking furnace using irradiation of beta ray or gamma ray with hot air crosslinking after mixing carbon or ferrite powder into a silicone rubber composition.
Other crosslinking processes such as a steam continuous crosslinking, a hot liquid vulcanizing (HLV), fluidized bed vulcanizing (FBV), and a liquid curing medium (LCM) are also applicable.

In an extrusion molding, for example, for manufacturing electric wire and cable coatings, uncrosslinked moldings may be crosslinked by hot air for from 5 seconds to 1 hour in the range of 200-500°C under ordinary pressure, or may be crosslinked for 1 hour at 200°C before subject to secondaly-crosslinking for several minutes at 400°C.

In crosslinking method for sponge rubber allowed to sufficient heat at a crosslinking temperature for silicone rubber composition or more besides a thermal decomposition temperature for foaming agent or more. For example, silicone rubber composition is heated to crosslink at 100-500°C for several seconds to 1 hour if necessary, silicone rubber composition can be allowed to secondaly-crosslinking by step-cure dividing heating condition into two steps or more, for example, heating at 150-300°C for about from 10 minute to 10 hours subsequently.

A foaming method includes the method of aging at a low temperature before foaming using a composition consisting of organohydrogenpolysiloxane, platinum catalyst and organic foaming agent.

A molding method for conductive roller include the method of press molding at a temperature of 90-170°C within the reaction ratio of crosslinkable group in the ranges of 1-80%, then the moldings took out from mold, before foaming and residual crosslinking at a temperature of 140 to 300°C.

In a compression molding, for example, uncrosslinked moldings may be heated for 5-60 minutes under a pressure of about 50-100kg /cm² at a temperature of about 100-200°C, though the molding condition can be suitably selected depending on the crosslinking system, the molding machine and so on.
Otherwise, the asymmetry organic peroxide may be added into a semicrosslinkable organopolysiloxane composition and made into a particurate form before being heated at a temperature below 100°C to produce a semicrosslinked, particulate molding material which retains the activity of the asymmetry organic peroxide and which contains residual crosslinkable functional group on the organopolysiloxane. Then, after filling a mold, the molding material may be completely crosslinked at a temperature of at least 100°C under suitable applied pressure.

A method of molding unified material by adhering different silicone rubbers include the method of applying liquid of the asymmetry organic peroxide on the surface of unlcrossinked silicone rubber composition and then allowed to compression molding at the decomposition temperature of the said asymmetry organic peroxide or higher, or the method of coating or laminating a rubber composition on the surface of uncrosslinked or crosslinked silicone rubber and thereafter lying thermoplastic resin on and adhering at a temperature of 100-300°C under the application of pressure of 0.1-10kg /cm², or the method of filling a semicrosslinked silicone rubber particulate (consisting of organohydrogenpolysiloxane having silicone-bonded hydrogen atom of 2 or more in the molecule and organopolysiloxane having silicone-bonded vinyl group of 2 or more as main component, the ratio of the former silicone-bonded hydrogen atom in organohydrogenpolysiloxane to the latter silicone-bonded vinyl group in organopolysiloxane is combined to be in the range of 0.20-0.95) into the cavity of a mold, then introducing a thermally crosslinkable silicone rubber composition and closing the mold, and subsequently heating the above mold with the application of pressure in order to form a single body or element by crosslinking the above thermally crosslinkable silicone rubber composition at the same time that crosslinking of the above semicrosslinked silicone rubber particulate is brought to completion.

In a hollow molding, the process of a silicone rubber tube formation can be applied, in which silicone rubber is extruded into a tube or a thin-film by blowing a gas such as air, at the same time it is thermally crosslinked, to produce sheet or film moldings.

A method of impregnating and coating rubber solution include the coating by spray or dip method using uncrosslinked organopolysiloxane gum solution with organic solvents such as benzene, toluene, xylene, hexane, heptane, kerosene, methanol, ethanol, isopropanol, butanol, 1,1,1-trichloroethane, perchloroethylene, methylene chloride and tetrachloroethylene of singly or as a suitable combination of two or more.

A method of coating silicone elastomer onto cloth include the method of knife coating, calendering, dip coating, spray, and brushing using coating-machines such as knife coater, comma coater, gravure coater and offset roller coater.

The various molding methods, besides the above-described methods, can be used in accordance with the intended products.

For example, planar exotherm can be obtained by coating silicone rubber composition on either surface of heat-resistant seat material such as metal foil or glass cloth and adhering exotherm wire covered by silicone rubber to the above surface by heating under the application of pressure to form heater wire for froster in refrigerator, freezing warehouse, or show-case.

The forming method of conductive polymer coating include the method to contact organopolysiloxane containing oxidizers such as ferric chloride, lead dioxide and quinone with gas of monomer having heterocyclic groups of five-membered ring or six-membered ring such as pyrroles or thiophenes, carbazole and dibenzothiophene.

A illuminant in crosslinking method by irradiaton of ultraviolet rays include lamps such as low-pressure mercury lamp, high-pressure mercury lamp or extra high-pressure mercury lamp, xenon mercury lamp and metal halide lamp, either of ozone generating type or ozoneless type, and The method of treating glass fiber by irradiation of ultraviolet rays include the method of coating the mixture consisting of ethylenically unsaturated group-containing organopolysiloxane having molecular terminal capped with the group selected fromdivinylmethylsilyl group, trivinylsilyl group andmethylphenylvinyl group, and the asymmetry organic peroxide onto glass fibers such as glass sleeve, glass cloth, glass roving, glass mat or braided glass wire, before crosslinking by irradiating ultraviolet rays having wave number in the range of 100-300nm.

A silicone rubber-resin unified key-pad, which have excellent adhesion, hard to cause contact-trouble caused by penetration of hand-sweat, excellent durability of printed symbols and visibility at night, is obtained by the rubber contact moldings method of coating cyanoacrylate adhesives on a crosslinked silicone rubber and then crosslinking with uncrosslinked resin together.

Silicone rubber moldings obtained by the above methods can be used in the fields such as electric or electronic instruments, office work machines, automobiles, aircrafts, buildings, constructions, medical supplies and foods.

In the field of electric or electronic instrument, silicone rubber moldings can be used for cable coatings such as electric power cable, control cable for nuclear power generation or vessel, ignition cable for automobile, metal sheath cable, plastic sheath cable, high voltage cable and optical fiber, internal wire for electric instrument, lead wire, internal wire for parts having high temperature of electric instruments such as oven range, electric heater and electric pot, heater wire for defroster in refrigerator, warehouse for freezing and show-case, heater wire for pipeline or cooking vessel, resistance heater for mat or tape and clothes or bedclothes, far-infrared ray releasing materials,
cap, boots, packing, gasket and o-ring for household utensils such as television, cathode-ray tube of television or display, iron, electronic jar, electric pot, microwave oven, dripolator, hair curler, hot plate, air-conditioning machine, flood light, electric parts, pressure pot and water heater module material for electric or electronic instrument, conductive parts, insulation-rubber tube, conductive-rubber tube, EMI shield gasket, gasket for electromagnetic shield, waveguide gasket, concentric connector, diaphragm, wire harness seeled connector, plates for supporting nichrome wire such as hair drier, iron and toaster, heat-resistable-decorative-protective coated material for household utensils such as heat-resistance washer, bulkhead board for microwave oven, switch, microwave oven and drier,
electric motor coated to the glass cloth, dry type transformer, middle or coil and rare or earth insulation for for household utensils, throat insulator for H-type insulation machine, terminal assembly, microwave covering board for microwave oven,

A radiation material instulled between integrated circuit elements such as power transistor, thyristor, IC, LSI, CPU and MPU, which is used for personal computer, word prosessor, especially for lap-top type or notebook type portable personal computer generating considerable amount of heat, and materials or radiation boad,
junction on the surface of the semiconductor devices such as electric or electronicparts includingmesa type PNP transistor and planar type PNP transistor, diode, IC and capacitor coil, junction coating and buffer coating for Aluminum electrode, delicate bonding wire and optical fiber to protection,
masking agent to humidity proof, dust proof, heat resistant, insulating, cold resistance and wetherability for sealant, solder resist and through hole of print circuit board,
adhering sealing agent for electric or electronic parts such as fixation of coil to circuit board, potting and adhering seal for sensor module,
pressure sensitive electro conductive mat switch for elasticic electrode and automatic door, pressure sensitive electro conductive senser for robot, electrode material for medical equipment, electronic device material for temperature sensitive element, resistance element and capacitor element, contact point material for electric or electronic parts, and electric instrument.

A glass material for high voltage electrical insulators such as electrical insulating bushing and cable head, made of specific ruber composition based on silicone rubber and silicone-EPDM or EVA hybrid rubber, for power distribution and transmission lines is attracted attention as altanative to ceramics.

In the field of office work machine, silicone rubber moldings can be used as rubber parts of rollers, forming silicone rubber molding layer on roller-shaped core, such as conductive, developing, copying, loading paper, fixation, pressurizing, electron remover, cleaning and applying oil roller, cleaning belt and fixing belt for photocopy macine, facsimile and printer, utilizing heat resistance and releasability of silicone rubber,
telephones such as push-button telephone, cordless telephone, cellular and automobile telephon, keyboad switches (rubber contact) for calculator, computer terminal keyboard, word processor, electric pocketbook, switch or remote controller such as television, video and sound machine, electronic organ and so on, and elastic parts for printing type utilizing elasticity, low strain and fatigue resistance of silicone rubber.

In the field of automobile and aircraft, silicone rubber moldings can be used as heat resistant, oil resistant, cold resistant, vibration resistant, sound resistant and impact absorbing rubber include joint boots, plug boots, anode cap, bellows boots , rocker cover gasket, o-ring, oil seal, oil packing, oil filter, valve stem seal, sealing, shaft seal, connector, diaphragm, high temperature gasket, rubber roll, hose, packing, wiper blade and weather strip materials for vehicles such as automobile and train, industry machine, device, aircraft, ship, harbor material and miniature motor, rubber parts for engine or exhaust used with high temperature oil, packing for heating fireplace, rubber packing parts for LNG carrier or environmental test vessel used at lower temperature of lower than -50°C, vibration resistant rubber such as damping parts for pick-up-arm used for sound instrument and electric or electronic instrument, adhering fixation or seal for screw, bolt, fixing parts and flange, and air bag made of silicone rubber-coated nylon cloth.

In the field of building or construction, silicone rubber moldings can be used as gaskets such as corner gasket, lute sticker gasket and zipper gasket having excellent wetherability, flame resistance and low compressionset to seal for chink and joint of building or construction industry use, material with excellent fire resistance, hybrid gasket for prevent pollution, sound proof for institutions, impact absorbance, insulation, insulation for insulation pipe of water supply, hot water supply and air conditioner system, steam pipe, air film structuraling material, material for structures such as gymnasium and garage, sponge seat for electrification prevention mat, conductive rubber with electrification or electrostatic prevention of lute sticker wall and floor for IC or LSI factory, electronic instrument plant, clean room and hospital, and protection material installed on mooring institution to soften of shock aused when vessel contact to pier, dock and quay.

In the field of medical supply or food industry, silicone rubber moldings can be used as tube, balloon, medicine stopper, O-ring, pump, valve packing, part for blood circuits, part for plasma skimming vessel, part for blood filter, catheter, acoustic lens for supersonicprobe of supersonic diagnostic equipment, orthopedics material, hollow fiber for artificial heart and lung, dialyzer part for artificial kidney and artificial stomach seat, part of artificial organ, wire coating for oral use in an orthodontic field, sponge for medical supply, pad material for medical supply, X-rays covering, ctheter for contrastradiography, contrast and opaque media of the intravascular, others of the anti-thrombus (anti-blood clotting), nipple, sealant for lunch box or food container between container and cap, tube for vending machine, toys, rubber stopper, rubber gloves, part for kitchen instrument, base oil for cosmetics to make-up, utilizing physiological inertness, light or gas permeability, releasability and durability of silicone rubber.

In the field of rubber lining, silicone rubber moldings can be used as anti-corrosion, soundproof and abrasion resistance material utilizing chemical resistance and low liquid or gas permeability of silicone rubber. In the field of chemical industry, water treatment device, air pollution control and rubber coated fabric for food industry, as a product of rubber coated on fiber base clothes, which is useful as known industrial materials, such as sheet, curtain, raincoat, tent, ski wear, mountain climbing wear, gardening house material, rubber raft, air mattress and liferaft.

Silicone rubber moldings can also be used as swimming, diving articles, leisure articles such as a golf gripe, handle gripe, pen gripe, eye cap for camera, head phone, skid of the glasses, thermal contraction tube, molding goods (doorknob covering and handrail covering), fiberglass treatment, coupling material for glass laminate, optical glass lens and silicone ceramics coupling agent for fiber reinforced plastics obtained by filament winding and hand-lay up form.

The present invention will be described by way of nonlimitative examples and comparative examples.

### <Example 1>

### (The synthesis of 4-methylbenzoylperoxy isopropyl monocarbonate)

36.5g of diethyl ether, 18.3g (0.12mole) of 4-methylperbenzoic acid prepared by well-known method, and 12.3g (0.1mole) of isopropyl chloroformate were placed in a 300ml four-necked flask equipped with a stirrer and a thermometer, and then 60. (0.15mole) of 10% NaOH aqueous solution was added dropwise at 5°C for 30 minutes with stirring. After stirring was continued for an additional 60 minutes, the precipitate was filtered and washed with water until alkaline was not shown in the filtrate, and then recrystallized from dichloromethane/methylalcohol, dried under reduced pressure.
17.3g of white crystal of 4-methylbenzoylperoxy isopropyl monocarbonate (active oxygen content: 6.68%, purity: 99.4%, yield: 72.3%) was obtained.

The obtained 4-methylbenzoylperoxy isopropyl monocarbonate was characterized by the melting point and the nuclear magnetic resonance (NMR) spectra as shown below.
melting point: 60.5-62°C, ¹H-NMR(CDCl₃) : 1.39(d, 6H), 2.42(s, 3H), 5.04(m, 1H), 7.28(d, 2H), 7.89(d, 2H)

### <Example 2>

### (The synthesis of 4-methylbenzoylperoxy ethyl monocarbonate)

7.8g of white crystal of 4-methylbenzoylperoxy ethyl monocarbonate (active oxygen content: 6.81%, purity: 95.4%, yield: 33.2%) was obtained in the same manner as in example 1 except that 10.9g (0.1mole) of ethyl chloroformate was used instead of isopropyl chloroformate.

The obtained 4-methylbenzoylperoxy ethyl monocarbonate was characterized as shown below.
melting point: 37-38°C, ¹H-NMR(CDCl₃) : 1.39(t, 3H), 2.43(s, 3H), 4.40(q, 2H), 7.28(d, 2H), 7.88(d, 2H)

### <Example 3>

### (The synthesis of 4-methylbenzoylperoxy n-propyl monocarbonate)

15.4g of white crystal of 4-methylbenzoylperoxy n-propyl monocarbonate (active oxygen content: 6.63%, purity: 98.7%, yield: 63.8%) was obtained in the same manner as in example 1 except that 12.3g (0.1mole) of n-propyl chloroformate was used instead of isopropyl chloroformate.

The obtained 4-methylbenzoylperoxy n-propyl monocarbonate was characterized as shown below.
melting point: 42-43°C, ¹H-NMR (CDCl₃): 0.98(t, 3H), 1.76(m, 2H), 2.41(s, 3H), 4.29(t, 2H), 7.27(d, 2H), 7.88(d, 2H)

### <Example 4>

### (The synthesis of 4-methylbenzoylperoxy 2-ethylhexyl monocarbonate)

30.2g of colorless liquid of 4-methylbenzoylperoxy 2-ethylhexyl monocarbonate (active oxygen content: 5.10%, purity: 98.2%, yield: 96.2%) was obtained in the same manner as in Example 1 except that 19.3g (0.1mole) of 2-ethylhexyl chloroformate was used instead of isopropyl chloroformate.

The obtained 4-methylbenzoylperoxy 2-ethylhexyl monocarbonate was characterized as shown below.

¹H-NMR (CDCl₃) : 0.92(m, 6H), 1.38(m, 8H), 1.69(m, 1H), 2.42(s, 3H), 4.28(d, 2H), 7.26(d, 2H), 7.90(d, 2H)

### <Example 5>

### (The synthesis of 4-methylbenzoylperoxy cetyl monocarbonate)

37.8g of white crystal of 4-methylbenzoylperoxy cetyl monocarbonate (active oxygen content: 3.76%, purity: 98.9%, yield: 65.5%) was obtained in the same manner as in Example 1 except that 30.5g (0.1mole) of cetyl chloroformate was used instead of isopropyl chloroformate.

The obtained 4-methylbenzoylperoxy cetyl monocarbonate was characterized as shown below.
melting point: 46.5-47.5°C, ¹H-NMR(CDCl₃:) 0.88(t, 3H), 1.25(m, 26H), 1.74(q, 2H), 4.33(t, 2H), 2.43(s, 3H), 7.28(d, 2H), 7.89(d, 2H)

### <Example 6>

### (The synthesis of benzoylperoxy isopropyl monocarbonate)

16.6g of diethyl ether, 16.6g (0.12mole) of perbenzoic acid prepared by well-known method, and 12.3g (0.1mole) of isopropyl chloroformate were placed in a 300ml four-necked flask equipped with a stirrer and a thermometer, and then 60. (0.15mole) of 10% NaOH aqueous solution was added dropwise at 5°C for 30 minutes with stirring. After stirring was continued for an additional 60 minutes, the precipitate was filtered and washed with water until alkaline was not shown in the filtrate, and then recrystallized from dichloromethane/methylalcohol, dried under reduced pressure.
11.9g of white crystal of benzoylperoxy isopropyl monocarbonate (active oxygen content: 6.99%, purity: 98.0%, yield: 52.0%) was obtained.

The obtained benzoylperoxy isopropyl monocarbonate was characterized as shown below.
melting point: 57-58 °C, ¹H-NMR (CDCl₃) : 1.39(d, 6H), 5.02(m, 1H), 7.48(t, 2H), 7.60(t, 1H), 7.98(d, 2H)

### <Example 7>

### (The synthesis of benzoylperoxy 2-ethylhexyl monocarbonate)

27.88 of colorless liquid of benzoylperoxy 2-ethylhexyl monocarbonate (active oxygen content: 5.02%, purity: 92.4%, yield: 87.3%) was obtained in the same manner as in Example 6 except that 19.3g (0.1mole) of 2-ethylhexyl chloroformate was used instead of isopropyl chloroformate.

The obtained benzoylperoxy 2-ethylhexyl monocarbonate was characterized as shown below.
¹H-NMR (CDCl₃) : 0.92(m, 6H), 1.34(m, 8H) , 1.68(m, 1H), 4.20(d, 2H), 7.44(t, 2H), 7.58(t, 1H), 7.96(d, 2H)

### <Example 8>

### (The synthesis of benzoylperoxy cetyl monocarbonate)

30.8g of white crystal of benzoylperoxy cetyl monocarbonate (active oxygen content: 3.88%, purity: 98.7%, yield: 74.8%) was obtained in the same manner as in Example 6 except that 30.5g (0.1mole) of cetyl chloroformate was used instead of isopropyl chloroformate.

The obtained benzoylperoxy cetyl monocarbonate was characterized as shown below.
melting point: 42.5-43.5°C, ¹H-NMR(CDCl₃) : 0.88(t, 3H), 1.26(m, 26H), 1.74 (q, 2H), 4.34(t, 2H), 7.49(t, 2H), 7.64(t, 1H), 8.00(d, 2H)

### <Example 9>

### (The synthesis of di(4-methylbenzoylperoxy) hexamethylene biscarbonate)

73.2g of diethyl ether, 18.3g (0.12mole) of 4-methylperbenzoic acid prepared by well-known method, and 12.2g (0.05mole) of 1, 6-hexamethylenedichloroformate were placed in a 300ml four-necked flask equipped with a stirrer and a thermometer, and then 60.0g (0.15mole) of 10% NaOH aqueous solution was added dropwise at 5°C for 30 minutes with stirring. After stirring was continued for an additional 60 minutes, the precipitate was filtered and washed with water until alkaline was not shown in the filtrate, and then recrystallized from chloroform/methylalcohol, dried under reduced pressure.
20.3g of white crystal of di (4-methylbenzoylperoxy) hexamethylene biscarbonate (active oxygen content: 6.67%, purity: 98.9%, yield: 84.6%) was obtained.

The obtained di(4-methylbenzoylperoxy) hexamethylene biscarbonate was characterized as shown below.
melting point: 85-88°C, ¹H-NMP(CDCl₃) 1.42(m, 4H), 1.74(m, 4H), 2.41(s, 6H), 4.32(t, 4H), 7.28(d, 4H), 7.88(d, 4H)

### <Example 10>

### (The synthesis of di(2-methylbenzoylperoxy) hexamethylene biscarbonate)

18.1g of white crystal of di(2-methylbenzoylperoxy) hexamethylene biscarbonate (active oxygen content: 6.64%, purity: 98.4%, yield: 75.1%) was obtained in the same manner as in Example 9 except that 18.3g (0.12mole) of 2-methylperbenzoic acid was used instead of 4-methylperbenzoic acid.

The obtained di(2-methylbenzoylperoxy) hexamethylene biscarbonate was characterized as shown below.
meltingpoint: 61-63°C, ¹H-NMP(CDCl₃) : 1.46(m, 4H), 1.77(m, 4H), 2.58(s, 6H), 4.35(t, 4H), 7.28(m, 4H), 7.48(t, 2H), 7.83(d, 2H)

### <Example 11>

### (The synthesis of di(3-methylbenzoylperoxy) hexamethylene biscarbonate)

19.1g of white crystal of di(3-methylbenzoylperoxy) hexamethylene biscarbonate (active oxygen content: 6.50%, purity: 96.4%, yield: 77.6%) was obtained in the same manner as in Example 9 except that 18.3g (0.12mole) of 3-methylperbenzoic acid was used instead of 4-methylperbenzoic acid.

The obtained di(3-methybenzoylperoxy) hexamethylene biscarbonate was characterized as shown below.
melting point: 85-88°C, ¹H-NMR(CDCl₃:) 1.43(m, 4H), 1.75(m, 4H), 2.40(s, 6H), 4.33(t, 4H), 7.39(m, 4H), 7.80(d, 4H)

### <Example 12>

### (The synthesis of di(3,5-dimethylbenzoylperoxy) hexamethylene biscarbonate)

20.4g of white crystal of di(3,5-dimethylbenzoylperoxy) hexamethylene biscarbonate (active oxygen content: 6.05%, purity: 95.1%, yield: 77.2%) was obtained in the same manner as in Example 9 except that 19.9g (0.12mole) of 3,5-dimethylperbenzoic acid was used instead of 4-methylperbenzoic acid.

The obtained di(3,5-dimethybenzoylperoxy) hexamethylene biscarbonate was characterized as shown below.
meltingpoint: 90-93°C, ¹H-NMR(CDCl₃) : 1.44(m, 4H), 1.75(m, 4H), 2.35(s, 12H), 4.33(t, 4H), 7.26(s, 2H), 7.61(s, 4H)

### <Example 13>

### (The synthesis of di (4-tert-butyl benzoyl peroxy) hexamethylenebis carbonate)

18.4g of white crystal of di(4-tert-butylbenzoylperoxy) hexamethylene biscarbonate (active oxygen content: 5.50%, purity: 96.1%, yield: 63.3%) was obtained in the same manner as in Example 9 except that 23.3g (0.12mole) of 4-tert-butylperbenzoic acid was used instead of 4-methylperbenzoic acid.

The obtained di (4-tert-butylbenzoylperoxy) hexamethylene biscarbonate was characterized as shown below.
melting point: 93-95°C, ¹H-NMR(CDCl₃): 1.34(s, 18H), 1.44(m, 4H), 1.75(m, 4H), 4.34(t, 4H), 7.51(d, 4H), 7.94(d, 4H)

### <Example 14>

### (The synthesis of di(4-methoxybenzoylperoxy) hexamethylene biscarbonate)

21.4g of white crystal of di(4-methoxybenzoylperoxy) hexamethylene biscarbonate (active oxygen content: 6.15%, purity: 97.4%, yield: 82.3%) was obtained in the same manner as in Example 9 except that 20.2g (0.12mole) of 4-methoxyperbenzoic acid was used instead of 4-methylperbenzoic acid.

The obtained di(4-methoxybenzoylperoxy) hexamethylene biscarbonate was characterized as shown below.
melting point: 75-78°C, ¹H-NMR(CDCl₃) 1.43(m, 4H), 1.75(m, 4H), 3.88(s, 6H), 4.33(t, 4H), 6.96(d, 4H), 7.97(d, 4H)

### <Example 15>

### (The synthesis of di(benzoylperoxy) hexamethylene biscarbonate)

17.8g of white crystal of di(benzoylperoxy) hexamethylene biscarbonate (active oxygen content: 6.94% , purity: 96.9%, yield: 77.3%) was obtained in the same manner as in Example 9 except that 16.6g (0.12mole) of perbenzoic acid was used instead of 4-methylperbenzoic acid.

The obtained di(benzoylperoxy) hexamethylene biscarbonate was characterized as shown below.
melting point: 73-75°C, ¹H-NMR(CDCl₃) : 1.42(m, 4H), 1.74(m, 4H), 4.33(t, 4H), 7.46(t, 4H), 7.64(t, 2H), 7.99(d, 2H)

### <Example 16>

### (The synthesis of 1,1,1-tris (4-methylbenzoylperoxycarbonyloxymethyl) propane)

123.1g of toluene, 27.4g (0.18mole) of 4-methylperbenzoic acid prepared by well-known method, and 16.1g (0.05mole) of trimethylolpropane trischloroformate were placed in a 300ml four-necked flask equipped with a stirrer and a thermometer, and then 90.0g (0.225mole) of 10% NaOH aqueous solution was added dropwise at 5°C for 30 minutes with stirring. After stirring was continued for an additional 60 minutes, the precipitate was filtered and washed with water until alkaline was not shown in the filtrate, and then recrystallized from chloroform/methylalcohol, dried under reduced pressure.
17.7g of white crystal of 1,1,1-tris(4-methylbenzoylperoxycarbonyloxymethyl)propane (active oxygen content: 6.65%, purity: 92.6%, yield: 49.0%) was obtained.

The obtained 1,1,1-tris(4-methylbenzoylperoxycarbonyloxymethyl)propane was characterized as shown below.
melting point: 92-95 °C, ¹H-NMR(CDCl₃): 0.95(t, 3H), 1.56(q, 2H), 2.42(s, 9H), 4.34(s, 6H), 7.28(d, 6H), 7.88(d, 6H)

### <Example 17>

### (The synthesis of 4-methylbenzoylperoxy cyclohexyl monocarbonate)

13.7g of white crystal of 4-methylbenzoylperoxy cyclohexyl monocarbonate (active oxygen content: 5.73%, purity: 99.7%, yield: 49.1%) was obtained in the same manner as in Example 1 except that 16.3g (0.1mole) of cyclohexyl chloroformate was used instead of isopropyl chloroformate.

The obtained 4-methylbenzoylperoxy cyclohexyl monocarbonate was characterized as shown below.
melting point: 61-63.5°C, ¹H-NMR(CDCl₃): 1.37(m, 3H), 1.58(m, 3H), 1.74(m, 2H), 1.93(m, 2H), 2.42(s, 3H), 4.81(q, 1H), 7.28(d, 2H), 7.89(d, 2H)

### <Example 18>

### (The synthesis of 4-methylbenzoylperoxy 4-tert-butylcyclohexyl monocarbonate)

17.4g of white crystal of 4-methylbenzoylperoxy 4-tert-butylcyclohexyl monocarbonate (active oxygen content: 4.76%, purity: 99.6%, yield: 51.8%) was obtained in the same manner as in Example 1 except that 21.9g (0.1mole) of 4-tert-butylcyclohexyl chloroformate was used instead of isopropyl chloroformate.

The obtained 4-methylbenzoylperoxy 4-tert-butycyclohexyl monocarbonate was characterized as shown below.
melting point: 82-86°C, ¹H-NM (CDCl₃): 0.85(s, 9H), 1.10(m, 3H), 1.45(m, 3H), 1.83(m, 2H), 2.18(m, 2H), 2.42(s, 3H), 4.67(m, 1H), 7.28(d, 2H), 7.89(d, 2H)

### <Example 19>

### (The synthesis of 2,2-bis(4-(4-methylbenzoylperoxycarbonyloxy)cyclohexyl)propane)

3.2g of white crystal of 2,2-bis (4-(4-methylbenzoylperoxycarbonyloxy)cyclohexyl)propane (active oxygen content: 5.08%, purity: 94.6%, yield: 10.1%) was obtained in the same manner as in Example 9 except that 191.0g of toluene was used instead of diethyl ether and 18.3g (0.05mole) of 2,2-bis(4-chloroformyloxycyclohexyl)propane was used instead of isopropyl chloroformate.

The obtained 2,2-bis(4-(4-methylbenzoylperoxycarbonyloxy)cyclohexyl)propane was characterized as shown below.
melting point: 131-133°C, ¹H-NMR(CDCl₃): 0.73(s, 6H), 1.10-2.19(m, 18H), 2.43(s, 6H), 4.67(m, 2H), 7.27(d, 4H), 7.89(d, 4H)

### <Example 20>

### (The synthesis of 4-methylbenzoylperoxy 2-ethoxyethyl monocarbonate)

16.5g of white solid of 4-methylbenzoylperoxy 2-ethoxyethyl monocarbonate (active oxygen content: 5.91%, purity: 99.1%, yield: 60.9%) was obtained in the same manner as in Example 1 except that 15.3g (0.1mole) of 2-ethoxyethyl chloroformate was used instead of isopropyl chloroformate.

The obtained 4-methylbenzoylperoxy 2-ethoxyethyl monocarbonate was characterized as shown below.
melting point: 37.5-38.5°C, ¹H-NMR(CDCl₃:) 1.19(t, 3H), 2.42(s, 3H), 3.53(q, 2H), 3.70(t, 2H), 4.46(t, 2H), 7.27(d, 2H), 7.88(d, 2H)

### <Example 21>

### (The synthesis of 4-methylbenzoylperoxy 3-methoxybutyl monocarbonate)

21.0g of colorless liquid of 4-methylbenzoylperoxy 3-methoxybutyl monocarbonate (active oxygen content: 5.49%, purity: 96.8%, yield: 72.0%) was obtained in the same manner as in Example 1 except that 16.7g (0.1mole) of 3-methoxybutyl chloroformate was used instead of isopropyl chloroformate.

The obtained 4-methylbenzoylperoxy 3-methoxybutyl monocarbonate was characterized as shown below.
¹H-NMR(CDCl₃): 1.17(d, 3H), 1.87(q, 2H), 2.41(s, 3H), 3.30(s, 3H), 3.45(m, 1H), 4.46(m, 2H), 7.28(d, 2H), 7.88(d, 2H)

### <Example 22>

### (The synthesis of benzoylperoxy 2-butoxyethyl monocarbonate)

27.0g of colorless liquid of benzoylperoxy 2-butoxyethyl monocarbonate (active oxygen content: 5.40%, purity: 95.3%, yield: 91.1%) was obtained in the same manner as in Example 6 except that 18.1g (0.1mole) of 2-butoxyethyl chloroformate was used instead of isopropyl chloroformate.

The obtained benzoylperoxy 2-butoxyethyl monocarbonate was characterized as shown below.
¹H-NMP(CDCl₃): 0.88(t, 3H), 1.48(m, 4H), 3.46(t, 2H), 3.70(t, 2H), 4.46(t, 2H), 7.48(t, 2H), 7.58(t, 1H), 8.02(d, 2H)

### <Example 23>

### (The synthesis of di(4-methylbenzoylperoxy) 3-oxapentamethylene biscarbonate)

73.0g of diethyl ether, 36.5g (0.24mole) of 4-methylperbenzoic acid prepared by well-known method, and 23.1g (0.1mole) of diethyleneglycoldichloroformate were placed in a 300ml four-necked flask equipped with a stirrer and a thermometer, and then 120g (0.3mole) of 10% NaOH aqueous solution was added dropwise at 5°C for 30 minutes with stirring. After stirring was continued for an additional 60 minutes, organic layer was separated and washed with water until alkaline was not shown in the layer before poured into petroleum ether, and then the formed precipitate was filtered and dried under reduced pressure. 23.2g of white crystal of di(4-methylbenzoylperoxy) 3-oxapentamethylene biscarbonate (active oxygen content: 6.85%, purity: 99.0%, yield: 49.7%) was obtained.

The obtained di(4-methylbenzoylperoxy) 3-oxapentamethylene biscarbonate was characterized as shown below.
melting point: 36.5-38°C, ¹H-NMR(CDCl₃:) 3.79(t, 2H), 2.42(s, 3H), 4.47(t, 2H), 7.28(d, 2H), 7.89(d, 2H)

### <Example 24>

### (The preparation of a crosslinking agent of silicone paste comprising the asymmetry organic peroxide of the present invention and the preparation of a silicone rubber compound employing the same)

The crosslinking agent of silicone oil paste was prepared by thoroughly mixing 4-methylbenzoylperoxy isopropyl monocarbonate obtained in Example 1 with dimethylsilicone oil followed by homogenization using a three-roll mill.

The silicone rubber compound was prepared by adding the obtained crosslinking agent of silicone oil paste comprising 4-methylbenzoylperoxy isopropyl carbonate to a silicone rubber base compound for application in extrusion molding (silicone rubber KE561-U obtainable from Shin-Etsu Chemical Co., Ltd. (Inc.)) in an amount shown in Table 1 or Table 2, and thoroughly mixing it using a two-roll mill.

The crosslinking agent of silicone paste comprising 4-methylbenzoylperoxy isopropyl carbonate in an amount shown in Table 2, and 2 parts by weight of azobisbutyronitrile were added to 100 parts by weight of silicone rubber base compound for sponge (silicone rubber KE904-FU obtainable from Shin-Etsu Chemical Co.,Ltd. (Inc.)). Thereafter, it was thoroughly mixed using a two-roll mill to produce a silicone rubber compound seat having thickness of 2mm.

### (Evaluations of crosslinking characters of the silicone rubber and properties of the crosslinked silicone rubber molding)

The crosslinking characters of the silicone rubber were evaluated at 120°C using a curelastometer, and shown in Table 1.
Wherein M_{H} is the maximum torque value, T₁₀ is the time to the 10% of maximum torque value, T₉₀ is the time to the 90% of maximum torque value.

Properties of the crosslinked silicone rubber molding by press-molding were evaluated using test pieces pressed at 110°C for 10 minutes, and shown in Table 1.
Tensile strength and elongation were measured in the same manner as JIS K6251, hardness was measured in the same manner as JIS K6253.

The surface condition of the hot air crosslinked silicone rubber molding was evaluated using test pieces, obtained by hot air crosslinking in a gear oven at 250°C for 10 minutes, for tackiness and blooming after seven days. The results were shown in Tabe 2.

The foaming rate and compression set (50% compression at 180°C/for 22 hours) of sponge were evaluated using sponge rubbers obtained by hot air crosslinking of silicone rubber compound seats at 250°C for 10 minutes in a gear oven. The results were shown in Table 2. Compression set was measured in the same manner as JIS K6262.

### <Examples 25-46>

### (The preparations of crosslinking agents of silicone paste comprising the asymmetry organic peroxide of the present invention and the preparations of silicone rubber compounds employing the same)

The crosslinking agents of silicone paste, comprising a (substituted)benzoylperoxy alkyl monocarbonate or a (substituted)benzoylperoxy alkylene carbonate obtained in Examples 2-23, and the silicone rubber compounds were prepared in the same manner as Example 24. The crosslinking characters of the silicone rubbers and the properties of the crosslinked silicone rubber moldings were evaluated, and the results were shown in Table 1 and Table 2.

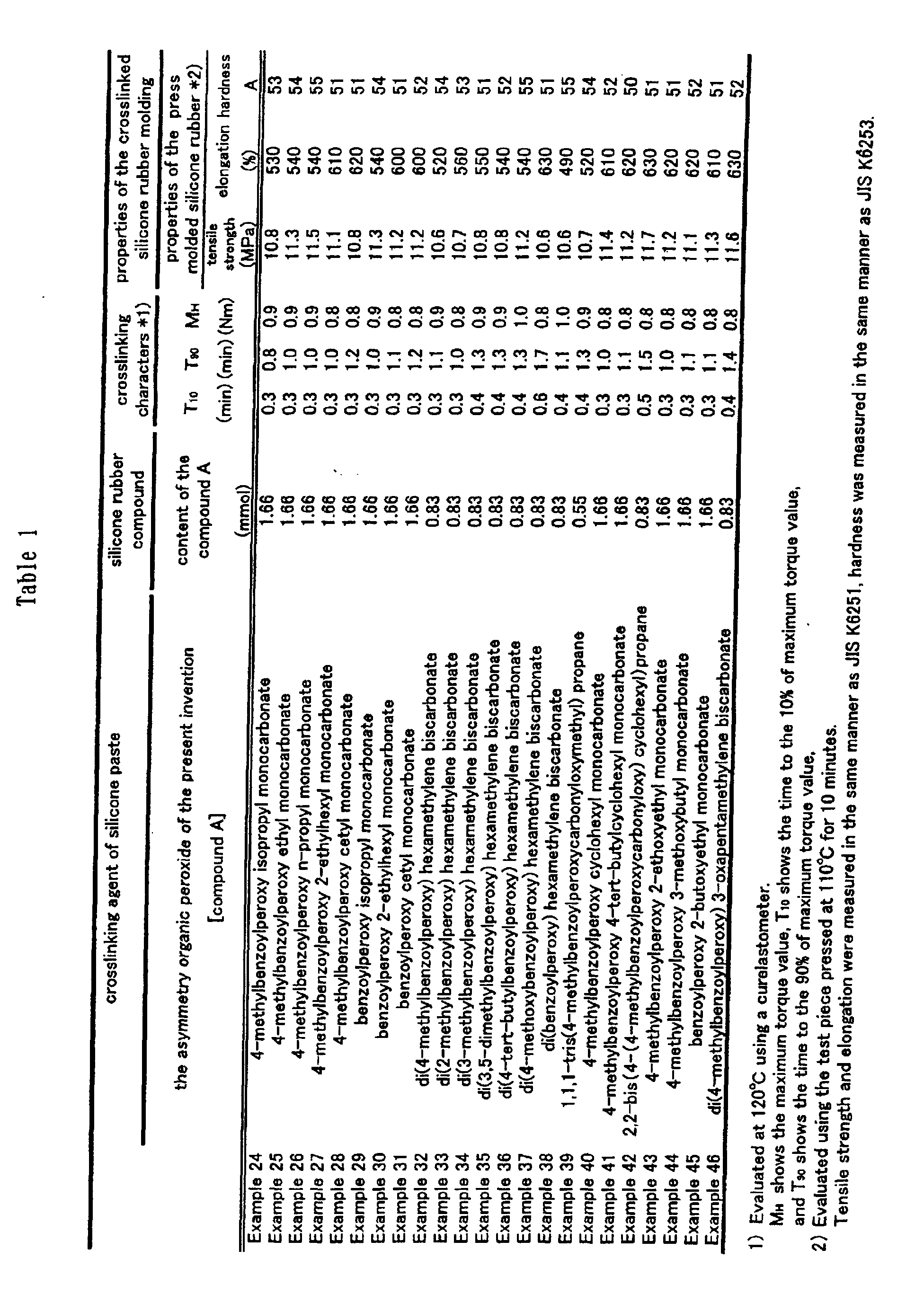

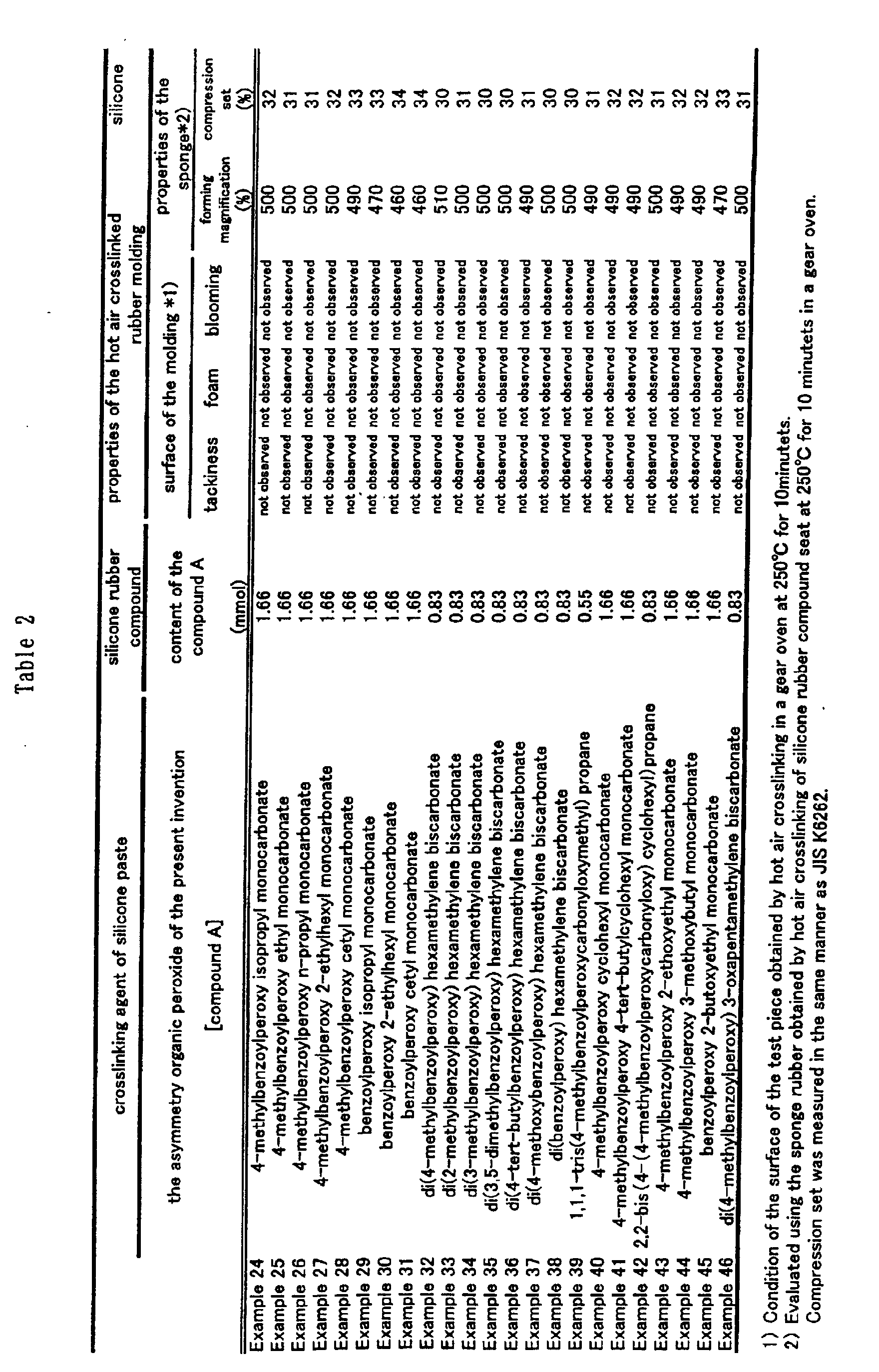

### <Example 47>

### (The preparation of a crosslinking agent of silicone paste comprising several kinds of the asymmetry organic peroxides of the present invention and the preparation of a silicone rubber compound employing the same.)

The crosslinking agent of silicone paste comprising a substitutedbenzoylperoxy alkyl monocarbonate and a di(substitutedbenzoylperoxy) alkylene biscarbonate was prepared by mixing the silicone paste comprising 4-methylbenzoylperoxy isopropyl monocarbonate obtained in Example 24 with the silicone paste comprising di(4-methylbenzoylperoxy) hexamethylene biscarbonate obtained in Example 32 at the content ratio shown in Table 3 and Table 4.

The silicone rubber compound was prepared in the same manner as in Example 24, and the crosslinking characters of the silicone rubber and the properties of the crosslinked silicone rubber molding were evaluated. The results were shown in Table 3 and Table 4.

### <Example 48>

### (The preparation of a crosslinking agent of silicone paste comprising several kinds of the asymmetry organic peroxides of the present invention and the preparation of a silicone rubber compound employing the same.)

The crosslinking agent of silicone paste comprising a substitutedbenzoylperoxy alkyl monocarbonate and a benzoylperoxy alkyl monocarbonate was prepared by mixing the silicone paste comprising 4-methylbenzoylperoxy isopropyl monocarbonate obtained in Example 24 with the silicone paste comprising benzoylperoxy isopropyl monocarbonate obtained in Example 29 at the content ratio shown in Table 3 and Table 4.

The silicone rubber compound was prepared in the same manner as in Example 24, and the crosslinking characters of the silicone rubber and the properties of the crosslinked silicone rubber molding were evaluated. The results were shown in Table 3 and Table 4.

### <Example 49>

### (The preparation of a crosslinking agent of silicone paste comprising the asymmetry organic peroxide of the present invention and other conventional organic peroxide, and the preparation of a silicone rubber compound employing the same.)

4-Methylbenzoyl peroxide was prepared by the well-known reaction of 4-methylbenzoyl chloride and sodium peroxide, and then dried.
The silicone paste was prepared by thoroughly mixing 4-methylbenzoyl peroxide with dimethylsilicone oil followed by homogenization using a three-roll mill.

The crosslinking agent of silicone paste comprising the asymmetry organic peroxide of the present invention and a substitutedbenzoyl peroxide was prepared by mixing the silicone paste comprising 4-methylbenzoylperoxy isopropyl monocarbonate obtained in Example 24 with the obtained silicone paste of 4-methylbenzoyl peroxide at the content ratio shown in Table 3 and Table 4.

The silicone rubber compound was prepared using the crosslinking agent in the same manner as in Example 24, and the crosslinking characters of the silicone rubber and the properties of the crosslinked silicone rubber moldings were evaluated. The results were shown in Table 3 and Table 4.

### <Example 50>

### (The prepartion of a crosslinking agent of silicone paste comprising the asymmetry organic peroxide of the present invention and other conventional organic peroxide, and the preparation of a silicone rubber compound employing the same.)

The crosslinking agent of silicone paste, comprising the asymmetry organic peroxide of the present invention and a substitutedbenzoyl peroxide, was prepared by mixing the silicone paste comprising di(4-methylbenzoylperoxy) hexamethylene biscarbonate obtained in Example 32 with the silicone paste comprising 4-methylbenzoyl peroxide obtained in Example 49 at the content ratio shown in Table 3 and Table 4.

The silicone rubber compound was prepared using the crosslinking agent in the same manner as in Example 24, and the crosslinking characters of the silicone rubber and the properties of the crosslinked silicone rubber molding were evaluated. The results were shown in Table 3 and Table 4.

### <Example 51>

### (The preparation of a crosslinking agent of silicone paste comprising the asymmetry organic peroxide of the present invention and other conventional organic peroxide, and the preparation of a silicone rubber compound employing the same.)

A silicone paste was prepared by thoroughly mixing dicumyl peroxide, which was prepared by the well-known method and then dried, with dimethylsilicone oil followed by homogenization using a three-roll mill.
The crosslinking agent of silicone paste comprising di(4-methylbenzoylperoxy) hexamethylene biscarbonate and dicumyl peroxide was prepared by mixing the silicone paste comprising di(4-methylbenzoylperoxy) hexamethylene biscarbonate obtained in Example 32 with the obtained silicone paste of dicumyl peroxide at the content ratio shown in Table 4.

The silicone rubber compound was prepared using the crosslinking agent in the same manner as in Example 24, and the crosslinking characters of the silicone rubber and the properties of the crosslinked silicone rubber molding were evaluated. The results were shown in Table 4.

### <Example 52>

### (The preparation of a crosslinking agent of silicone paste comprising the asymmetry organic peroxide of the present invention and other conventional organic peroxide, and the preparation of a silicone rubber compound employing the same.)

A silicone paste was prepared by thoroughly mixing 2,5-dimethyl-2,5-bis(tert-butylperoxy)hexane, which was prepared by well-known method, with dimethylsilicone oil.
The crosslinking agent of silicone paste comprising di(4-methylbenzoylperoxy) hexamethylene biscarbonate and 2,5-dimethyl-2,5-bis(tert-butylperoxy)hexane was prepared by mixing the silicone paste comprising di (4-methylbenzoylperoxy) hexamethylene biscarbonate obtained in Example 32 with the obtained silicone paste of 2,5-dimethyl-2,5-bis(tert-butylperoxy)hexane at the content ratio shown in Table 3.

The silicone rubber compound was prepared using the crosslinking agent in the same manner as in Example 24, and the crosslinking characters of the silicone rubber and the properties of the crosslinked silicone rubber molding were evaluated. The results were shown in Table 4.

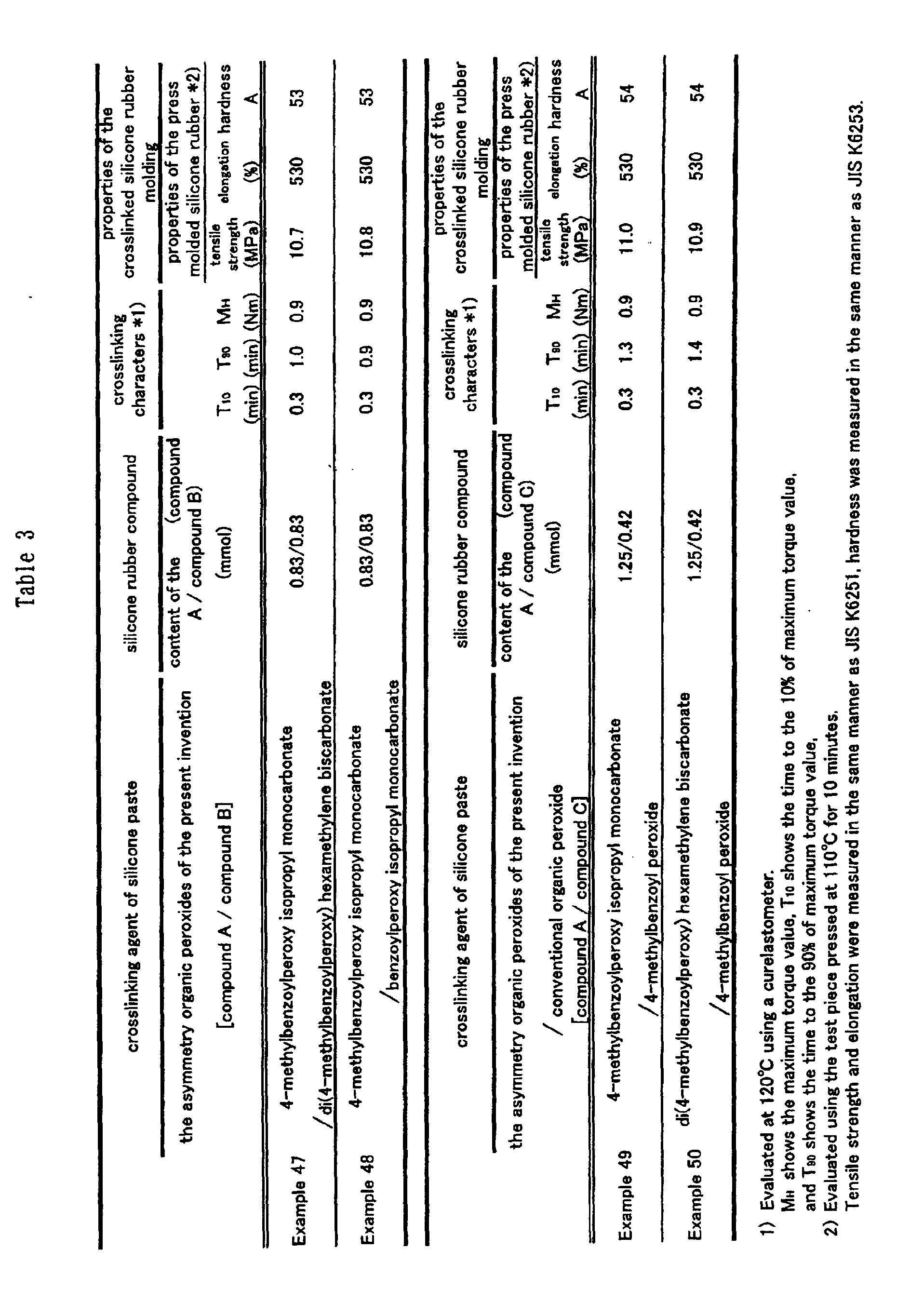

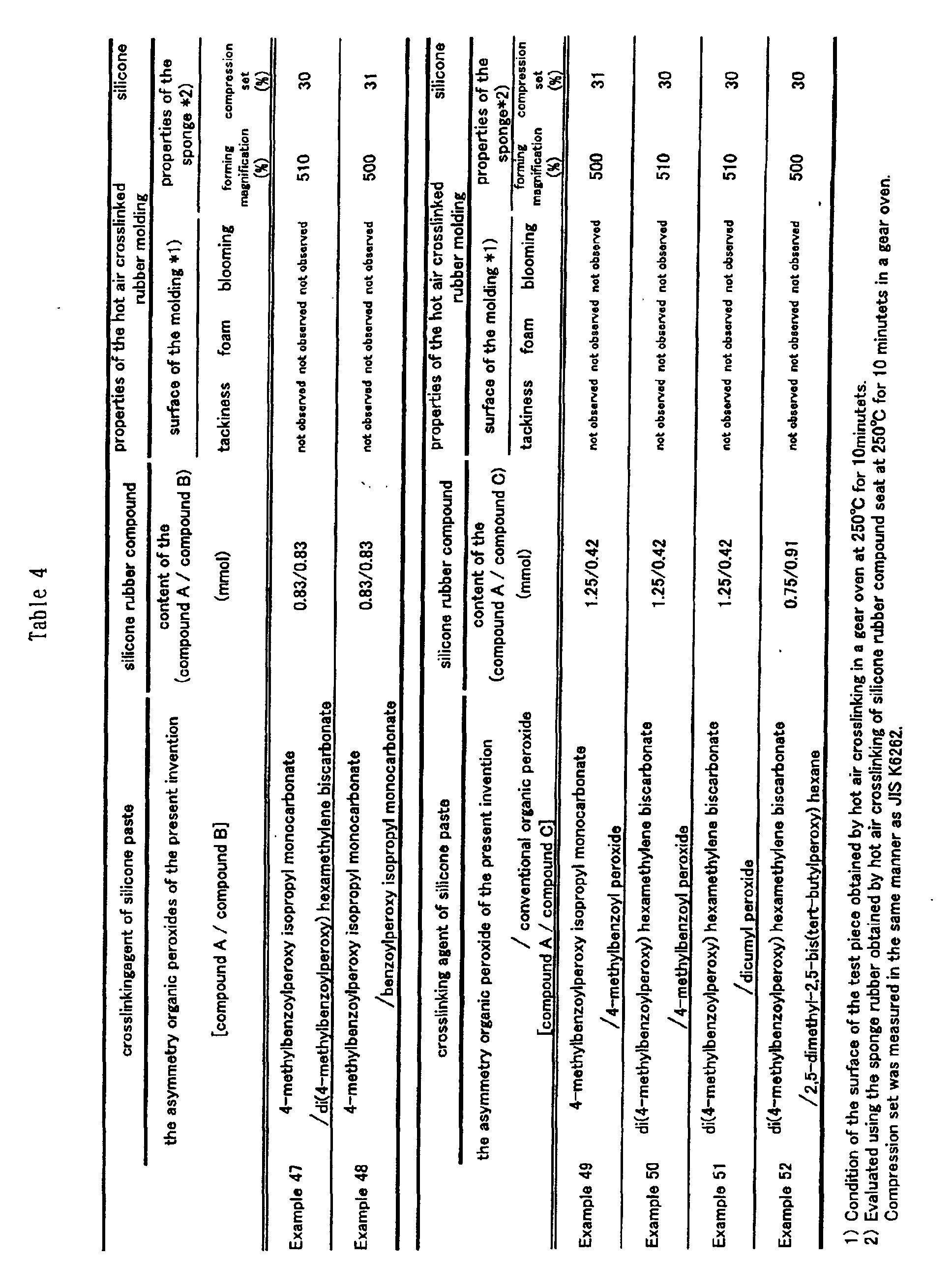

### <Comparative Example 1>

### (The preparation of a crosslinking agent of silicone paste comprising a conventional organic peroxide, and the preparation of a silicone rubber compound employing the same)

The crosslinking agent of silicone paste was prepared by thoroughly mixing 2,4-dichlorobenzoyl peroxide, which was prepared by the well-known reaction of 2,4-dichlorobenzoyl chloride and sodiumperoxide and then dried, with dimethyl silicone oil, followed by homogenization using a three-roll mill.

The silicone rubber compound was prepared by adding the crosslinking agent of silicone paste comprising 2,4-dichlorobenzoyl peroxide to a silicone rubber base compound for extrusion molding (silicone rubber KE-561-U obtainable from Shin-Etsu Chemical Co., Ltd. (Inc.),) in an amount shown in Table 5 and mixing fully using a two-roll mill.

The silicone rubber compound sheet having thickness of 2mm was prepared by adding the crosslinking agent of silicone paste comprising 2,4-dichlorobenzoyl peroxide in the amount shown in Table 6 together with 2 parts by weight of azobisbutyronitrile to 100 parts by weight of a silicone rubber base compound for sponge(silicone rubber KE904-FU obtainable from Shin-Etsu Chemical Co., Ltd. (Inc.),) and mixing fully using a two-roll mill.

### (Evaluations of crosslinking characters of the silicone rubber and the properties of the crosslinked silicone rubber moldings)

The crosslinking characters of the silicone rubber and the properties of the crosslinked silicone rubber moldings obtained by press molding, of the silicone rubber moldings obtained by hot air crosslinking, and the sponge rubber obtained by hot air-crosslinking were evaluated in the same manner as in Example 24. The results were shown in Table 5 and Table 6.

### <Comparative Examples 2-5>

### (The preprations of crosslinking agents of silicone paste comprising a conventional organic peroxide, and the preparations of silicone rubber compounds employing the same)

Benzoyl peroxide, 4-methylbenzoyl peroxide, 2-methylbenzoyl peroxide and 2-methoxybenzoyl peroxide were synthesized independently by well-known methods, and the crosslinking agents of silicone paste were prepared in the same manner as in Comparative Example 1.

The crosslinking characters of the silicone rubbers and the properties of the crosslinked silicone rubber moldings were evaluated in the same manner as in Comparative Example 1. The results were shown in Table 5 and Table 6.

### <Comparative Example 6>

### (The prepartion of a crosslinking agent of silicone paste comprising a conventional organic peroxide, and the preparation of a silicone rubber compound employing the same)

The crosslinking agent of silicone paste was prepared by throughly mixing di(4-tert-butylcyclohexyl) peroxy dicarbonate, which was prepared by the well-known reaction of 4-tert-butylcyclohexyl chloroformate and sodiumperoxide and then dried, with dimethyl silicone oil, followed by homogenization using a three-roll mill.

The crosslinking characters of the silicone rubber and the properties of the crosslinked silicone rubber molding were evaluated in the same manner as in Comparative Example 1. The results were shown in Table 5 and Table 6.

### <Comparative Example 7>

### (The preparation of a crosslinking agent of silicone paste comprising a conventional organic peroxide and the preparation of a silicone rubber compound employing the same)

The crosslinking agent of silicone paste comprising dimyristyl peroxy dicarbonate was prepared in the same manner as in Comparative Example 6 except that myristyl chloroformate was used instead of 4-tert-butylcyclohexyl chloroformate.

The crosslinking characters of the silicone rubber and the properties of the crosslinked silicone rubber molding were evaluated in the same manner as in Comparative Example 1. The results were shown in Table 5 and Table 6.

### <Comparative Example 8>

### (The preparation of 4-methylbenzoylperoxy phenyl monocarbonate)

21.6g of white crystal of 4-methylbenzoylperoxy phenyl monocarbonate (active oxygen content: 5.83%, purity: 99.1%, yield: 78.6%) was obtained in the same manner as in Example 1 except that 15.7g (0.1mol.) of phenyl chloroformate was used instead of isopropyl chloroformate.

The obtained 4-methylbenzoylperoxy phenyl monocarbonate was characterized as shown below.
melting point: 70-72 °C, ¹H-NMR(CDCl₃): 2.37(s, 3H), 7.25(m, 5H), 7.36(d, 2H), 7.89(d, 2H)

The crosslinking agent of silicone paste and the silicone rubber compound were prepared using the obtained 4-methylbenzoylperoxy phenyl monocarbonate, before the crosslinking chracters of the silicone rubber and the properties of the crosslinked silicone rubber molding were evaluated in the same manner as in Comparative Example 1. The results were shown in Table 5 and Table 6.

### <Comparative Example 9>

### (The preparation of 4-methylbenzoylperoxy benzyl monocarbonate)

18.7g of white crystal of 4-methylbenzoylperoxy benzyl monocarbonate (active oxygen content: 5.56%, purity: 99.5%, yield: 65.0%) was obtained in the same manner as in Example 1 except that 48.8g of toluene solution containing 17.1g (0.1mole) of benzyl chloroformate was used instead of isopropyl chloroformate.

The obtained 4-methylbenzoylperoxy benzyl monocarbonate was characterized as shown below.
melting point: 96-98 °C, ¹H-NMR(CDCl₃): 2.38(s, 3H), 5.30(s, 2H), 7.24(d, 2H), 7.36(m, 5H), 7.86(d, 2H)

The crosslinking agent of silicone paste and the silicone rubber compound were prepared using the obtained 4-methylbenzoylperoxy benzyl monocarbonate, before the crosslinking characters of the silicone rubber and the properties of the crosslinked silicone rubber molding were evaluated in the same manner as in Comparative Example 1. The results were shown in Table 5 and Table 6.

### <Comparative Example 10>

### (The preparation of di(lauroylperoxy) hexamethylene biscarbonate)

19.0g of white crystal of di(lauroylperoxy) hexamethylene biscarbonate ( active oxygen content:5.08%, purity:95.7%, yield:60.3%) was obtained in the same manner as in Example 9 except that 26.3g (0.12mole) of lauric peracid was used instead of 4-methylperbenzoic acid.

The obtained di(lauroylperoxy) hexamethylene biscarbonate was characterized as shown below.
melting point: 45-47°C, ¹H-NMP(CDCl₃): 0.88(t, 6H), 1.26(m, 36H), 1.70(m, 8H), 2.42(t, 4H), 4.30(t, 4H)

The crosslinking agent of silicone paste and the silicone rubber compound were prepared using the obtained di(lauroylperoxy) hexamethylene biscarbonate, before the crosslinking characters of the silicone rubber and the properties of the crosslinked silicone rubber molding were evaluated in the same manner as in Comparative Example 1. The results were shown in Table 5 and Table 6.

### <Comparative Examples 11,12>

### (The preparations of silicone rubber compounds employing crosslinking agents of silicone paste comprising several kinds of conventional organic peroxides)

The silicone rubber compound was prepared by adding the crosslinking agent of silicone paste comprising benzoyl peroxide obtained in Comparative Example 2 together with the crosslinking agent of silicone paste comprising di(4-tert-butylcyclohexyl)peroxy dicarbonate obtained in Comparative Example 6 to a silicone rubber base compound in the amount shown in Table 3, followed by thoroughly mixing it using a two-roll mill (Comparative Example 11).

The silicone rubber compound was prepared by adding the crosslinking agent of silicone paste comprising benzoyl peroxide obtained in Comparative Example 2 togather with the crosslinking agent of silicone paste comprising dimyristyl peroxy dicarbonate obtained in comparative Example 7 to a silicone rubber base compound in the amount shown in Table 3, followed by thoroughly missing it using a two-roll mill (Comparative Example 12).
The crosslinking characters of the silicone rubbers and and the properties of the crosslinked silicone rubber moldings were evaluated in the same manner as in Comparative Example 1. The results were shown in Table 5 and Table 6.

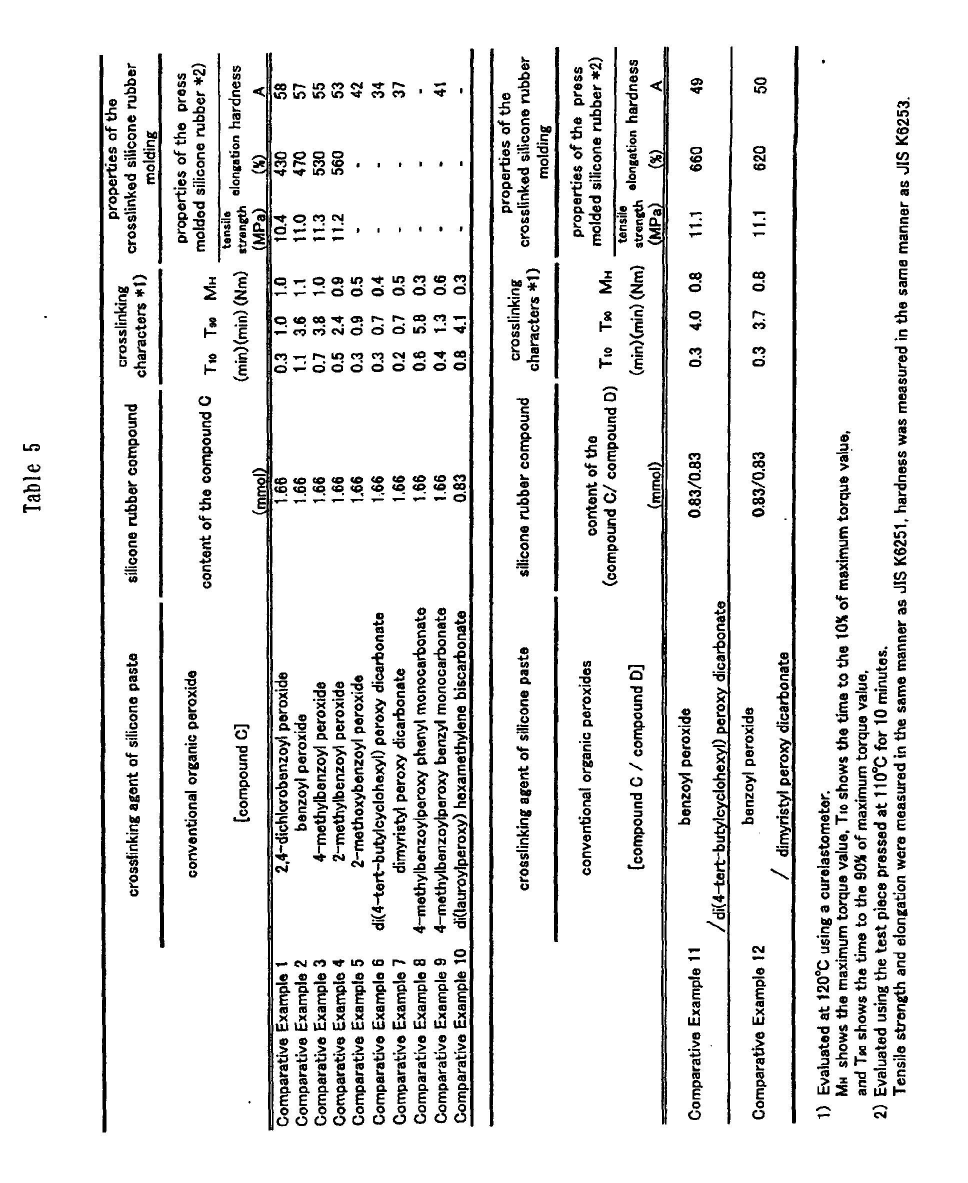

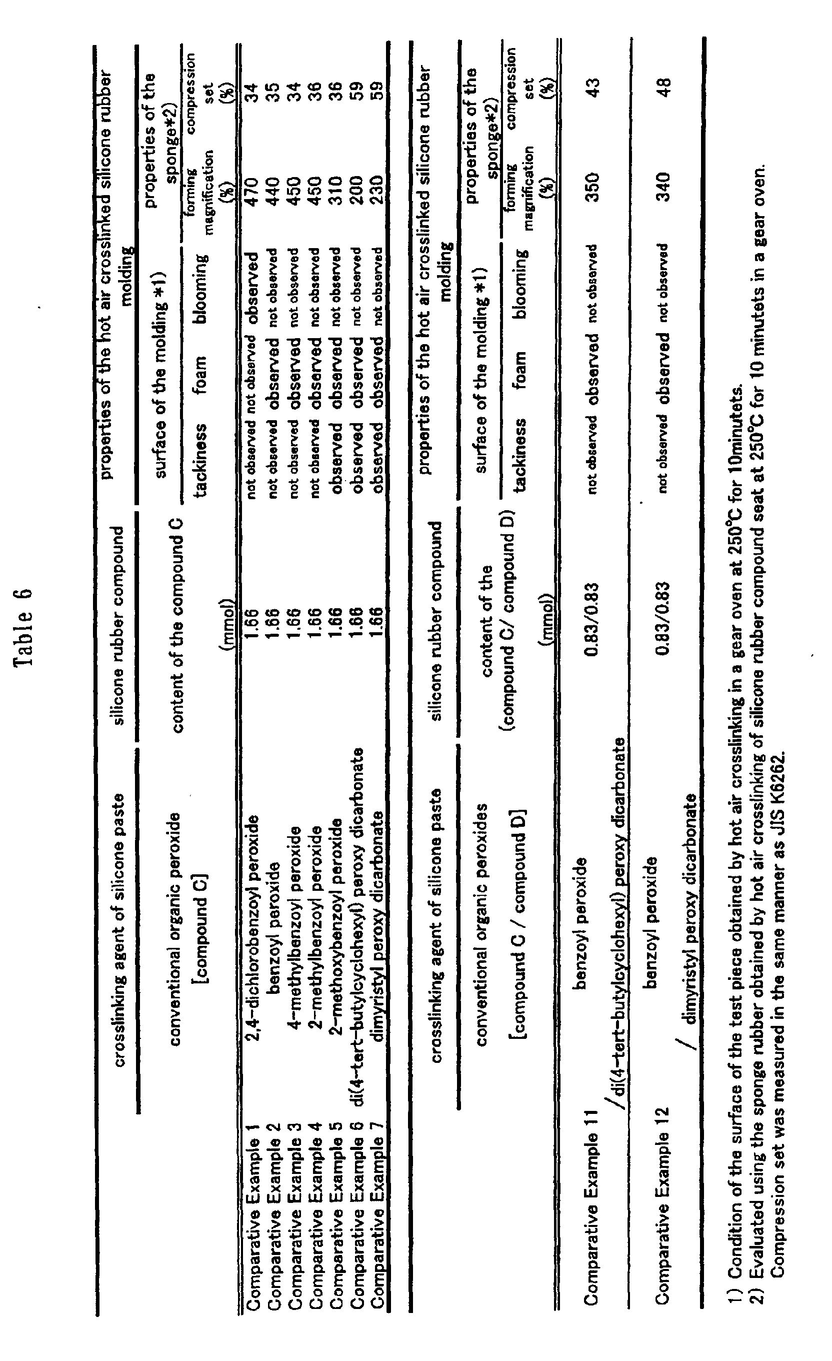

The crosslinking speeds of the silicone rubbers using the crosslinking agents comprising the asymmetry organic peroxides of the present invention shown in Examples 24-50 were at the same level with those of the silicone rubber using the crosslinking agent comprising 2,4-dichlorobenzoyl peroxide shown in Comparative Example 1, and were higher than those of the silicone rubbers using the crosslinking agents comprising benzoyl peroxide, 4-methylbenzoyl peroxide or 2-methylbenzoyl peroxide shown in Comparative Examples 2-4.
Hot air crosslinked silicone rubber moldings using the crosslinking agents comprising the asymmetry organic peroxides of the present invention had no tacky surface, and no foam were observed while their crosslinkings unlike in Comparative Examples 2-4, and further, no blooming were observed on their surfaces unlike the molding prepared in Comparative Example 1. In addition to that, the properties of the press molded silicone rubbers and the sponge rubbers were excellent.
Especially, it was confirmed that sponge shaped-crosslinked silicone rubber moldings using the crosslinking agents of the present invention comprising a (substituted) benzoylperoxy alkyl monocarbonate shown in Examples 24-28, 40, 41, 43 and 44, as well as those using the crosslinking agents comprising a (substituted)benzoylperoxy alkylene carbonate shown in Examples 32-39, 42 and 46, had high foaming rate, low compression set, no coloration, homogeneous cells smaller than 1mm, and smooth no tacky surface.

In cases where the crosslinking agents of the present invention were used, the crosslinking efficiencies, which was shown as M_{H} (maximum torque value), were high and the crosslinked silicone rubber moldings had no tacky surface. On the other hand, in cases where the crosslinking agents comprising 2-methoxybenzoyl peroxide, or a peroxy dicarbonate such as di(4-tert-butylcyclohexyl)peroxy dicarbonate and dimyristylperoxy dicarbonate were used shown in Comparative Example 5 and Comparative Examples 6 and 7 respectively, the crosslinking efficiencies were low and the crosslinked silicone rubber moldings had tacky surfaces.
It was confirmed that the crosslinking agent comprising the asymmetry organic peroxide having specific structure has excellent crosslinking characters.

Further, it was confirmed that the crosslinking speeds of silicone rubbers were fast and the crosslinking efficiencies were high in cases where the crosslinking agents of the present invention, which do not have any phenyl group and benzyl group bonded to a monocarbonate group, were used. On the other hand, in case where the crosslinking agent having the sutucture in which a phenyl group is bonded to a monocarbonate group such as (substituted)benzoylperoxy phenyl monocarbonates was used as shown in Comparative Example 8 where the crosslinking agent comprising 4-methylbenzoylperoxy phenyl monocarbonate was used, the crosslinking speed of silicone rubber was slow and the crosslinking efficiency was low and in case where the crosslinking agent having the sutucture in which a benyl group is bonded to a monocarbonate group such as (substituted)benzoylperoxy benzyl monocarbonates was used, as shown in Comparative Example 9 where the cross linking agent comprising 4-methylbenzoylperoxy benyl monocarbonate was used, the crosslinking efficiency was low.

Still, it was confirmed that the crosslinking speed of silicone rubber was fast and the crosslinking efficiency was high in case where the crosslinking agent of the present invention having the structure of (substituted)benzoyl group was used. On the other hand, in case where a peroxy alkylene biscarbonate having the structure of aliphatic acyl group was used, like shown in Comparative Example 10 where di(lauroylperoxy) hexamethylene biscarbonate was used, the crosslinking speed of silicone rubber was slow and the crosslinking efficiency was low.

Still further, it was confirmed that the crosslinking agents of the present invention comprising the specific asymmetry organic peroxides have excellent crosslinking characters, because the crosslinking speeds of silicone rubbers using the crosslinking agents of the present invention were faster and the crosslinking efficiencies were higher than those in cases where the crosslinking agents comprising a diacyl peroxide together with a peroxy dicarbonate (the ratio of diacyl peroxide to peroxy dicarbonate is 1:1) were used, as shown in Comparative Examples 11 and 12 where benzoyl peroxide was used with di (4-tert-butylcyclohexyl)peroxy dicarbonate or dimyristylperoxy dicarbonate, respectively were used as shown in.

That is to say, it was confirmed that the crosslinking agent comprising the asymmetry organic peroxide of the present invention is applicable to press-molding, or any other conventional methods for crosslinking of silicone rubber.
Specifically, use of the crosslinking agent of the present invention, which has high crosslinking efficiency and high crosslinking speed, in hot air-crosslinking of silicone rubber for electrical wire and cable coatings and tubes would greatly improve the productivity, which mainly reflects the crosslinking speed. Furthermore, the crosslinked silicone rubber moldings obtainable from the process will have no tacky surface, no foaming (except sponge rubbers), and no blooming, and the sponge rubbers obtainable from the process will have excellent quality (such as lower compression set).

### APPLICABILITY OF THE INVENTION

As described above, the asymmetry organic peroxide of the present invention, which has no chlorine atom in the molecule, does not cause any anxiety for sanitary problems of crosslinked silicone rubber moldings, unlike the case 2,4-dichlorobenzoyl peroxide is used where high toxity compounds like polychlorinated biphenyl can be formed as by-products.
The present crosslinking process of silicone rubber moldings using the crosslinking agent as described above has excellent productivity because of the high crosslinking efficiency and the high crosslinking speed.
And the silicone rubber moldings obtained by using the crosslinking agent of the present invention as described above will have no blooming unlike those obtained by using 2,4-dichlorobenzoyl peroxide. Thus, higher production efficiency can be attained because of no needs of aftercrosslinking.

## Claims

1. An asymmetry organic peroxide having at least one structure unit of (substituted)benzoylperoxy carbonyloxy group represented by the following formula (1) in the molecule thereof. 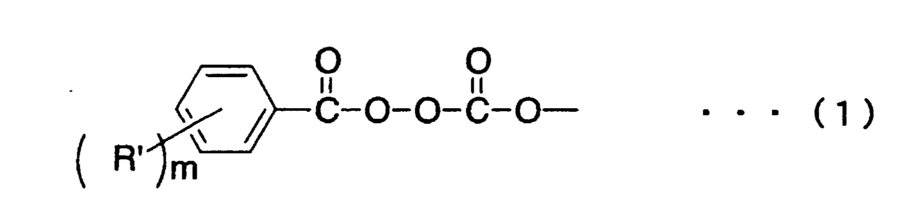 (wherein m stands for an integer in a range from 0 to 5, and R' corresponds to either of the following (A), (B) or (C):
(A) m stands for 0, in other words, R' stands for a hydrogen atom.
(B) m stands for an integer in a range of 1 to 5, and R' stands for an alkyl group of 1 to 6 carbon atoms, an alkoxy group of 1 to 6 carbon atoms or a phenyl group.
(C) m stands for an integer in a range of 1 to 5, and R' stands for a silyl group substituted by either of an alkyl group of 1 to 6 carbon atoms, an alkoxy group of 1 to 6 carbon atoms, a phenyl group or a vinyl group.
wherein R' can be the same or different in each (substituted)benzoyl group).

2. An asymmetry organic peroxide comprising a (substituted)benzoylperoxy alkyl carbonate or a (substituted)benzoylperoxy alkylene carbonate represented by the following formula (2). 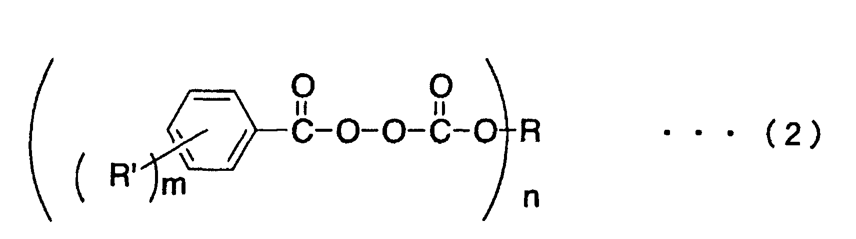 (wherein n stands for an integer in a range from 1 to 6, m stands for an integer in a range from 0 to 5, n and R corresponds to either of the following (a) , (b) or (c), and m and R' corresponds to either of the following (A), (B) or (C):
(a) in case of n is 1, R stands for an alkyl group of 1 to 20 carbon atoms, whreas in case of n is in a range of 2 to 6, R stands for a linear or branched hydrocarbon group of 1 to 20 carbon atoms.
(b) in case of n is 1, R stands for a cycloalkyl group of 3 to 10 carbon atoms or a substituted cycloalkyl group of 4 to 10 carbon atoms substituted by an alkyl group of 1 to 4 carbon atoms, whereas in case of n is in a range of 2 to 4, R stands for a cyclohexanediyldimethylene group, a cyclohexylene group, a bicyclohexylene group or a hydrocarbon group of 13 to 25 carbon atoms substituted by 2 to 4 of cyclohexylene groups.
(c) in case of n is 1, R stands for an alkoxyalkyl group represented by the formula -R¹-O-R² (wherein, R¹ stands for an alkylene group of 2 to 5 carbon atoms, R² stands for an alkyl group of 1 to 8 carbon atoms), whereas in case of n is 2, R stands for an oxoalkylene group represented by the formula -(R³-O-)ₖ-R³- (wherein k stands for 1 or 2, and R³ stands for an alkylene group of 2 to 5 carbon atoms).
(A) m stands for 0, in other words, R' stands for a hydrogen atom.
(B) m stands for an integer of 1 to 5, and R' stands for an alkyl group of 1 to 6 carbon atoms, an alkoxy group of 1 to 6 carbon atoms or a phenyl group.
(C) m stands for an integer of 1 to 5, and R' stands for a silyl group substituted by either of an alkyl group of 1 to 6 carbon atoms, an alkoxy group of 1 to 6 carbon atoms, a phenyl group or a vinyl group.
wherein R' can be the same or different in each (substituted)benzoyl group, and in each (substituted)benzoyl group in one molecule.

3. An asymmetry organic peroxide comprising a substitutedbenzoylperoxy alkyl monocarbonate represented by the following formula (2). 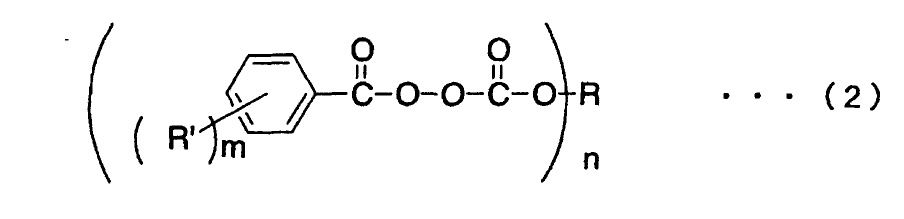 (wherein n stands for 1, m stands for an integer in a range from 1 to 5, R corresponds to either of the following (a), (b) or (c), and R' corresponds to either of the following (B) or (C):
(a)R stands for an alkyl group of 1 to 20 carbon atoms.
(b)R stands for a cycloalkyl group of 3 to 10 carbon atoms or a substitutedcycloalkyl group of 4 to 10 carbon atoms substituted by an alkyl group of 1 to 4 carbon atoms.
(c) R stands for an alkoxyalkyl group represented by the formula -R¹-O-R² (wherein R¹ stands for an alkylene group of 2 to 5 carbon atoms, R² stands for an alkyl group of 1 to 8 carbon atoms)
(B) R' stands for an alkyl group of 1 to 6 carbon atoms, an alkoxy group of 1 to 6 carbon atoms or a phenyl group.
(C) R' stands for a silyl group substituted by either of an alkyl group of 1 to 6 carbon atoms, an alkoxy groupof 1 to 6 carbon atoms, a phenyl group or a vinyl group.
wherein R' can be the same or different in each (substituted)benzoyl group.

4. An asymmetry organic peroxide comprising a (substituted)benzoylperoxy alkylene carbonate represented by the following formula (2). 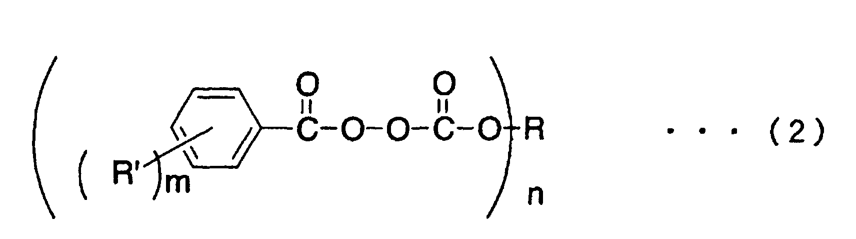 (wherein n stands for an integer in a range from 2 to 6, m stands for an integer in a range from 0 to 5, n and R corresponds to either of the following (a) or (c), and m and R' corresponds to either of the following (A), (B) or (C):
(a)R stands for a linear or branched hydrocarbon group of 1 to 20 carbon atoms.
(c) R stands for an oxoalkylene group represented by the formula -(R³-O-)ₖ-R³- (wherein k stands for 1 or 2, and R³ stands for an alkylene group of 2 to 5 carbon atoms) .
(A) m stands for 0, in other words, R' stands for a hydrogen atom.
(B) m stands for an integer in a range of 1 to 5, and R' stands for an alkyl group of 1 to 6 carbon atoms, an alkoxy group of 1 to 6 carbon atoms or a phenyl group.
(C) m stands for an integer in a range of 1 to 5, and R' stands for a silyl group substituted by either of an alkyl group of 1 to 6 carbon atoms, an alkoxy group of 1 to 6 carbon atoms, a phenyl group or a vinyl group.
wherein R' can be the same or different in each (substituted)benzoyl group, and in each (substituted)benzoyl group in one molecule.

5. A crosslinking agent comprising at least one of the asymmetry organic peroxide according to any one of Claims 1 to 4.

6. A crosslinking agent comprising a mixture of the asymmetry organic peroxide according to any one of Claims 1 to 4 with the other organic peroxide

7. A crosslinking agent according to either Claim 5 or Claim 6, further comprising silicone oil singly or in combination with microparticulate silica.

8. A crosslinking processs of silicone rubber using the crosslinking agent according to any one of Claims 5 to 7, in which an organic peroxide is mixed with silicone rubber and heated.
